# EUROPEAN PATENT SPECIFICATION

(11) **EP 3 502 107 B1**
(45) Date of publication and mention of the grant of the patent: **26.01.2022**
(21) Application number: 18213749.7
(22) Date of filing: 18.12.2018
(51) Int. Cl.: C07D 405/12, C07D 307/91, H01L 51/50

(54) **1-AMINODIBENZOFURAN-BASED COMPOUND AND ORGANIC LIGHT-EMITTING DEVICE INCLUDING THE SAME**
1-AMINODIBENZOFURAN-BASIERTE VERBINDUNG UND ORGANISCHE LICHTEMITTIERENDE VORRICHTUNG DAMIT
COMPOSÉ BASÉ SUR LE 1-AMINODIBENZOFURANE ET DISPOSITIF ÉLECTROLUMINESCENT ORGANIQUE LE COMPRENANT

(30) Priority: 20.12.2017 KR 20170176483; 09.11.2018 KR 20180137609
(43) Date of publication of application: 26.06.2019
(73) Proprietor: Samsung Display Co., Ltd., Yongin-si Gyeonggi-do (KR)
(72) Inventor: Kim, Jongwoo, Gyeonggi-do (KR); Baek, Jangyeol, Gyeonggi-do (KR); Jeong, Eunjae, Gyeonggi-do (KR); Han, Sanghyun, Gyeonggi-do (KR); Youngkook, Kim, Gyeonggi-do (KR); Hwang, Seokhwan, Gyeonggi-do (KR)
(74) Representative: Russell, Tim

(56) References cited:
- EP-A1- 2 930 168
- EP-A1- 3 018 128
- EP-A1- 3 305 782
- EP-A1- 3 321 341
- WO-A1-2013/039184
- WO-A1-2013/039184
- WO-A1-2014/088285
- WO-A1-2015/130069
- WO-A1-2017/012687
- WO-A1-2017/012687
- WO-A1-2017/016632
- WO-A1-2017/038728
- WO-A1-2017/142308
- WO-A1-2017/148564
- WO-A1-2017/148565
- WO-A1-2017/207596
- WO-A1-2018/091435
- WO-A1-2018/216903
- WO-A1-2019/054599
- WO-A1-2019/095920
- WO-A1-2019/124903
- CN-A- 105 349 134
- CN-A- 107 382 749
- US-A1- 2013 087 776
- US-A1- 2016 028 020
- US-A1- 2016 093 810
- US-A1- 2016 126 469
- US-A1- 2016 181 543
- US-A1- 2016 365 517
- US-A1- 2017 213 970
- US-A1- 2017 213 971
- US-A1- 2017 213 972
- US-A1- 2017 279 050

## Description

### CROSS-REFERENCE TO RELATED APPLICATION

### BACKGROUND

### 1. Field

One or more embodiments relate to an amine-based compound and an organic light-emitting device comprising the same.

### 2. Description of the Related Art

Organic light-emitting devices (OLEDs) are self-emission devices that, as compared with related art devices, have wide viewing angles, high contrast ratios, short response times, and excellent brightness, driving voltage, and response speed characteristics, and produce full-color images.

OLEDs may comprise a first electrode disposed on a substrate, and may comprise a hole transport region, an emission layer, an electron transport region, and a second electrode sequentially disposed on the first electrode. Holes provided from the first electrode may move toward the emission layer through the hole transport region. Electrons provided from the second electrode may move toward the emission layer through the electron transport region. Carriers, such as holes and electrons, may recombine in the emission layer to produce excitons. These excitons transit from an excited state to a ground state to thereby generate light.

### SUMMARY

In a first aspect, the present invention provides an organic light-emitting device according to claim 1.

### BRIEF DESCRIPTION OF THE DRAWINGS

FIG. 1 is a schematic cross-sectional view of an embodiment of an organic light-emitting device.
FIG. 2 is a schematic cross-sectional view of an embodiment of an organic light-emitting device.
FIG. 3 is a schematic cross-sectional view of an embodiment of an organic light-emitting device.
FIG. 4 is a schematic cross-sectional view of an embodiment of an organic light-emitting device.

### DETAILED DESCRIPTION

Reference will now be made in more detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of', when preceding a list of elements, modify the entire list of elements and do not modify the individual elements of the list. Further, the use of "may" when describing embodiments of the present invention refers to "one or more embodiments of the present invention."

As used herein, the terms "substantially," "about," and similar terms are used as terms of approximation and not as terms of degree, and are intended to account for the inherent deviations in measured or calculated values that would be recognized by those of ordinary skill in the art. Also, any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein. Accordingly, Applicant reserves the right to amend this specification, including the claims, to expressly recite any sub-range subsumed within the ranges expressly recited herein.

As the inventive concept allows various modifications and includes various embodiments, examplary embodiments will be illustrated in the drawings and described in more detail in the written description. Effects, features, and a method of achieving the inventive concept will become apparent by reference to the examplary embodiments of the inventive concept, together with the accompanying drawings. The inventive concept may, however, be embodied in many different forms and should not be construed as being limited to the examplary embodiments set forth herein.

Hereinafter, the inventive concept will be described in more detail by explaining examplary embodiments of the inventive concept with reference to the attached drawings. Like reference numerals in the drawings denote like elements, and thus their description will not be repeated.

In the embodiments described in the present specification, an expression used in the singular encompasses the expression of the plural, unless it has a clearly different meaning in the context.

In the present specification, it is to be understood that the terms such as "including," "having," and "comprising" are intended to indicate the existence of the features or components disclosed in the specification, and are not intended to preclude the possibility that one or more other features or components may exist or may be added.

It will be understood that when a layer, region, or component is referred to as being "on" or "onto" another layer, region, or component, it may be directly or indirectly formed over the other layer, region, or component. That is, for example, intervening layers, regions, or components may be present.

Sizes of components in the drawings may be exaggerated for convenience of explanation. In other words, because sizes and thicknesses of components in the drawings are arbitrarily illustrated for convenience of explanation, the following embodiments are not limited thereto.

All subject matter not present in the claims is seen as not forming part of the invention and being present as reference information.

An exemplary amine-based compound can be represented by one of Formulae 1A and 1B:

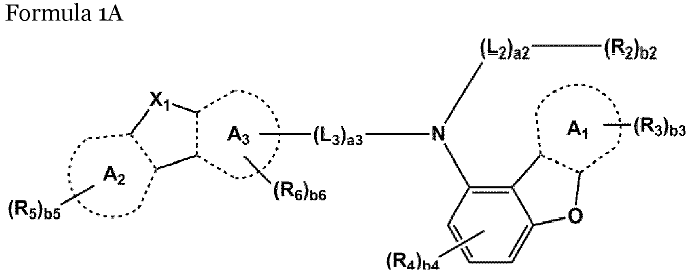

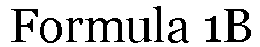

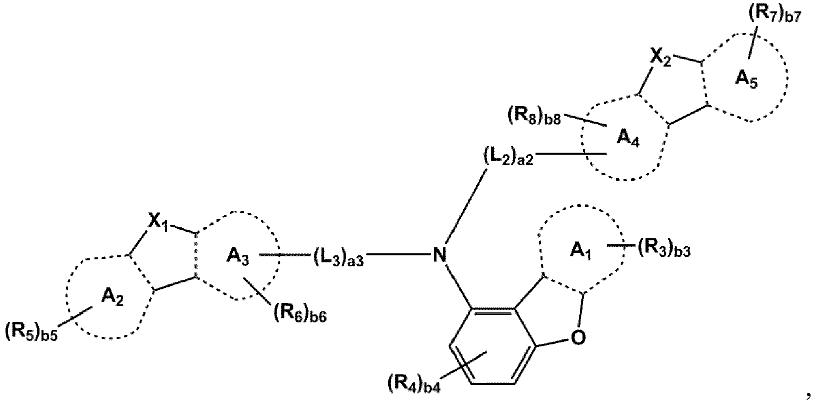

wherein, in Formulae 1A and 1B, A₁ to A₅ are independently selected from a C₅-C₃₀ carbocyclic group and a C₁-C₃₀ heterocyclic group.

In some embodiments, in Formulae 1A and 1B, A₁ to A₅ may each independently be selected from a benzene group, an indene group, a naphthalene group, an anthracene group, a fluorene group, a phenanthrene group, a triphenylene group, a pyrene group, a chrysene group, a naphthacene group, a pyrrole group, an imidazole group, a pyrazole group, a pyridine group, a pyrimidine group, a pyrazine group, a pyridazine group, an indole group, an isoindole group, an indazole group, a quinoline group, an isoquinoline group, a benzoquinoline group, a naphthyridine group, a quinoxaline group, a quinazoline group, a cinnoline group, a carbazole group, a phenanthridine group, an acridine group, a phenanthroline group, a phenazine group, a benzimidazole group, a furan group, a benzofuran group, a thiophene group, a benzothiophene group, a thiazole group, an isothiazole group, a benzothiazole group, an isoxazole group, an oxazole group, a triazole group, an oxadiazole group, a triazine group, a benzoxazole group, a dibenzofuran group, a dibenzothiophene group, a benzocarbazole group, and a dibenzocarbazole group.

In some embodiments, in Formulae 1A and 1B, A₁ to A₅ may each independently be selected from a benzene group, an indene group, a naphthalene group, an anthracene group, a fluorene group, a phenanthrene group, a pyridine group, a pyrimidine group, a pyrazine group, a pyridazine group, and a carbazole group, but embodiments are not limited thereto.

In some embodiments, in Formulae 1A and 1B, A1 to A5 may each independently be selected from a benzene group and a naphthalene group, but embodiments are not limited thereto.

In some embodiments, in Formulae 1A and 1B, A₁ to A₅ may each independently be a benzene group, but embodiments are not limited thereto.

In Formulae 1A and 1B, X₁ may be O, S, N-(L₁)ₐ₁-(R₁)_{b1}, or C(R₉)(R₁₀), and X₂ may be O, S, or C(R₁₁)(R₁₂).

In some embodiments, in Formulae 1A and 1B, X₁ may be N-(L₁)ₐ₁-(R₁)_{b1}, and X2 may be O, S, or C(R₁₁)(R₁₂); or X₁ may be O, and X₂ may be C(R₁₁)(R₁₂), but embodiments are not limited thereto.

In Formulae 1A and 1B, L₁ to L₃ may each independently be selected from a single bond, a substituted or unsubstituted C₅-C₆₀ carbocyclic group, and a substituted or unsubstituted C₁-C₆₀ heterocyclic group.

In some embodiments, in Formulae 1A and 1B, L₁ to L₃ may each independently be selected from a single bond, a benzene group, a pentalene group, an indene group, a naphthalene group, an azulene group, a heptalene group, an indacene group, an acenaphthalene group, a fluorene group, a spiro-bifluorene group, a benzofluorene group, a phenalene group, a phenanthrene group, an anthracene group, a fluoranthene group, a triphenylene group, a pyrene group, a chrysene group, a naphthacene group, a picene group, a perylene group, a pentaphenylene group, a hexacene group, a pyrrole group, an imidazole group, a pyrazole group, a pyridine group, a pyrazine group, a pyrimidine group, a pyridazine group, an isoindole group, an indole group, an indazole group, a purine group, a quinoline group, an isoquinoline group, a benzoquinoline group, a phthalazine group, a naphthyridine group, a quinoxaline group, a quinazoline group, a cinnoline group, a carbazole group, a phenanthridine group, an acridine group, a phenanthroline group, a phenazine group, a benzimidazole group, a furan group, a benzofuran group, a thiophene group, a benzothiophene group, a thiazole group, an isothiazole group, a benzothiazole group, an iso-oxazole group, an oxazole group, a triazole group, a tetrazole group, an oxadiazole group, a triazine group, a benzoxazole group, a dibenzofuran group, a dibenzothiophene group, a benzocarbazole group, and a dibenzocarbazole group; and

a benzene group, a pentalene group, an indene group, a naphthalene group, an azulene group, a heptalene group, an indacene group, an acenaphthalene group, a fluorene group, a spiro-bifluorene group, a benzofluorene group, a phenalene group, a phenanthrene group, an anthracene group, a fluoranthene group, a triphenylene group, a pyrene group, a chrysene group, a naphthacene group, a picene group, a perylene group, a pentaphenylene group, a hexacene group, a pyrrole group, an imidazole group, a pyrazole group, a pyridine group, a pyrazine group, a pyrimidine group, a pyridazine group, an isoindole group, an indole group, an indazole group, a purine group, a quinoline group, an isoquinoline group, a benzoquinoline group, a phthalazine group, a naphthyridine group, a quinoxaline group, a quinazoline group, a cinnoline group, a carbazole group, a phenanthridine group, an acridine group, a phenanthroline group, a phenazine group, a benzimidazole group, a furan group, a benzofuran group, a thiophene group, a benzothiophene group, a thiazole group, an isothiazole group, a benzothiazole group, an iso-oxazole group, an oxazole group, a triazole group, a tetrazole group, an oxadiazole group, a triazine group, a benzoxazole group, a dibenzofuran group, a dibenzothiophene group, a benzocarbazole group, and a dibenzocarbazole group, each substituted with at least one selected from deuterium, -F, -Cl, -Br, -I, a hydroxyl group, a cyano group, a nitro group, an amino group, an amidino group, a hydrazino group, a hydrazono group, a C₁-C₂₀ alkyl group, a C₁-C₂₀ alkoxy group, a C₆-C₂₀ aryl group, a C₁-C₂₀ heteroaryl group, and - Si(Q₃₁XQ₃₂)(Q₃₃),

wherein, Q₃₁ to Q₃₃ may each independently be selected from hydrogen, deuterium, -F, -Cl, -Br, -I, a hydroxyl group, a cyano group, a nitro group, an amino group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with a C₁-C₆₀ alkyl group, a C₁-C₆₀ heteroaryl group, a monovalent non-aromatic condensed C8 to C60 polycyclic group, a monovalent non-aromatic condensed 8- to 60-membered heteropolycyclic group, a biphenyl group, and a terphenyl group.

In some embodiments, in Formulae 1A and 1B, L₁ to L₃ may each independently be selected from a single bond and groups represented by Formulae 3-1 to 3-46: 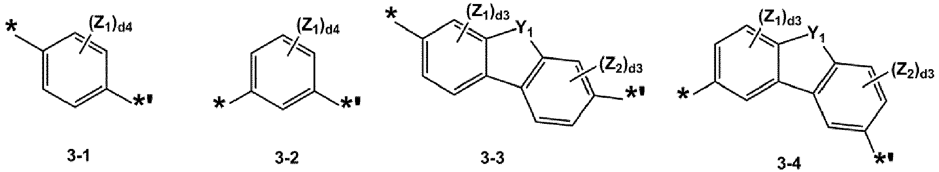 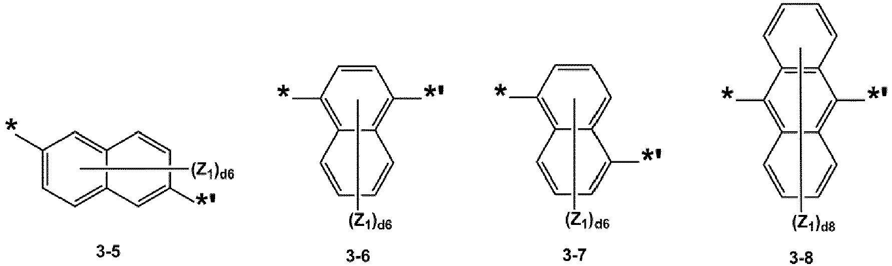 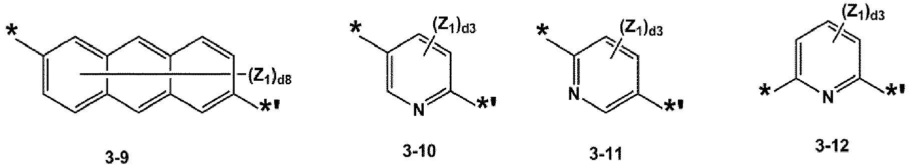 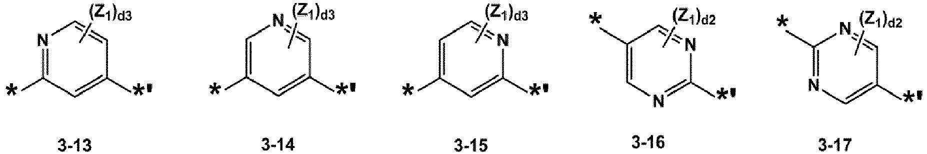 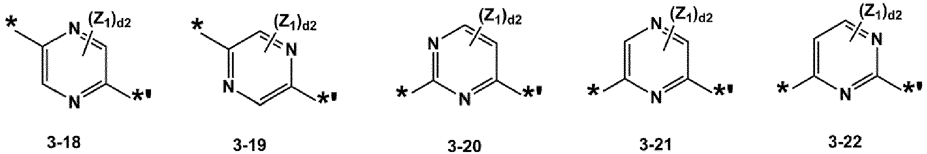 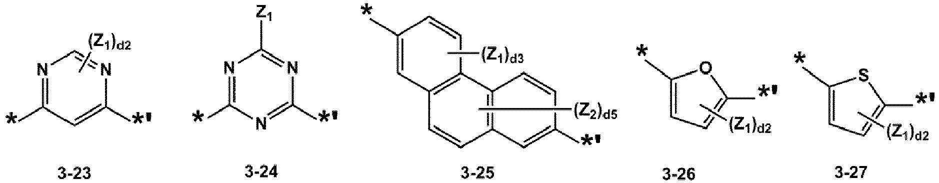 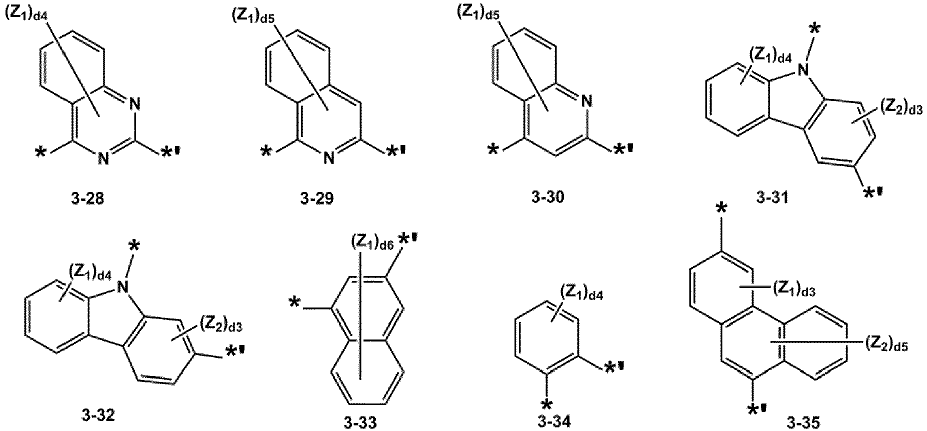 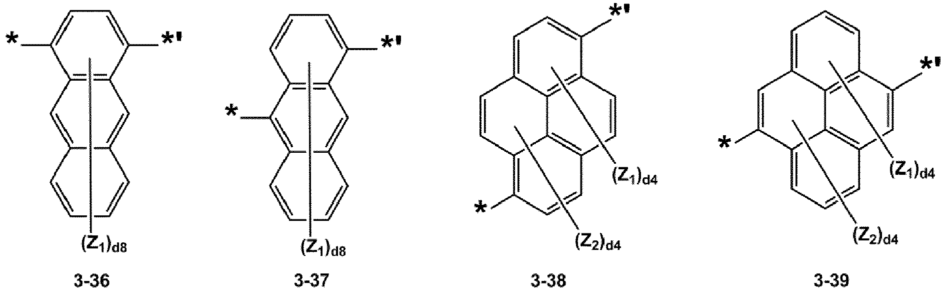 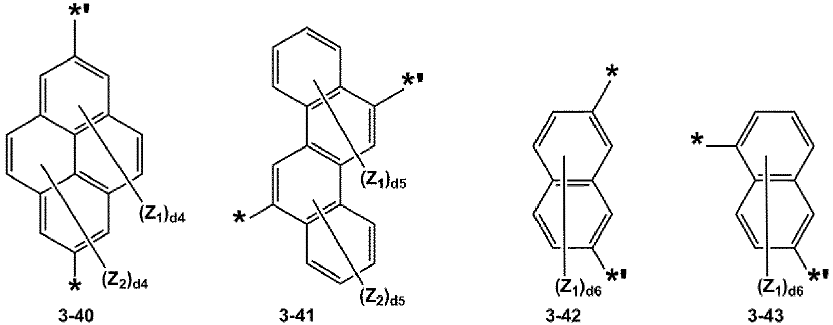 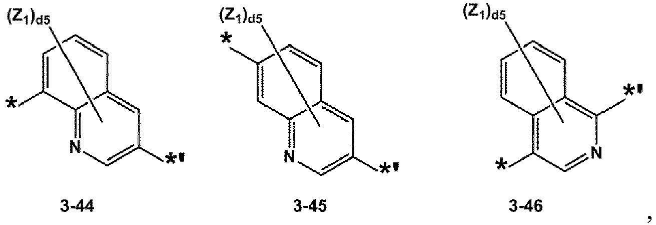 wherein, in Formulae 3-1 to 3-46,
Y₁ may be O, S, C(Z₃)(Z₄), N(Z₅), or Si(Z₆)(Z₇),
Z₁ to Z₇ may each independently be selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a naphthyl group, a fluorenyl group, a spiro-bifluorenyl group, a phenalenyl group, a phenanthrenyl group, an anthracenyl group, a fluoranthenyl group, a triphenylenyl group, a pyridinyl group, a pyrazinyl group, a pyrimidinyl group, a quinolinyl group, an isoquinolinyl group, a benzoquinolinyl group, a naphthyridinyl group, a quinoxalinyl group, a quinazolinyl group, a carbazolyl group, a phenanthridinyl group, an acridinyl group, a phenanthrolinyl group, a phenazinyl group, a triazinyl group, a dibenzofuranyl group, a dibenzothiophenyl group, and -Si(Q₃₁)(Q₃₂)(Q₃₃),
d2 may be an integer from 0 to 2; when d2 is 2, at least two Z₁ groups may be identical to or different from each other,
d₃ may be an integer from 0 to 3; when d₃ is 2 or greater, at least two Z₁ groups (in (Z₁)_{d3}) may be identical to or different from each other, and at least two Z₂ groups (in (Z₂)_{d3}) may be identical to or different from each other,
d₄ may be an integer from 0 to 4; when d₄ is 2 or greater, at least two Z₁ groups (in (Z₁)_{d4}) may be identical to or different from each other, and at least two Z₂ groups (in (Z₂)_{d4}) may be identical to or different from each other,
d₅ may be an integer from 0 to 5; when d₅ is 2 or greater, at least two Z₁ groups (in (Z₁)_{d5}) may be identical to or different from each other, and at least two Z₂ groups (in (Z₂)_{d5}) may be identical to or different from each other,
d6 may be an integer from 0 to 6; when d6 is 2 or greater, at least two Z₁ groups (in (Z₁)_{d6}) may be identical to or different from each other, and at least two Z₂ groups (in (Z₂)_{d6}) may be identical to or different from each other,
d8 may be an integer from 0 to 8; when d8 is 2 or greater, at least two Z₁ groups may be identical to or different from each other, and
^{∗} and ^{∗}' each indicate a binding site to an adjacent atom.

In some examples, L₁ to L₃ may each independently be selected from a single bond and groups represented by Formulae 3-1 to 3-7, 3-31 to 3-34, 3-42, and 3-43.

In some examples, L₁ to L₃ may each independently be selected from a single bond and groups represented by Formulae 3-1, 3-2, and 3-34.

In some examples, in Formulae 3-1 to 3-46, Z₁ to Z₇ may each independently be selected from hydrogen, deuterium, -F, a cyano group, a methyl group, an ethyl group, an n-propyl group, a tert-butyl group, a phenyl group, and a naphthyl group, but embodiments are not limited thereto.

In some examples, in Formulae 1A and 1B, L₁ to L₃ may each independently be selected from a single bond and a benzene group; and
a benzene group substituted with at least one selected from deuterium, -F, -Cl, -Br, -I, a hydroxyl group, a cyano group, a nitro group, an amino group, an amidino group, a hydrazino group, a hydrazono group, a C₁-C₂₀ alkyl group, a C₁-C₂₀ alkoxy group, a C₆-C₂₀ aryl group, a C₁-C₂₀ heteroaryl group, and -Si(Q₃₁)(Q₃₂)(Q₃₃), but embodiments are not limited thereto.

In Formulae 1A and 1B, a1 to a3 are each independently an integer from 1 to 5. a1 indicates the number of L₁ groups; when a1 is 2 or greater, at least two L₁ groups may be identical to or different from each other. Descriptions for a2 and a3 may be understood by referring to (may be the same as) the descriptions for a1 provided herein.

In some examples, in Formulae 1A and 1B, a1 to a3 may each independently be an integer from 1 to 3.

For example, a1 to a3 may each be 1, but examplesare not limited thereto.

For the avoidance of doubt, when a1 is 2 or more, the moiety -(L₁)a1- does not comprise a plurality of adjacent single bonds.

In Formulae 1A and 1B, R₁ to R₈ each independently are selected from hydrogen, deuterium, -F, -Cl, -Br, -I, a hydroxyl group, a cyano group, a nitro group, an amino group, an amidino group, a hydrazino group, a hydrazono group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₁-C₆₀ heteroaryloxy group, a substituted or unsubstituted monovalent non-aromatic condensed C8 to C60 polycyclic group, a substituted or unsubstituted monovalent non-aromatic condensed 8-to 60-membered heteropolycyclic group, -Si(Q₁)(Q₂)(Q₃), -B(Q₁)(Q₂), and -C(=O)(Q₁),
R₉ and R₁₀ and/or R₁₁ and R₁₂ are as described herein in connection with R₁ to R₈ or are bound to each other to form a substituted or unsubstituted C₅-C₃₀ carbocyclic group or a substituted or unsubstituted C₁-C₃₀ heterocyclic group,
wherein, Q₁ to Q₃ are each independently selected from hydrogen, deuterium, - F, -Cl, -Br, -I, a hydroxyl group, a cyano group, a nitro group, an amino group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with a C₁-C₆₀ alkyl group, a C₁-C₆₀ heteroaryl group, a monovalent non-aromatic condensed C8 to C60 polycyclic group, a monovalent non-aromatic condensed 8- to 60-membered heteropolycyclic group, a biphenyl group, and a terphenyl group.

In some examples, in Formulae 1A and 1B, R₁ and R₂ may each independently be selected from a C₆-C₆₀ aryl group, a C₁-C₆₀ heteroaryl group, a monovalent non-aromatic condensed C8 to C60 polycyclic group, and a monovalent non-aromatic condensed 8- to 60-membered heteropolycyclic group; and
a C₆-C₆₀ aryl group, a C₁-C₆₀ heteroaryl group, a monovalent non-aromatic condensed C8 to C60 polycyclic group, and a monovalent non-aromatic condensed 8- to 60-membered heteropolycyclic group, each substituted with at least one selected from deuterium, -F, -Cl, -Br, -I, a C₁-C₆₀ alkyl group, a C₁-C₆₀ alkoxy group, a C₆-C₆₀ aryl group, a C₁-C₆₀ heteroaryl group, a monovalent non-aromatic condensed C8 to C60 polycyclic group, a monovalent non-aromatic condensed 8- to 60-membered heteropolycyclic group, -N(Q₃₁)(Q₃₂), and -Si(Q₃₁)(Q₃₂)(Q₃₃).

In some examples, in Formulae 1A and 1B, R₁ and R₂ may each independently be selected from a phenyl group, a pentalenyl group, an indenyl group, a naphthyl group, an azulenyl group, a heptalenyl group, an indacenyl group, an acenaphthyl group, a fluorenyl group, a spiro-fluorenyl group, a spiro-bifluorenyl group, a benzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzoxazolyl group, a benzimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a benzoxazolyl group, a dibenzofuranyl group, a dibenzothiophenyl group, a benzocarbazolyl group, and a dibenzocarbazolyl group; and
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a spiro-bifluorenyl group, a benzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzoxazolyl group, a benzimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a benzoxazolyl group, a dibenzofuranyl group, a dibenzothiophenyl group, a benzocarbazolyl group, and a dibenzocarbazolyl group, each substituted with at least one selected from deuterium, -F, -Cl, -Br, -I, a hydroxyl group, a cyano group, a nitro group, an amino group, an amidino group, a hydrazino group, a hydrazono group, a C₁-C₂₀ alkyl group, a C₁-C₂₀ alkoxy group, a C₆-C₂₀ aryl group, a C₁-C₂₀ heteroaryl group, and - Si(Q₃₁)Q₃₂)(Q₃₃), but embodiments are not limited thereto.

In some examples, in Formulae 1A and 1B, R₁ and R₂ may each independently be selected from groups represented by Formulae 5-1 to 5-79, but embodiments are not limited thereto: 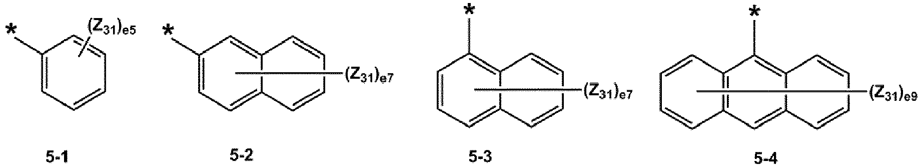 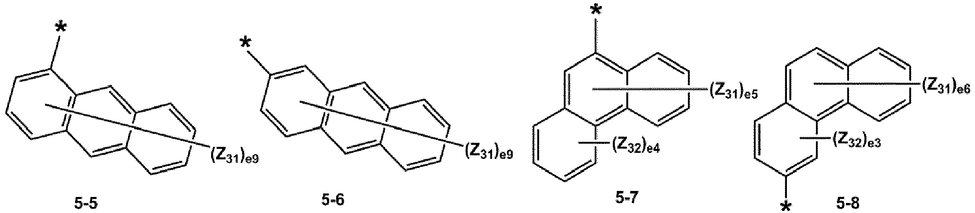 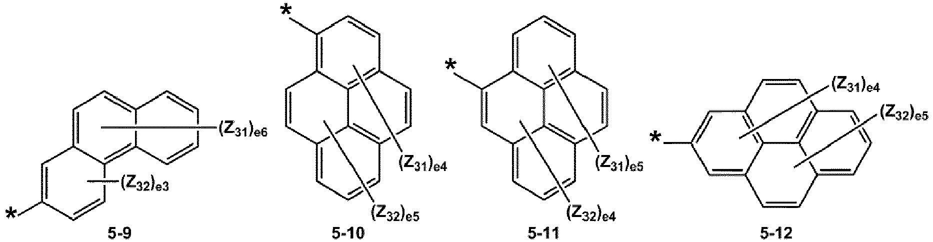 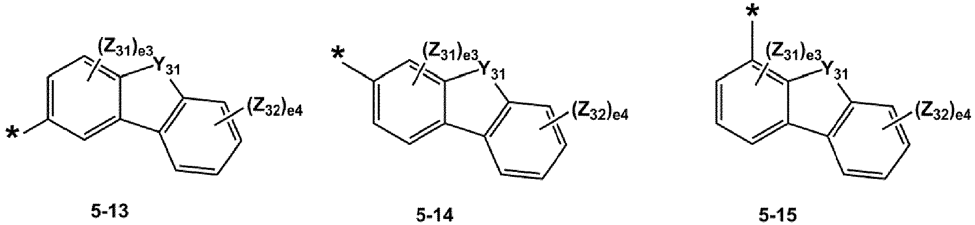 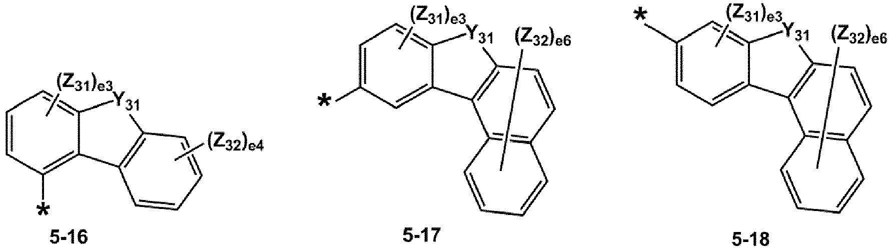 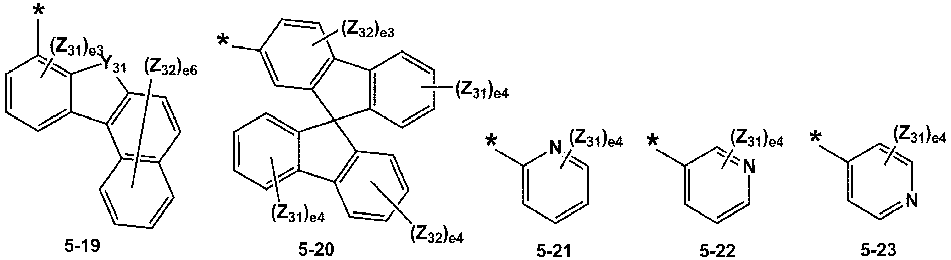 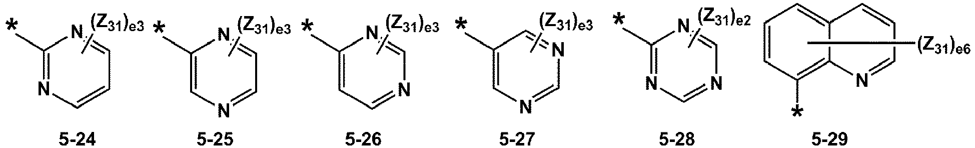 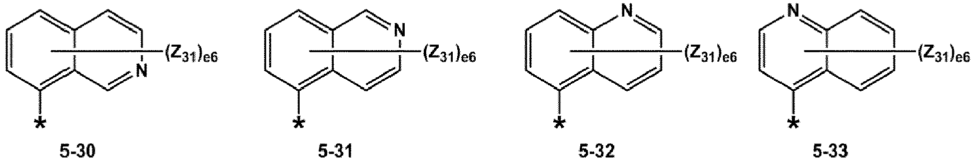 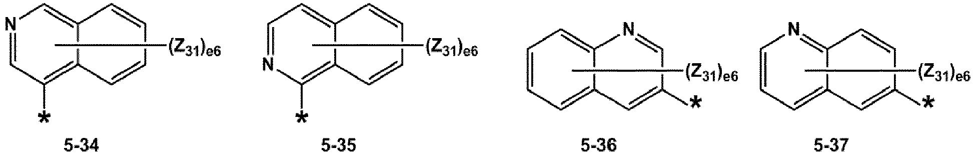 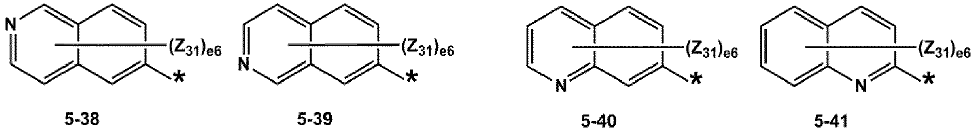 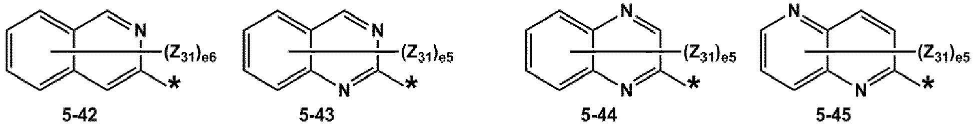 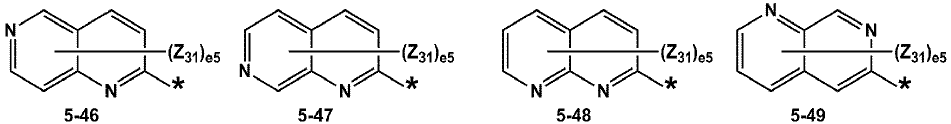 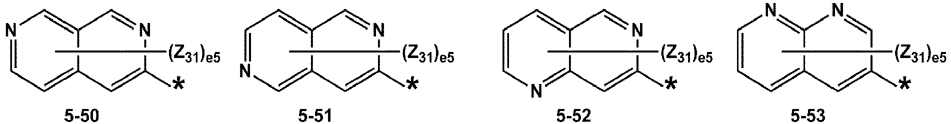 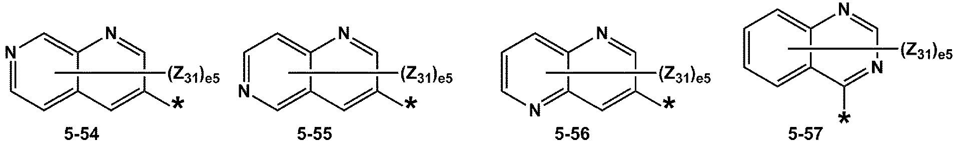 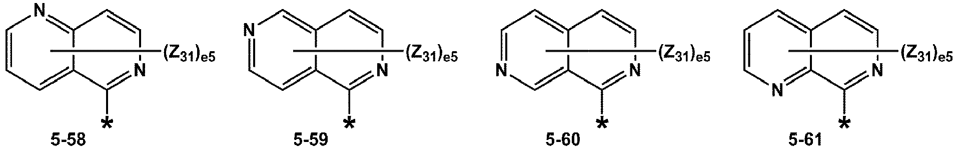 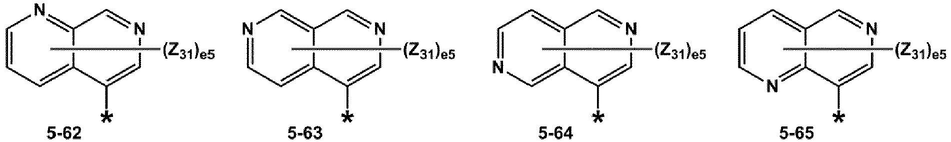 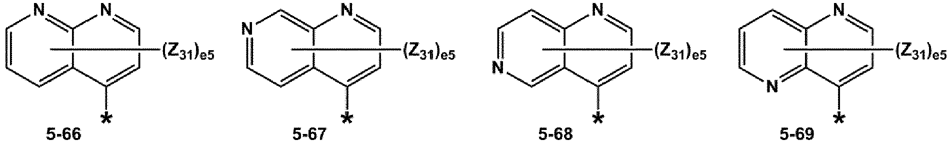 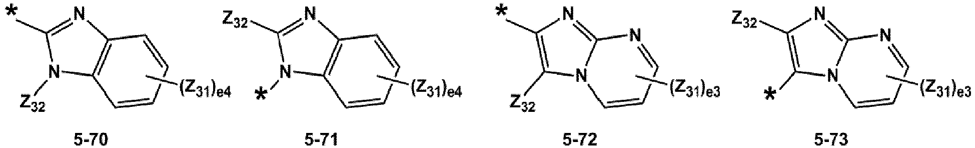 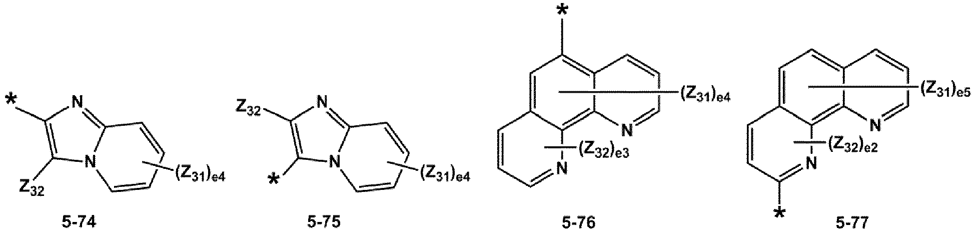 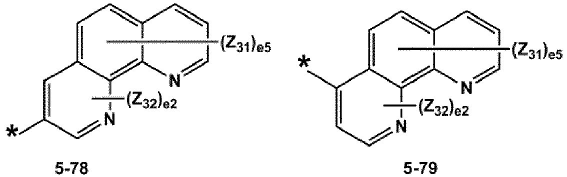 ,
wherein, in Formulae 5-1 to 5-79,
Y₃₁ may be O, S, C(Z₃₃)(Z₃₄), N(Z₃₅), or Si(Z₃₆)(Z₃₇),
Z₃₁, Z₃₂ and Z₃₅ to Z₃₇ may each independently be selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a naphthyl group, a fluorenyl group, a spiro-bifluorenyl group, a spiro-fluorene-benzofluorenyl group, a benzofluorenyl group, a dibenzofluorenyl group, a phenalenyl group, a phenanthrenyl group, an anthracenyl group, a fluoranthenyl group, a triphenylenyl group, a pyridinyl group, a pyrazinyl group, a pyrimidinyl group, a quinolinyl group, an isoquinolinyl group, a benzoquinolinyl group, a naphthyridinyl group, a quinoxalinyl group, a quinazolinyl group, a carbazolyl group, a phenanthridinyl group, an acridinyl group, a phenanthrolinyl group, a phenazinyl group, a triazinyl group, a dibenzofuranyl group, a dibenzothiophenyl group, and -Si(Q₃₁)(Q₃₂)(Q₃₃),
Z₃₃ and Z₃₄ may be as defined for Z₃₁, Z₃₂ and Z₃₅ to Z₃₇, or may be bound to form a substituted or unsubstituted C₅-C₃₀ carbocyclic group or a substituted or unsubstituted C₁-C₃₀ heterocyclic group,
e2 may be an integer from 0 to 2; when e2 is 2, at least two Z₃₁ groups may be identical to or different from each other, and at least two Z₃₂ groups may be identical to or different from each other,
e3 may be an integer from 0 to 3; when e3 is 2 or greater, at least two Z₃₁ groups (in (Z₃₁)ₑ₃) may be identical to or different from each other, and at least two Z₃₂ groups (in (Z₃₂)ₑ₃) may be identical to or different from each other,
e₄ may be an integer from 0 to 4; when e4 is 2 or greater, at least two Z₃₁ groups (in (Z₃₁)ₑ₄) may be identical to or different from each other, and at least two Z₃₂ groups (in (Z₃₂)ₑ₄) may be identical to or different from each other,
e₅ may be an integer from 0 to 5; when e₅ is 2 or greater, at least two Z₃₁ groups (in (Z₃₁)ₑ₅) may be identical to or different from each other, and at least two Z₃₂ groups (in (Z₃₂)ₑ₅) may be identical to or different from each other,
e6 may be an integer from 0 to 6; when e6 is 2 or greater, at least two Z₃₁ groups (in (Z₃₁)ₑ₆) may be identical to or different from each other, and at least two Z₃₂ groups (in (Z₃₂)ₑ₆) may be identical to or different from each other,
e₇ may be an integer from 0 to 7; when e7 is 2 or greater, at least two Z₃₁ groups may be identical to or different from each other,
e9 may be an integer from 0 to 9; when e9 is 2 or greater, at least two Z₃₁ groups may be identical to or different from each other, and
^{∗}indicates a binding site to an adjacent atom.

In some examples, in Formulae 1A and 1B, R₁ and R₂ may each independently be selected from groups represented by Formulae 5-1 to 5-20, but examplesare not limited thereto.

In some examples, in Formulae 1A and 1B, R₁ and R₂ may each independently be selected from groups represented by Formulae 5-1 to 5-3, 5-13 to 5-16, and 5-20, but examplesare not limited thereto.

In some examples, in Formulae 5-1 to 5-79, Z₃₁ to Z₃₇ may each independently be selected from hydrogen, deuterium, -F, a cyano group, a methyl group, an ethyl group, an n-propyl group, a tert-butyl group, a phenyl group, and a naphthyl group, but examplesare not limited thereto.

In some examples, in Formulae 1A and 1B, R₁ and R₂ may each independently be selected from groups represented by Formulae 6-1 to 6-42, but examplesare not limited thereto: 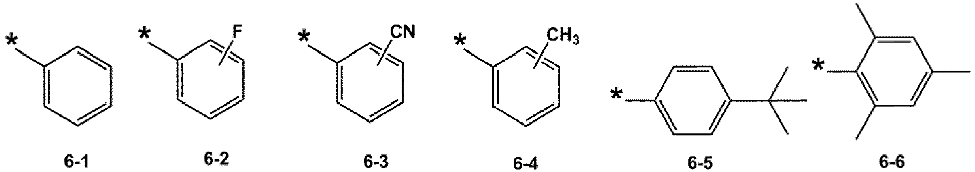 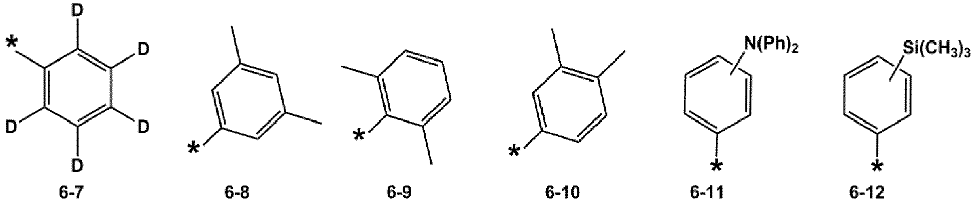 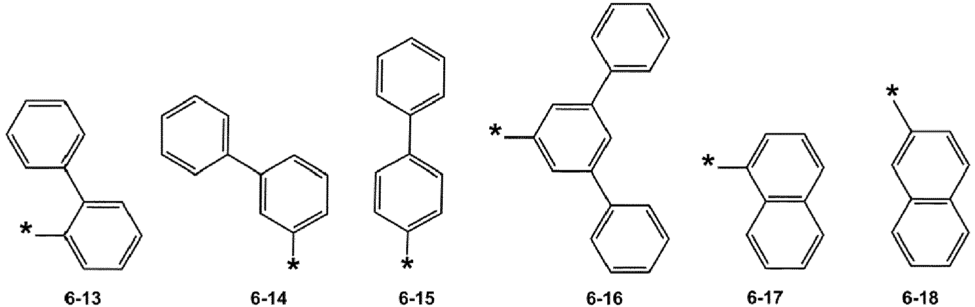 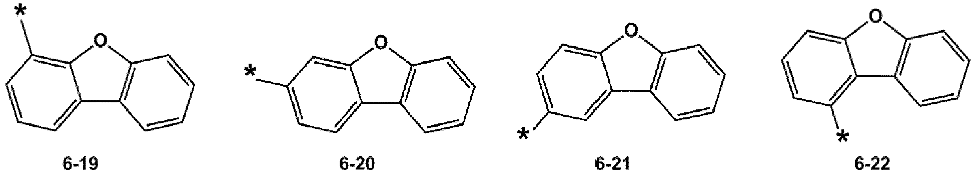 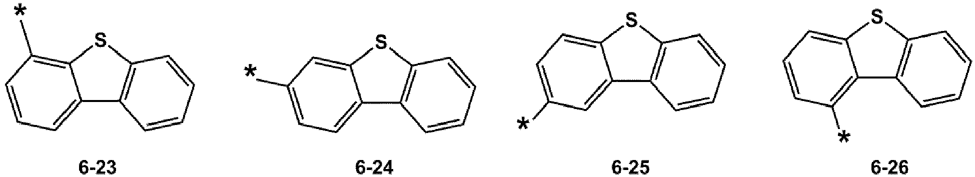 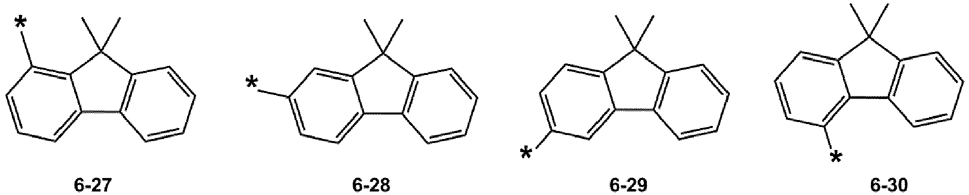 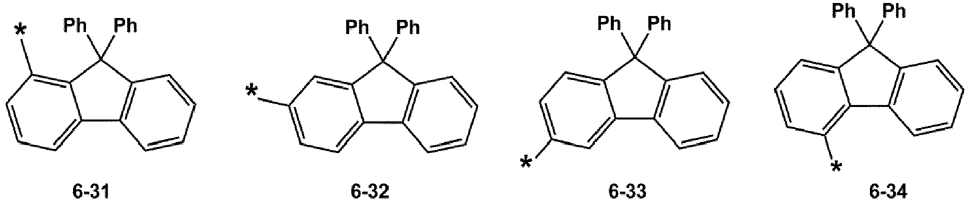 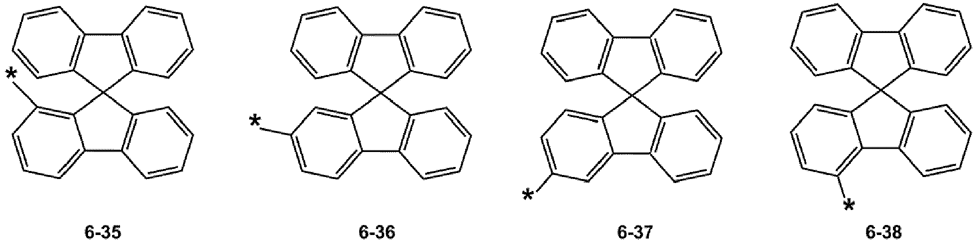 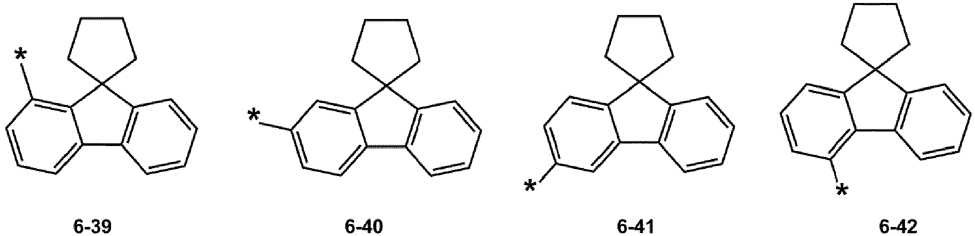 ,
wherein, in Formulae 6-1 to 6-42,
"Ph" represents a phenyl group, and
^{∗} indicates a binding site to an adjacent atom.

In Formulae 1A and 1B, R₃ to R₁₂ may each independently be selected from hydrogen, deuterium, -F, -Cl, -Br, -I, a C₁-C₆₀alkyl group, a C₁-C₆₀alkoxy group, a C₆-C₆₀ aryl group, a C₆-C₆₀ aryloxy group, a C₆-C₆₀ arylthio group, a C₁-C₆₀heteroaryl group, a monovalent non-aromatic condensed C8 to C60 polycyclic group, a monovalent non-aromatic condensed 8- to 60-membered heteropolycyclic group, -Si(Q₁)(Q₂)(Q₃), and - N(Q₁)(Q₂);
a C₁-C₆₀ alkyl group and a C₁-C₆₀alkoxy group, each substituted with at least one selected from deuterium, -F, -Cl, -Br, -I, a cyano group, and a nitro group; and
a C₆-C₆₀ aryl group, a C₁-C₆₀ heteroaryl group, a monovalent non-aromatic condensed C8 to C60 polycyclic group, and a monovalent non-aromatic condensed 8- to 60-membered heteropolycyclic group, each substituted with at least one selected from deuterium, -F, -Cl, -Br, -I, a cyano group, a nitro group, a methyl group, a methoxy group, a phenyl group, a naphthyl group, and a biphenyl group, but examplesare not limited thereto.

In some examples, R₃ to R₁₂ may each independently be selected from hydrogen, deuterium, -F, -Cl, -Br, -I, a methyl group, an ethyl group, a propyl group, an iso-propyl group, an n-butyl group, an isobutyl group, a sec-butyl group, a ter-butyl group, a pentyl group, an iso-amyl group, a hexyl group, a phenyl group, and a biphenyl group, but examplesare not limited thereto.

In some embodiments, R₂ is not a group represented by

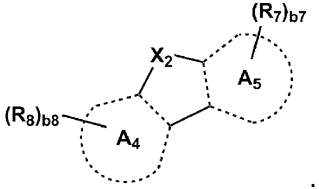

In Formulae 1A and 1B, b1 to b3 and b5 to b8 are each independently an integer from 1 to 10, and b4 is an integer from 1 to 3.
b1 indicates the number of R₁ groups; when b1 is 2 or greater, at least two R₁ groups may be identical to or different from each other. Descriptions for b2 to b8 may each be understood by referring to (may be the same as) the descriptions for b1 provided herein.

The substituted C₅-C₆₀ carbocyclic group, the substituted C₁-C₆₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted C₁-C₆₀ heteroaryloxy group, the substituted monovalent non-aromatic condensed C8 to C60 polycyclic group, and the substituted monovalent non-aromatic condensed 8- to 60-membered heteropolycyclic group are each independently substituted by one or more substituents (e.g. 1, 2,3, 4 or 5 substiuents)selected from:
deuterium, -F, -Cl, -Br, -I, a hydroxyl group, a cyano group, a nitro group, an amino group, an amidino group, a hydrazino group, a hydrazono group, a C₁-C₆₀ alkyl group, a C₂-C₆₀ alkenyl group, a C₂-C₆₀ alkynyl group, and a C₁-C₆₀alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, -Br, - I, a hydroxyl group, a cyano group, a nitro group, an amino group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed C8 to C60 polycyclic group, a monovalent non-aromatic condensed 8- to 60-membered heteropolycyclic group, -Si(Q₁₁)(Q₁₂)(Q₁₃), -N(Q₁₁)(Q₁₂), - B(Q₁₁)(Q₁₂), -C(=O)(Q₁₁), -S(=O)₂(Q₁₁), and -P(=O)(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed C8 to C60 polycyclic group, a monovalent non-aromatic condensed 8- to 60-membered heteropolycyclic group, a biphenyl group, and a terphenyl group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed C8 to C60 polycyclic group, a monovalent non-aromatic condensed 8- to 60-membered heteropolycyclic group, a biphenyl group, and a terphenyl group, each substituted with at least one selected from deuterium, -F, -Cl, -Br, -I, a hydroxyl group, a cyano group, a nitro group, an amino group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed C8 to C60 polycyclic group, a monovalent non-aromatic condensed 8- to 60-membered heteropolycyclic group, a biphenyl group, a terphenyl group, -Si(Q₂₁)(Q₂₂)(Q₂₃), - N(Q₂₁)(Q₂₂), -B(Q₂₁(Q₂₂), -C(=O)(Q₂₁), -S(=O)₂(Q₂₁), and -P(=O)(Q₂₁)(Q₂₂); and
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and - P(=O)(Q₃₁)(Q₃₂),
wherein Q₁ to Q₃, Q₁₁ to Q₁₃, Q₂₁ to Q₂₃, and Q₃₁ to Q₃₃ are each independently selected from hydrogen, deuterium, -F, -Cl, -Br, -I, a hydroxyl group, a cyano group, a nitro group, an amino group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with a C₁-C₆₀ alkyl group, a C₁-C₆₀ heteroaryl group, a monovalent non-aromatic condensed C8 to C60 polycyclic group, a monovalent non-aromatic condensed 8- to 60-membered heteropolycyclic group, a biphenyl group, and a terphenyl group.

According to an embodiment, the amine-based compound is represented by Formula 1D,: 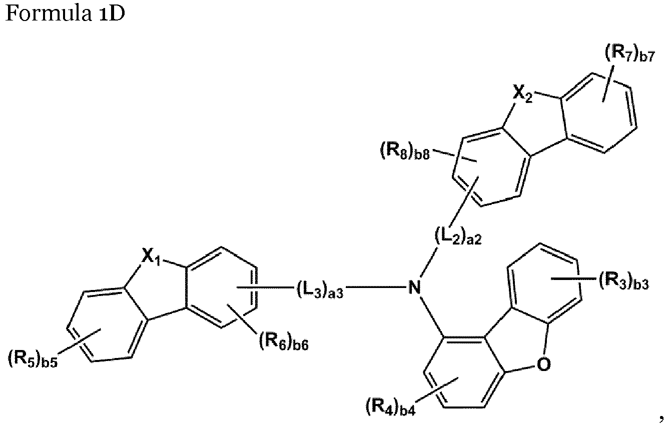
wherein, in Formulae and 1D,
descriptions for substituent groups may be understood by referring to the claims
b4, b6, and b8 may each independently be an integer from 1 to 3, and
b3, b5, and b7 may each independently be an integer from 1 to 4.

In some examples, the compound represented by one of Formulae 1A and 1B maybe represented by one of Formulae 1A-1 to 1A-4 and 1B-1 to 1B-16, but embodiments are not limited thereto: 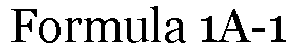 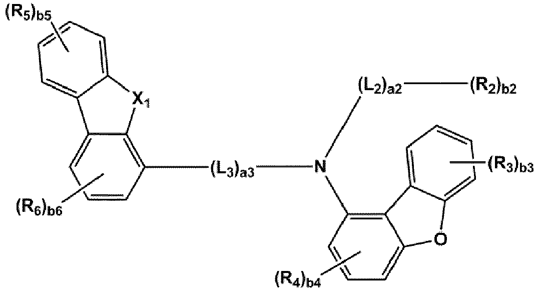 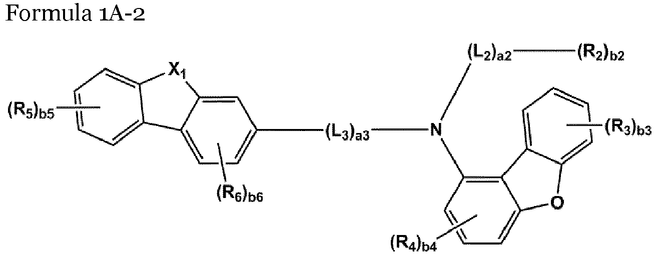 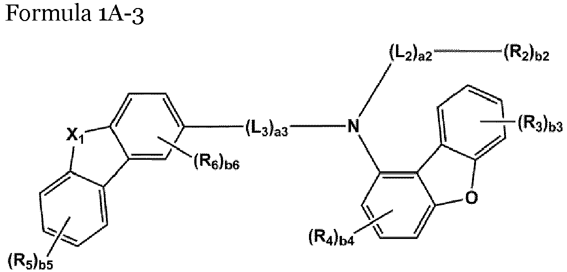 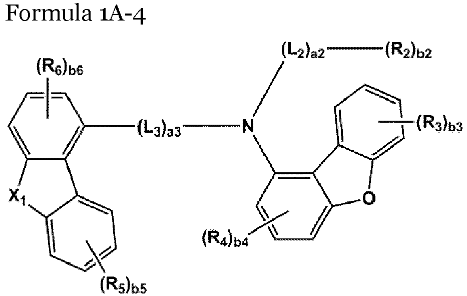 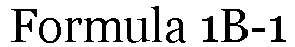 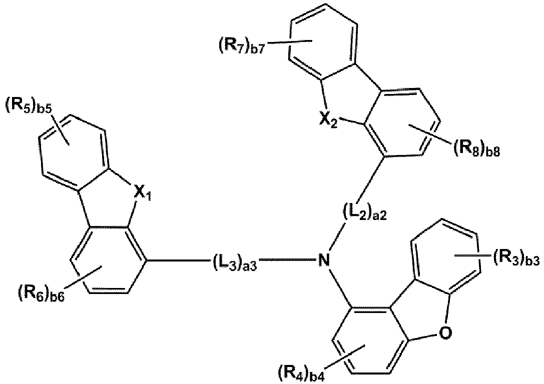 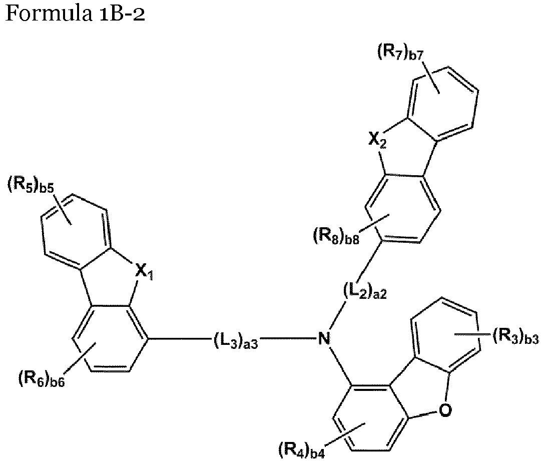 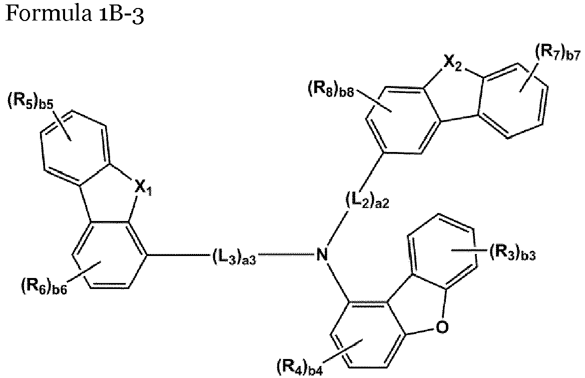 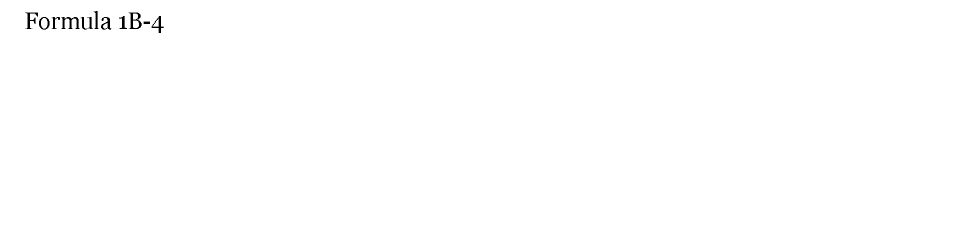 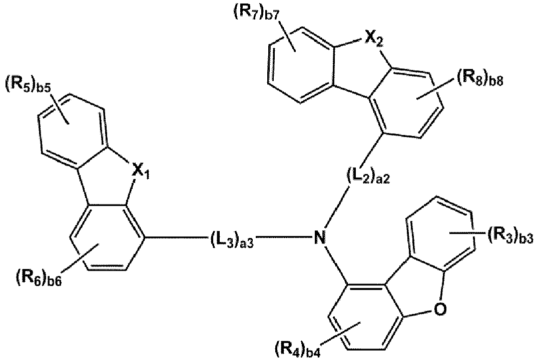 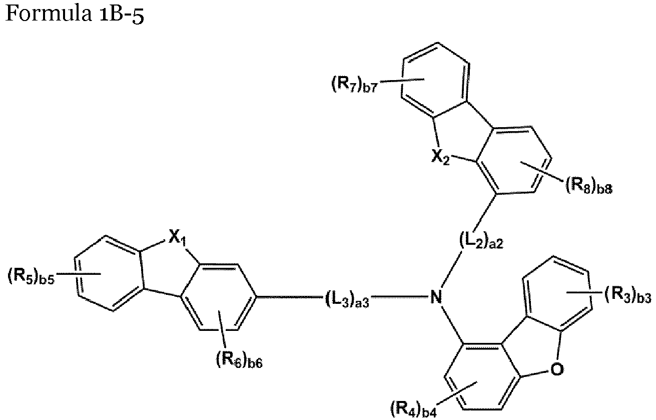 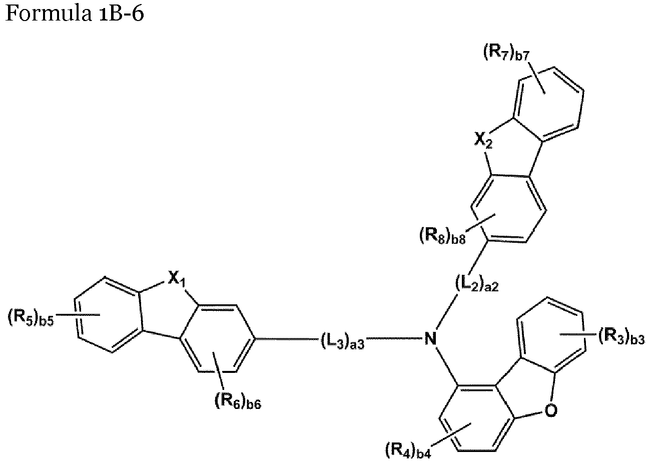 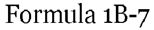 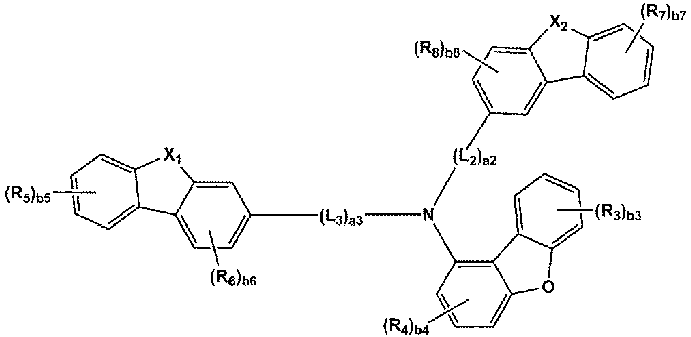 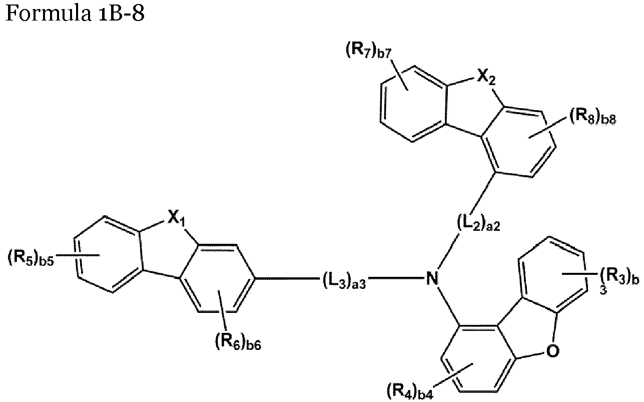 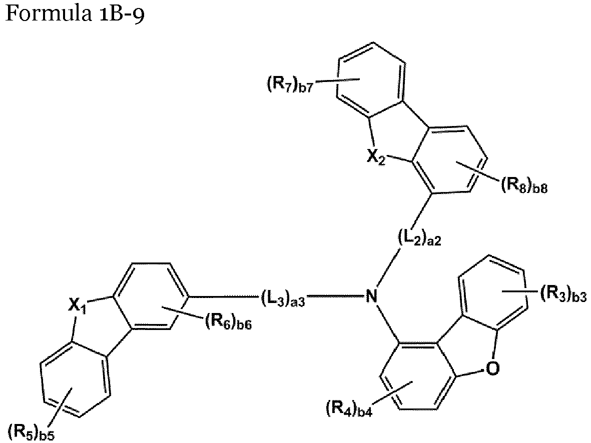 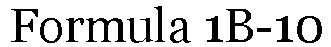 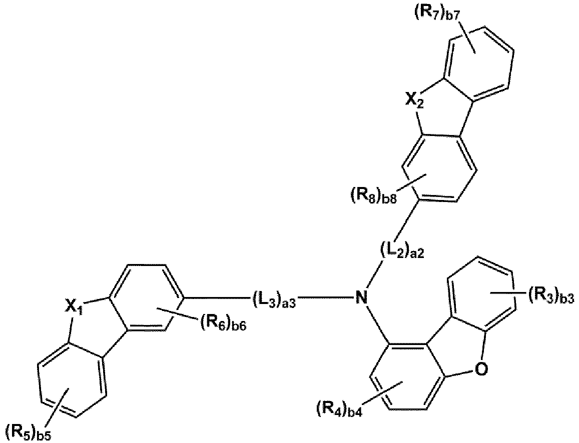 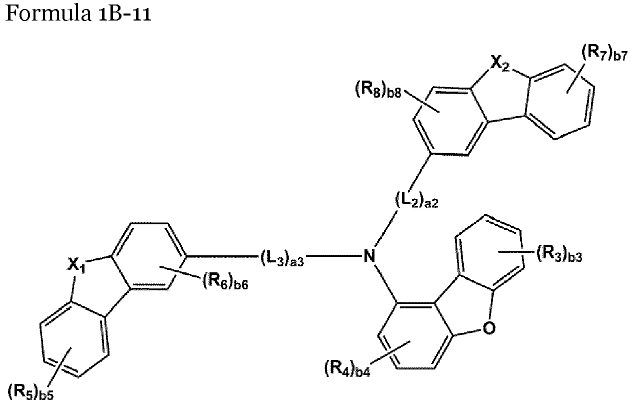 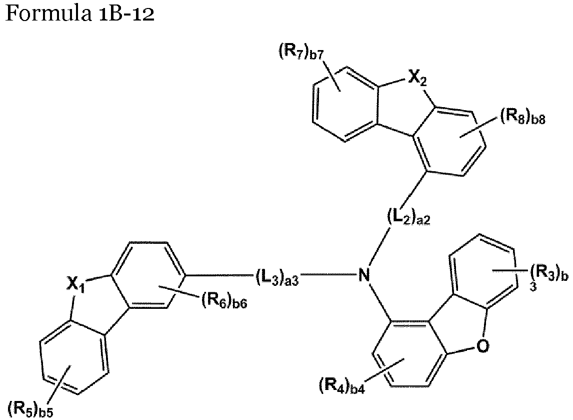 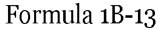 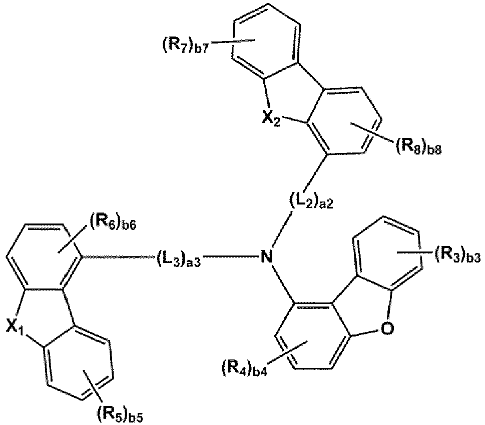 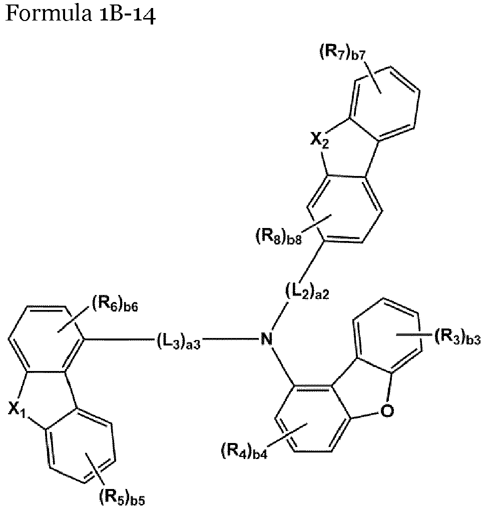 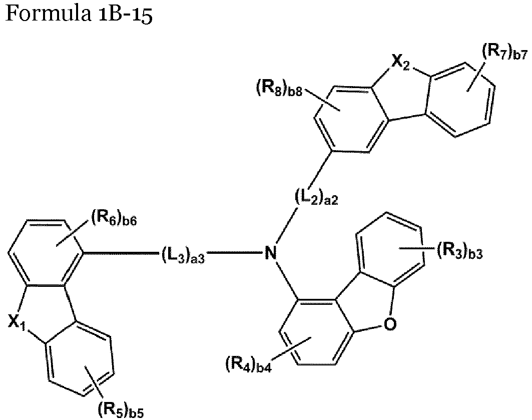 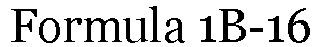 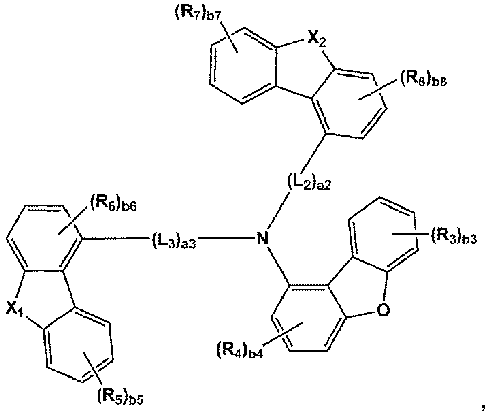 wherein, in Formulae 1A-1 to 1A-4 and 1B-1 to 1B-16,
descriptions for X₁, X₂, L₁ L₂, a2, a3, R₂ to R₈, and b2 may be understood by referring to (may be the same as) the descriptions for those of Formulae 1A and 1B,
b4, b6, and b8 may each independently be an integer from 1 to 3, and
b3, b5, and b7 may each independently be an integer from 1 to 4.

In some examples, in Formulae 1A-1 to 1A-4 and 1B-1 to 1B-16, X₁ may be N-(L₁)ₐ₁-(R₁)_{b1}, and X₂ may be O, S, or C(R₁₁)(R₁₂),
X₁ may be O, and X2 may be C(R₁₁)(R₁₂), or
X₁ may be C(R₉)(R₁₀), and X₂ may be C(R₁₁)(R₁₂), but examplesare not limited thereto.

In some examples, in Formulae 1A-1 to 1A-4 and 1B-1 to 1B-16, L₁ to L₃ may each independently be selected from a single bond and a benzene group; and
a benzene group substituted with at least one selected from deuterium, -F, -Cl, -Br, -I, a hydroxyl group, a cyano group, a nitro group, an amino group, an amidino group, a hydrazino group, a hydrazono group, a C₁-C₂₀ alkyl group, a C₁-C₂₀ alkoxy group, a C₆-C₂₀ aryl group, a C₁-C₂₀ heteroaryl group, and -Si(Q₃₁)(Q₃₂)(Q₃₃), but examplesare not limited thereto.

In some examples, in Formulae 1A-1 to 1A-4 and 1B-1 to 1B-16, R₁ and R₂ may each independently be selected from Formulae 6-1 to 6-42, but examplesare not limited thereto.

In some examples, in Formulae 1A and 1B, R₉ and R₁₀ and/or R₁₁ and R₁₂ may be bound to each other to form a group represented by one of Formulae 7-1 to 7-3:

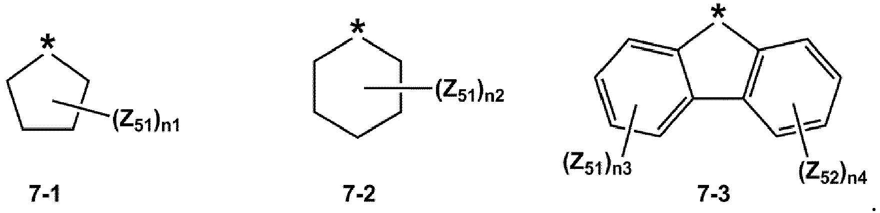

In Formulae 7-1 to 7-3,
Z₅₁ and Z₅₂ may each independently be selected from hydrogen, deuterium, -F, - Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a naphthyl group, a fluorenyl group, a spiro-bifluorenyl group, a spiro-fluorene-benzofluorenyl group, a benzofluorenyl group, a dibenzofluorenyl group, a phenalenyl group, a phenanthrenyl group, an anthracenyl group, a fluoranthenyl group, a triphenylenyl group, a pyridinyl group, a pyrazinyl group, a pyrimidinyl group, a quinolinyl group, an isoquinolinyl group, a benzoquinolinyl group, a naphthyridinyl group, a quinoxalinyl group, a quinazolinyl group, a carbazolyl group, a phenanthridinyl group, an acridinyl group, a phenanthrolinyl group, a phenazinyl group, a triazinyl group, a dibenzofuranyl group, a dibenzothiophenyl group, and -Si(Q₃₁)(Q₃₂)(Q₃₃),
n1 may be an integer from 1 to 8,
n2 may be an integer from 1 to 10,
n3 and n4 may each independently be an integer from 1 to 4, and
^{∗} indicates a carbon atom bound to R₉ and R₁₀ or bound to R₁₁ and R₁₂.

In some embodiments, the compound represented by one of Formula 1Dmay be selected from Compounds 42-45, 47-50, 56-59, 61-64, 70-73, 75-78, 84-87, and 89-92. Compounds 1-41, 46, 51-55, 60, 65-69, 74, 79-83, 88, 93to 265, 267 to 284, 207A, 208A and 226A are included only for reference and do not form part of the claimed invention:

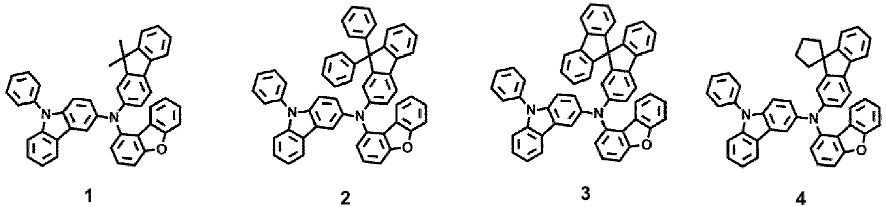

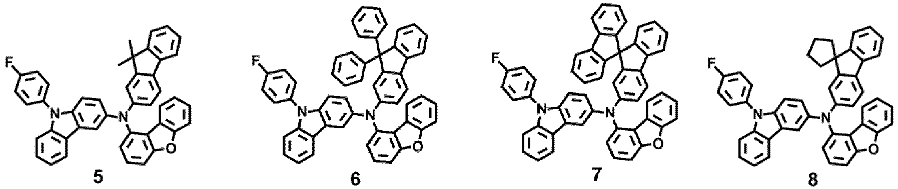

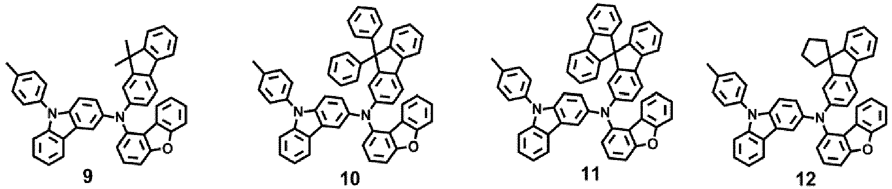

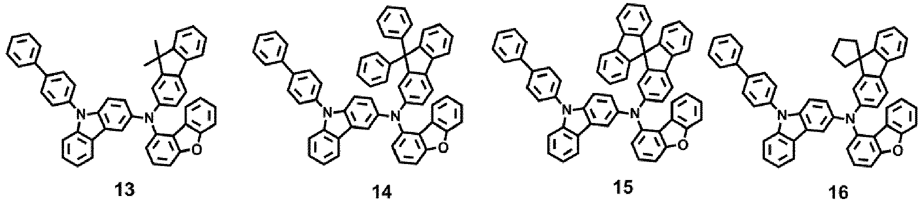

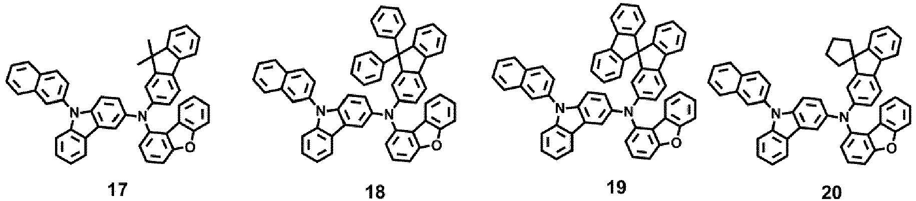

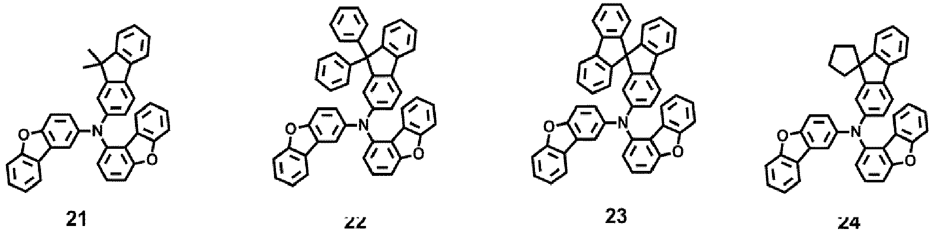

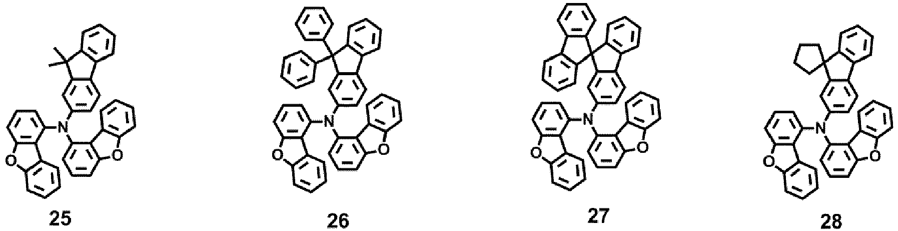

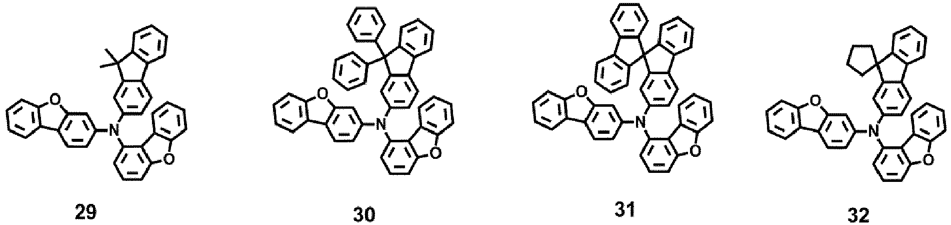

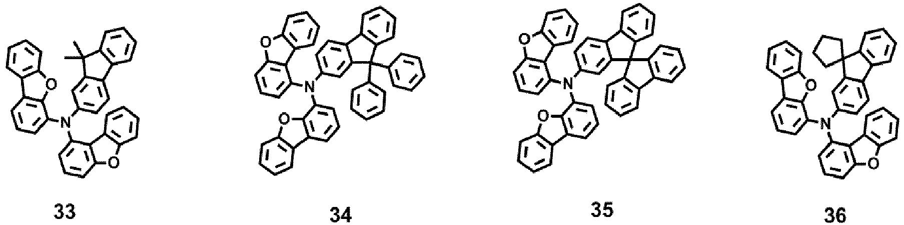

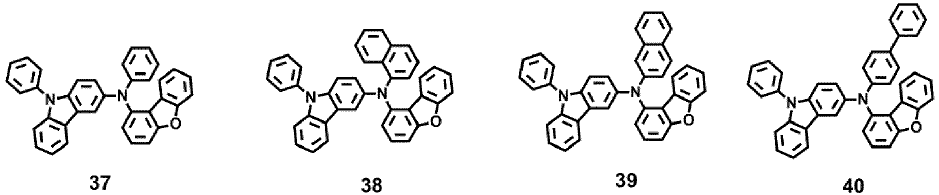

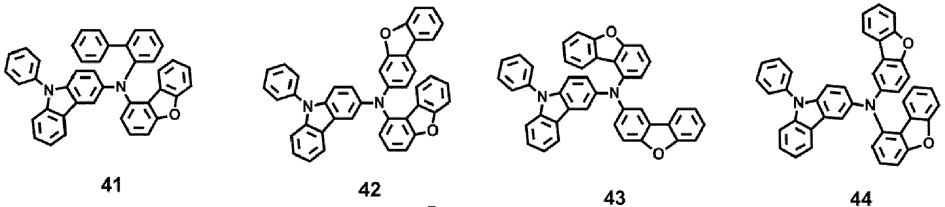

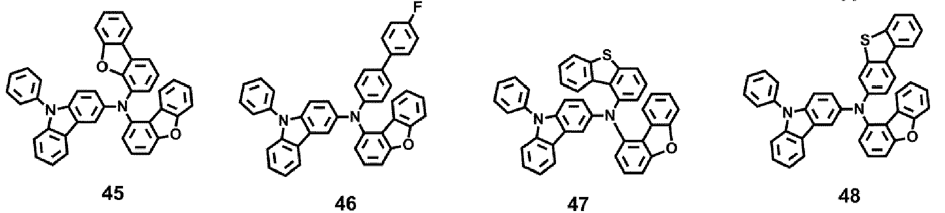

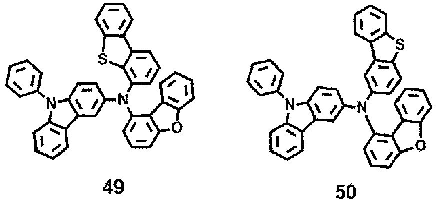

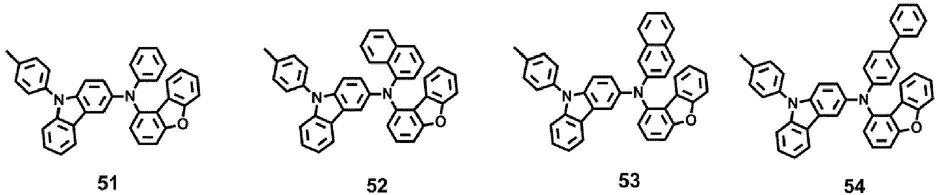

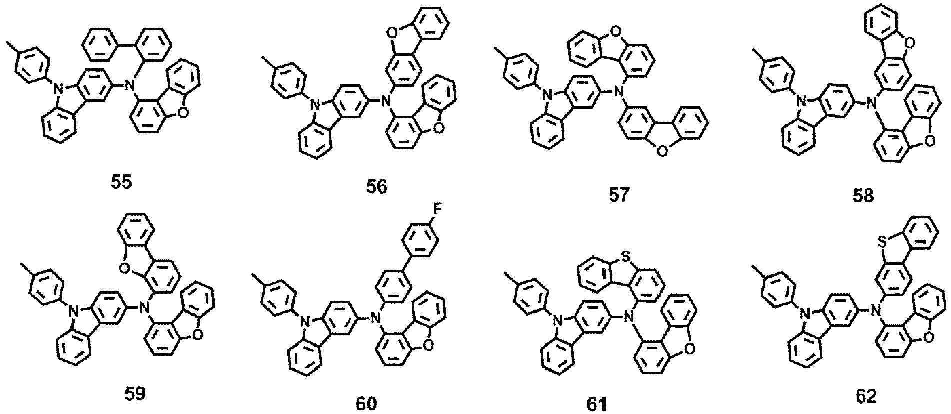

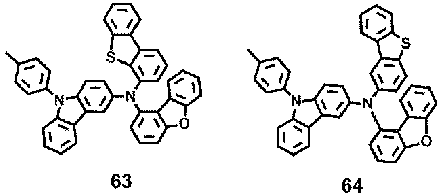

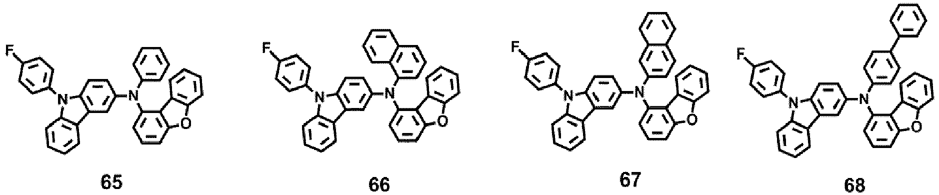

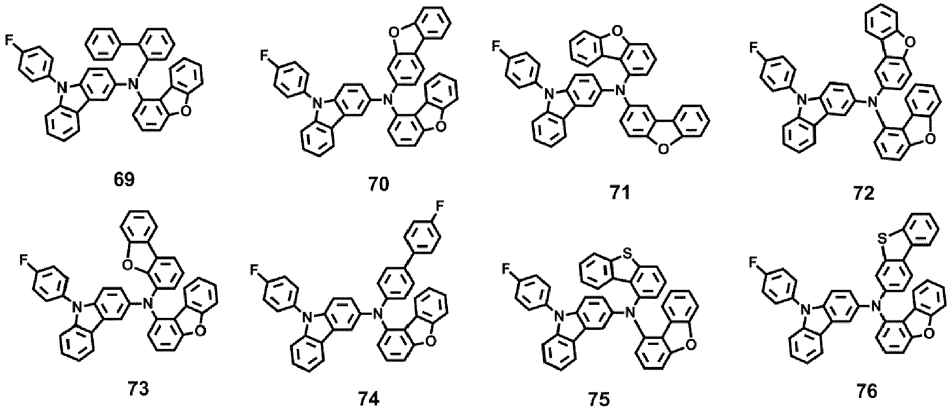

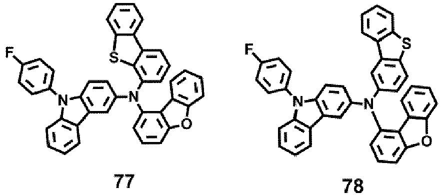

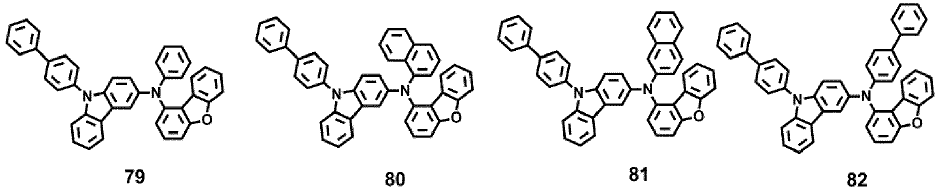

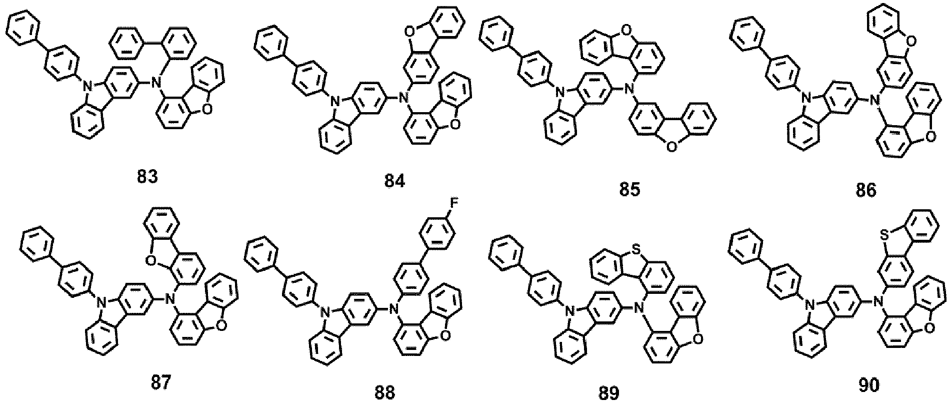

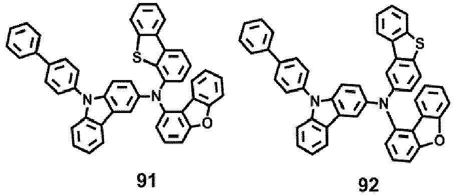

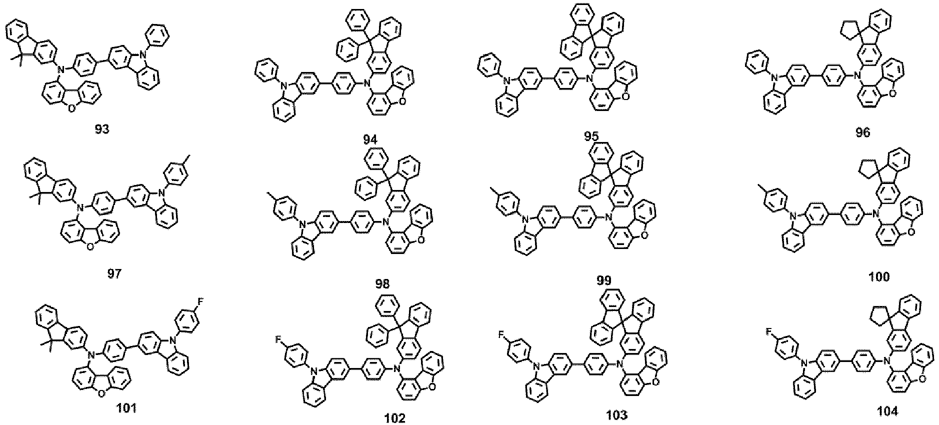

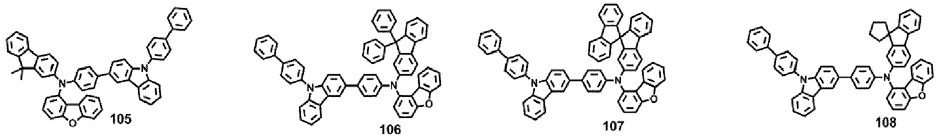

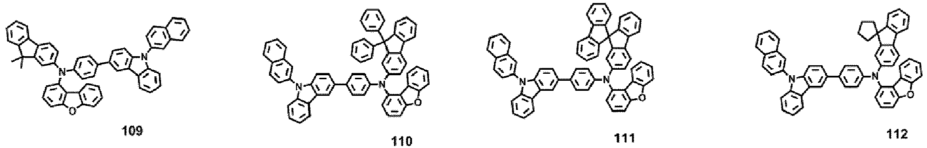

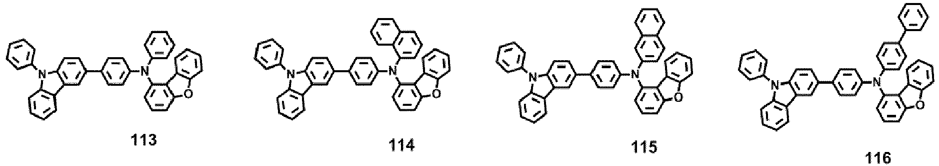

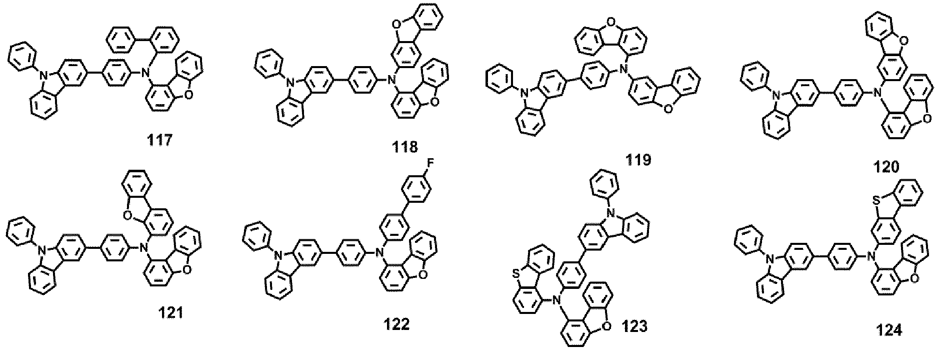

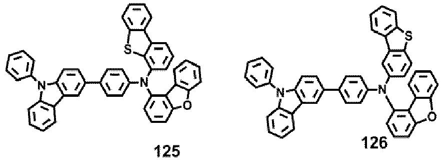

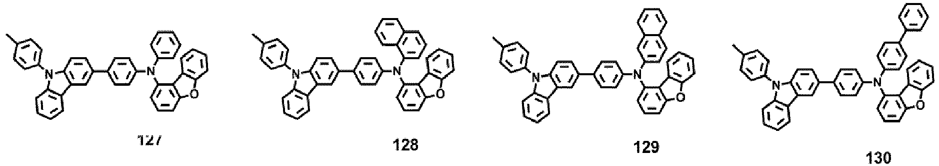

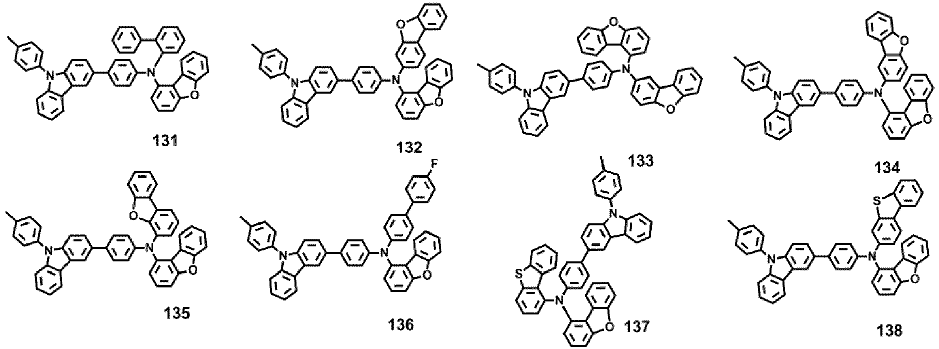

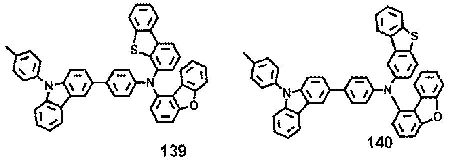

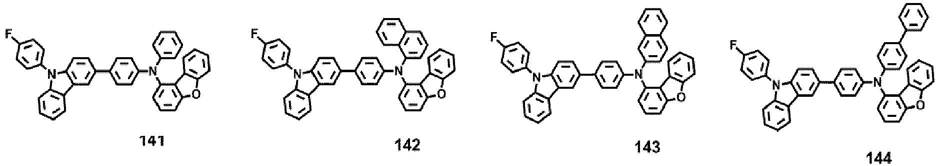

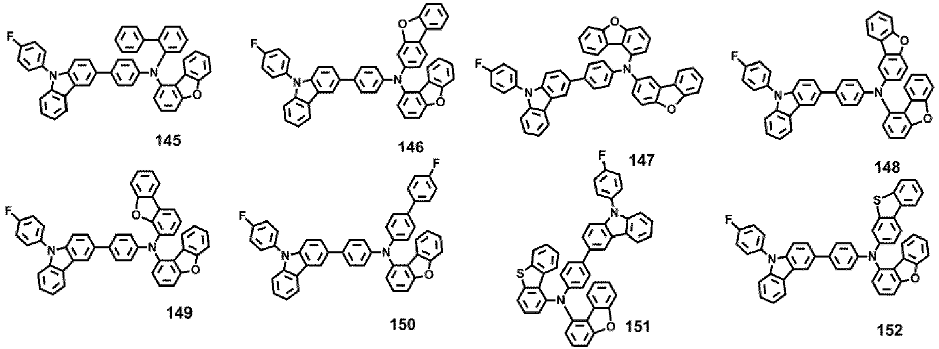

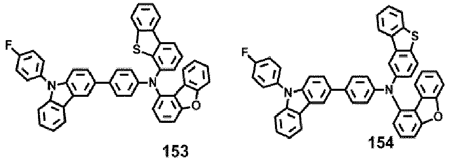

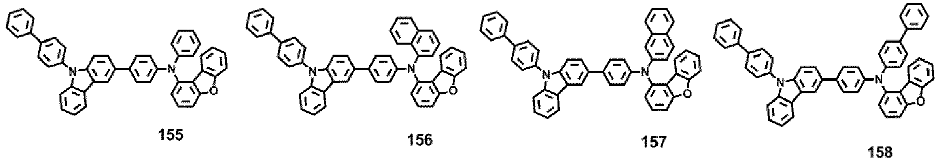

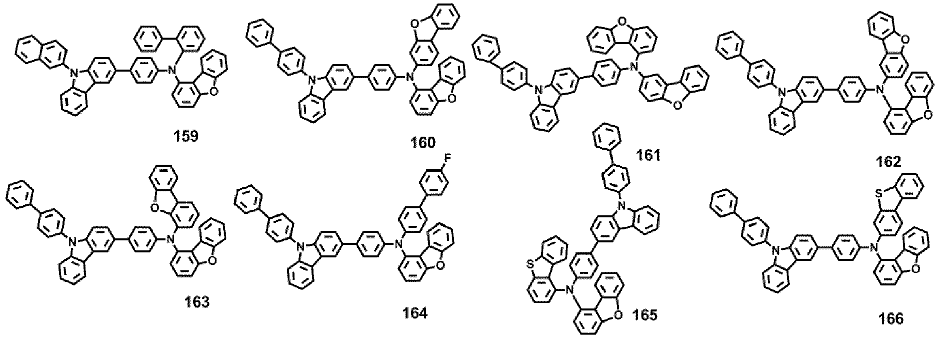

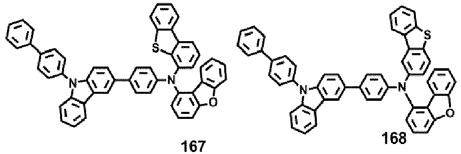

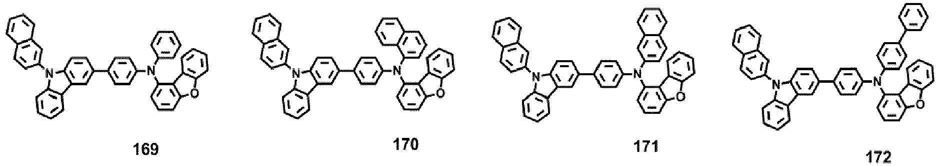

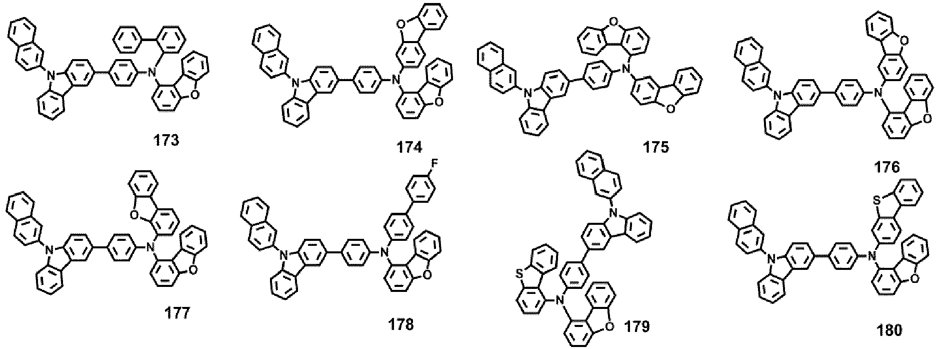

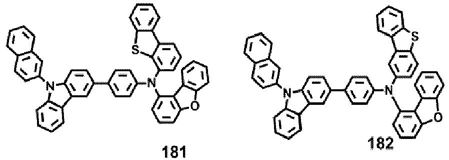

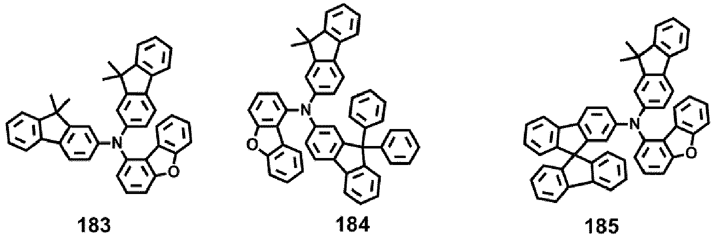

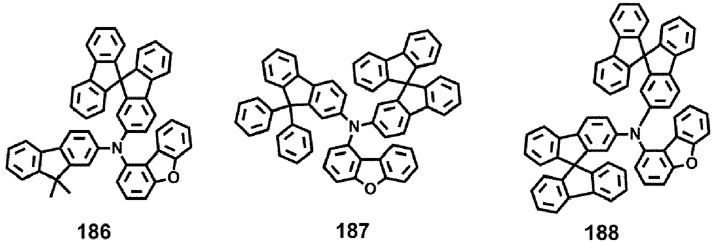

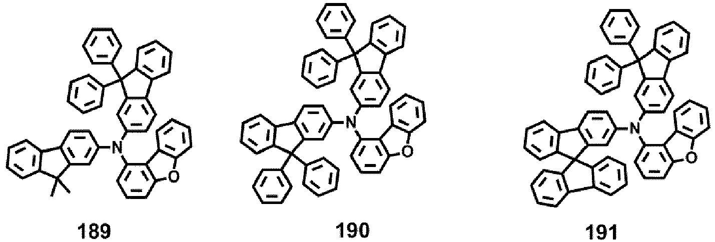

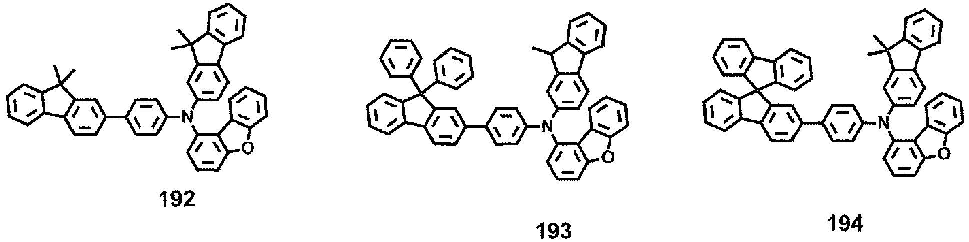

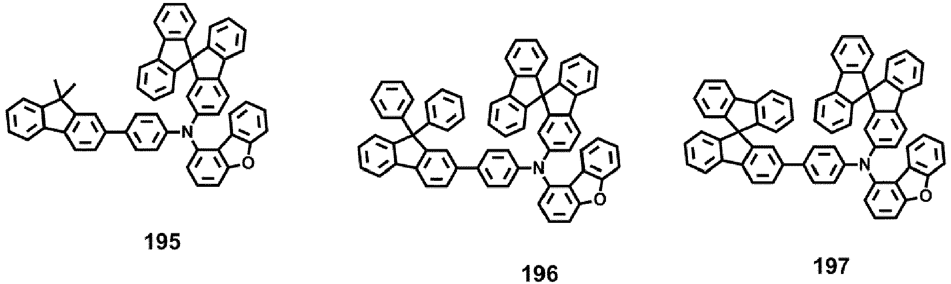

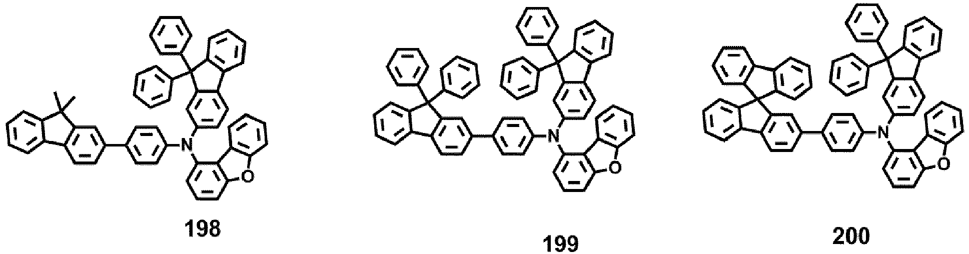

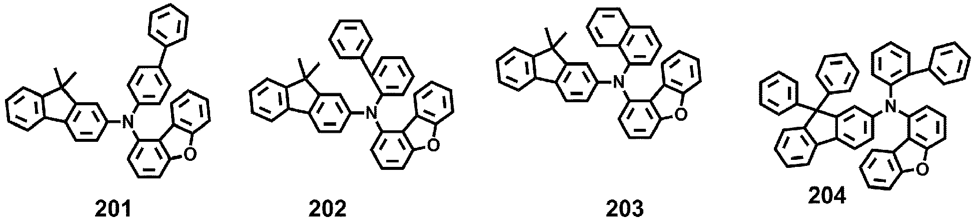

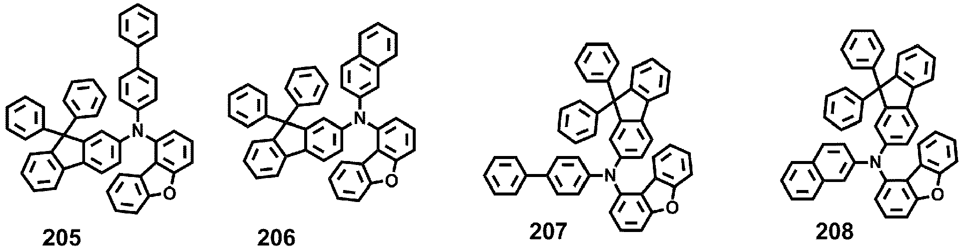

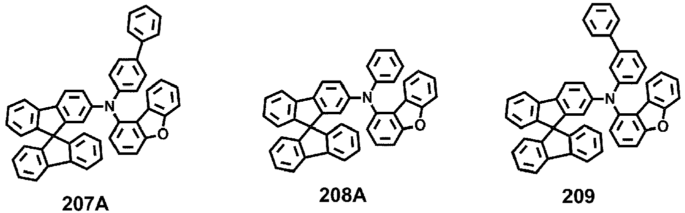

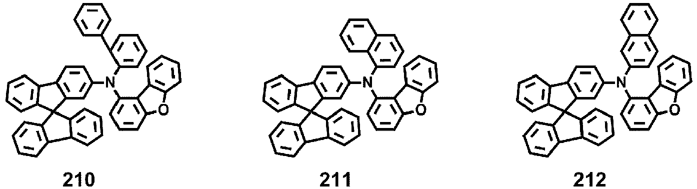

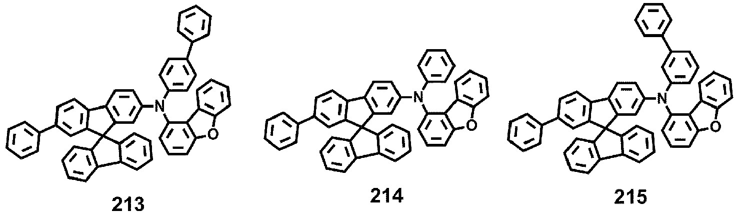

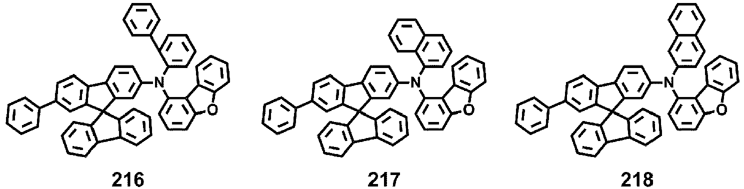

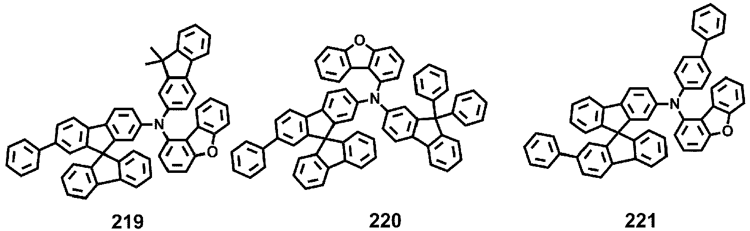

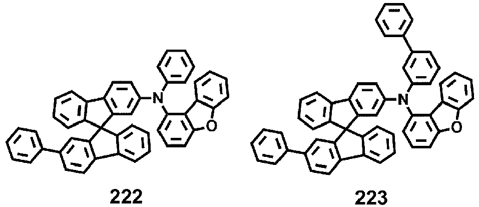

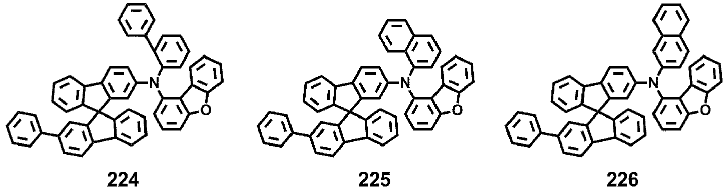

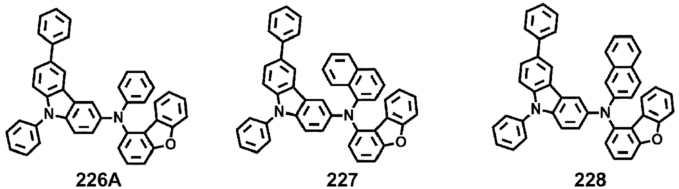

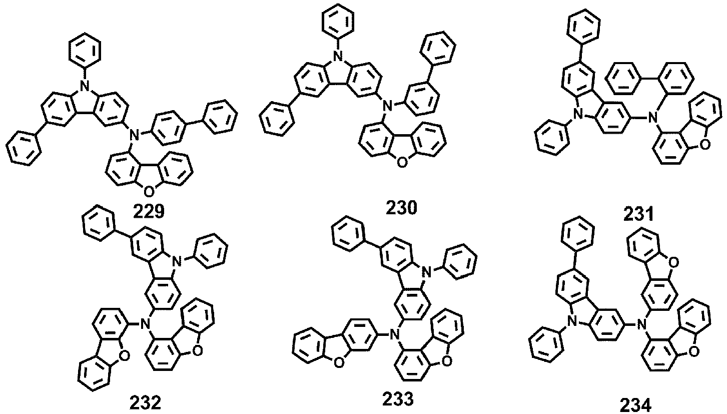

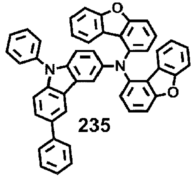

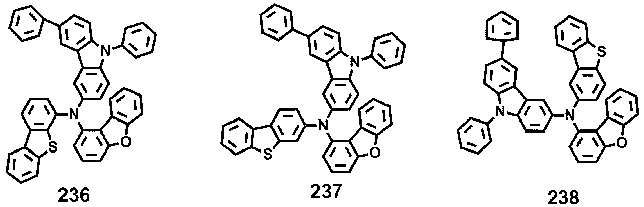

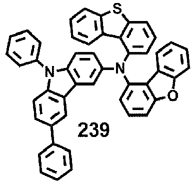

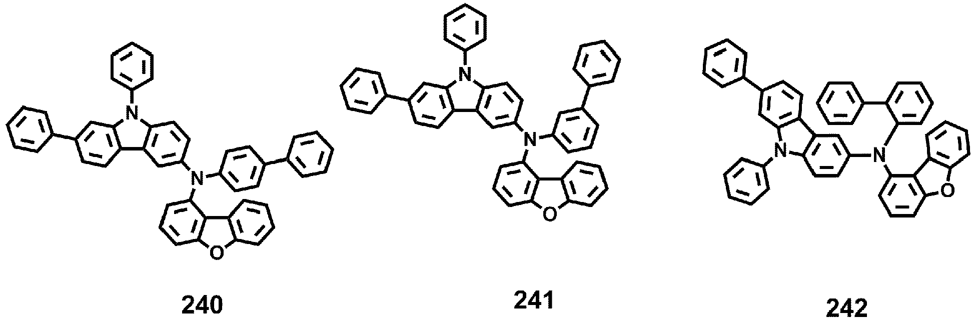

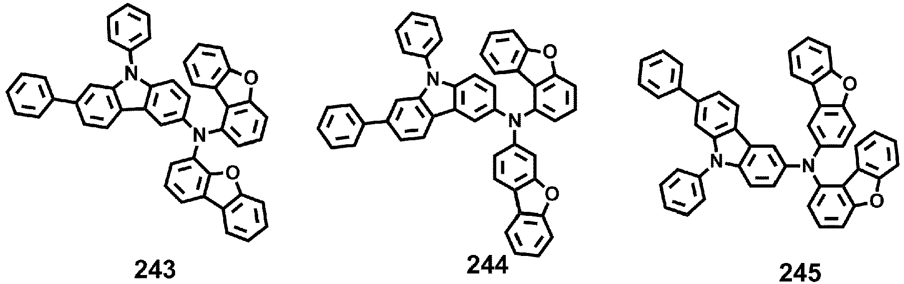

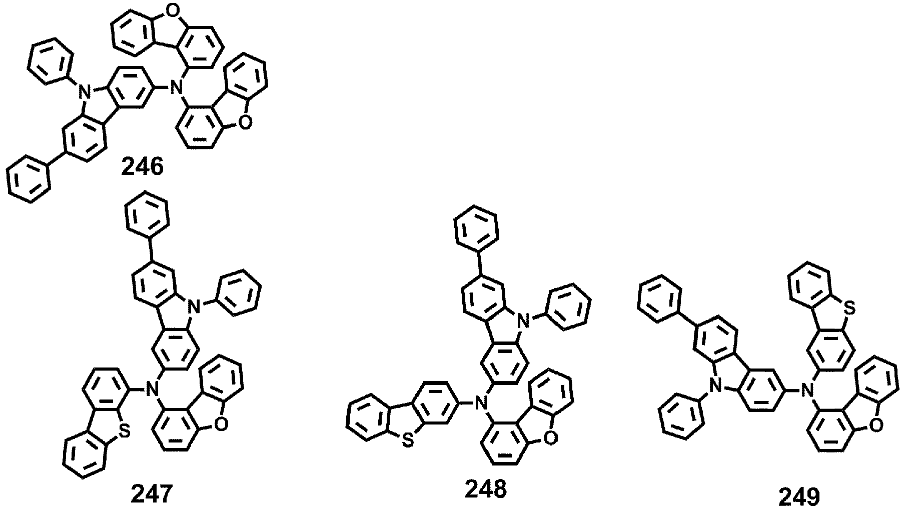

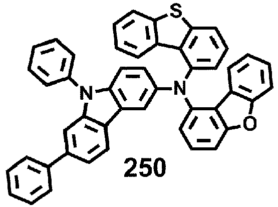

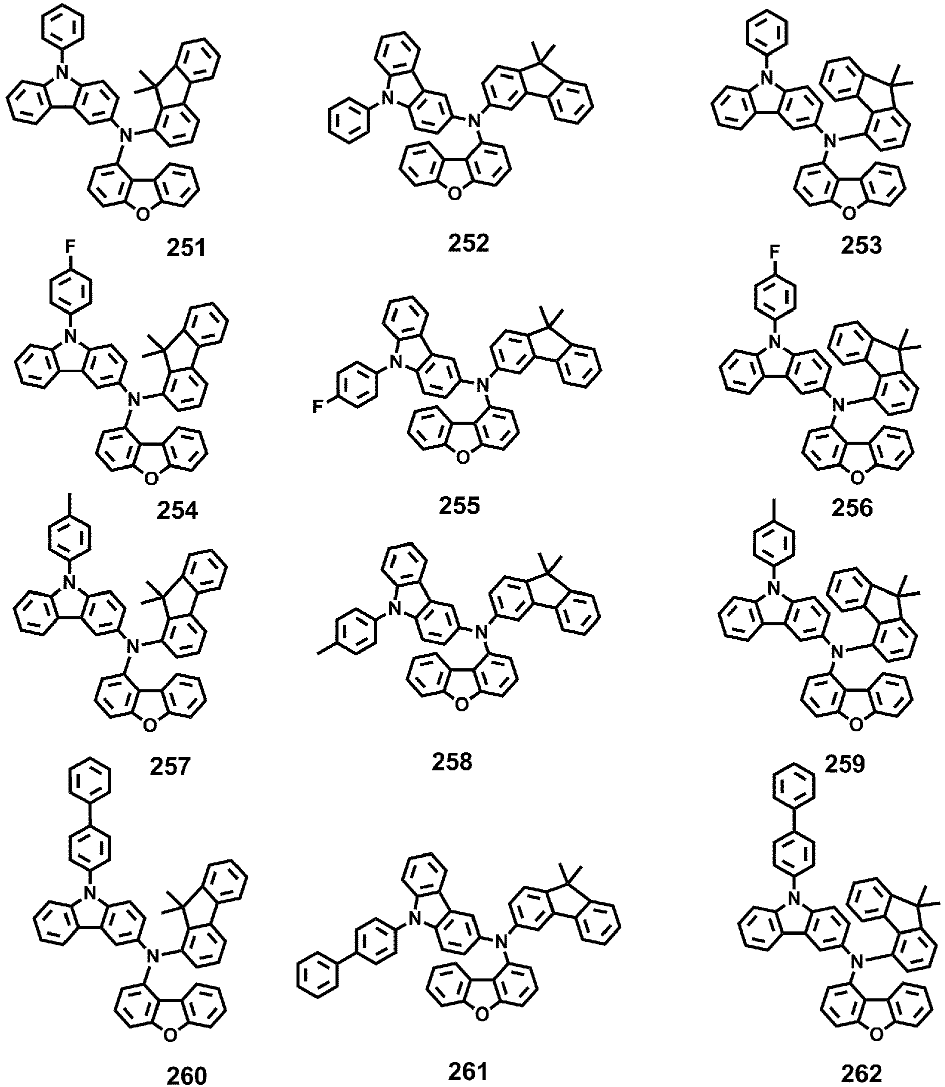

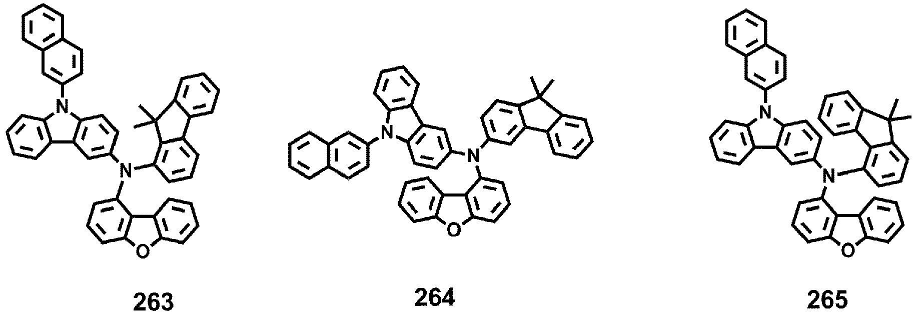

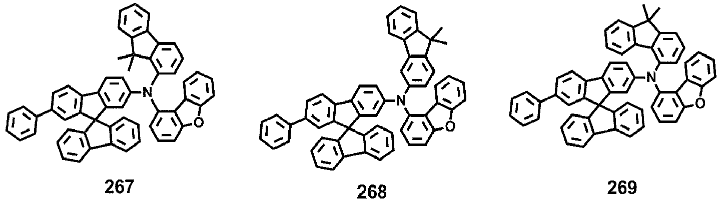

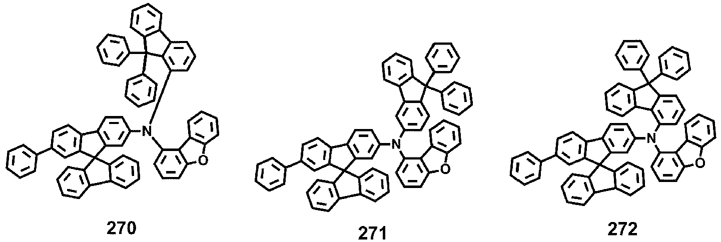

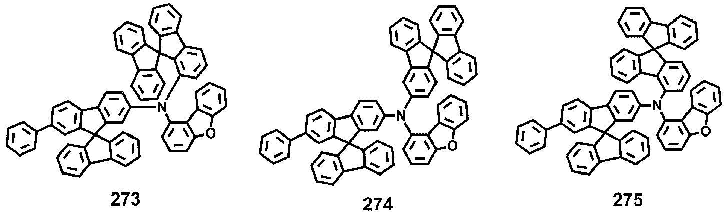

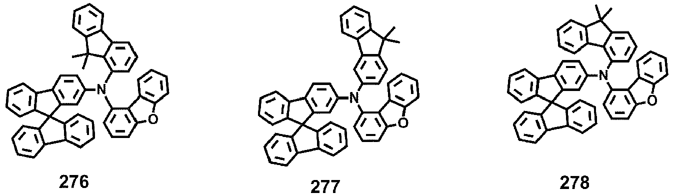

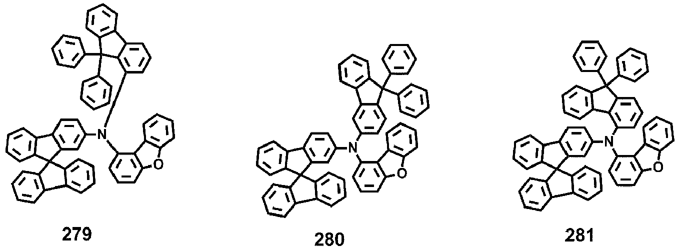

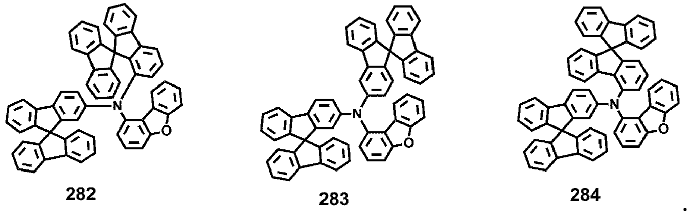

The amine-based compound represented by one of Formulae 1A and 1B has a structure in which nitrogen (N) is bound to a substituent represented by Formula 1A', and thus the compound may have a molecular structure having a large steric hindrance. Accordingly, the amine-based compound may maintain optimum intermolecular density. Further, the amine-based compound may have excellent hole transportability due to the increased interaction of an unshared electron pair of oxygen (O), which is relatively sterically exposed:

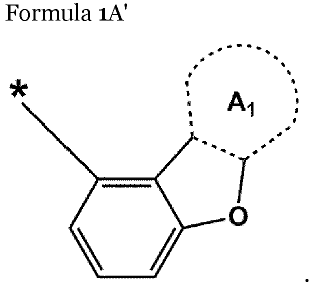

Furthermore, the amine-based compound comprises an amine group in a molecule. Thus, the amine-based compound may have a suitable energy level, as compared with a compound comprising at least two amine groups. Accordingly, the amine-based compound may have improved hole mobility due to reduced hole transport barrier and hole injection barrier.

Therefore, an electronic device, e.g., an organic light-emitting device, employing the amine-based compound may have a low driving voltage, high efficiency, and long lifespan.

Methods of synthesizing the amine-based compound represented by one of Formulae 1A and 1B and 1D should be readily apparent to those of ordinary skill in the art by referring to Examples described herein.

Accordingly, there is provided an organic light-emitting device comprising a first electrode; a second electrode facing the first electrode; and an organic layer between the first electrode and the second electrode, wherein the organic layer comprises an emission layer and at least one amine-based compound represented by Formula 1D.

As used herein, the expression "(for example, the organic layer) comprising at least one amine-based compound" refers to that "(the organic layer) comprising an amine-based compound represented by one of Formulae 1A and 1B, or at least two different amine-based compounds represented by one of Formulae 1A and 1B".

For example, the organic layer may contain only Compound 1 as the amine-based compound. In this embodiment, Compound 1 may be contained in the emission layer of the organic light-emitting device. In some embodiments, the organic layer may contain Compounds 1 and 2 as the amine-based compounds. In this embodiment, Compounds 1 and 2 may be present in the same layer (for example, Compounds 1 and 2 may be both present in an emission layer), or in different layers (for example, Compound 1 may be present in an emission layer, and Compound 2 may be present in an electron transport layer).

In some embodiments, the first electrode may be an anode, the second electrode may be a cathode, and the organic layer may further comprise a hole transport region between the first electrode and the emission layer and an electron transport region between the emission layer and the second electrode, wherein the hole transport region may comprise a hole injection layer, a hole transport layer, an emission auxiliary layer, an electron blocking layer, or a combination thereof, and the electron transport region may comprise a hole blocking layer, an electron transport layer, an electron injection layer, or a combination thereof.

In some embodiments, the hole transport region may comprise the amine-based compound.

In some embodiments, the hole transport region may comprise a hole injection layer and a hole transport layer, wherein at least one of the hole injection layer and the hole transport layer may comprise the amine-based compound.

In some embodiments, the hole transport region may comprise a hole transport layer, wherein the hole transport layer may comprise the amine-based compound.

In some embodiments, examples of the p-dopant may include at least one selected from a quinone derivative, a metal oxide, and a cyano group-containing compound.

In some embodiments, the hole transport region may comprise a hole injection layer comprising the p-dopant.

The term "organic layer" as used herein refers to a single and/or a plurality of layers between the first electrode and the second electrode in an organic light-emitting device. A material comprised in the "organic layer" is not limited to an organic material.

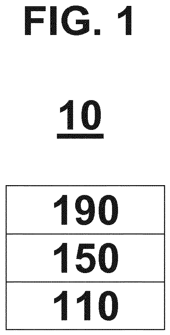

### Description of FIG. 1

FIG. 1 illustrates a schematic cross-sectional view of an organic light-emitting device 10 according to an embodiment. The organic light-emitting device 10 may comprise a first electrode 110, an organic layer 150, and a second electrode 190.

Hereinafter, the structure of the organic light-emitting device 10 according to an embodiment and a method of manufacturing an organic light-emitting device 10 according to an embodiment will be described in connection with FIG. 1.

### First electrode 110

In FIG. 1, a substrate may be additionally disposed under the first electrode **110** or above the second electrode 190. The substrate may be a glass substrate or a plastic substrate, each having excellent mechanical strength, thermal stability, transparency, surface smoothness, ease of handling, and water resistance.

The first electrode **110** may be formed by vacuum-depositing or sputtering, onto the substrate, a material for forming the first electrode 110. When the first electrode **110** is an anode, the material for the first electrode **110** may be selected from materials with a high work function to facilitate hole injection.

The first electrode **110** may be a reflective electrode, a semi-transmissive electrode, or a transmissive electrode. When the first electrode **110** is a transmissive electrode, as a material for forming the first electrode 110, indium tin oxide (ITO), indium zinc oxide (IZO), tin oxide (SnO₂), zinc oxide (ZnO), and any combination thereof may be utilized, but embodiments are not limited thereto. When the first electrode **110** is a semi-transmissive electrode or a reflective electrode, as a material for forming the first electrode 110, magnesium (Mg), aluminum (Al), aluminum-lithium (Al-Li), calcium (Ca), magnesium-indium (Mg-In), magnesium-silver (Mg-Ag), and any combination thereof may be utilized, but embodiments are not limited thereto.

The first electrode **110** may have a single-layered structure, or a multi-layered structure comprising two or more layers. In some embodiments, the first electrode **110** may have a triple-layered structure of ITO/Ag/ITO, but embodiments are not limited thereto.

### Organic layer 150

The organic layer **150** may be on the first electrode 110. The organic layer **150** may comprise an emission layer.

The organic layer **150** may further comprise a hole transport region between the first electrode **110** and the emission layer and an electron transport region between the emission layer and the second electrode 190.

### Hole transport region in organic layer 150

The hole transport region may have i) a single-layered structure including a single layer including a single material, ii) a single-layered structure including a single layer comprising a plurality of different materials, or iii) a multi-layered structure having a plurality of layers, each comprising a single or a plurality of different materials.

The hole transport region may comprise at least one selected from a hole injection layer, a hole transport layer, an emission auxiliary layer, and an electron blocking layer.

For example, the hole transport region may have a single-layered structure including a single layer comprising a plurality of different materials or a multi-layered structure, e.g., a structure of hole injection layer/hole transport layer, a structure of hole injection layer/hole transport layer/emission auxiliary layer, a structure of hole injection layer/emission auxiliary layer, a structure of hole transport layer/emission auxiliary layer, or a structure of hole injection layer/hole transport layer/electron blocking layer, wherein layers of each structure are sequentially stacked on the first electrode **110** in each stated order, but embodiments are not limited thereto.

The hole transport region may comprise the amine-based compound represented by one of Formulae 1A and 1B.

The hole transport region may comprise, in addition to the amine-based compound represented by one of Formulae 1A and 1B, at least one selected from m-MTDATA, TDATA, 2-TNATA, NPB (NPD), β-NPB, TPD, spiro-TPD, spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), (polyaniline)/poly(4-styrenesulfonate) (PANI/PSS), a compound represented by Formula 201, and a compound represented by Formula 202: 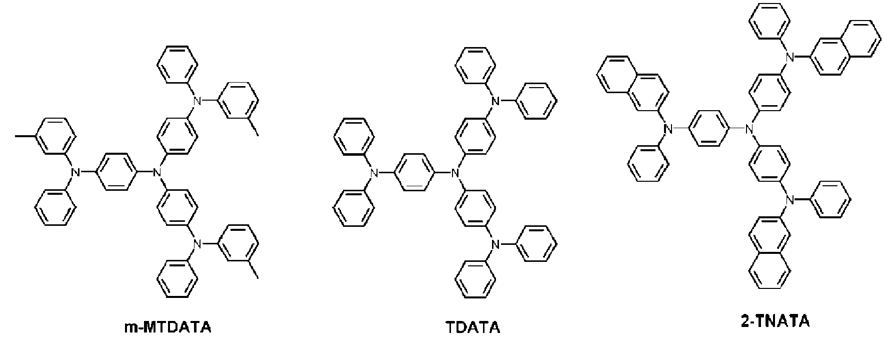 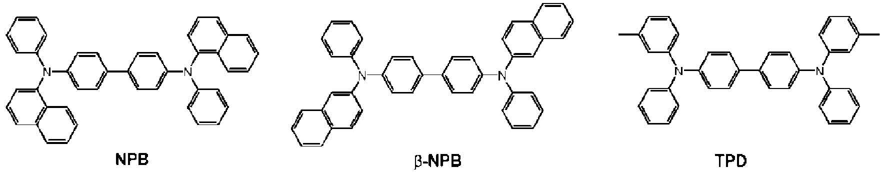 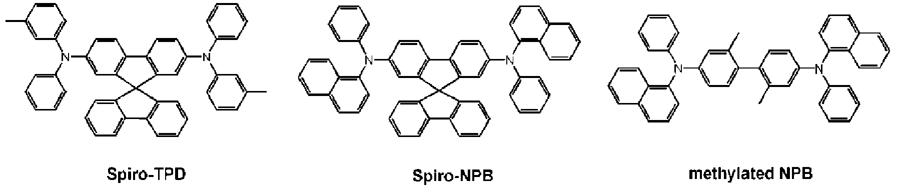 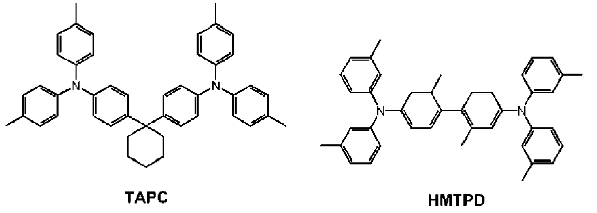 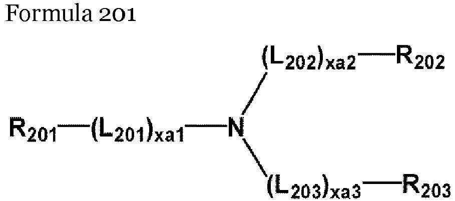 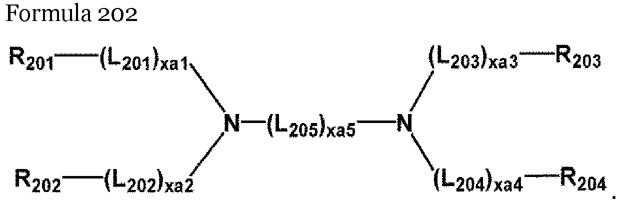 In Formulae 201 and 202,
L₂₀₁ to L₂₀₄ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed C8 to C60 polycyclic group, and a substituted or unsubstituted divalent non-aromatic condensed 8-to 60-membered heteropolycyclic group,
L₂₀₅ may be selected from ^{∗}-O-^{∗}', ^{∗}-S-^{∗}', ^{∗}-N(Q₂₀₁)-^{∗}', a substituted or unsubstituted C₁-C₂₀ alkylene group, a substituted or unsubstituted C₂-C₂₀ alkenylene group, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed C8 to C60 polycyclic group, and a substituted or unsubstituted divalent non-aromatic condensed 8- to 60-membered heteropolycyclic group,
xa1 to xa4 may each independently be an integer from 0 to 3,
xa5 may be an integer from 1 to 10, and
R₂₀₁ to R₂₀₄ and Q₂₀₁ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed C8 to C60 polycyclic group, and a substituted or unsubstituted monovalent non-aromatic condensed 8- to 60-membered heteropolycyclic group.

In some embodiments, in Formula 202, R₂₀₁ and R₂₀₂ may optionally be linked to each other via a single bond, a dimethyl-methylene group, or a diphenyl-methylene group, and R₂₀₃ and R₂₀₄ may optionally be linked to each other via a single bond, a dimethyl-methylene group, or a diphenyl-methylene group.

In one embodiment, in Formulae 201 and 202, L₂₀₁ to L₂₀₅ may each independently be selected from
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group; and
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃), and -N(Q₃₁)(Q₃₂),
wherein Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

In one or more embodiments, xa1 to xa₄ may each independently be 0, 1, or 2.

In one or more embodiments, xa5 may be 1, 2, 3, or 4.

In one or more embodiments, R₂₀₁ to R₂₀₄ and Q₂₀₁ may each independently be selected from a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and
a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃), and -N(Q₃₁)(Q₃₂),
wherein descriptions for Q₃₁ to Q₃₃ may be understood by referring to (may be the same as) the descriptions for those provided herein.

In one or more embodiments, in Formula 201, at least one of R₂₀₁ to R₂₀₃ may each independently be selected from
a fluorenyl group, a spiro-bifluorenyl group, a carbazolyl group, a dibenzofuranyl group, and a dibenzothiophenyl group; and
a fluorenyl group, a spiro-bifluorenyl group, a carbazolyl group, a dibenzofuranyl group, and a dibenzothiophenyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a carbazolyl group, a dibenzofuranyl group, and a dibenzothiophenyl group, but embodiments are not limited thereto.

In one or more embodiments, in Formula 202, i) R₂₀₁ may be linked to R₂₀₂ via a single bond, and/or ii) R₂₀₃ may be linked to R₂₀₄ via a single bond.

In one or more embodiments, in Formula 202, at least one of R₂₀₁ to R₂₀₄ may be selected from
a carbazolyl group; and
a carbazolyl group substituted with at least one selected from deuterium, -F, - Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a carbazolyl group, a dibenzofuranyl group, and a dibenzothiophenyl group, but embodiments are not limited thereto.

The compound represented by Formula 201 may be represented by Formula 201A:

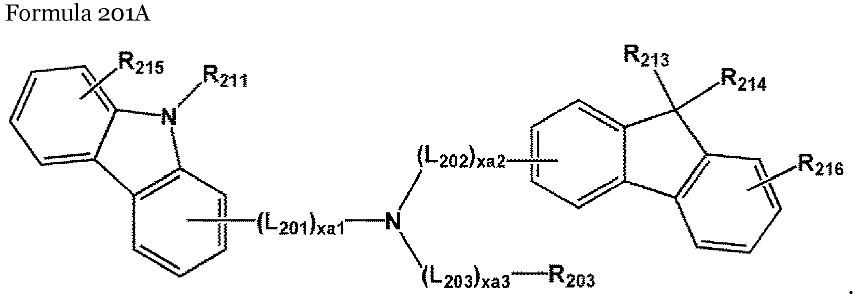

In some embodiments, the compound represented by Formula 201 may be represented by Formula 201A(1), but embodiments are not limited thereto:

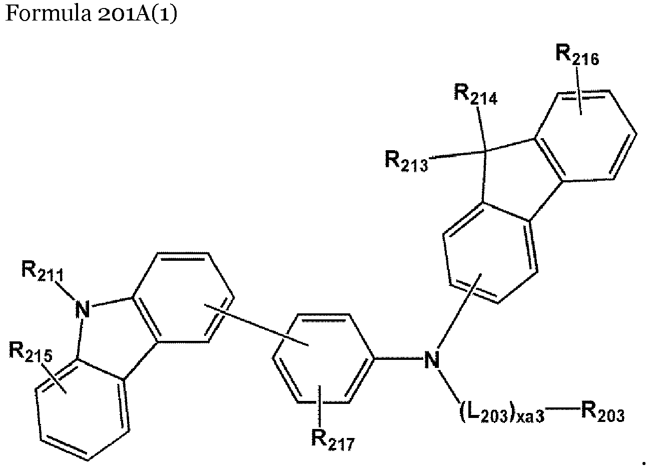

In some embodiments, the compound represented by Formula 201 may be represented by Formula 201A-1, but embodiments are not limited thereto:

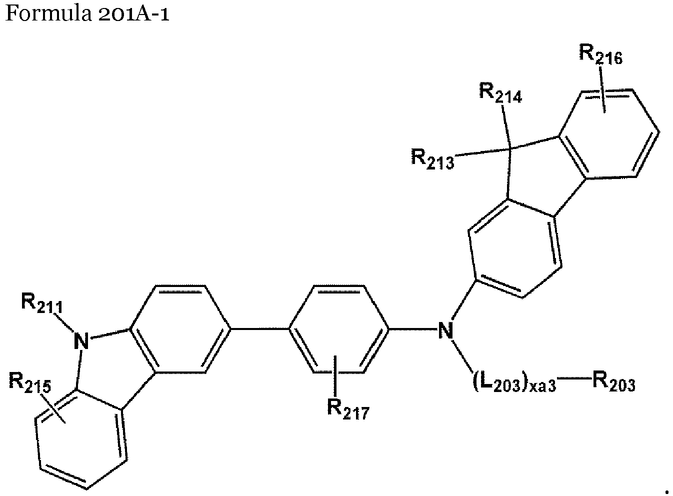

The compound represented by Formula 202 may be represented by Formula 202A:

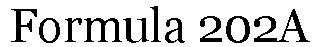

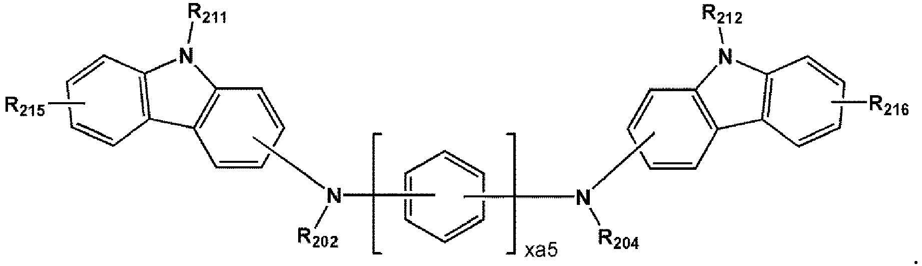

In some embodiments, the compound represented by Formula 202 may be represented by Formula 202A-1:

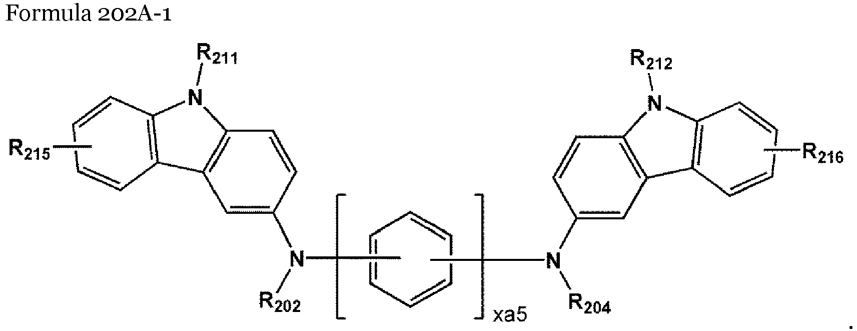

In Formulae 201A, 201A(1), 201A-1, 202A, and 202A-1,
descriptions for L₂₀₁ to L₂₀₃, xa1 to xa3, xa5, and R₂₀₂ to R₂₀₄ may each be understood by referring to (may be the same as) the descriptions for those provided herein,
descriptions for R₂₁₁ and R₂₁₂ may each be be understood by referring to (may be the same as) the descriptions for those for R₂₀₃ provided herein, and
R₂₁₃ to R₂₁₇ may each independently be selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

The hole transport region may comprise at least one compound selected from Compounds HT1 to HT39, but embodiments are not limited thereto:

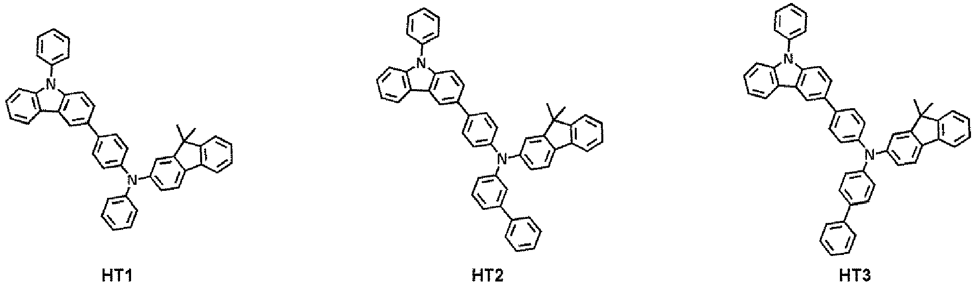

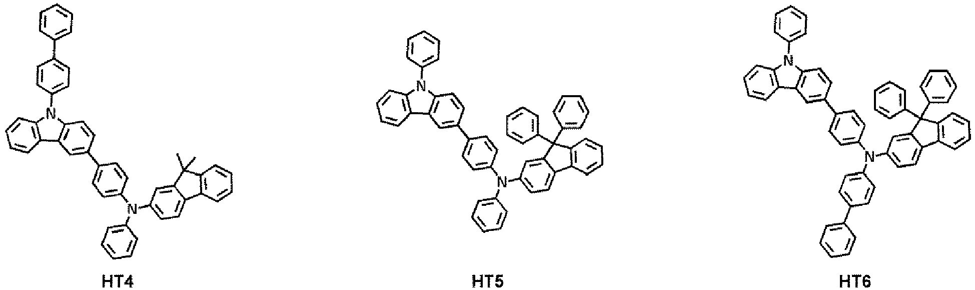

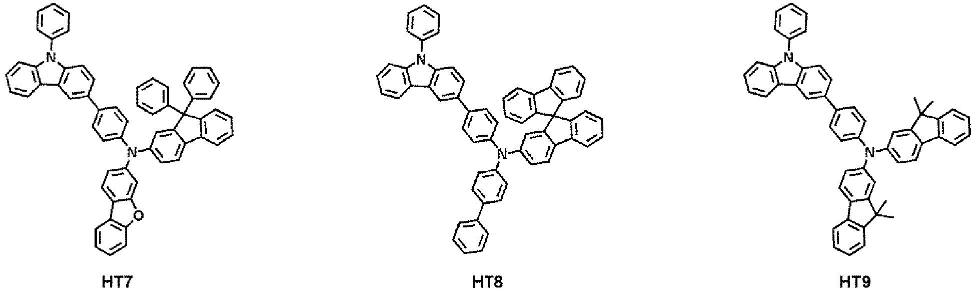

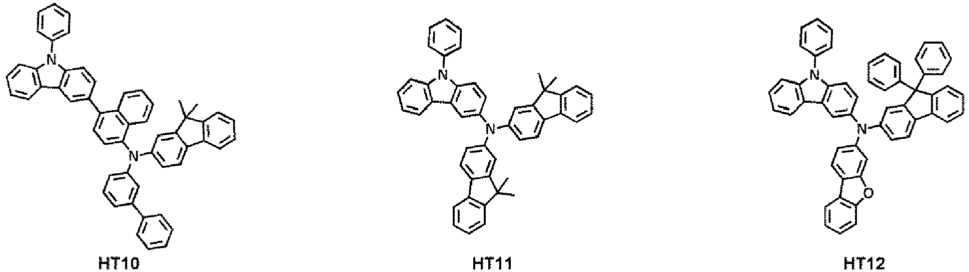

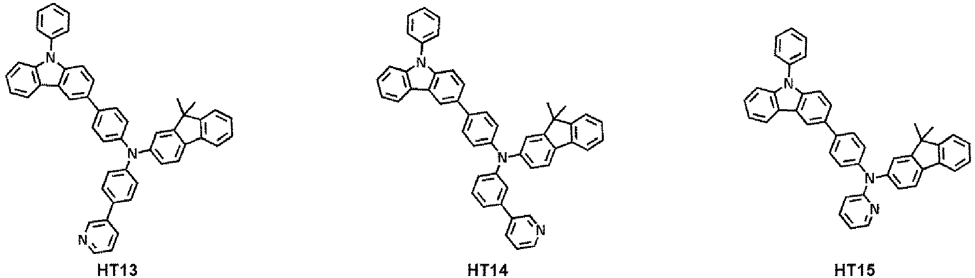

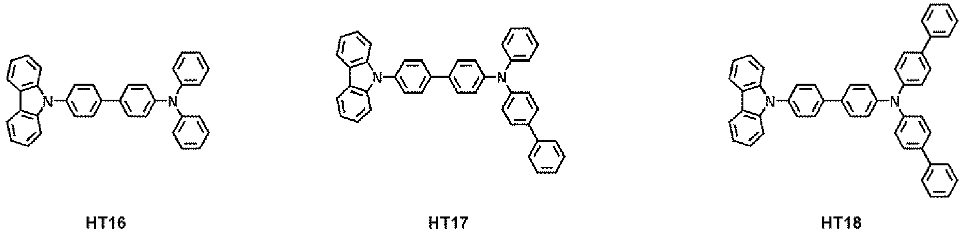

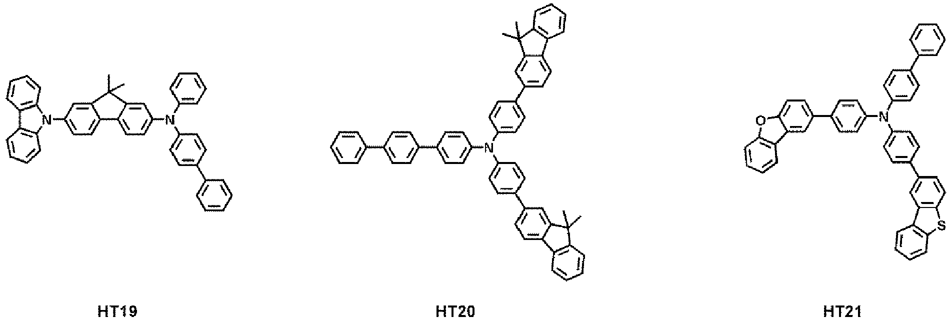

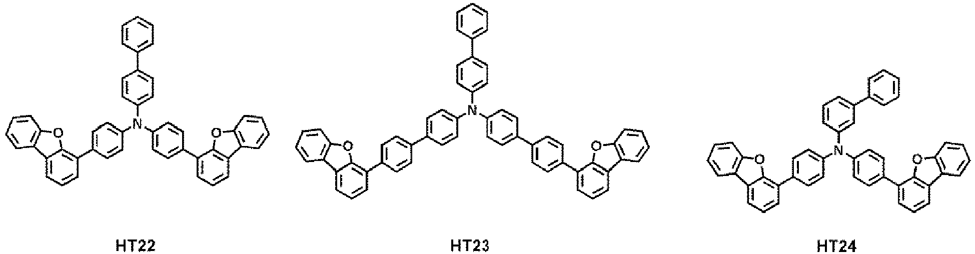

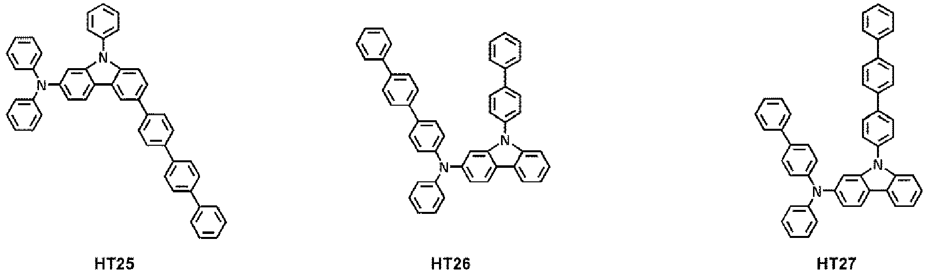

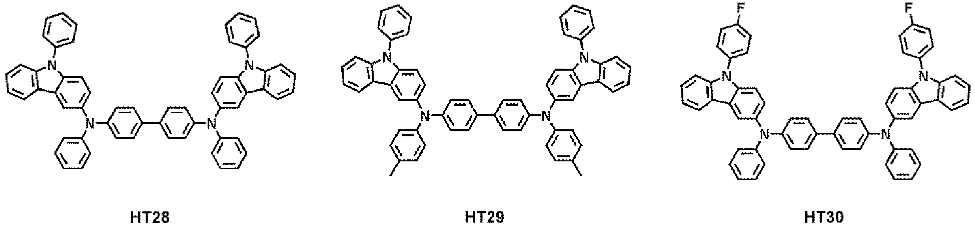

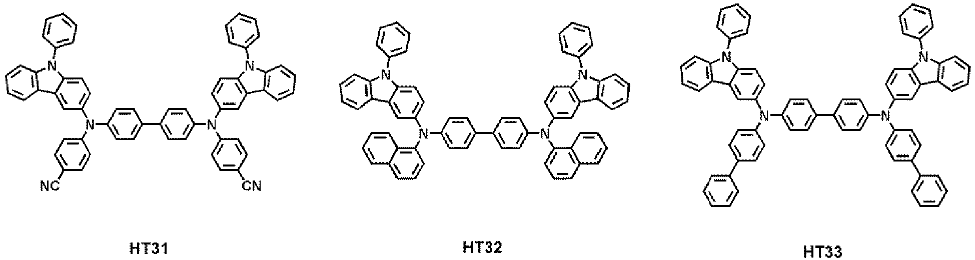

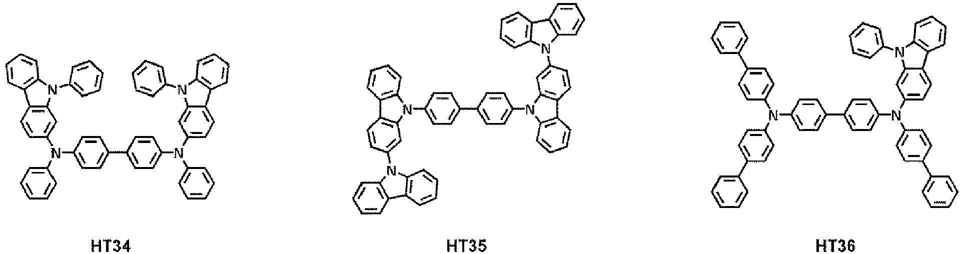

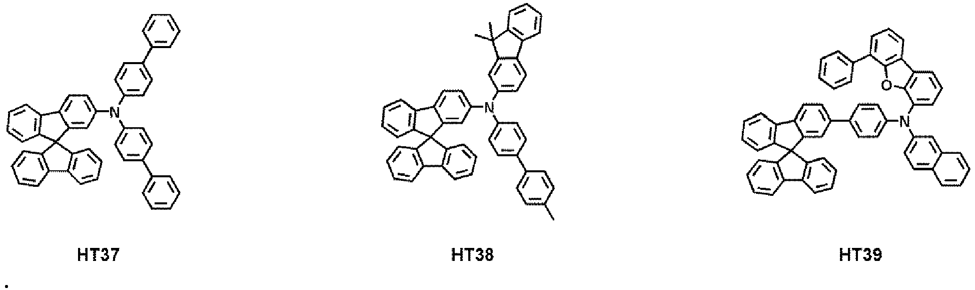

The thickness of the hole transport region may be in a range of 100 (Angstroms) Å to 10,000 Å, and in some embodiments, 100 Å to 1,000 Å. When the hole transport region comprises at least one of a hole injection layer and a hole transport layer, a thickness of the hole injection layer may be in a range of 100 Å to 9,000 Å, for example, 100 Å to 1,000 Å, and a thickness of the hole transport layer may be in a range of 50 Å to 2,000 Å, for example, 100 Å to 1,500 Å. When the thicknesses of the hole transport region, the hole injection layer, and the hole transport layer are within the above-described ranges, excellent hole transport characteristics may be obtained without a substantial increase in driving voltage.

The emission auxiliary layer may increase light emission efficiency by compensating for an optical resonance distance according to the wavelength of light emitted by an emission layer. The electron blocking layer may reduce or eliminate the flow of electrons from an electron transport region. The emission auxiliary layer and the electron blocking layer may comprise the aforementioned materials.

### p-dopant

The hole transport region may comprise a charge generating material as well as the aforementioned materials, to improve conductive properties of the hole transport region. The charge generating material may be substantially homogeneously or non-homogeneously dispersed in the hole transport region.

The charge generating material may comprise, for example, a p-dopant.

In some embodiments, the LUMO energy level of the p-dopant may be about - 3.5 eV or less.

Examples of the p-dopant may include at least one selected from a quinone derivative, a metal oxide, and a cyano group-containing compound, but embodiments are not limited thereto.

In some embodiments, examples of the p-dopant may include:
a quinone derivative, such as tetracyanoquinodimethane (TCNQ) or 2,3,5,6-tetrafluoro-7,7,8,8-tetracyanoquinodimethane (F4-TCNQ);
a metal oxide, such as tungsten oxide or molybdenum oxide;
1,4,5,8,9,12-hexaazatriphenylene-hexacarbonitrile (HAT-CN); and
a compound represented by Formula 221, but embodiments are not limited thereto:

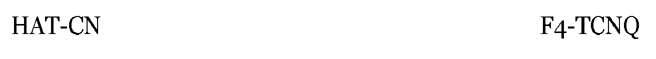

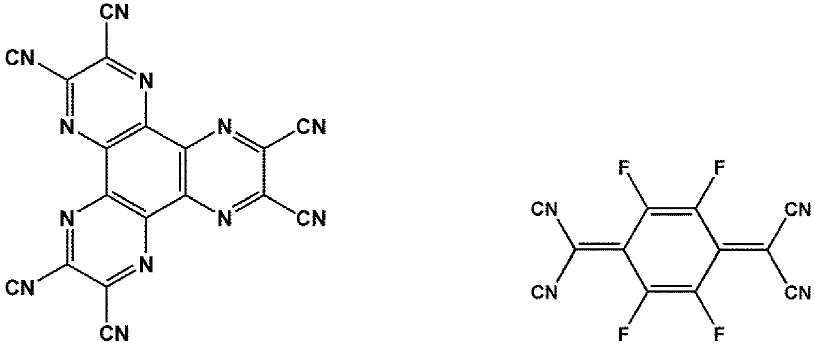

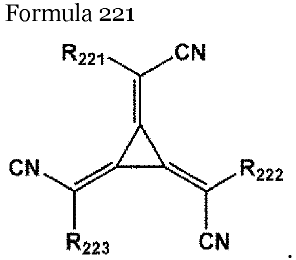

In Formula 221,
R₂₂₁ to R₂₂₃ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₁-C₆₀ heteroaryl group, a substituted or unsubstituted monovalent non-aromatic condensed C8 to C60 polycyclic group, and a substituted or unsubstituted monovalent non-aromatic condensed 8- to 60-membered heteropolycyclic group, provided that at least one of R₂₂₁ to R₂₂₃ may include at least one substituent selected from a cyano group, -F, -Cl, -Br, -I, a C₁-C₂₀ alkyl group substituted with -F, a C₁-C₂₀ alkyl group substituted with -Cl, a C₁-C₂₀ alkyl group substituted with -Br, and a C₁-C₂₀ alkyl group substituted with -I.

### Emission layer in organic layer 150

When the organic light-emitting device 10 is a full color organic light-emitting device, the emission layer may be patterned into a red emission layer, a green emission layer, or a blue emission layer, according to a sub-pixel. In some embodiments, the emission layer may have a stacked structure. The stacked structure may comprise two or more layers selected from a red emission layer, a green emission layer, and a blue emission layer. The two or more layers may be in direct contact with each other. Alternatively, the two or more layers may be separated from each other. In one or more embodiments, the emission layer may comprise two or more materials. The two or more materials may comprise a red light-emitting material, a green light-emitting material, or a blue light-emitting material. The two or more materials may be mixed with each other in a single layer. The two or more materials mixed with each other in the single layer may emit white light.

The emission layer may comprise a host and a dopant. The dopant may comprise at least one of a fluorescent dopant and a phosphorescent dopant.

The amount of the dopant in the emission layer may be, in general, in a range of about 0.01 parts to about 15 parts by weight based on 100 parts by weight of the host, but embodiments are not limited thereto.

The thickness of the emission layer may be in a range of about 100 Å to about 1,000 Å, and in some embodiments, about 200 Å to about 600 Å. When the thickness of the emission layer is within the above-described ranges, improved luminescence characteristics may be obtained without a substantial increase in driving voltage.

### Host in emission layer

The host may include a compound represented by Formula 301:

Formula 301 [Ar₃₀₁]_{xb11}-[(L₃₀₁)_{xb1}-R₃₀₁]_{xb21}

wherein, in Formula 301,
Ar₃₀₁ may be a substituted or unsubstituted C₅-C₆₀ carbocyclic group or a substituted or unsubstituted C₁-C₆₀ heterocyclic group,
xb11 may be 1, 2, or 3,
L₃₀₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed C8 to C60 polycyclic group, and a substituted or unsubstituted divalent non-aromatic condensed 8- to 60-membered heteropolycyclic group,
xb1 may be an integer from 0 to 5,
R₃₀₁ may be selected from deuterium, -F, -Cl, -Br, -I, a hydroxyl group, a cyano group, a nitro group, an amidino group, a hydrazino group, a hydrazono group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed C8 to C60 polycyclic group, a substituted or unsubstituted monovalent non-aromatic condensed 8- to 60-membered heteropolycyclic group, -Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), - S(=O)₂(Q₃₀₁), and -P(=O)(Q₃₀₁)(Q₃₀₂),
xb2i may be an integer from 1 to 5,
wherein Q₃₀₁ to Q₃₀₃ may each independently be selected from a C₁-C₁₀ alkyl group, a C₁-C₁₀ alkoxy group, a phenyl group, a biphenyl group, a terphenyl group, and a naphthyl group, but embodiments are not limited thereto.

In some embodiments, Ar₃₀₁ in Formula 301 may be selected from
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group; and
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), -N(Q₃₁)(Q₃₂), -B(Q₃₁)(Q₃₂), - C(=O)(Q₃₁), -S(=O)₂(Q₃₁), and -P(=O)(Q₃₁)(Q₃₂),
wherein Q₃₁ to Q₃₃ may each independently be selected from a C₁-C₁₀ alkyl group, a C₁-C₁₀ alkoxy group, a phenyl group, a biphenyl group, a terphenyl group, and a naphthyl group, but embodiments are not limited thereto.

When xb11 in Formula 301 is 2 or greater, at least two Ar₃₀₁ groups may be linked via a single bond.

In one or more embodiments, the compound represented by Formula 301 may be represented by Formula 301-1 or 301-2:

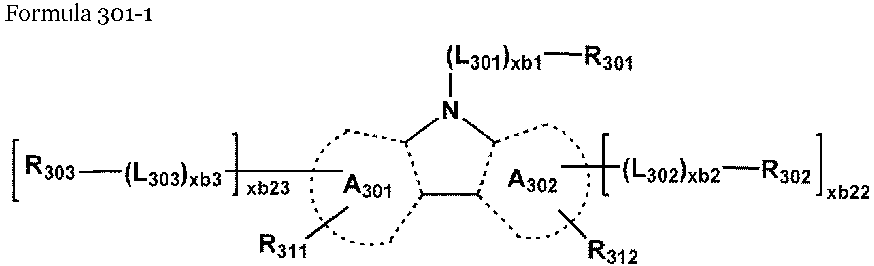

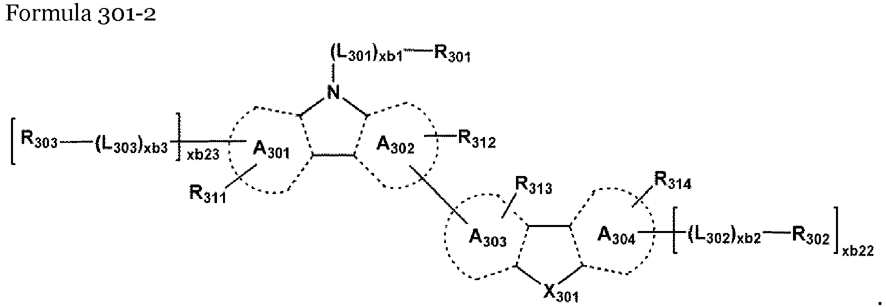

In Formulae 301-1 to 301-2,
A₃₀₁ to A₃₀₄ may each independently be selected from a benzene group, a naphthalene group, a phenanthrene group, a fluoranthene group, a triphenylene group, a pyrene group, a chrysene group, a pyridine group, a pyrimidine group, an indene group, a fluorene group, a spiro-bifluorene group, a benzofluorene group, a dibenzofluorene group, an indole group, a carbazole group, a benzocarbazole group, a dibenzocarbazole group, a furan group, a benzofuran group, a dibenzofuran group, a naphthofuran group, a benzonaphthofuran group, a dinaphthofuran group, a thiophene group, a benzothiophene group, a dibenzothiophene group, a naphthothiophene group, a benzonapthothiophene group, and a dinaphthothiophene group,
X₃₀₁ may be O, S, or N-[(L₃₀₄)_{xb4}-R₃₀₄],
R₃₁₁ to R₃₁₄ may each independently be selected from hydrogen, deuterium, -F, - Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), -N(Q₃₁)(Q₃₂), - B(Q₃₁)(Q₃₂), -C(=O)(Q₃₁), -S(=O)₂(Q₃₁), and -P(=O)(Q₃₁)(Q₃₂),
xb22 and xb23 may each independently be 0, 1, or 2,
descriptions for L₃₀₁, xbi, R₃₀₁, and Q₃₁ to Q₃₃ may each independently be understood by referring to (may be the same as) the descriptions for those provided herein,
descriptions for L₃₀₂ to L₃₀₄ may be understood by referring to (may be the same as) those for L₃₀₁ provided herein,
descriptions for xb2 to xb4 may each independently be understood by referring to (may be the same as) those for xb1 provided herein, and
descriptions for R₃₀₂ to R₃₀₄ may each independently be understood by referring to (may be the same as) those for R₃₀₁ provided herein.

In some embodiments, in Formulae 301, 301-1, and 301-2, L₃₀₁ to L₃₀₄ may each independently be selected from
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), - N(Q₃₁)(Q₃₂), -B(Q₃₁)(Q₃₂), -C(=O)(Q₃₁), -S(=O)₂(Q₃₁), and -P(=O)(Q₃₁)(Q₃₂),
wherein descriptions for Q₃₁ to Q₃₃ may each independently be understood by referring to (may be the same as) the descriptions for those provided herein.

In some embodiments, in Formulae 301, 301-1, and 301-2, R₃₀₁ to R₃₀₄ may each independently be selected from
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂), -B(Q₃₁)(Q₃₂), -C(=O)(Q₃₁), - S(=O)₂(Q₃₁), and -P(=O)(Q₃₁)(Q₃₂),
wherein descriptions for Q₃₁ to Q₃₃ may each independently be understood by referring to (may be the same as) the descriptions for those provided herein.

In some embodiments, the host may include an alkaline earth metal complex. For example, the host may include a beryllium (Be) complex, e.g., Compound H55, a magnesium (Mg) complex, or a zinc (Zn) complex.

The host may include at least one selected from 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-carbazolylbenzene (mCP), 1,3,5-tri(carbazol-9-yl)benzene (TCP), and Compounds Hi to H55, but embodiments are not limited thereto:

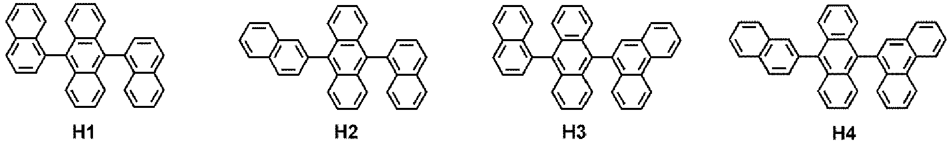

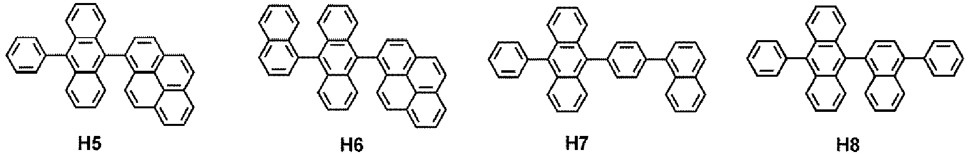

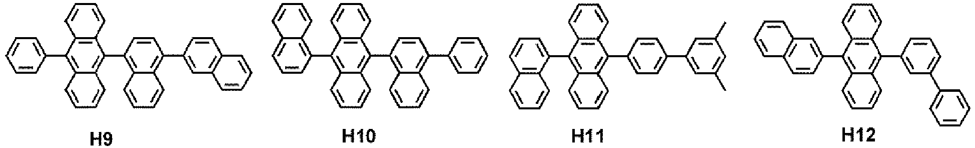

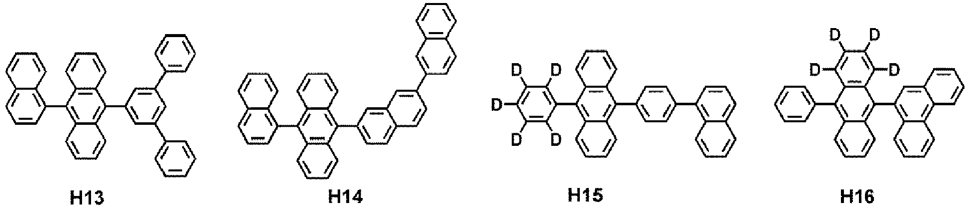

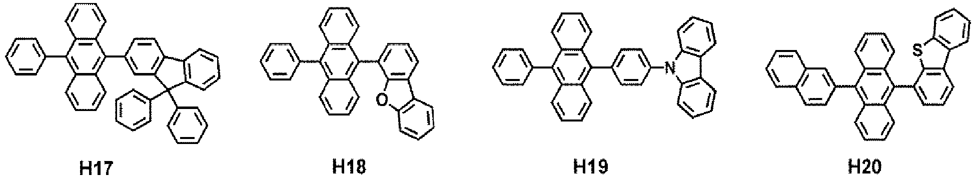

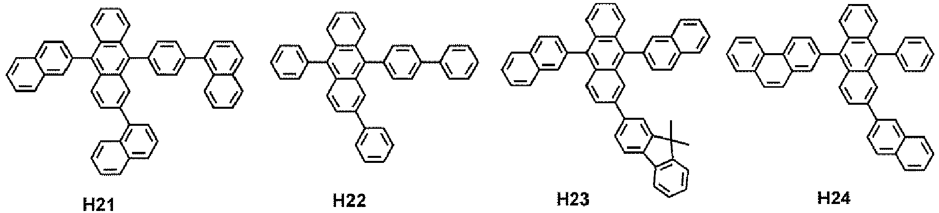

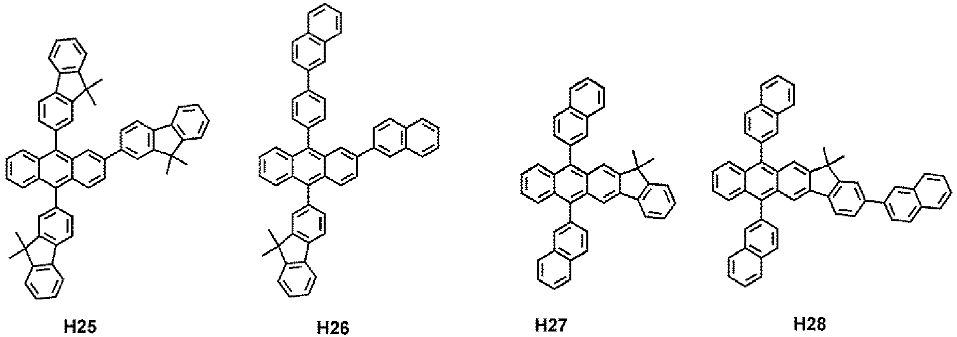

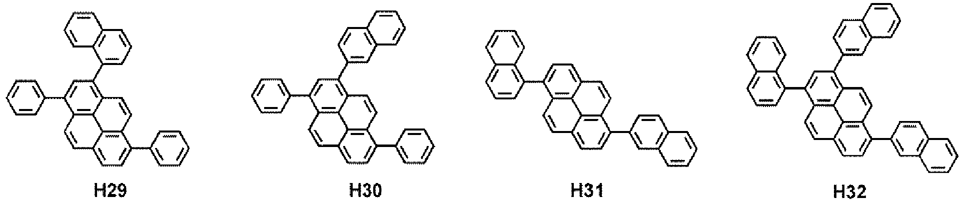

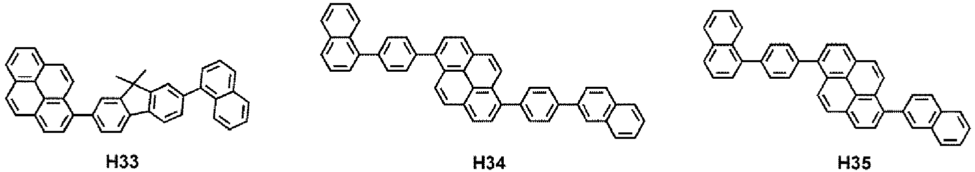

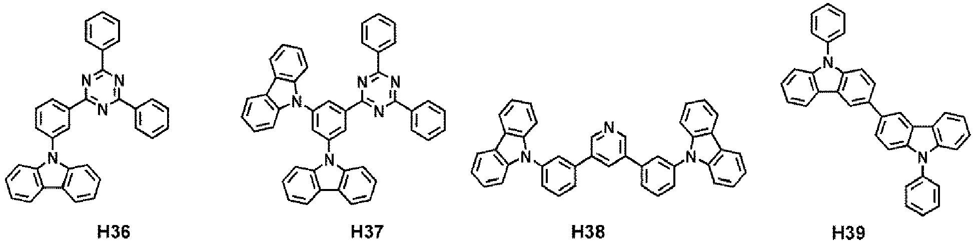

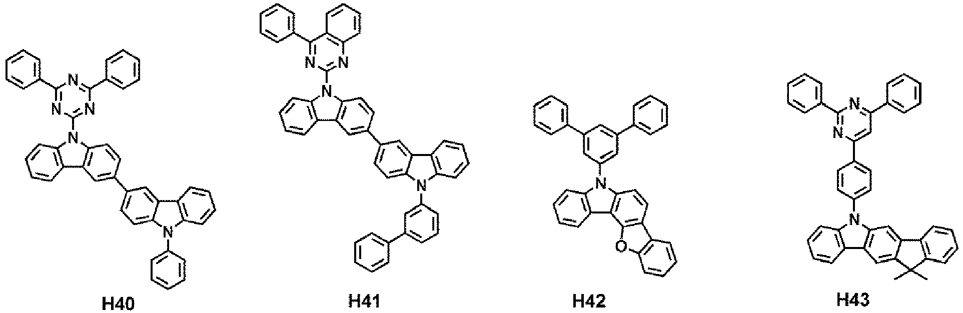

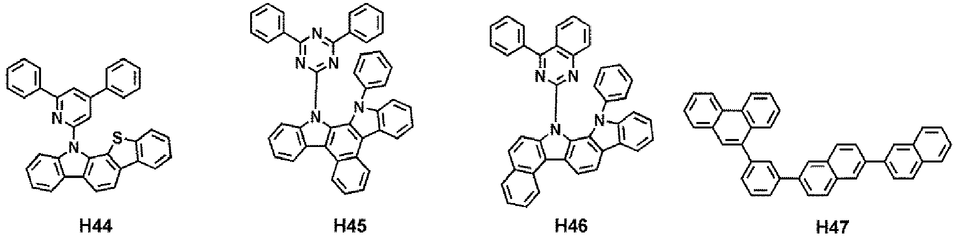

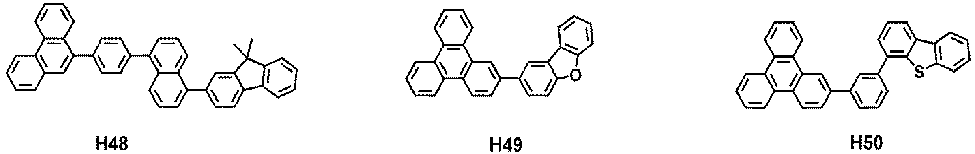

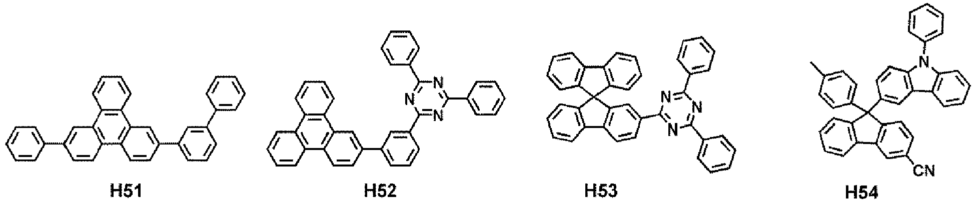

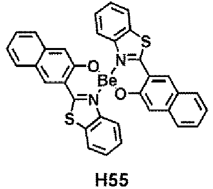

### Phosphorescent dopant comprised in emission layer of organic layer 150

Examples of the phosphorescent dopant may include an organic metal complex represented by Formula 401:

Formula 401 M(L₄₀₁)_{xc1}(L₄₀₂)_{xc2}

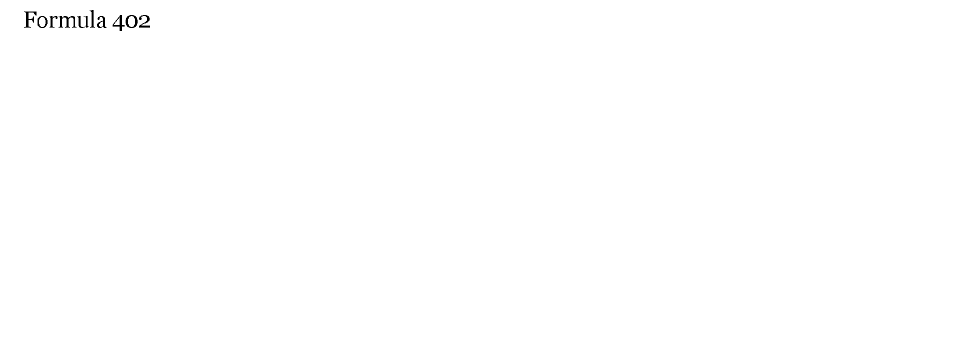

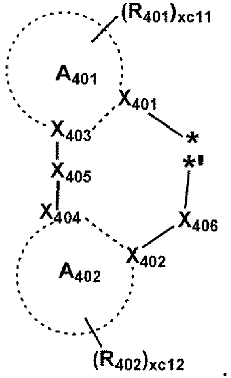

In Formulae 401 and 402,
M may be selected from iridium (Ir), platinum (Pt), palladium (Pd), osmium (Os), titanium (Ti), zirconium (Zr), hafnium (Hf), europium (Eu), terbium (Tb), rhodium (Rh), and thulium (Tm),
L₄₀₁ may be selected from ligands represented by Formula 402, xc1 may be an integer selected from 1, 2, and 3; when xc1 is two or greater, at least two L₄₀₁ groups may be identical to or different from each other,
L₄₀₂ may be an organic ligand, xc2 may be an integer from 0 to 4; when xc2 is 2 or greater, at least two L₄₀₂ groups may be identical to or different from each other,
X₄₀₁ to X₄₀₄ may each independently be nitrogen or carbon,
X₄₀₁ and X₄₀₃ may be linked via a single bond or a double bond, X₄₀₂ and X₄₀₄ may be linked via a single bond or a double bond,
A₄₀₁ and A₄₀₂ may each independently be a C₅-C₆₀ carbocyclic group or a C₁-C₆₀ heterocyclic group,
X₄₀₅ may be a single bond, ^{∗}-O-^{∗}', ^{∗}-S-^{∗'}, ^{∗}-C(=O)-^{∗}', ^{∗}-N(Q₄₁₁)-^{∗}', ^{∗}-C(Q₄₁₁)(Q₄₁₂)-^{∗}', ^{∗}-C(Q₄₁₁)=C(Q₄₁₂)-^{∗}', ^{∗}-C(Q₄₁₁)=^{∗}', or ^{∗}=C=^{∗}', wherein Q₄₁₁ and Q₄₁₂ may each independently be hydrogen, deuterium, a C₁-C₂₀ alkyl group, a C₁-C₂₀ alkoxy group, a phenyl group, a biphenyl group, a terphenyl group, or a naphthyl group,
X₄₀₆ may be a single bond, O, or S,
R₄₀₁ and R₄₀₂ may each independently be selected from hydrogen, deuterium, - F, -Cl, -Br, -I, a hydroxyl group, a cyano group, a nitro group, an amidino group, a hydrazino group, a hydrazono group, a substituted or unsubstituted C₁-C₂₀ alkyl group, a substituted or unsubstituted C₁-C₂₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed C8 to C60 polycyclic group and a substituted or unsubstituted monovalent non-aromatic condensed 8- to 60-membered heteropolycyclic group, -Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), - S(=O)₂(Q₄₀₁), and -P(=O)(Q₄₀₁)(Q₄₀₂), wherein Q₄₀₁ to Q₄₀₃ may each independently be selected from a C₁-C₁₀ alkyl group, a C₁-C₁₀ alkoxy group, a C₆-C₂₀ aryl group, and a C₁-C₂₀ heteroaryl group,
xc11 and xc12 may each independently be an integer from 0 to 10, and
^{∗} and ^{∗}' in Formula 402 each indicate a binding site to M in Formula 401.
In one embodiment, A₄₀₁ and A₄₀₂ in Formula 402 may each independently be selected from a benzene group, a naphthalene group, a fluorene group, a spiro-bifluorene group, an indene group, a pyrrole group, a thiophene group, a furan group, an imidazole group, a pyrazole group, a thiazole group, an isothiazole group, an oxazole group, an isoxazole group, a pyridine group, a pyrazine group, a pyrimidine group, a pyridazine group, a quinoline group, an isoquinoline group, a benzoquinoline group, a quinoxaline group, a quinazoline group, a carbazole group, a benzimidazole group, a benzofuran group, a benzothiophene group, an isobenzothiophene group, a benzoxazole group, an isobenzoxazole group, a triazole group, a tetrazole group, an oxadiazole group, a triazine group, a dibenzofuran group, and a dibenzothiophene group.

In one or more embodiments, in Formula 402, i) X₄₀₁ may be nitrogen, and X₄₀₂ may be carbon, or ii) X₄₀₁ and X₄₀₂ may each be nitrogen.

In one or more embodiments, in Formula 402, R₄₀₁ and R₄₀₂ may each independently be selected from
hydrogen, deuterium, -F, -Cl, -Br, -I, a hydroxyl group, a cyano group, a nitro group, an amidino group, a hydrazino group, a hydrazono group, a C₁-C₂₀ alkyl group, and a C₁-C₂₀ alkoxy group;
a C₁-C₂₀ alkyl group and a C₁-C₂₀ alkoxy group, each substituted with at least one selected from deuterium, -F, -Cl, -Br, -I, a hydroxyl group, a cyano group, a nitro group, an amidino group, a hydrazino group, a hydrazono group, a phenyl group, a naphthyl group, a cyclopentyl group, a cyclohexyl group, an adamantyl group, a norbornanyl group, and a norbornenyl group;
a cyclopentyl group, a cyclohexyl group, an adamantyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group;
a cyclopentyl group, a cyclohexyl group, an adamantyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, an adamantanyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group; and
-Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), -S(=O)₂(Q₄₀₁) and -P(=O)(Q₄₀₁)(Q₄₀₂),
wherein Q₄₀₁ to Q₄₀₃ may each independently be selected from a C₁-C₁₀ alkyl group, a C₁-C₁₀ alkoxy group, a phenyl group, a biphenyl group, and a naphthyl group, but embodiments are not limited thereto.

In one or more embodiments, when xc1 in Formula 401 is 2 or greater, two A₄₀₁(s) in at least two L₄₀₁(s) may optionally be linked to each other via X₄₀₇, which is a linking group; or two A₄₀₂(s) in at least two L₄₀₁(s) may optionally be linked to each other via X₄₀₈, which is a linking group (see Compounds PD1 to PD4 and PD7). X₄₀₇ and X₄₀₈ may each independently be selected from a single bond, ^{∗}-O-^{∗}', ^{∗}-S-^{∗}', ^{∗}-C(=O)-^{∗}', ^{∗}-N(Q₄₁₃)-^{∗}', ^{∗}-C(Q₄₁₃)(Q₄₁₄)-^{∗}', and ^{∗}-C(Q₄₁₃)=C(Q₄₁₄)-^{∗}', wherein Q₄₁₃ and Q₄₁₄ may each independently be hydrogen, deuterium, a C₁-C₂₀ alkyl group, a C₁-C₂₀ alkoxy group, a phenyl group, a biphenyl group, a terphenyl group, or a naphthyl group, but embodiments are not limited thereto.

L₄₀₂ in Formula 401 may be any suitable monovalent, divalent, or trivalent organic ligand. In some embodiments, L₄₀₂ may be selected from halogen, diketone (e.g., acetylacetonate), a carboxylic acid (e.g., picolinate), -C(=O), isonitrile, -CN, and phosphorus (e.g., phosphine or phosphite), but embodiments are not limited thereto.

In some embodiments, examples of the phosphorescent dopant may include, for example, at least one selected from Compounds PD1 to PD25, but embodiments are not limited thereto:

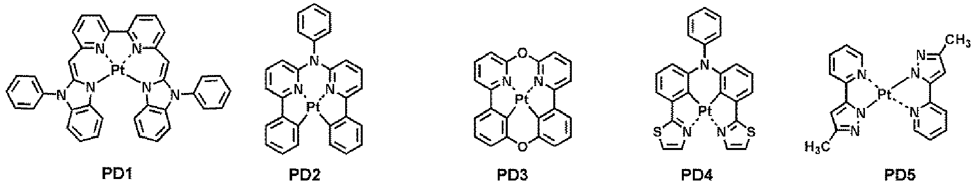

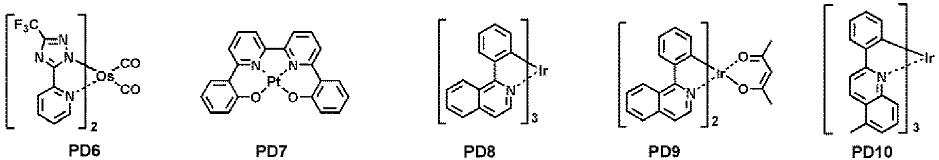

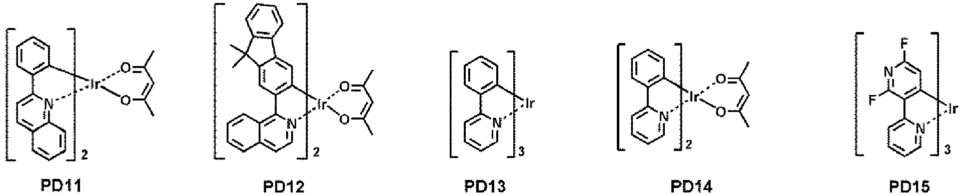

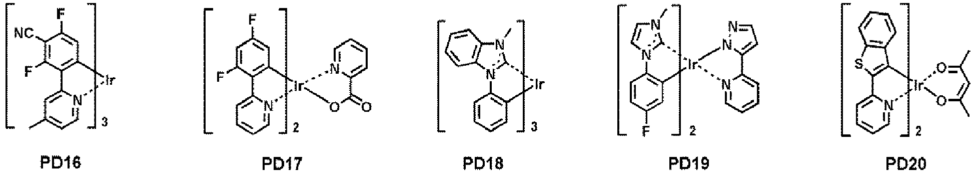

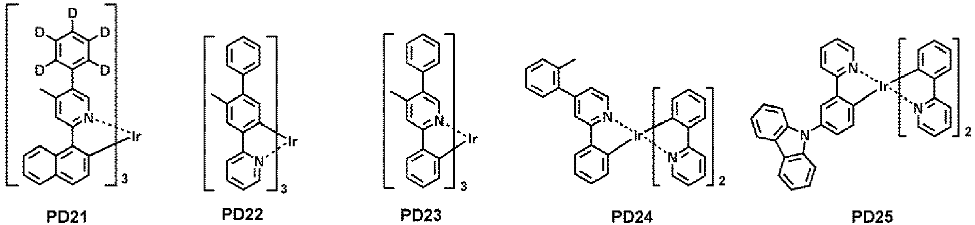

### Fluorescent dopant in emission layer

Examples of the fluorescent dopant may include an arylamine compound or a styrylamine compound.

The host may include a compound represented by Formula 501:

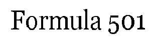

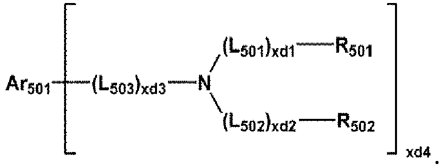

In Formula 501,
Ar₅₀₁ may be a substituted or unsubstituted C₅-C₆₀ carbocyclic group or a substituted or unsubstituted C₁-C₆₀ heterocyclic group,
L₅₀₁ to L₅₀₃ may be each independently selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed C8 to C60 polycyclic group, and a substituted or unsubstituted divalent non-aromatic condensed 8-to 60-membered heteropolycyclic group,
xd1 to xd3 may each independently be an integer from 0 to 3,
R₅₀₁ and R₅₀₂ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed C8 to C60 polycyclic group, and a substituted or unsubstituted monovalent non-aromatic condensed 8- to 60-membered heteropolycyclic group, and
xd4 may be an integer from 1 to 6.

In some embodiments, Ar₅₀₁ in Formula 501 may be selected from
a naphthalene group, a heptalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, and an indenophenanthrene group; and
a naphthalene group, a heptalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, and an indenophenanthrene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, and a naphthyl group.

In one or more embodiments, in Formula 501, L₅₀₁ to L₅₀₃ may each independently be selected from
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

In one or more embodiments, in Formula 501, R₅₀₁ and R₅₀₂ may each independently be selected from
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, and -Si(Q₃₁)(Q₃₂)(Q₃₃),
wherein Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

In one or more embodiments, xd4 in Formula 501 may be 2, but embodiments are not limited thereto.

In some embodiments, the fluorescent dopant may be selected from Compounds FD₁ to FD₂₂:

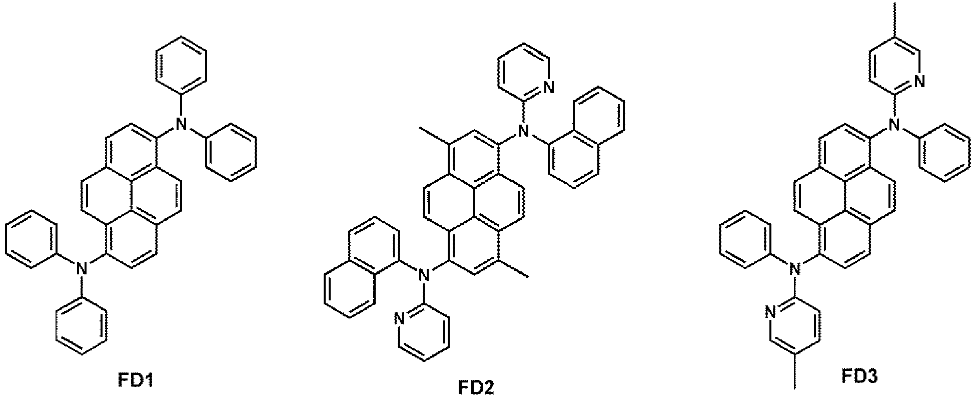

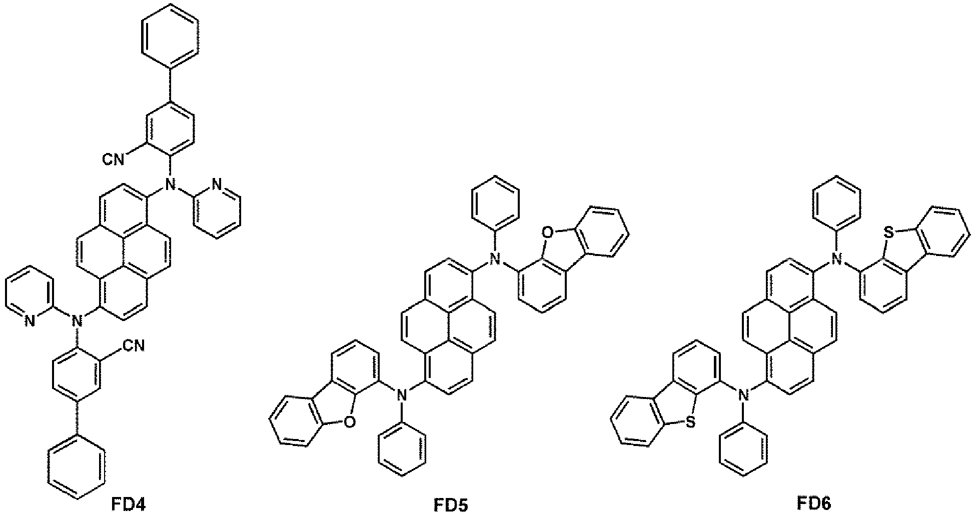

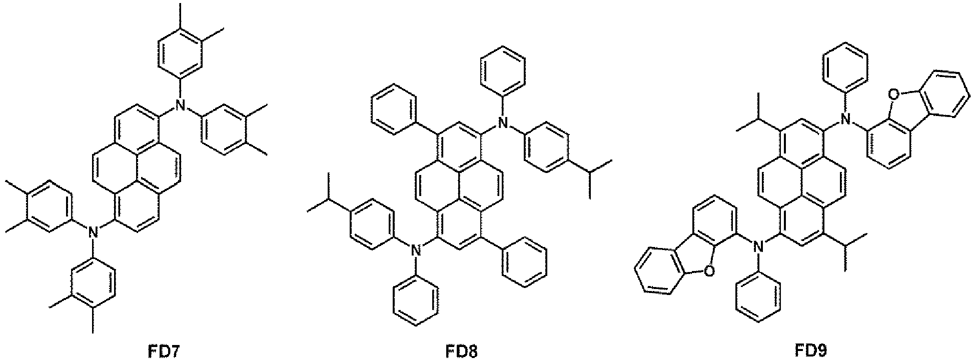

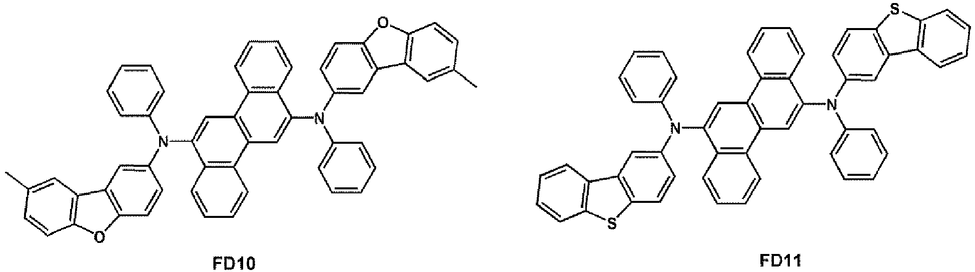

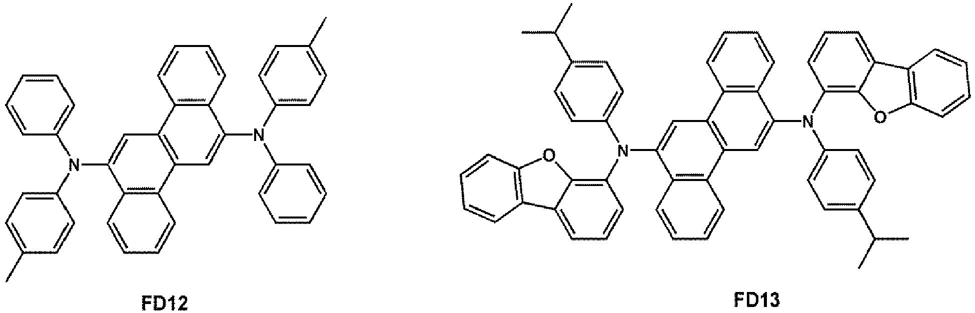

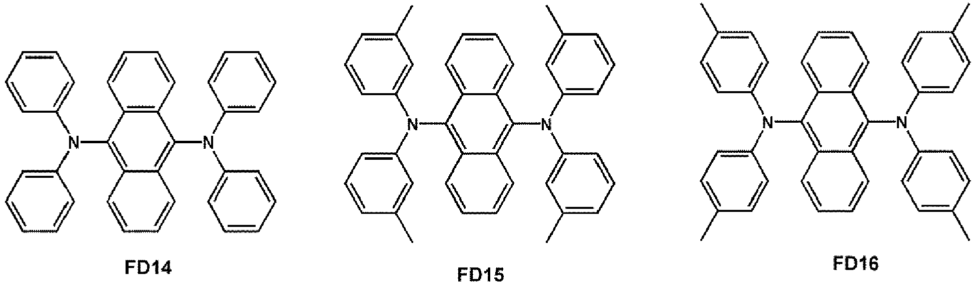

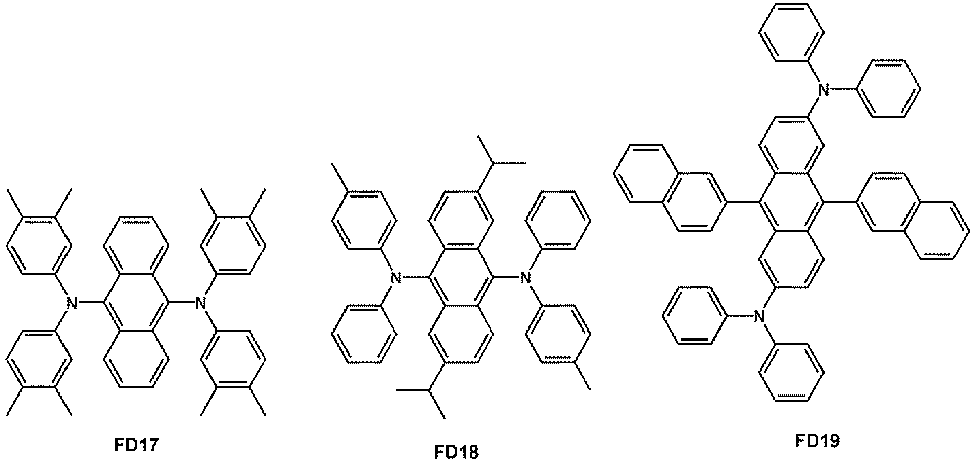

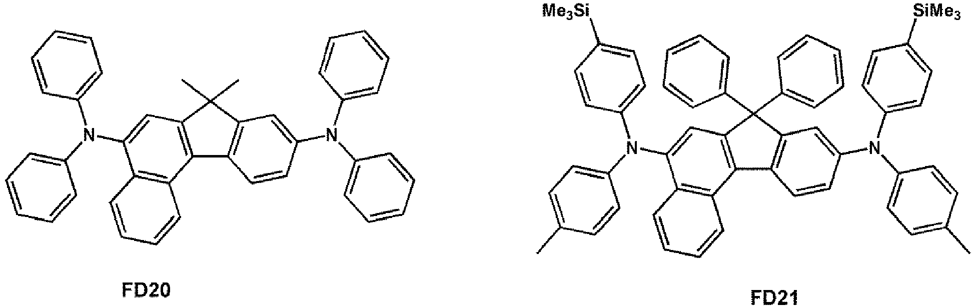

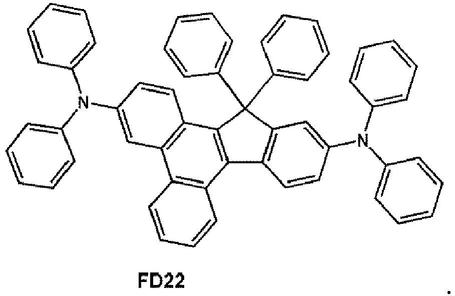

In some embodiments, the fluorescent dopant may be selected from the following compounds, but embodiments are not limited thereto:

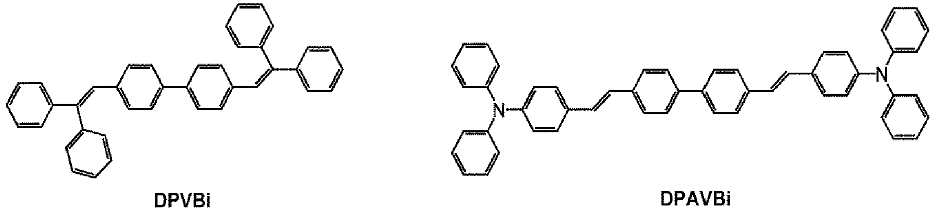

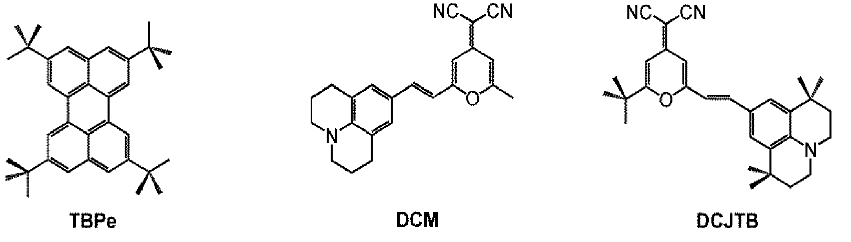

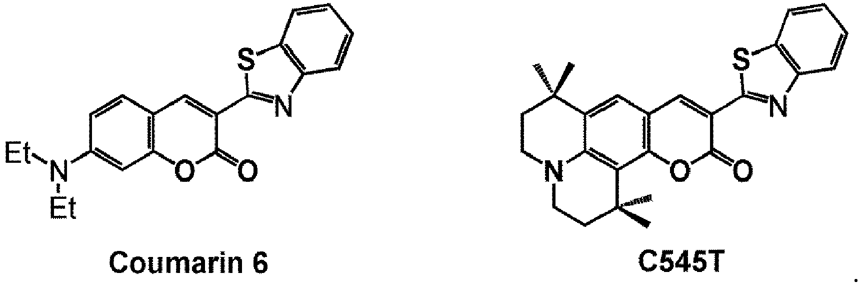

### Electron transport region in organic layer 150

The electron transport region may have i) a single-layered structure including a single layer comprising a single material, ii) a single-layered structure including a single layer comprising a plurality of different materials, or iii) a multi-layered structure having a plurality of layers comprising a plurality of different materials.

The electron transport region may comprise at least one selected from a buffer layer, a hole blocking layer, an electron control layer, an electron transport layer, and an electron injection layer, but embodiments are not limited thereto.

In some embodiments, the electron transport region may have a structure of an electron transport layer/electron injection layer, a structure of a hole blocking layer/electron transport layer/electron injection layer, a structure of an electron control layer/electron transport layer/electron injection layer, or a structure of a buffer layer/electron transport layer/electron injection layer, wherein layers of each structure are sequentially stacked on the emission layer in each stated order, but embodiments are not limited thereto.

The electron transport region, e.g., a buffer layer, a hole blocking layer, an electron control layer, or an electron transport layer in the electron transport region, may comprise a metal-free compound. The metal-free compound may include at least one π electron-depleted nitrogen-containing ring.

The term "π electron-depleted nitrogen-containing ring" as used herein refers to a C₁-C₆₀ heterocyclic group having at least one ^{∗}-N=^{∗}' moiety as a ring-forming moiety.

For example, the "π electron-depleted nitrogen-containing ring" may be i) a 5-membered to 7-membered heteromonocyclic group having at least one ^{∗}-N=^{∗}' moiety, ii) a heteropolycyclic group in which two or more 5-membered to 7-membered heteromonocyclic groups, each having at least one ^{∗}-N=^{∗}' moiety, are condensed, or iii) a heteropolycyclic group in which at least one 5-membered to 7-membered heteromonocyclic group, having at least one ^{∗}-N=^{∗}' moiety, is condensed with at least one C₅-C₆₀ carbocyclic group.

Examples of the π electron-depleted nitrogen-containing ring may include an imidazole, a pyrazole, a thiazole, an isothiazole, an oxazole, an isoxazole, a pyridine, a pyrazine, a pyrimidine, a pyridazine, an indazole, a purine, a quinoline, an isoquinoline, a benzoquinoline, a phthalazine, a naphthyridine, a quinoxaline, a quinazoline, a cinnoline, a phenanthridine, an acridine, a phenanthroline, a phenazine, a benzimidazole, an iso-benzothiazole, a benzoxazole, an isobenzoxazole, a triazole, a tetrazole, an oxadiazole, a triazine, a thiadiazole, an imidazopyridine, an imidazopyrimidine, and an azacarbazole, but embodiments are not limited thereto.

In some embodiments, the electron transport region may comprise a compound represented by Formula 601:

Formula 601 [Ar₆₀₁]ₓₑ₁₁-[(L₆₀₁)ₓₑ₁-R₆₀₁]ₓₑ₂₁

wherein, in Formula 601,
Ar₆₀₁ may be a substituted or unsubstituted C₅-C₆₀ carbocyclic group or a substituted or unsubstituted C₁-C₆₀ heterocyclic group,
xe11 may be 1, 2, or 3,
L₆₀₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed C8 to C60 polycyclic group, and a substituted or unsubstituted divalent non-aromatic condensed 8- to 60-membered heteropolycyclic group,
xe1 may be an integer from 0 to 5,
R₆₀₁ may be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed C8 to C60 polycyclic group, a substituted or unsubstituted monovalent non-aromatic condensed 8- to 60-membered heteropolycyclic group, -Si(Q₆₀₁)(Q₆₀₂)(Q₆₀₃), -C(=O)(Q₆₀₁), -S(=O)₂(Q₆₀₁), and - P(=O)(Q₆₀₁)(Q₆₀₂),
wherein Q₆₀₁ to Q₆₀₃ may each independently be a C₁-C₁₀ alkyl group, a C₁-C₁₀ alkoxy group, a phenyl group, a biphenyl group, a terphenyl group, or a naphthyl group, and
xe2i may be an integer from 1 to 5.

In one embodiment, at least one of Ar₆₀₁ groups in the number of xe11 and R₆₀₁ groups in the number of xe2i may comprise the π electron-depleted nitrogen-containing ring.

In some embodiments, Ar₆₀₁ in Formula 601 may be selected from
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an iso-benzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group; and
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an iso-benzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), - S(=O)₂(Q₃₁), and -P(=O)(Q₃₁)(Q₃₂),
wherein Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

When xe11 in Formula 601 is 2 or greater, at least two Ar₆₀₁ groups may be linked via a single bond.

In one or more embodiments, Ar₆₀₁ in Formula 601 may be an anthracene group.

In some embodiments, the compound represented by Formula 601 may be represented by Formula 601-1:

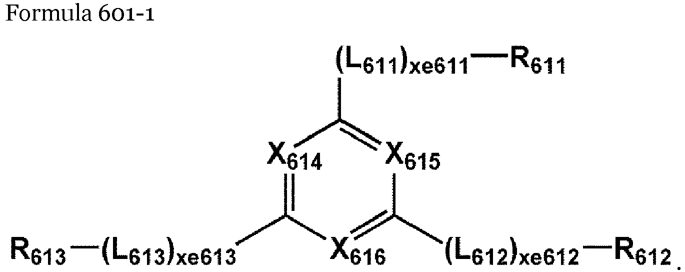

In Formula 601-1,
X₆₁₄ may be N or C(R₆₁₄), X₆₁₅ may be N or C(R₆₁₅), X₆₁₆ may be N or C(R₆₁₆), and at least one selected from X₆₁₄ to X₆₁₆ may be N,
descriptions for L₆₁₁ to L₆₁₃ may each independently be understood by referring to (may be the same as) the descriptions for those for L₆₀₁ provided herein,
descriptions for xe611 to xe₆₁₃ may each independently be understood by referring to (may be the same as) the descriptions for those for xe1 provided herein,
descriptions for R₆₁₁ to R₆₁₃ may each independently be understood by referring to (may be the same as) the descriptions for those for R₆₀₁ provided herein, and
R₆₁₄ to R₆₁₆ may each independently be selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, and a naphthyl group.

In one embodiment, in Formulae 601 and 601-1, L₆₀₁ and L₆₁₁ to L₆₁₃ may each independently be selected from
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, but embodiments are not limited thereto.

In one or more embodiments, in Formulae 601 and 601-1, xe1 and xe611 to xe613, may each independently be 0, 1, or 2.

In one or more embodiments, in Formulae 601 and 601-1, R₆₀₁ and R₆₁₁ to R₆₁₃ may each independently be selected from
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group;
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and
-S(=O)₂(Q₆₀₁) and -P(=O)(Q₆₀₁)(Q₆₀₂),
wherein descriptions for Q₆₀₁ and Q₆₀₂ may each independently be understood by referring to (may be the same as) the descriptions for those provided herein.

The electron transport region may comprise at least one compound selected from Compounds ET1 to ET36, but embodiments are not limited thereto:

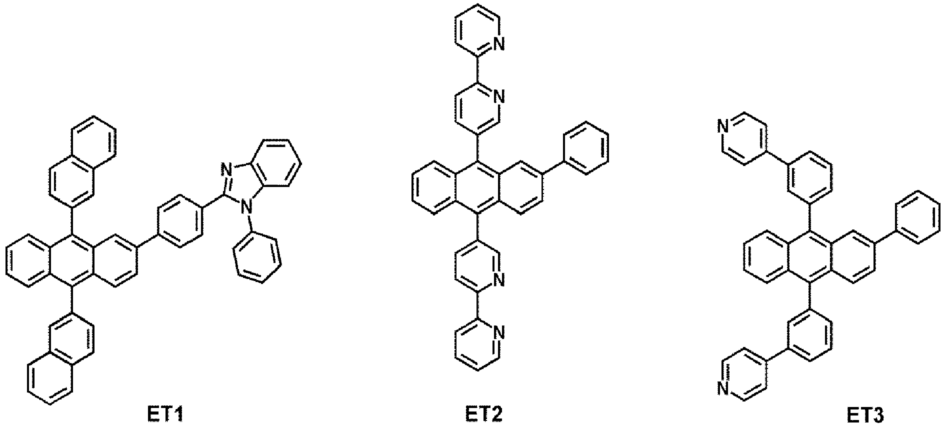

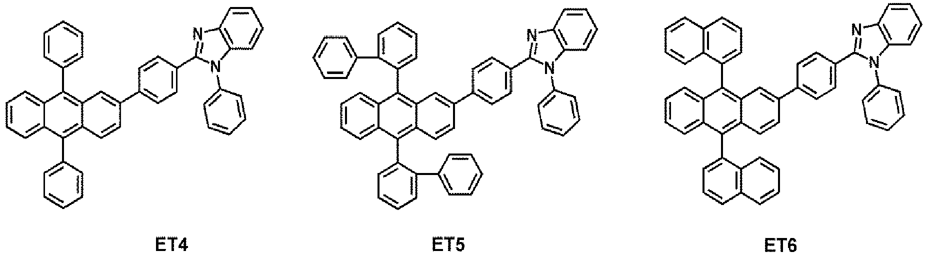

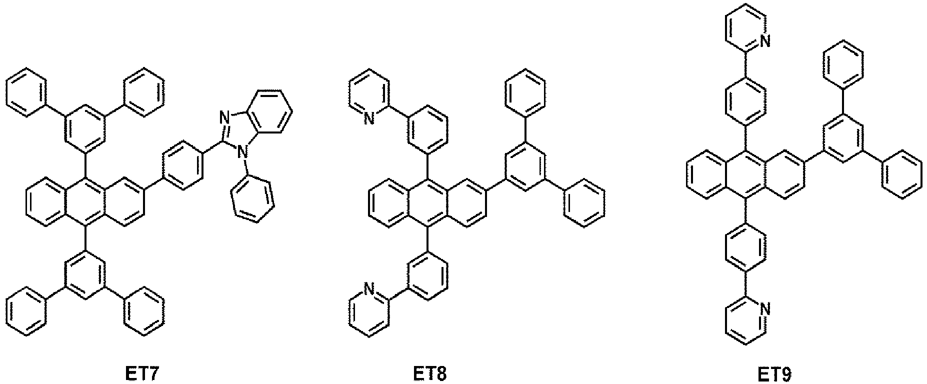

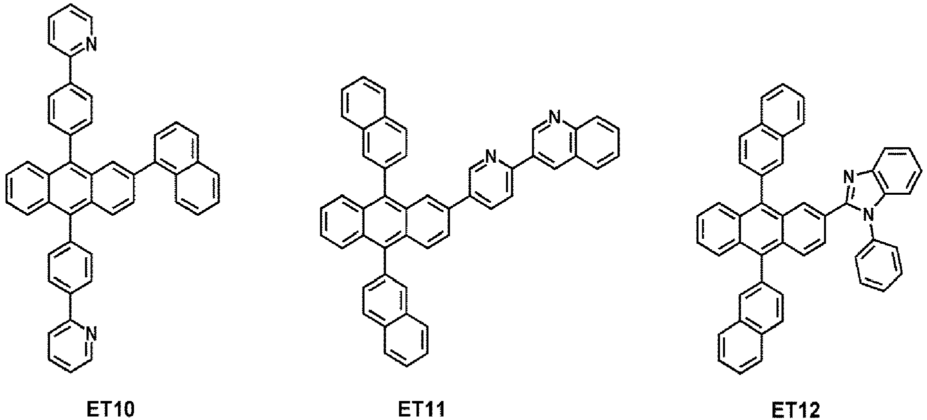

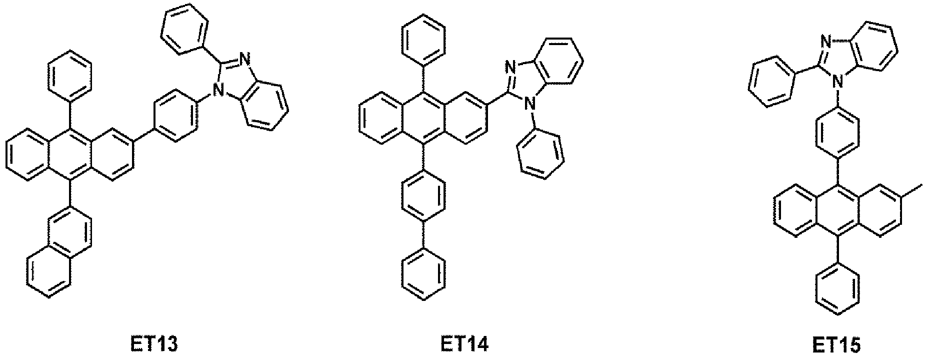

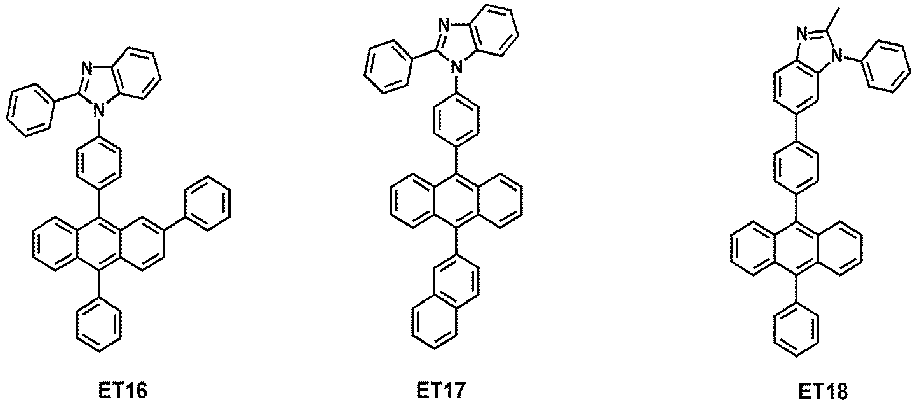

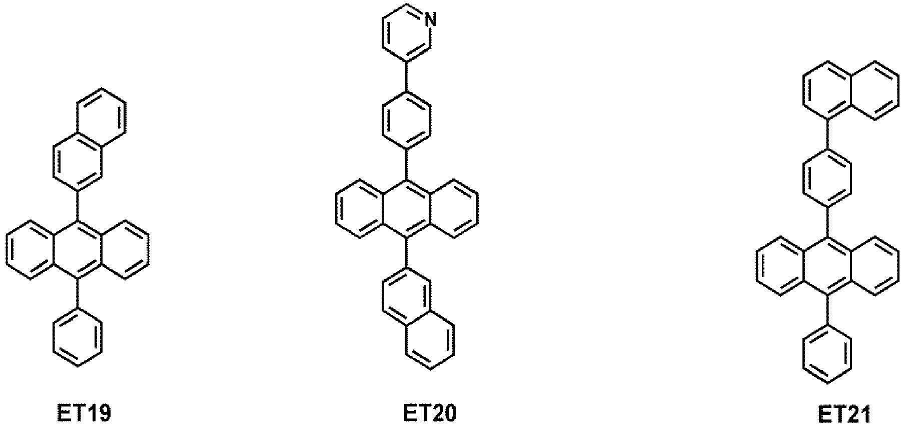

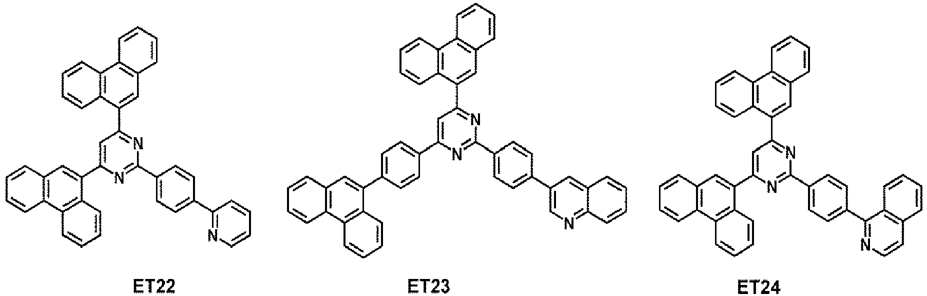

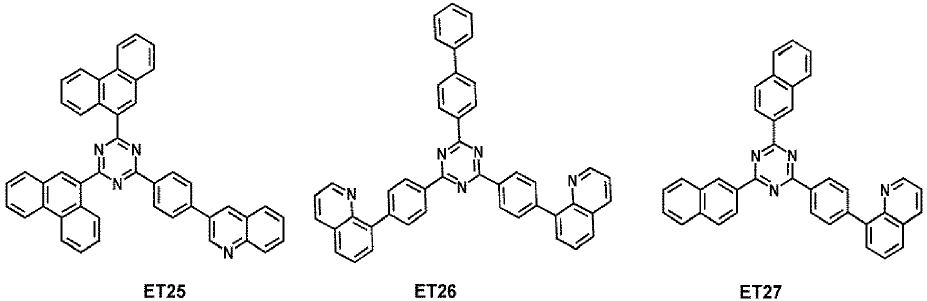

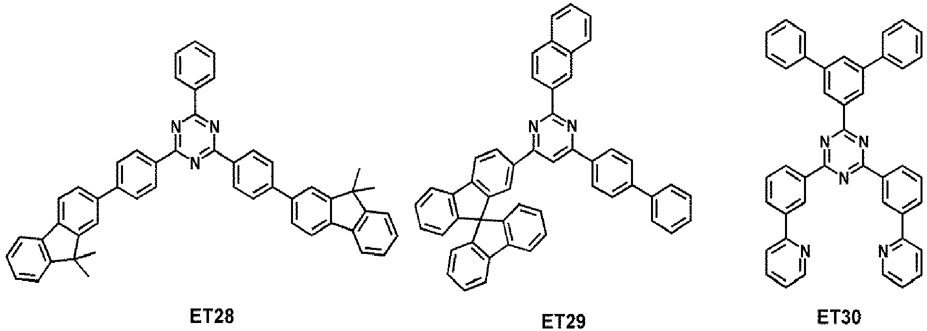

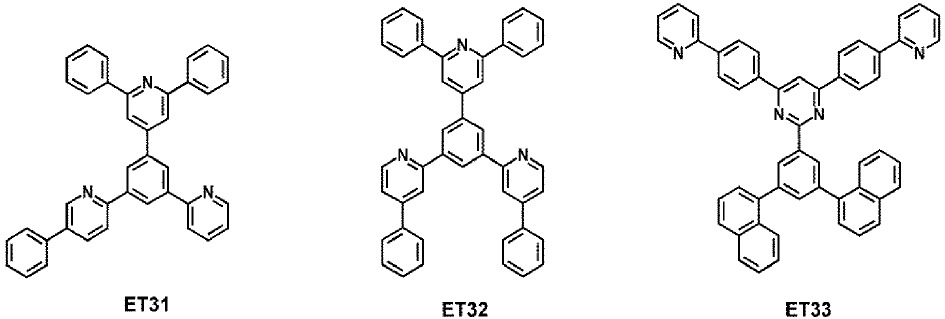

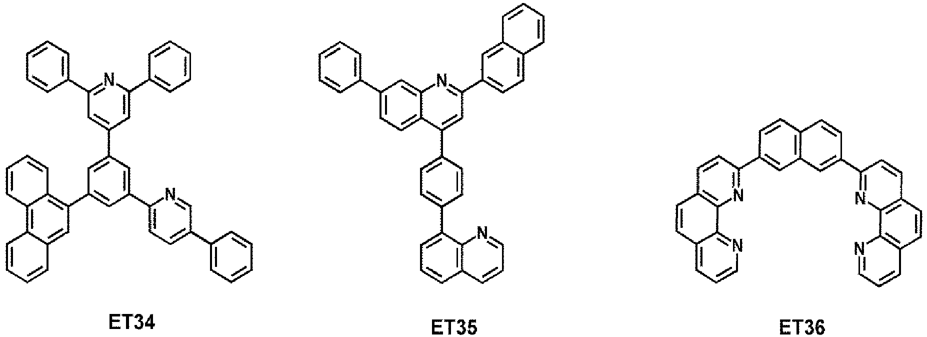

In some embodiments, the electron transport region may comprise at least one compound selected from 2,9-dimethyl-4,7-diphenyl-1,10-phenanthroline (BCP), 4,7-diphenyl-1,10-phenanthroline (Bphen), Alq₃, BAlq, 3-(biphenyl-4-yl)-5-(4-*tert-*butylphenyl)-4-phenyl-4*H*-1,2,4-triazole (TAZ), and NTAZ:

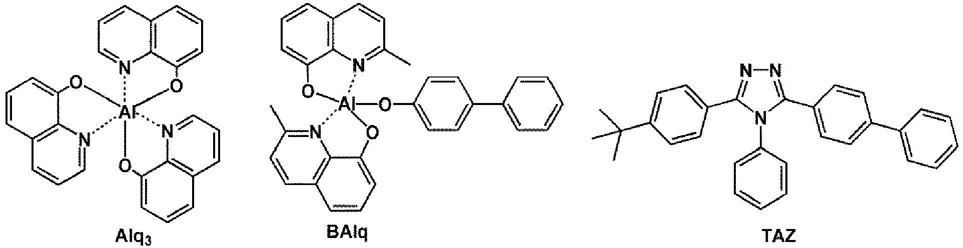

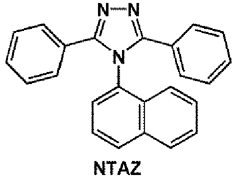

The thicknesses of the buffer layer, the hole blocking layer, or the electron control layer may each independently be in a range of about 20 Å to about 1,000 Å, and in some embodiments, about 30 Å to about 300 Å. When the thicknesses of the buffer layer, the hole blocking layer or the electron control layer are within the above-described ranges, excellent hole blocking characteristics or excellent electron controlling characteristics may be obtained without a substantial increase in driving voltage.

The thickness of the electron transport layer may be in a range of about 100 Å to about 1,000 Å, and in some embodiments, about 150 Å to about 500 Å. When the thickness of the electron transport layer is within the above-described ranges, excellent electron transport characteristics may be obtained without a substantial increase in driving voltage.

The electron transport region (e.g., the electron transport layer in the electron transport region) may further comprise, in addition to the materials described above, a material comprising metal (e.g., a metal ion).

The material comprising metal may comprise at least one selected from an alkali metal complex and an alkaline earth metal complex. The alkali metal complex may comprise a metal ion selected from a lithium (Li) ion, a sodium (Na) ion, a potassium (K) ion, a rubidium (Rb) ion, and a cesium (Cs) ion. The alkaline earth metal complex may comprise a metal ion selected from a beryllium (Be) ion, a magnesium (Mg) ion, a calcium (Ca) ion, an strontium (Sr) ion, and a barium (Ba) ion. Each ligand coordinated with the metal ion of the alkali metal complex and the alkaline earth metal complex may independently be selected from a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyl oxadiazole, a hydroxyphenyl thiadiazole, a hydroxyphenyl pyridine, a hydroxyphenyl benzimidazole, a hydroxyphenyl benzothiazole, a bipyridine, a phenanthroline, and a cyclopentadiene, but embodiments are not limited thereto.

Examples of the material comprising metal may include a Li complex. Examples of the Li complex may include, e.g., Compound ET-Di (lithium quinolate, LiQ) or Compound ET-D2:

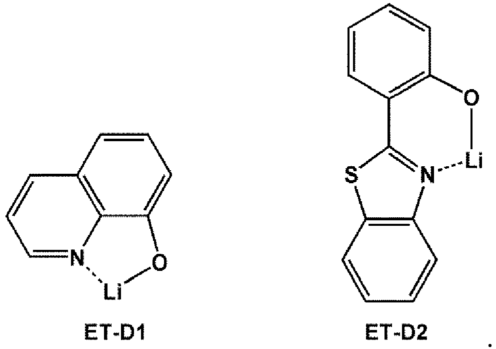

The electron transport region may comprise an electron injection layer that facilitates injection of electrons from the second electrode 190. The electron injection layer may be in direct contact with the second electrode 190.

The electron injection layer may have i) a single-layered structure including a single layer including a single material, ii) a single-layered structure including a single layer comprising a plurality of different materials, or iii) a multi-layered structure having a plurality of layers, each comprising a plurality of different materials.

The electron injection layer may comprise an alkali metal, an alkaline earth metal, a rare earth metal, an alkali metal compound, an alkaline earth metal compound, a rare earth metal compound, an alkali metal complex, an alkaline earth metal complex, a rare earth metal complex, or a combination thereof.

The alkali metal may be selected from Li, Na, K, Rb, and Cs. In one embodiment, the alkali metal may be Li, Na, or Cs. In one or more embodiments, the alkali metal may be Li or Cs, but embodiments are not limited thereto.

The alkaline earth metal may be selected from Mg, Ca, Sr, and Ba.

The rare earth metal may be selected from Sc, Y, Ce, Tb, Yb, and Gd.

The alkali metal compound, the alkaline earth metal compound, and the rare earth metal compound may each independently be selected from oxides and halides (e.g., fluorides, chlorides, bromides, or iodines) of the alkali metal, the alkaline earth metal, and the rare earth metal, respectively.

The alkali metal compound may be selected from alkali metal oxides (such as Li₂O, Cs₂O, or K₂O), and alkali metal halides (such as LiF, NaF, CsF, KF, LiI, NaI, CsI, KI, or RbI). In one embodiment, the alkali metal compound may be selected from LiF, Li₂O, NaF, LiI, NaI, CsI, and KI, but embodiments are not limited thereto.

The alkaline earth metal compound may be selected from alkaline earth metal compounds such as BaO, SrO, CaO, BaₓSr₁₋ₓO (where 0<x<1), and BaₓCa₁₋ₓO (where 0<x<1). In one embodiment, the alkaline earth metal compound may be selected from BaO, SrO, and CaO, but embodiments are not limited thereto.

The rare earth metal compound may be selected from YbF₃, ScF₃, ScO₃, Y₂O₃, Ce₂O₃, GdF₃, and TbF₃. In one embodiment, the rare earth metal compound may be selected from YbF₃, ScF₃, TbF₃, YbI₃, ScI₃, and TbI₃, but embodiments are not limited thereto.

The alkali metal complex, the alkaline earth metal complex, and the rare earth metal complex may each comprise ions of the above-described alkali metal, alkaline earth metal, and rare earth metal. Each ligand coordinated with the metal ion of the alkali metal complex, the alkaline earth metal complex, and the rare earth metal complex may independently be selected from a hydroxyquinoline, a hydroxyisoquinoline, a hydroxybenzoquinoline, a hydroxyacridine, a hydroxyphenanthridine, a hydroxyphenyl oxazole, a hydroxyphenyl thiazole, a hydroxyphenyl oxadiazole, a hydroxyphenyl thiadiazole, a hydroxyphenyl pyridine, a hydroxyphenyl benzimidazole, a hydroxyphenyl benzothiazole, a bipyridine, a phenanthroline, and a cyclopentadiene, but embodiments are not limited thereto.

The electron injection layer may comprise (e.g., consist of) an alkali metal, an alkaline earth metal, a rare earth metal, an alkali metal compound, an alkaline earth metal compound, a rare earth metal compound, an alkali metal complex, an alkaline earth metal complex, a rare earth metal complex, or a combination thereof, as described above. In some embodiments, the electron injection layer may further comprise an organic material. When the electron injection layer further comprises an organic material, the alkali metal, the alkaline earth metal, the rare earth metal, the alkali metal compound, the alkaline earth metal compound, the rare earth metal compound, the alkali metal complex, the alkaline earth metal complex, the rare earth metal complex, or a combination thereof may be homogeneously or non-homogeneously dispersed in a matrix comprising the organic material.

The thickness of the electron injection layer may be in a range of about 1 Å to about 100 Å, and in some embodiments, about 3 Å to about 90 Å. When the thickness of the electron injection layer is within the above-described ranges, excellent electron injection characteristics maybe obtained without a substantial increase in driving voltage.

### Second electrode 190

The second electrode 190 may be on the organic layer 150. In an embodiment, the second electrode 190 may be a cathode that is an electron injection electrode. In this embodiment, a material for forming the second electrode 190 may be a material having a low work function, for example, a metal, an alloy, an electrically conductive compound, or a combination thereof.

The second electrode 190 may comprise at least one selected from lithium (Li), silver (Ag), magnesium (Mg), aluminum (Al), aluminum-lithium (Al-Li), calcium (Ca), magnesium-indium (Mg-In), magnesium-silver (Mg-Ag), ITO, and IZO, but embodiments are not limited thereto. The second electrode 190 may be a transmissive electrode, a semi-transmissive electrode, or a reflective electrode.

The second electrode 190 may have a single-layered structure, or a multi-layered structure comprising two or more layers.

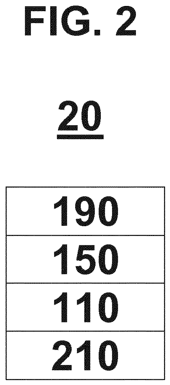

### Description of FIGS. 2 to 4

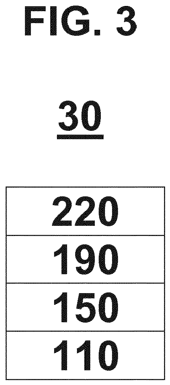

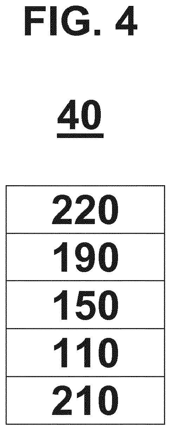

Referring to FIG. 2, an organic light-emitting device 20 has the structure of a first capping layer 210, the first electrode 110, the organic layer 150, and the second electrode 190, wherein the layers are sequentially stacked in this stated order. Referring to FIG. 3, an organic light-emitting device 30 has the structure of the first electrode 110, the organic layer 150, the second electrode 190, and a second capping layer 220, wherein the layers are sequentially stacked in this stated order. Referring to FIG. 4, an organic light-emitting device 40 has the structure of the first capping layer 210, the first electrode 110, the organic layer 150, the second electrode 190, and the second capping layer 220, wherein the layers are stacked in this stated order.

The first electrode 110, the organic layer 150, and the second electrode 190 illustrated in FIGS. 2 to 4 may be substantially the same as those illustrated in FIG. 1.

In the organic light-emitting devices 20 and 40, light emitted from the emission layer in the organic layer 150 may pass through the first electrode 110 (which may be a semi-transmissive electrode or a transmissive electrode) and through the first capping layer 210 to the outside. In the organic light-emitting devices 30 and 40, light emitted from the emission layer in the organic layer 150 may pass through the second electrode 190 (which may be a semi-transmissive electrode or a transmissive electrode) and through the second capping layer 220 to the outside.

The first capping layer 210 and the second capping layer 220 may improve the external luminescence efficiency based on the principle of constructive interference.

The first capping layer 210 and the second capping layer 220 may each independently be an organic capping layer comprising an organic material, an inorganic capping layer comprising an inorganic material, or a composite capping layer comprising an organic material and an inorganic material.

At least one of the first capping layer 210 and the second capping layer 220 may each independently comprise at least one material selected from carbocyclic compounds, heterocyclic compounds, amine-based compounds, porphine derivatives, phthalocyanine derivatives, naphthalocyanine derivatives, alkali metal complexes, and alkaline earth metal complexes. The carbocyclic compound, the heterocyclic compound, and the amine-based compound may optionally be substituted with a substituent containing at least one element selected from O, N, S, Se, Si, F, Cl, Br, and I. In one embodiment, at least one of the first capping layer 210 and the second capping layer 220 may each independently comprise an amine-based compound.

In one or more embodiments, at least one of the first capping layer 210 and the second capping layer 220 may each independently comprise a compound represented by Formula 201 or a compound represented by Formula 202.

In one or more embodiments, at least one of the first capping layer 210 and the second capping layer 220 may each independently comprise a compound selected from Compounds HT28 to HT33 and Compound CP1 to CP5, but embodiments are not limited thereto:

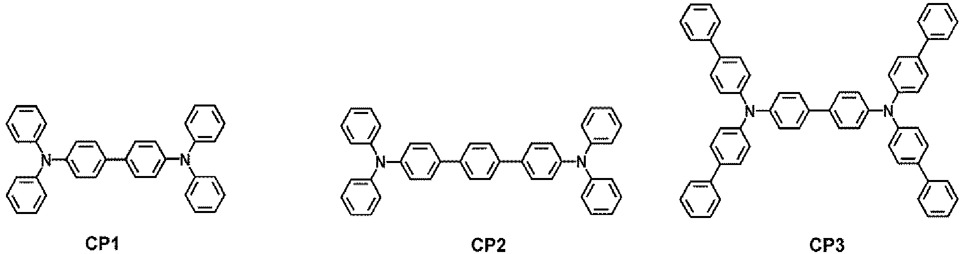

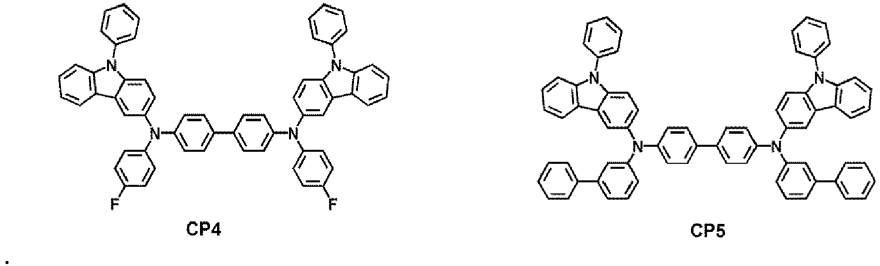

Hereinbefore, the organic light-emitting device has been described with reference to FIGS. 1 to 4, but embodiments are not limited thereto.

The layers constituting the hole transport region, the emission layer, and the layers constituting the electron transport region may be formed in a specific region (e.g., in a respective region) utilizing one or more suitable methods, such as vacuum deposition, spin coating, casting, Langmuir-Blodgett (LB) deposition, ink-jet printing, laser printing, and/or laser-induced thermal imaging.

When the layers constituting the hole transport region, the emission layer, and/or the layers constituting the electron transport region are each formed by vacuum deposition, the vacuum deposition may be performed at a deposition temperature in a range of about 100°C to about 500°C at a vacuum degree in a range of about 10⁻⁸ torr to about 10⁻³ torr, and at a deposition rate in a range of about 0.01 Angstroms per second (Å/sec) to about 100 Å/sec, depending on the material to be included in each layer and the structure of each layer to be formed.

When the layers constituting the hole transport region, the emission layer, and/or the layers constituting the electron transport region are each formed by spin coating, the spin coating may be performed at a coating rate of about 2,000 revolutions per minute (rpm) to about 5,000 rpm and at a heat treatment temperature of about 80°C to about 200°C, depending on the material to be included in each layer and the structure of each layer to be formed.

### General definitions of substituents

The term "C₁-C₆₀ alkyl group" as used herein refers to a linear or branched aliphatic hydrocarbon monovalent group having 1 to 60 carbon atoms, e.g. 1 to 30 carbon atoms, 1 to 20 carbon atoms, 1 to 10 carbon atoms, 1 to 6 carbon atoms or 1 to 4 carbon atoms. Examples thereof may include a methyl group, an ethyl group, a propyl group, an iso-butyl group, a sec-butyl group, a tert-butyl group, a pentyl group, an iso-amyl group, and a hexyl group. The term "C₁-C₆₀ alkylene group" as used herein refers to a divalent group having the same structure as the C₁-C₆₀ alkyl group.

The term "C₂-C₆₀ alkenyl group" as used herein refers to a hydrocarbon group having at least one carbon-carbon double bond in the middle or at the terminus of the C2-C₆₀ alkyl group. Examples thereof may include an ethenyl group, a propenyl group, and a butenyl group. The term "C₂-C₆₀ alkenylene group" as used herein refers to a divalent group having the same structure as the C₂-C₆₀ alkenyl group.

The term "C₂-C₆₀ alkynyl group" as used herein refers to a hydrocarbon group having at least one carbon-carbon triple bond in the middle or at the terminus of the C2-C₆₀ alkyl group. Examples thereof may include an ethynyl group and a propynyl group. The term "C₂-C₆₀ alkynylene group" as used herein refers to a divalent group having the same structure as the C₂-C₆₀ alkynyl group.

The term "C₁-C₆₀ alkoxy group" as used herein refers to a monovalent group represented by -OA₁₀₁ (wherein A₁₀₁ is a C₁-C₆₀ alkyl group). Examples thereof may include a methoxy group, an ethoxy group, and an isopropyloxy group.

The term "C₃-C₁₀ cycloalkyl group" as used herein refers to a monovalent monocyclic saturated hydrocarbon group having 3 to 10 carbon atoms. Examples thereof may include a cyclopropyl group, a cyclobutyl group, a cyclopentyl group, a cyclohexyl group, and a cycloheptyl group. The term "C₃-C₁₀ cycloalkylene group" as used herein refers to a divalent group having the same structure as the C₃-C₁₀ cycloalkyl group.

The term "C₁-C₁₀ heterocycloalkyl group" as used herein refers to a monovalent monocyclic group having at least one heteroatom (e.g. 1, 2, 3, 4 or 5 heteroatoms) selected from N, O, Si, P, and S as a ring-forming atom and 1 to 10 carbon atoms, e.g. 1, 2, 3, 4 or 5 ring carbon atoms. Examples thereof may include a 1,2,3,4-oxatriazolidinyl group, a tetrahydrofuranyl group, and a tetrahydrothiophenyl group. The term "C₁-C₁₀ heterocycloalkylene group" as used herein refers to a divalent group having the same structure as the C₁-C₁₀ heterocycloalkyl group.

The term "C₃-C₁₀ cycloalkenyl group" as used herein refers to a monovalent monocyclic group having 3 to 10 carbon atoms and at least one carbon-carbon double bond in its ring, wherein the molecular structure as a whole is non-aromatic. Examples thereof may include a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₁-C₁₀ heterocycloalkenyl group" as used herein refers to a monovalent monocyclic group having at least one heteroatom (e.g. 1, 2, 3, 4 or 5 heteroatoms) selected from N, O, Si, P, and S as a ring-forming atom, 1 to 10 carbon atoms, e.g. 1, 2, 3, 4 or 5 ring carbon atoms, and at least one double bond in its ring. Examples of the C₁-C₁₀ heterocycloalkenyl group may include a 4,5-dihydro-1,2,3,4-oxatriazolyl group, a 2,3-dihydrofuranyl group, and a 2,3-dihydrothiophenyl group. The term "C₁-C₁₀ heterocycloalkenylene group" as used herein refers to a divalent group having the same structure as the C₁-C₁₀ heterocycloalkenyl group.

The term "C₆-C₆₀ aryl group" as used herein refers to a monovalent group having a carbocyclic aromatic system having 6 to 60 carbon atoms, e.g. 6 to 20 ring carbon atoms, 6 to 14 ring cabon atoms or 6 to 10 ring carbon atoms. The term "C₆-C₆₀ arylene group" as used herein refers to a divalent group having a carbocyclic aromatic system having 6 to 60 carbon atoms, e.g. 6 to 20 ring carbon atoms, 6 to 14 ring cabon atoms or 6 to 10 ring carbon atoms. Examples of the C₆-C₆₀ aryl group may include a phenyl group, a naphthyl group, an anthracenyl group, a phenanthrenyl group, a pyrenyl group, and a chrysenyl group. When the C₆-C₆₀ aryl group and the C₆-C₆₀ arylene group each independently comprise two or more rings, the respective rings may be fused.

The term "C₁-C₆₀ heteroaryl group" as used herein refers to a monovalent group having a heterocyclic aromatic system having at least one heteroatom (e.g. 1, 2, 3, 4 or 5 hereoatoms) selected from N, O, Si, P, and S as a ring-forming atom and 1 to 60 carbon atoms, e.g. 1 to 19 ring carbon atoms, 1 to 13 ring cabon atoms or 1 to 9 ring carbon atoms. The term "C₁-C₆₀ heteroarylene group" as used herein refers to a divalent group having a heterocyclic aromatic system having at least one heteroatom (e.g. 1, 2, 3, 4 or 5 hereoatoms) selected from N, O, Si, P, and S as a ring-forming atom and 1 to 60 carbon atoms, e.g. 1 to 19 ring carbon atoms, 1 to 13 ring cabon atoms or 1 to 9 ring carbon atoms. Examples of the C₁-C₆₀ heteroaryl group may include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each independently comprise two or more rings, the respective rings may be fused.

The term "C₆-C₆₀ aryloxy group" as used herein refers to a group represented by -OA₁₀₂ (where A₁₀₂ is a C₆-C₆₀ aryl group). The term "C₆-C₆₀ arylthio group" as used herein refers to a group represented by -SA₁₀₃ (where A₁₀₃ is a C₆-C₆₀ aryl group).

The term "monovalent non-aromatic condensed C8 to C60 polycyclic group" as used herein refers to a monovalent group that has two or more rings condensed and 8 to 60 carbon atoms as ring forming atoms (e.g., 8 to 20, 8 to 14 or 8 to 10 ring carbon atoms), wherein the entire molecular structure is non-aromatic (e.g., the molecular structure does not have overall aromaticity). An example of the monovalent non-aromatic condensed C8 to C60 polycyclic group may be a fluorenyl group. The term "divalent non-aromatic condensed C8 to C60 polycyclic group" as used herein refers to a divalent group having the same structure as the monovalent non-aromatic condensed C8 to C60 polycyclic group.

The term "monovalent non-aromatic condensed 8- to 60-membered heteropolycyclic group" as used herein refers to a monovalent group that has having 8- to 60- ring-forming atoms and two or more condensed rings and at least one heteroatom (e.g. 1, 2, 3, 4 or 5 hereoatoms) selected from N, O, Si, P, and S, in addition to carbon atoms (e.g., 1 to 59, 2 to 59, 2 to 19, 2 to 13 or 2 to 9 ring carbon atoms), as a ring-forming atom, wherein the entire molecular structure is non-aromatic (e.g., the molecular structure does not have overall aromaticity). An example of the monovalent non-aromatic condensed 8- to 60-membered heteropolycyclic group is a carbazolyl group. The term "divalent non-aromatic condensed 8- to 60-membered heteropolycyclic group" as used herein refers to a divalent group having the same structure as the monovalent non-aromatic condensed 8- to 60-membered heteropolycyclic group.

The term "C₅-C₆. carbocyclic group" as used herein refers to a monocyclic group or a polycyclic group having 5 to 60 carbon atoms e.g. 5 to 20 ring carbon atoms, 5 to 14 ring cabon atoms or 5 to 10 ring carbon atoms, in which the only ring-forming atoms are carbon atoms. The C₅-C₆₀ carbocyclic group may be an aromatic carbocyclic group or a non-aromatic carbocyclic group. The term "C₅-C₆₀ carbocyclic group" as used herein refers to a ring (e.g., a benzene group), a monovalent group (e.g., a phenyl group), or a divalent group (e.g., a phenylene group). In one or more embodiments, depending on the number of substituents connected to the C₅-C₆₀ carbocyclic group, the C₅-C₆₀ carbocyclic group may be a trivalent group or a quadrivalent group.

The term "C₁-C₆₀ heterocyclic group" as used herein refers to a group having substantially the same structure as the C₅-C₆₀ carbocyclic group, except that at least one heteroatom (e.g. 1, 2, 3, 4 or 5 hereoatoms) selected from N, O, Si, P, and S is used as a ring-forming atom, in addition to 1 to 60 carbon atoms (e.g.,1 to 20 ring carbon atoms, 1 to 14 ring cabon atoms or 1 to 10 ring carbon atoms).

Certain groups are specified herein as being substituted or unsubstituted. Unless otherwise specified, when a group is substituted it is typically substituted with 1, 2, 3 or 4 substituents. For example, when a group is substituted it may be substituted with 1, 2 or 3 substituents; 1 or 2 substituents; or 1 substituent. Groups which are not specified as being substituted or unsubstituted are typically unsubstituted. Unless otherwise specified, when a group is substituted it is typically substituted with at least one substituent (e.g. 1, 2, 3 or 4 substituents) selected from:

deuterium (-D), -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, and a C₁-C₆₀ alkoxy group;

a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, -Br, - I, a hydroxyl group, a cyano group, a nitro group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed C8 to C60 polycyclic group, a monovalent non-aromatic condensed 8- to 60-membered heteropolycyclic group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), and -P(=O)(Q₁₁)(Q₁₂);

a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed C8 to C60 polycyclic group, and a monovalent non-aromatic condensed 8- to 60-membered heteropolycyclic group;

a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed C8 to C60 polycyclic group, and a monovalent non-aromatic condensed 8- to 60-membered heteropolycyclic group, each substituted with at least one selected from deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed C8 to C60 polycyclic group, a monovalent non-aromatic condensed 8-to 60-membered heteropolycyclic group, -Si(Q₂₁)(Q₂₂)(Q₂₃), -N(Q₂₁)(Q₂₂), -B(Q₂₁)(Q₂₂), - C(=O)(Q₂₁), -S(=O)₂(Q₂₁), and -P(=O)(Q₂₁)(Q₂₂); and

-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁) and - P(=O)(Q₃₁)(Q₃₂),

wherein Q₁₁ to Q₁₃, Q₂₁ to Q₂₃, and Q₃₁ to Q₃₃ may each independently be selected from hydrogen, deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed C8 to C60 polycyclic group, a monovalent non-aromatic condensed 8- to 60-membered heteropolycyclic group, a biphenyl group, and a terphenyl group.

The term "Ph" as used herein represents a phenyl group. The term "Me" as used herein represents a methyl group. The term "Et" as used herein represents an ethyl group. The term "ter-Bu" or "Bu^{t}" as used herein represents a *tert*-butyl group. The term "OMe" as used herein represents a methoxy group.

The term "biphenyl group" as used herein refers to a phenyl group substituted with a phenyl group. The "biphenyl group" belongs to "a substituted phenyl group" having a "C₆-C₆₀ aryl group" as a substituent.

The term "terphenyl group" as used herein refers to a phenyl group substituted with a biphenyl group. The "terphenyl group" belongs to "a substituted phenyl group" having a "C₆-C₆₀ aryl group substituted with a C₆-C₆₀ aryl group" as a substituent.

The symbols ^{∗} and ^{∗}' as used herein, unless defined otherwise, each indicate a binding site to an adjacent atom in the corresponding formula.

Hereinafter, compounds and an organic light-emitting device according to one or more embodiments will be described in more detail with reference to Synthesis Examples and Examples. The wording "B was utilized instead of A" used in describing Synthesis Examples means that an amount of B utilized was identical to an amount of A utilized in terms of molar equivalents.

### Synthesis Examples

### Synthesis Example 1: Synthesis of Compound 1

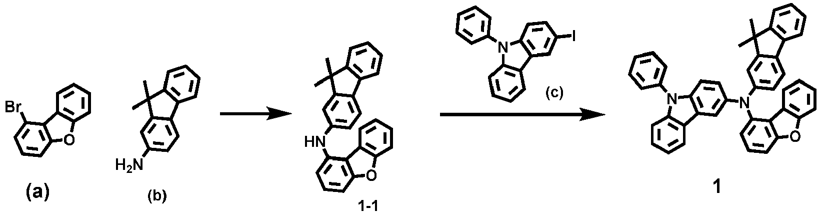

### (1) Synthesis of Intermediate 1-1

5 grams (g) of 1-bromodibenzo[*b*,*d*]furan (Intermediate (a)), 6.4 g of 9,9-dimethyl-9*H*-fluoren-2-amine (Intermediate (b)), 6.4 g of potassium tert-butoxide (KₜOBu), 0.3 g of P(tBu)₃, and 0.4 g of Pd₂(dba)₃ were diluted in toluene, followed by stirring at a temperature of 100°C under reflux. After a 20-hour lapse, the temperature was lowered to room temperature. Subsequently, the reaction was terminated utilizing water. An organic layer was extracted therefrom three times utilizing ethyl acetate. Then, the organic layer was dried utilizing anhydrous magnesium sulfate, followed by filtration under reduced pressure. The obtained residue was separated and purified through column chromatography to thereby obtain 6.5 g of Intermediate 1-1 (yield: 86%). The compound thus obtained was identified by liquid chromatography-mass spectrometry (LC-MS). C27H21NO : calc'd : M+ 375.16 foun'd : 375.26

### (2) Synthesis of Compound 1

Compound 1 was obtained in substantially the same manner as in Synthesis of Intermediate 1-1, except that 3.8 g of Intermediate 1-1 was utilized instead of Intermediate (b), and 3.7 g of 3-iodo-9-phenyl-9H-carbazole (Intermediate (c)) was utilized instead of 1-bromodibenzo[*b*,*d*]furan (Intermediate (a)).

C45H32N2O : calc'd : M+ 616.25 foun'd : 616.35

### Synthesis Example 2: Synthesis of Compound 2

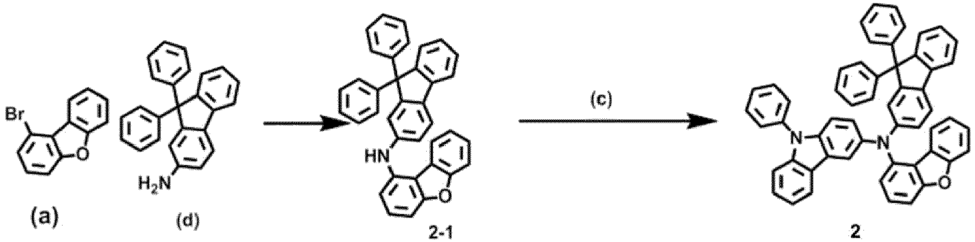

### (1) Synthesis of Intermediate 2-1

8.2 g of Intermediate 2-1 was obtained in substantially the same manner as in Synthesis of Intermediate 1-1, except that 8 g of Intermediate (d) was utilized instead of Intermediate (b) (yield: 82%). C37H25NO: calc'd : M+ 499.19 foun'd : 499.29

### (2) Synthesis of Compound 2

Compound 2 was obtained in substantially the same manner as in Synthesis of Compound 1, except that 5 g of Intermediate 2-1 was utilized instead of Intermediate 1-1. C55H36N2O : calc'd : M+ 740.28 foun'd : 740.38

### Synthesis Example 3: Synthesis of Compound 3

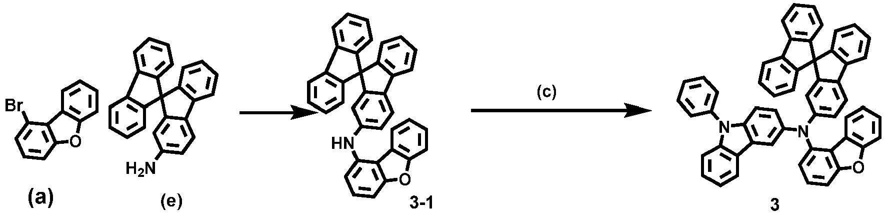

### (1) Synthesis of Intermediate 3-1

5.2 g of Intermediate 3-1 was obtained in substantially the same manner as in Synthesis of Intermediate 1-1, except that 8 g of Intermediate (e) was utilized instead of Intermediate (b) (yield: 52%). C37H25NO: calc'd : M+ 497.18 foun'd : 497.28

### (2) Synthesis of Compound 3

7.4 g of Compound 3 was obtained in substantially the same manner as in Synthesis of Compound 1, except that 5 g of Intermediate 3-1 was utilized instead of Intermediate 1-1 (yield: 75%). C55H36N2O : calc'd : M+ 740.28 foun'd : 740.38

### Synthesis Example 4: Synthesis of Compound 4

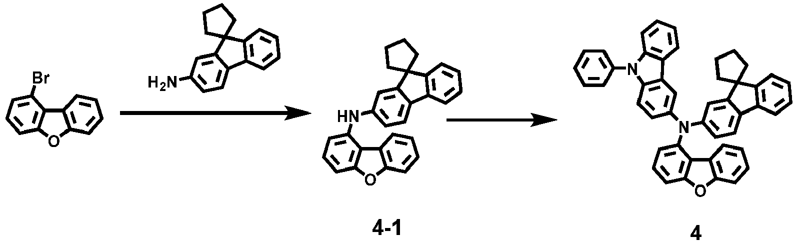

### (1) Synthesis of Intermediate 4-1

5.1 g of Intermediate 4-1 was obtained in substantially the same manner as in Synthesis of Intermediate 1-1, except that 4.9 g of 1-bromodibenzo[b,d]furan was utilized instead of 5 g of Intermediate (a), and 7.1 g of spiro[cyclopentane-1,9'-fluoren]-2'-amine was utilized instead of Intermediate (b) (yield: 64 %).

C29H23NO : calc'd : M+ 401.18 foun'd : 401.20

### (2) Synthesis of Compound 4

4.6 g of Compound 4 was obtained in substantially the same manner as in Synthesis of Intermediate 1-1, except that 4 g of Intermediate 4-1 was utilized instead of Intermediate (b), and 3.7 g of 3-iodo-9-phenyl-9H-carbazole was utilized instead of 5 g of Intermediate (a) (yield: 72 %).

C47H34N2O : calc'd : M+ 647.27 foun'd : 647.28

¹H NMR (500 MHz, CDCl₃) δ= 8.22(m, 1H), 7.79(dd, 2H), 7.72(dd, 1H), 7.67(d, 1H), 7.54-7.14(m, 17H), 6.95(d, 1H), 6.90(dd, 1H), 6.66(dd, 1H),. 6.54(dd, 1H), 2.19-2.08(m, 2H), 1.95-1.72(m, 6H)

### Synthesis Example 5: Synthesis of Compound 5

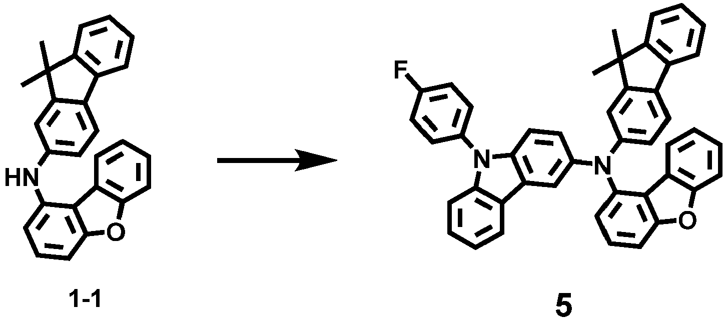

4.7 g of Compound 5 was obtained in substantially the same manner as in Synthesis of Intermediate 1-1, except that 3.8 g of Intermediate 1-1 was utilized instead of Intermediate (b), and 3.4 g of 3-bromo-9-(4-fluorophenyl)-9H-carbazole was utilized instead of Intermediate (a) (yield: 75%). C45H31FN2O : calc'd : M+ 634.24 foun'd : 634.34

### Synthesis Example 6: Synthesis of Compound 6

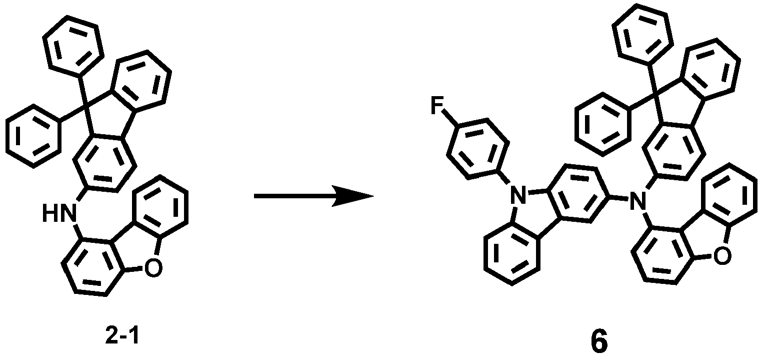

5.2 g of Compound 6 was obtained in substantially the same manner as in Synthesis of Intermediate 1-1, except that 5 g of Intermediate 2-1 was utilized instead of Intermediate (b), and 3.4 g of 3-bromo-9-(4-fluorophenyl)-9H-carbazole was utilized instead of Intermediate (a) (yield: 68%). C55H35FN2O : calc'd : M+ 758.27 foun'd : 758.37

### Synthesis Example 7: Synthesis of Compound 8

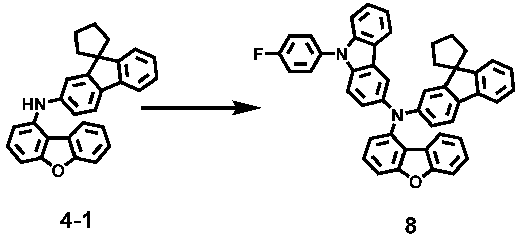

5 g of Compound 8 was obtained in substantially the same manner as in Synthesis of Compound 4, except that 4 g of Intermediate 4-1 and 3.9 g of 9-(4-fluorophenyl)-3-iodo-9H-carbazole were utilized (yield: 76 %).

C47H33FN2O : calc'd : M+ 660.26 foun'd : 660.27

¹H NMR (500 MHz, CDCl₃) δ= 8.22(m, 1H), 7.79(dd, 2H), 7.72(dd, 1H), 7.67(d, 1H), 7.48-7.23(m, 13H), 7.18-7.14(m, 1H), 7.09-7.04(m, 2H), 6.95(d, 1H), 6.90(dd, 1H), 6.66(dd, 1H),. 6.54(dd, 1H), 2.19-2.08(m, 2H), 1.95-1.72(m, 6H)

### Synthesis Example 8: Synthesis of Compound 16

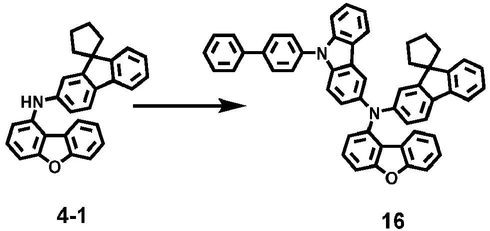

4.7 g of Compound 16 was obtained in substantially the same manner as in Synthesis of Compound 4, except that 4 g of Intermediate 4-1 and 4 g of 9-([1,1'-biphenyl]-4-yl)-3-bromo-9H-carbazole were utilized (yield: 66 %).

C53H38N2O : calc'd : M+ 718.30 foun'd : 718.33

¹H NMR (500 MHz, CDCl₃) δ= 8.22(m, 1H), 7.79(dd, 2H), 7.72(dd, 1H), 7.67(d, 1H), 7.63-7.59(m, 2H), 7.57-7.23(m, 18H), 7.18(dt, 1H), 6.95(d, 1H), 6.90(dd, 1H), 6.66(dd, 1H), 6.59(dd, 1H), 2.19-2.08(m, 2H), 1.95-1.72(m, 6H)

### Synthesis Example 9: Synthesis of Compound 21

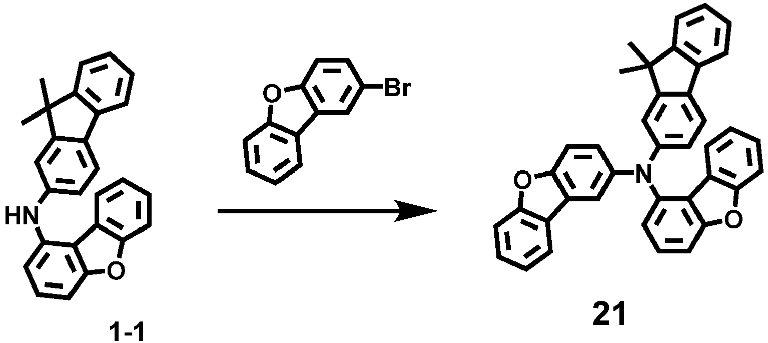

4.3 g of Compound 21 was obtained in substantially the same manner as in Synthesis of Intermediate 1-1, except that 3.8 g of Intermediate 1-1 was utilized instead of Intermediate (b), and 2.5 g of 2-bromodibenzo[b,d]furan was utilized instead of Intermediate (a) (yield: 80%). C45H31FN2O: calc'd : M+ 541.20 foun'd : 541.30

### Synthesis Example 10: Synthesis of Compound 37

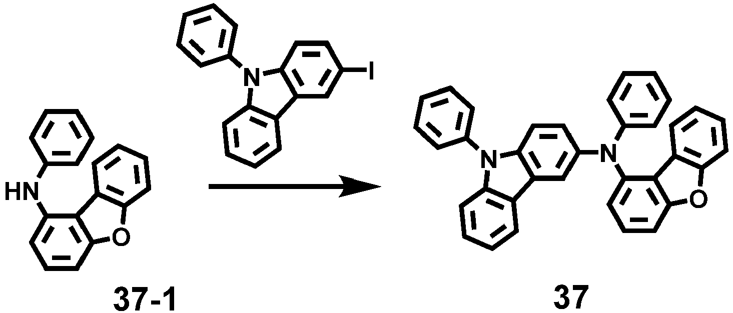

3.8 g of Compound 37 was obtained in substantially the same manner as in Synthesis of Intermediate 1-1, except that 2.6 g of Intermediate 37-1 was utilized instead of Intermediate (b), and 3.7 g of Intermediate (c) was utilized instead of Intermediate (a) (yield: 76%).

C36H24N2OM+ calc'd : 500.19 foun'd : 500.29

### Synthesis Example 11: Synthesis of Compound 93

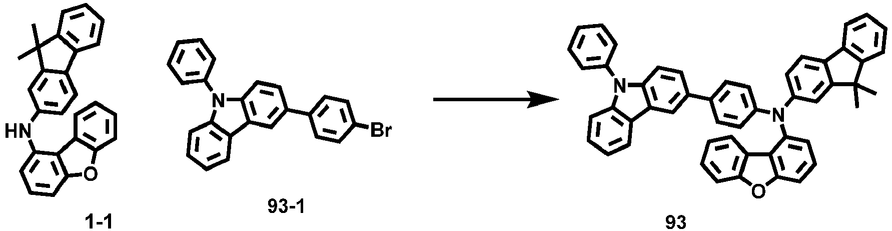

5.1 g of Compound 93 was obtained in substantially the same manner as in Synthesis of Intermediate 1-1, except that 3.8 g of Intermediate 1-1 was utilized instead of Intermediate (b), and 4 g of Intermediate 93-1 was utilized instead of Intermediate (a) (yield: 74%).

C51H36N2O M+ calc'd : 692.28 foun'd : 692.38

### Synthesis Example 12: Synthesis of Compound 94

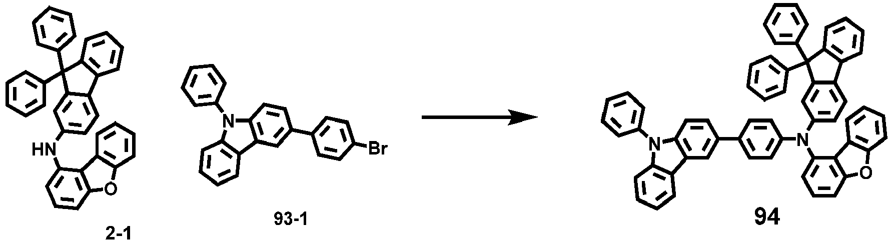

6.3 g of Compound 94 was obtained in substantially the same manner as in Synthesis of Intermediate 1-1, except that 5 g of Intermediate 2-1 was utilized instead of Intermediate (b), and 4 g of Intermediate 93-1 was utilized instead of Intermediate (a) (yield: 77%).

C61H40N2O M+ calc'd : 816.31 foun'd : 816.41

### Synthesis Example 13: Synthesis of Compound 95

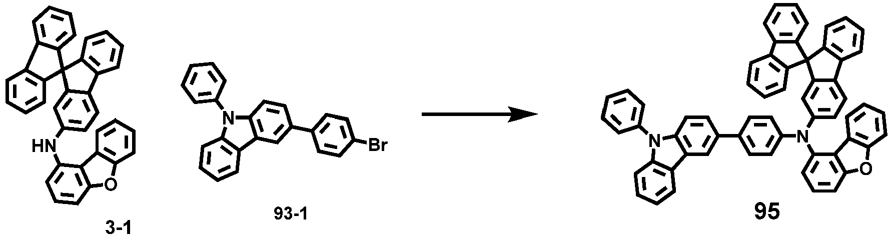

6.1 g of Compound 95 was obtained in substantially the same manner as in Synthesis of Intermediate 1-1, except that 5 g of Intermediate 3-1 was utilized instead of Intermediate (b), and 4 g of Intermediate 93-1 was utilized instead of Intermediate (a) (yield: 75%).

C61H38N2O M+ calc'd : 814.30 foun'd : 814.40

### Synthesis Example 14: Synthesis of Compound 101

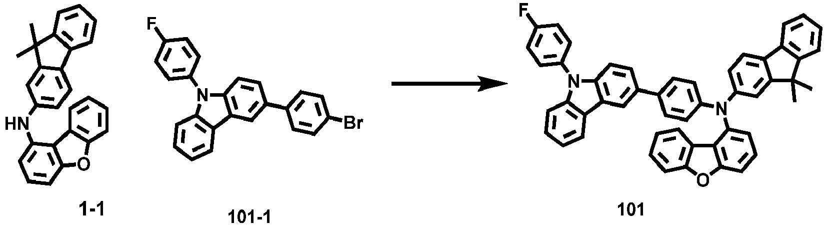

7.1 g of Compound 101 was obtained in substantially the same manner as in Synthesis of Intermediate 1-1, except that 3.8 g of Intermediate 1-1 was utilized instead of Intermediate (b), and 4.2 g of Intermediate 101-1 was utilized instead of Intermediate (a) (yield: 75%).

C51H35FN2O M+ calc'd : 710.27 foun'd : 710.37

### Synthesis Example 15: Synthesis of Compound 187

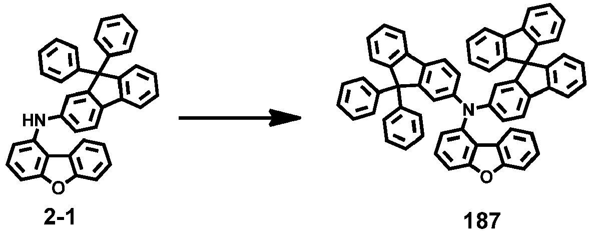

3.1 g of Compound 187 was obtained in substantially the same manner as in Synthesis of Intermediate 1-1, except that 2.5 g of Intermediate 2-1 was utilized instead of Intermediate (b), and 2 g of 2-bromo-9,9'-spirobi[fluorene] was utilized instead of Intermediate (a) (yield: 77%).

C62H39NO : calc'd : M+ 813.30 foun'd : 813.34

¹H NMR (500 MHz, CDCl₃) δ= 7.92-7.84(m, 4^{H}), 7.79(m, 1H), 7.72(dd, 1H), 7.61(d, 1H), 7.58(d, 1H), 7.48-7.41(m, 5H), 7.36-7.28(m, 3H), 7.21-7.06(m, 14H), 6.92(dd, 1H), 6.81-6.72(m, 5H), 6.66(dd, 1H), 6.57(d, 1H), 6.43(d, 1H)

### Synthesis Example 16: Synthesis of Compound 188

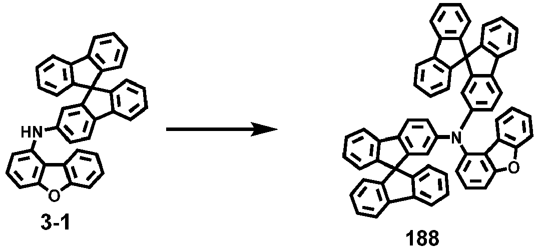

3.3 g of Compound 188 was obtained in substantially the same manner as in Synthesis of Intermediate 1-1, except that 2.5 g of Intermediate 3-1 was utilized instead of Intermediate (b), and 2 g of 2-bromo-9,9'-spirobi[fluorene] was utilized instead of Intermediate (a) (yield: 81 %).

C62H37NO : calc'd : M+ 811.29 foun'd : 811.31

¹H NMR (500 MHz, CDCl₃) δ= 7.92(dd, 4H), 7.89(dd, 2H), 7.79(td, 1H), 7.72(dd, 1H), 7.61(d, 2H), 7.48-7.41(m, 7H), 7.36-7.26(m, 3H), 7.21-7.15(m, 6H), 6.92(dd, 2H), 6.77-6.73(m, 6H), 6.68(dd, 1H), 6.43(d, 2H)

### Synthesis Example 17: Synthesis of Compound 204

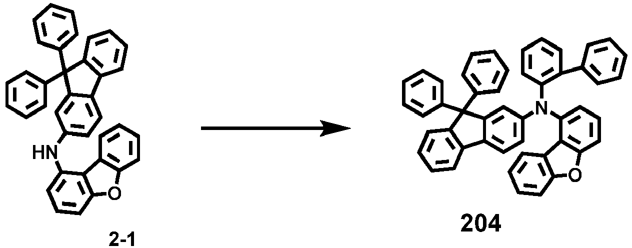

3.8 g of Compound 204 was obtained in substantially the same manner as in Synthesis of Intermediate 1-1, except that 5 g of Intermediate 2-1 was utilized instead of Intermediate (b), and 2.3 g of 2-bromo-1,1'-biphenyl was utilized instead of Intermediate (a) (yield: 58%).

C49H33NO : M+ calc'd : 651.26 foun'd : 651.36

### Synthesis Example 18: Synthesis of Compound 210

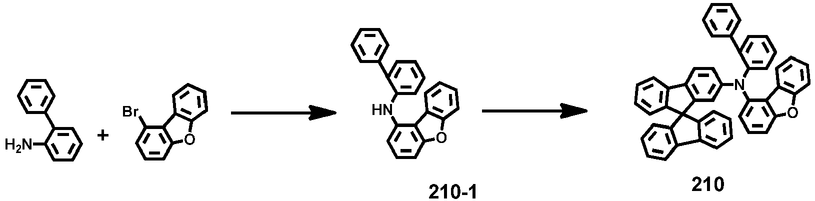

### (1) Synthesis of Intermediate 210-1

3 g of Intermediate **210-1** was obtained in substantially the same manner as in Synthesis of Intermediate 1-1, except that 2.5 g of [1,1'-biphenyl]-2-amine was utilized instead of Intermediate (b), and 2.5 g of 1-bromodibenzo[b,d]furan was utilized instead of 5 g of Intermediate (a) (yield: 91%).

C24H17NO: calc'd : M+ 335.13 foun'd : 335.14

### (2) Synthesis of Compound 210

5.1 g of Compound 210 was obtained in substantially the same manner as in Synthesis of Intermediate 1-1, except that 3 g of Intermediate 210-1 was utilized instead of Intermediate (b), and 3.7 g of 2-bromo-9,9'-spirobi[fluorene] was utilized instead of Intermediate (a) (yield: 86 %).

C49H31NO: calc'd : M+649.24 foun'd : 649.25

¹H NMR (500 MHz, CDCl₃) δ= 7.92(dd, 2H), 7.89(dd, 1H), 7.80(d, 1H), 7.72(d, 1H), 7.59-7.51(m, 5H), 7.49-7.41(m, 5H), 7.32-7.27(m, 3H), 7.21-7.14(m, 5H), 7.01(t, 1H), 6.77-6.73(m, 4H), 6.66(dd, 1H), 6.61(dd, 1H),5.47(d, 1H)

### Synthesis Example 19: Synthesis of Compound 213

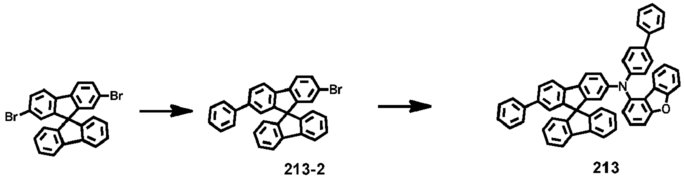

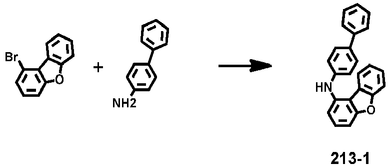

### (1) Synthesis of Intermediate 213-2

4.7 g of 2,7-dibromo-9,9'-spirobi[fluorene] and 3.4 g of phenyl boronic acid were dilluted with 60 mL of tetrahydrofuran and 20 mL of water. 4.1 g of K₂CO₃ and 580 mg of Pd(PPh₃)₄ were added dropwise thereto, followed by stirring at a temperature of 65 °C under reflux. Once the reaction was complete, the temperature was lowered to room temperature, and filtration was performed three times utilizing ethyl acetate under reduced pressure. Then, the residue obtained by distillation under reduced pressure was separated and purified through column chromatography to obtain 4.4 g of Intermediate 213-2 (yield: 93 %).

C31H19Br : calc'd : M+ 470.07 foun'd : 470.08

### (2) Synthesis of Intermediate 213-1

3 g of Intermediate 213-1 was obtained in substantially the same manner as in Synthesis of Intermediate 1-1, except that 2.5 g of [1,1'-biphenyl]-4-amine was utilized instead of Intermediate (b), and 2.5 g of 1-bromodibenzo[b,d]furan was utilized instead of 5 g of Intermediate (a) (yield: 91%).

C24H17NO: calc'd : M+ 335.13 foun'd : 335.14

### (3) Synthesis of Compound 213

5.1g of Compound 213 was obtained in substantially the same manner as in Synthesis of Intermediate 1-1, except that 4.4 g of Intermediate 213-2 was utilized instead of Intermediate (a), and 3 g of Intermediate 213-1 was utilized instead of Intermediate (b) (yield: 79 %).

C55H35NO: calc'd : M+ 725.27 foun'd : 725.30

¹H NMR (500 MHz, CDCl₃) δ= 7.93(dd, 2H), 7.79-7.78(111, 2H), 7.72-7.59(111, 6H), 7.54-7.28(m, 15H), 7.20(t, 2H), 6.89(m, 2H), 6.75(dd, 2H), 6.68(dd, 1H), 6.56-6.54(m, 2H), 5.68(d, 1H)

### Synthesis Example 20: Synthesis of Compound 219

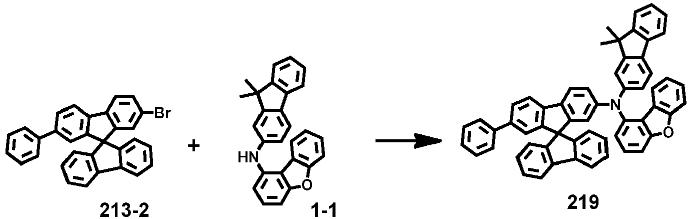

3.1 g of Compound 219 was obtained in substantially the same manner as in Synthesis of Intermediate 1-1, except that 2.4 g of Intermediate 213-2 was utilized instead of Intermediate (a), and 1.9 g of Intermediate 1-1 was utilized instead of Intermediate (b) (yield: 81 %).

C58H39NO : calc'd : M+ 765.30 foun'd : 765.34

¹H NMR (500 MHz, CDCl₃) δ= 7.93(dd, 2H), 7.79-7.67(m, 6H), 7.62(d, 1H), 7.53-7.28(m, 11H), 7.18(dt, 2H), 7.11(dd, 2H), 6.89-6.82(m, 3H), 6.75(dd, 2H), 6.68(dd, 1H), 6.50(d, 1H). 6.10(d, 1H), 1.61(s, 6H)

### Synthesis Example 21: Synthesis of Compound 240

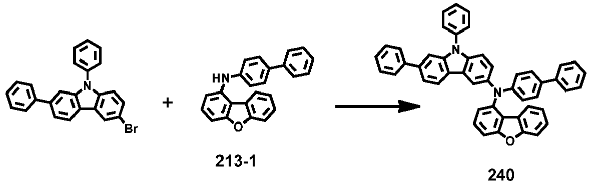

2.5 g of Compound 240 was obtained in substantially the same manner as in Synthesis of Intermediate 1-1, except that 2 g of 6-bromo-2,9-diphenyl-9H-carbazole was utilized instead of Intermediate (a), and 1.7 g of Intermediate 213-1 was utilized instead of Intermediate (b) (yield: 78 %).

C48H32N2O: calc'd : M+ 625.25 foun'd : 625.29

¹H NMR (500 MHz, CDCl₃) δ= 7.86(dd, 1H), 7.79-7.76(m, 2H), 7.73-7.70(m, 3H), 7.65-7.61(m, 2H), 7.55-7.28(m, 18H), 7.24-7.21(m, 2H), 6.70-6.65(m, 3H), 6.59(dd, 1H)

### Synthesis Example 22: Synthesis of Compound 251

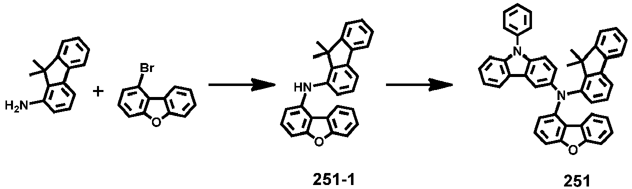

### (1) Synthesis of Intermediate 251-1

3.1 g of Intermediate 251-1 was obtained in substantially the same manner as in Synthesis of Intermediate 1-1, except that 2.1 g of 9,9-dimethyl-9H-fluoren-1-amine was utilized instead of Intermediate (b), and 2.5 g of 1-bromodibenzo[b,d]furan was utilized instead of 5 g of Intermediate (a) (yield: 83 %).

C27H21NO: calc'd : M+ 375.16 foun'd : 375.19

### (2) Synthesis of Compound 251

4 g of Compound 251 was obtained in substantially the same manner as in Synthesis of Intermediate 1-1, except that 3.1 g of Intermediate 251-1 was utilized instead of Intermediate (b), and 3.1 g of 3-iodo-9-phenyl-9H-carbazole was utilized instead of Intermediate (a) (yield: 78 %).

C45H32N2O: calc'd : M+ 616.25 foun'd : 616.29

¹H NMR (500 MHz, CDCl₃) δ= 8.22(d, 1H), 7.82(d,1 H), 7.77(td, 1H), 7.70(dd, 1H), 7.54-7.44(m, 7H), 7.39-7.22(m, 10H), 7.11(dd, 1H), 6.96(dd, 1H), 6.57-6.47(m, 3H), 1.68(s, 6H)

### Synthesis Example 23: Synthesis of Compound 253

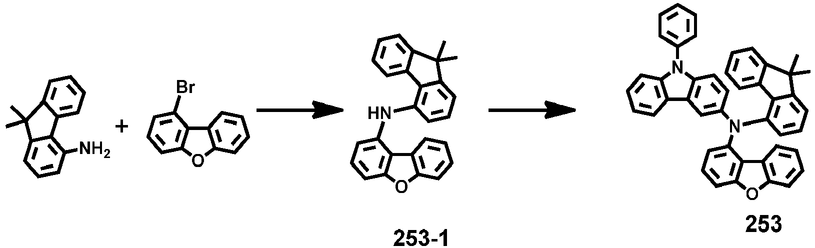

### (1) Synthesis of Intermediate 253-1

3.5 g of Intermediate 253-1 was obtained in substantially the same manner as in Synthesis of Intermediate 1-1, except that 2.1 g of 9,9-dimethyl-9H-fluoren-4-amine was utilized instead of Intermediate (b), and 2.5 g of 1-bromodibenzo[b,d]furan was utilized instead of 5 g of Intermediate (a) (yield: 92 %).

C27H21NO: calc'd : M+ 375.16 foun'd : 375.19

### (2) Synthesis of Compound 253

4.6 g of Compound 253 was obtained in substantially the same manner as in Synthesis of Intermediate 1-1, except that 3.5 g of Intermediate 253-1 was utilized instead of Intermediate (b), and 3.6 g of 3-iodo-9-phenyl-9H-carbazole was utilized instead of Intermediate (a) (yield: 81 %).

C45H32N2O: calc'd : M+ 616.25 foun'd : 616.29

¹H NMR (500 MHz, CDCl₃) δ= 8.22(d, 1H), 7.82(d,1 H), 7.72(d, 1H), 7.63(dd,1 H), 7.51-7.21(m, 16H), 7.14-7.07(m, 2H), 6.86(dd, 1H), 6.61-6.59(m, 1H), 6.53(dd, 1H), 6.24(td, 1H), 1.61(s, 6H)

### Synthesis Example 24: Synthesis of Compound 256

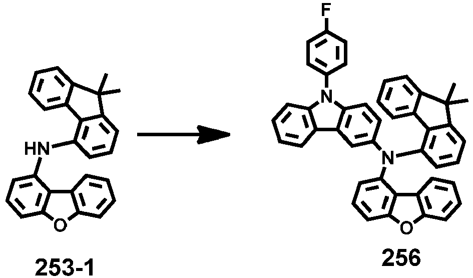

5.0 g of Compound 256 was obtained in substantially the same manner as in Synthesis of Intermediate 1-1, except that 3.5 g of Intermediate 253-1 was utilized instead of Intermediate (b), and 3.2 g of 3-bromo-9-(4-fluorophenyl)-9H-carbazole was utilized instead of Intermediate (a) (yield: 84 %).

C45H31FN2O: calc'd : M+ 634.24 foun'd : 634.25

¹H NMR (500 MHz, CDCl₃) δ= 8.23(d, 1H), 7.82(d, 1H), 7.72(td,1 H), 7.62(dd, 1H), 7.58-7.35(m, 3H), 7.34-7.21(m, 10H), 7.14-7.03(m, 4^{H}), 6.86(dd, 1H), 6.61(m, 1H), 6.53(dd,1 H), 6.24(dd, 1H, 1.61(s, 6H)

### Synthesis Example 25: Synthesis of Compound 268

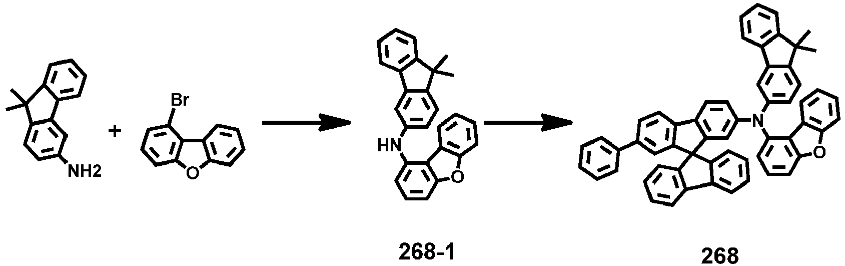

### (1) Synthesis of Intermediate 268-1

3.2 g of Intermediate 268-1 was obtained in substantially the same manner as in Synthesis of Intermediate 1-1, except that 2.1 g of 9,9-dimethyl-9H-fluoren-2-amine was utilized instead of Intermediate (b), and 2.5 g of 1-bromodibenzo[b,d]furan was utilized instead of 5 g of Intermediate (a) (yield: 85 %).

C27H21NO: calc'd : M+ 375.16 foun'd : 375.18

### (2) Synthesis of Compound 268

5.7 g of Compound 268 was obtained in substantially the same manner as in Synthesis of Intermediate 1-1, except that 3.2 g of Intermediate 268-1 was utilized instead of Intermediate (b), and 4.0 g of Intermediate 213-2 was utilized instead of Intermediate (a) (yield: 88 %).

C58H39NO: calc'd : M+ 765.30 foun'd : 765.32

¹H NMR (500 MHz, CDCl₃) δ= 7.93(dd, 2H), 7.79-7.66(m, 6H). 7.62(d, 1H), 7.53-7.29(m, 11H), 7.20(dt,2 H), 7.11-7.04(m, 4H), 6.89(d,1H), 6.87(t, 1H), 6.75(t, 1H), 6.74(d, 1H), 6.66(dd, 1H), 6.42(dd,1 H), 5.68(d,1 H), 1.52(s, 6H)

### Synthesis Example 26: Synthesis of Compound 269

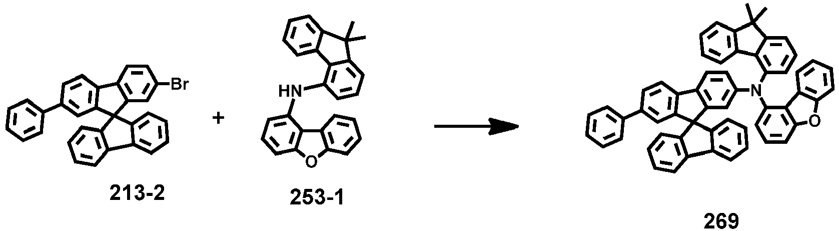

4.8 g of Compound 269 was obtained in substantially the same manner as in Synthesis of Intermediate 1-1, except that 3.2 g of Intermediate 253-1 was utilized instead of Intermediate (b), and 4.0 g of Intermediate 213-2 was utilized instead of Intermediate (a) (yield: 74 %).

C58H39NO: calc'd : M+ 765.30 foun'd : 765.32

¹H NMR (500 MHz, CDCl₃) δ= 7.93(d,2H), 7.82(d, 1H), 7.80(d, 1H), 7.72-7.58(m, 5H), 7.53-7.36(m, 7H), 7.33-7.05(m, 9H), 6.87-6.84(m, 2H), 6.79(dd,1 H), 6.75(dd,2 H), 6.61(m, 1H), 6.23(d, 1H), 5.49(d, 1H), 1.61(s, 6H)

### Synthesis Example 27: Synthesis of Compound 278

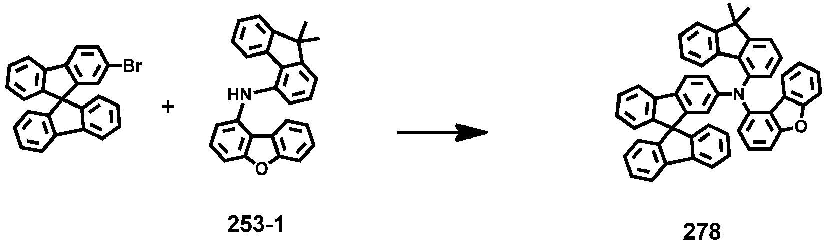

4.3 g of Compound 278 was obtained in substantially the same manner as in Synthesis of Intermediate 1-1, except that 3.2 g of Intermediate 253-1 was utilized instead of Intermediate (b), and 3.8 g of 2-bromo-9,9'-spirobi[fluorene] was utilized instead of Intermediate (a) (yield: 86 %).

C52H35NO: calc'd : M+ 689.27 foun'd : 689.28

¹H NMR (500 MHz, CDCl₃) δ= 7.92(d, 2H), 7.89(d, 1H), 7.82(d,1 H), 7.72(d, 1H), 7.62(dd, 1H), 7.59(d,1 H), 7.48-7.41(m, 4H), 7.32-7.06(m, 10H), 6.87(d, 1H), 6.81(dd,1 H), 6.77-6.73(m, 3H), 6.61(m,1 H), 6.23(d, 1H), 5.72(dd, 1H), 1.61(s, 6H)

### Synthesis Example 28: Synthesis of Compound 281

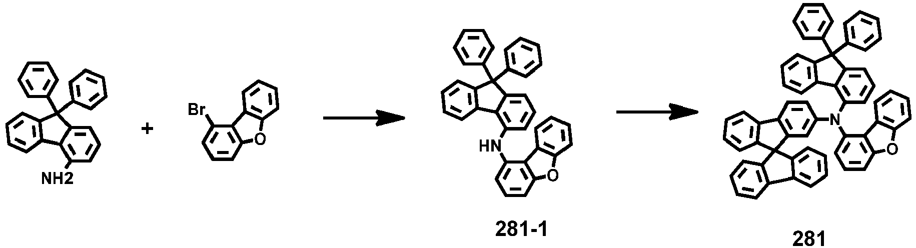

### (1) Synthesis of Intermediate 281-1

3.8 g of Intermediate 281-1 was obtained in substantially the same manner as in Synthesis of Intermediate 1-1, except that 3.3 g of 9,9-diphenyl-9H-fluoren-4-amine was utilized instead of Intermediate (b), and 2.5 g of 1-bromodibenzo[b,d]furan was utilized instead of 5 g of Intermediate (a) (yield: 77 %).

C37H25NO: calc'd : M+ 499.19 foun'd : 499.23

### (2) Synthesis of Compound 281

4.9 g of Compound 281 was obtained in substantially the same manner as in Synthesis of Intermediate 1-1, except that 3.8 g of Intermediate 281-1 was utilized instead of Intermediate (b), and 3.2 g of 2-bromo-9,9'-spirobi[fluorene] was utilized instead of Intermediate (a) (yield: 79 %).

C62H39NO: calc'd : M+ 813.30 foun'd : 813.33

¹H NMR (500 MHz, CDCl₃) δ= 7.92(d, 2H), 7.89(d,1 H), 7.82(d,1 H), 7.72(d,1 H), 7.59(dd, 2H), 7.49-7.40(m, 4H), 7.33-7.26(m, 4H), 7.21-7.05(m, 14H), 6.81-6.71(m, 6H), 6.62(m, 1H), 6.56(dd, 1H), 5.72(d, 1H), 5.68(t, 1H)

Compounds synthesized in Synthesis Examples 1 to 28 were identified by ¹H nuclear magnetic resonance (NMR). The results thereof are shown in Tables 1 and 2. Methods of synthesizing compounds other than compounds shown in Tables 1 and 2 may be easily understood to those skilled in the art by referring to the synthesis pathways and raw materials described above.

**Table 1**

| Compound | 1H NMR (400 Mhz) |
|---|---|
| 1 | 8.25(d, 1H), 7.85(d, 2H), 7.80-7.75(m,2H), 7.73-7.70(m, 1H), 7.56(d, 1H), 7.53-7.21(m, 14H), 7.15-7.09(m, 2H), 7.00(dd, 3H), 6.85(dd, 1H), 6.65(dd, 1H), 6.55(dd, 1H), 1.61(s, 6H) |
| 2 | 8.22-8.20(m, 1H), 7.86(td, 1H), 7.79-7.77(m, 1H), 7.72(m,1H), 7.58(d, 1H), 7.51-7.23(m, 15H), 7.21-7.05(m, 11H), 6.81(td, 1H), 6.73(dd, 1H), 6.65(dd, 1H), 6.53-6.48(m, 2H) |
| 3 | 8.22-8.20(m, 1H), 7.90(dd, 2H), 7.87(dd, 1H), 7.79(m, 1H), 7.72(m, 1H), 7.61(d,1H), 7.54-7.12(m, 20H), 6.92(dd, 1H), 6.77-6.73(m, 3H), 6.66(dd, 1H), 6.51(dd, 1H), 5.97(q, 1H) |
| 5 | 8.22-8.20(m, 1H), 7.85(m, 1H), 7.79-7.76(m, 2H), 7.71(m, 1H), 7.57(m, 1H), 7.46(dd, 1H), 7.40-7.21(m, 10H), 7.14-7.03(m, 4H), 7.01(dd,1H), 6.85(dd, 1H), 6.66(dd, 1H), 6.54(dd, 1H), 1.61(s, 6H) |
| 6 | 8.22-8.20(m, 1H), 7.86(td, 1H), 7.79-7.77(m, 1H), 7.72-7.70(m, 1H), 7.58(d, 1H). 7.48-7.23(m, 12H), 7.20-7.03(m, 13H), 6.81(dd, 1H), 6.73(dd, 1H), 6.66(dd, 1H), 6.53(q, 1H), 6.51(dd, 1H) |
| 21 | 7.84-7.82(m, 1H), 7.79-7.75(m, 2H), 7.73-7.70(m, 2H),7.68-7.65(m, 1H), 7.57-7.40(m, 5H), 7.34-7.25(m, 4H), 7.14-7.09(m, 2H), 7.03(dd, 1H), 6.85(m, 1H), 6.66(dd, 1H), 6.54(dd, 1H), 1.61(s, 6H) |
| 37 | 8.22-8.20(m, 1H), 7.79(m, 1H), 7.72(m, 1H), 7.54-7.21(m, 14H), 7.08-7.02(m, 2H), 6.67-6.53(m, 3H), 6.34-6.31(m, 2H) |
| 93 | 8.24-8.22(m, 1H), 8.15(m, 1H), 7.79-7.70(m, 4H), 7.67(d, 1H), 7.58-7.42(m, 8H), 7.39-7.25(m, 7H), 7.21-7.19(m, 1H), 7.14-7.09(m, 2H), 6.84(dd, 1H), 6.68(dd, 1H), 6.63-6.59(m, 2H), 6.49(m, 1H), 1.61(s, 6H) |
| 94 | 8.24-8.22(m, 1H), 8.15(m, 1H), 7.86(td, 1H), 7.79-7.70(m, 3H), 7.67(d, 1H), 7.60-7.53(m, 3H),7.50-7.25(m, 12H), 7.21-7.03(m, 12H), 6.81(td, 1H), 6.75(dd, 1H), 6.68(dd, 1H), 6.63-6.57(m, 3H), 6.44(d, 1H) |
| 95 | 8.24-8.22(m, 1H), 8.15(m, 1H), 8.02-7.87(m, 3H), 7.79-7.70(m, 3H), 7.67(d, 1H), 7.60-7.53(m, 3H),7.50-7.25(m, 14H), 7.23-7.15(m, 4H), 6.92(dd, 1H), 6.77-6.71(m, 3H), 6.68(dd, 1H), 6.63-6.57(m, 3H), 5.95(d, 1H) |
| 101 | 8.24-8.22(m, 1H), 8.15(m, 1H), 7.79-7.70(m, 4H), 7.67(d, 1H), 7.58-7.52(m, 3H), 7.48-7.44(m, 1H),7.39-7.25(m, 8H), 7.21-7.19(m, 1H), 7.14-7.03(m, 4H), 6.84(dd, 1H), 6.68(dd, 1H), 6.63-6.59(m, 2H), 6.49(m, 1H), 1.61(s, 6H) |
| 204 | 7.92-7.87(m, 3H), 7.79(m, 1H), 7.72(m,1H), 7.63-7.58(m, 3H), 7.52-7.24(m,12H), 7.21-7.15(m, 3H), 6.92(dd, 1H), 6.77-6.71(m, 3H), 6.68-6.66(dd, 1H), 6.58-6.54(m, 2H), 5.95(dd, 1H) |

**Table 2**

| Compound | 1H NMR (sooMhz) |
|---|---|
| 4 | 8.22(m, 1H), 7.79(dd, 2H), 7.72(dd, 1H), 7.67(d, 1H), 7.54-7.14(m, 17H), 6.95(d, 1H), 6.90(dd, 1H), 6.66(dd, 1H),. 6.54(dd, 1H), 2.19-2.08(m, 2H), 1.95-1.72(m, 6H) |
| 8 | 8.22(m, 1H), 7.79(dd, 2H), 7.72(dd, 1H), 7.67(d, 1H), 7.48-7.23(m, 13H), 7.18-7.14(m, 1H), 7.09-7.04(m, 2H), 6.95(d, 1H), 6.90(dd, 1H), 6.66(dd, 1H),. 6.54(dd, 1H), 2.19-2.08(m, 2H), 1.95-1.72(m, 6H) |
| 16 | 8.22(m, 1H), 7.79(dd, 2H), 7.72(dd, 1H), 7.67(d, 1H), 7.63-7.59(m, 2H), 7.57-7.23(m, 18H), 7.18(dt, 1H), 6.95(d, 1H), 6.90(dd, 1H), 6.66(dd, 1H), 6.59(dd, 1H), 2.19-2.08(m, 2H), 1.95-1.72(m, 6H) |
| 187 | 7.92-7.84(m, 4H), 7.79(m, 1H), 7.72(dd, 1H), 7.61(d, 1H), 7.58(d, 1H), 7.48-7.41(m, 5H), 7.36-7.28(m, 3H), 7.21-7.06(m, 14H), 6.92(dd, 1H), 6.81-6.72(m, 5H), 6.66(dd, 1H), 6.57(d, 1H), 6.43(d, 1H) |
| 188 | 7.92(dd, 4H), 7.89(dd, 2H), 7.79(td, 1H), 7.72(dd, 1H), 7.61(d, 2H), 7.48-7.41(m, 7H), 7.36-7.26(m, 3H), 7.21-7.15(m, 6H), 6.92(dd, 2H), 6.77-6.73(m, 6H), 6.68(dd, 1H), 6.43(d, 2H) |
| 210 | 7.92(dd, 2H), 7.89(dd, 1H), 7.80(d, 1H), 7.72(d, 1H), 7.59-7.51(m, 5H), 7.49-7.41(m, 5H), 7.32-7.27(m, 3H), 7.21-7.14(m, 5H), 7.01(t, 1H), 6.77-6.73(m, 4H), 6.66(dd, 1H), 6.61(dd, 1H),5.47(d, 1H) |
| 213 | 7.93(dd, 2H), 7.79-7.78(m, 2H), 7.72-7.59(m, 6H), 7.54-7.28(m, 15H), 7.20(t, 2H), 6.89(m, 2H), 6.75(dd, 2H), 6.68(dd, 1H), 6.56-6.54(m, 2H), 5.68(d, 1H) |
| 219 | 7.93(dd, 2H), 7.79-7.67(m, 6H), 7.62(d, 1H), 7.53-7.28(m, 11H), 7.18(dt, 2H), 7.11(dd, 2H), 6.89-6.82(m, 3H), 6.75(dd, 2H), 6.68(dd, 1H), 6.50(d, 1H). 6.10(d, 1H), 1.61(s, 6H) |
| 240 | 7.86(dd, 1H), 7.79-7.76(m, 2H), 7.73-7.70(m, 3H), 7.65-7.61(m, 2H), 7.55-7.28(m, 18H), 7.24-7.21(m, 2H), 6.70-6.65(m, 3H), 6.59(dd, 1H) |
| 251 | 8.22(d, 1H), 7.82(d,1 H), 7.77(td, 1H), 7.70(dd, 1H), 7.54-7.44(m, 7H), 7.39-7.22(m, 10H), 7.11(dd, 1H), 6.96(dd, 1H), 6.57-6-47(m, 3H), 1.68(s, 6H) |
| 253 | 8.22(d, 1H), 7.82(d,1 H), 7.72(d, 1H), 7.63(dd,1 H), 7.51-7.21(m, 16H), 7.14-7.07(m, 2H), 6.86(dd, 1H), 6.61-6.59(m, 1H), 6.53(dd, 1H), 6.24(td, 1H), 1.61(s, 6H) |
| 256 | 8.23(d, 1H), 7.82(d, 1H), 7.72(td,1 H), 7.62(dd, 1H), 7.58-7.35(m, 3H), 7.34-7.21(m, 10H), 7.14-7.03(m, 4H), 6.86(dd, 1H), 6.61(m, 1H), 6.53(dd,1 H), 6.24(dd, 1H, 1.61(s, 6H) |
| 268 | 7.93(dd, 2H), 7.79-7.66(m, 6H). 7.62(d, 1H), 7.53-7.29(m, 11H), 7.20(dt,2 H), 7.11-7.04(m, 4H), 6.89(d,1 H), 6.87(t, 1H), 6.75(t, 1H), 6.74(d, 1H), 6.66(dd, 1H), 6.42(dd,1 H), 5.68(d,1 H), 1.52(s, 6H) |
| 269 | 7.93(d, 2H), 7.82(d, 1H), 7.80(d, 1H), 7.72-7.58(m, 5H), 7.53-7.36(m, 7H), 7.33-7.05(m, 9H), 6.87-6.84(m, 2H), 6.79(dd,1 H), 6.75(dd,2 H), 6.61(m, 1H), 6.23(d, 1H), 5.49(d, 1H), 1.61(s, 6H) |
| 278 | 7.92(d, 2H), 7.89(d, 1H), 7.82(d,1 H), 7.72(d, 1H), 7.62(dd, 1H), 7.59(d,1 H), 7.48-7.41(m, 4H), 7.32-7.06(m, 10H), 6.87(d, 1H), 6.81(dd,1 H), 6.77-6.73(m, 3H), 6.61(m,1 H), 6.23(d, 1H), 5.72(dd, 1H), 1.61(s, 6H) |
| 281 | 7.92(d, 2H), 7.89(d,1 H), 7.82(d,1 H), 7.72(d,1 H), 7.59(dd, 2H), 7.49-7.40(m, 4H), 7.33-7.26(m, 4H), 7.21-7.05(m, 14H), 6.81-6.71(m, 6H), 6.62(m, 1H), 6.56(dd, 1H), 5.72(d, 1H), 5.68(t, 1H) |

### Examples

### Comparative Example 1

A Corning 15 Ohms per square centimeter (Ω/cm²) (1,200 Å) ITO glass substrate was cut to a size of 50 millimeters (mm)×50 mm×0.7 mm, sonicated in isopropyl alcohol and pure water for 5 minutes in each solvent, and cleaned by exposure to ultraviolet rays and ozone for 30 minutes to utilize the glass substrate as an anode. Then, the glass substrate was mounted to a vacuum-deposition apparatus.

2-TNATA was vacuum-deposited on the glass substrate to form a hole injection layer having a thickness of 600 Å. Thereafter, 4,4'-bis[N-(1-naphthyl)-N-phenylamino]biphenyl (hereinafter referred to as "NPB"), which is a hole transport material, as a hole transporting compound was vacuum-deposited on the hole injection layer to form a hole transport layer having a thickness of 300 Å.

9,10-di(naphthalen-2-yl)anthracene (hereinafter referred to as "DNA"), which is a known blue fluorescent host, and 4,4'-bis[2-(4-(N,N-diphenylamino)phenyl)vinyl]biphenyl (hereinafter referred to as "DPAVBi"), which is a known blue fluorescent dopant, were co-deposited on the hole transport layer in a weight ratio of about 98:2 to form an emission layer having a thickness of 300 Å.

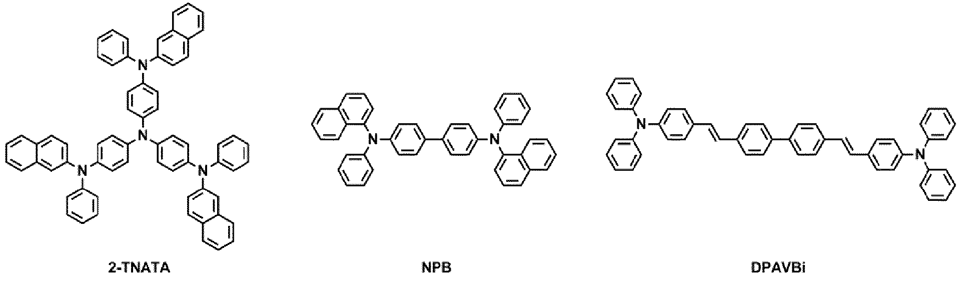

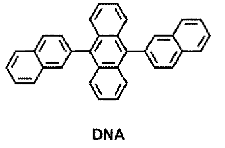

Subsequently, Alq₃ was deposited on the emission layer to form an electron transport layer having a thickness of 300 Å. Subsequently, LiF, which is halogenated alkali metal, was deposited on the electron transport layer to form an electron injection layer having a thickness of 10 Å. Finally, Al was vacuum-deposited on the electron injection layer to form a cathode having a thickness of 3,000 Å to form a LiF/Al electrode, thereby completing the manufacture of an organic light-emitting device.

### Examples 1 to 28

An organic light-emitting device was manufactured substantially in the same manner as in Comparative Example 1, except that Compounds shown in Table 3 were utilized instead of NPB in the formation of a hole transport layer.

### Comparative Example 2

An organic light-emitting device was manufactured substantially in the same manner as in Comparative Example 1, except that Compound A was utilized instead of NPB in the formation of a hole transport layer.

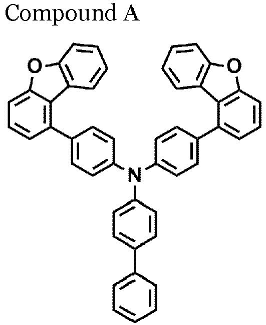

### Comparative Example 3

An organic light-emitting device was manufactured substantially in the same manner as in Comparative Example 1, except that Compound B was utilized instead of NPB in the formation of a hole transport layer.

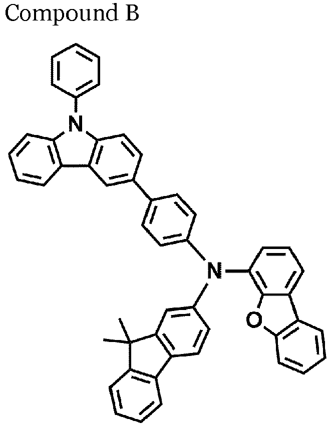

### Comparative Example 4

An organic light-emitting device was manufactured substantially in the same manner as in Comparative Example 1, except that Compound C was utilized instead of NPB in the formation of a hole transport layer.

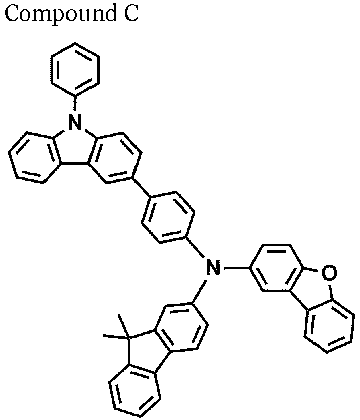

### Comparative Example 5

An organic light-emitting device was manufactured in substantially the same manner as in Comparative Example 1, except that Compound D was utilized instead of NPB in the formation of a hole transport layer.

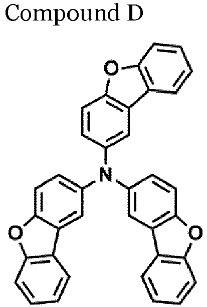

### Comparative Example 6

An organic light-emitting device was manufactured in substantially the same manner as in Comparative Example 1, except that Compound E was utilized instead of NPB in the formation of a hole transport layer.

### Compound E

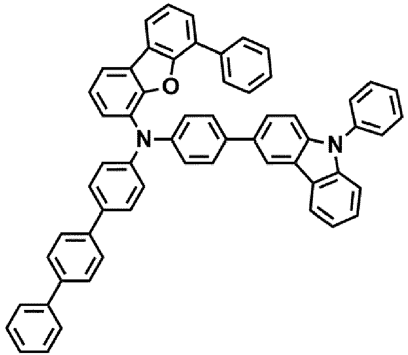

### Comparative Example 7

An organic light-emitting device was manufactured in substantially the same manner as in Comparative Example 1, except that Compound F was utilized instead of NPB in the formation of a hole transport layer.

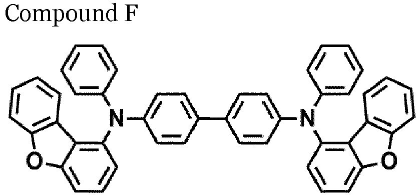

### Comparative Example 8

An organic light-emitting device was manufactured in substantially the same manner as in Comparative Example 1, except that Compound G was utilized instead of NPB in the formation of a hole transport layer.

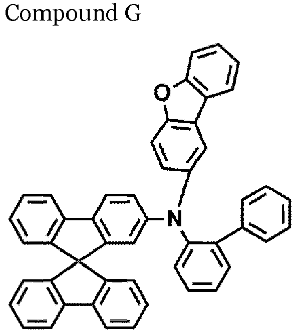

### Comparative Example 9

An organic light-emitting device was manufactured in substantially the same manner as in Comparative Example 1, except that Compound H was utilized instead of NPB in the formation of a hole transport layer.

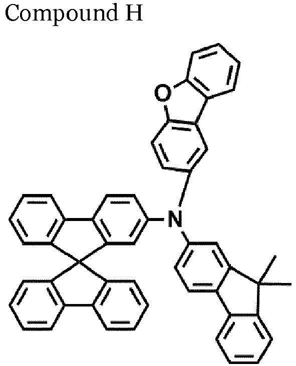

The performances (driving voltage, luminance, efficiency, and color-coordinate) of the organic light-emitting devices manufactured in Examples 1 to 28 and Comparative Examples 1 to 9 while driving at a current density of 50 mA/cm² were evaluated. The half lifespan was also measured at a current density of 100 mA/cm², which indicates time (hour) for the luminance of each organic light-emitting device to decline to 50% of its initial luminance. The evaluation results are shown in Table 3.

The luminance was measured utilizing a luminance meter PR650 powered by a current voltmeter (Keithley SMU 236).

The efficiency was measured utilizing a luminance meter PR650 powered by a current voltmeter (Keithley SMU 236).

**Table 3**

| | Hole transport material | Driving voltage (V) | Current density (mA/cm2) | Luminance (cd/ m2) | Efficiency (cd/A) | Emission color | Half lifespan (hr @100 mA/cm2) |
|---|---|---|---|---|---|---|---|
| Example 1 | Compound 1 | 4.32 | 50 | 3670 | 7.34 | Blue | 362 |
| Example 2 | Compound 2 | 4.21 | 50 | 3715 | 7.43 | Blue | 353 |
| Example 3 | Compound 3 | 4.22 | 50 | 3665 | 7.33 | Blue | 372 |
| Example 4 | Compound 4 | 4.85 | 50 | 3876 | 7.75 | Blue | 374 |
| Example 5 | Compound 5 | 4.26 | 50 | 3730 | 7.46 | Blue | 374 |
| Example 6 | Compound 6 | 4.26 | 50 | 3730 | 7.46 | Blue | 374 |
| Example 7 | Compound 8 | 4.67 | 50 | 3985 | 7.97 | Blue | 356 |
| Example 8 | Compound 16 | 4.72 | 50 | 3451 | 6.90 | Blue | 382 |
| Example 9 | Compound 21 | 4.25 | 50 | 3630 | 7.26 | Blue | 384 |
| Example 10 | Compound 37 | 4.41 | 50 | 3725 | 7.45 | Blue | 343 |
| Example 11 | Compound9 3 | 4.26 | 50 | 3630 | 7.26 | Blue | 366 |
| Example 12 | Compound 94 | 4.32 | 50 | 3670 | 7.34 | Blue | 378 |
| Example 13 | Compound 95 | 4.45 | 50 | 3440 | 6.88 | Blue | 325 |
| Example 14 | Compound 101 | 4.60 | 50 | 3490 | 6.98 | Blue | 330 |
| Example 15 | Compound 187 | 4.55 | 50 | 3655 | 7.31 | Blue | 375 |
| Example 16 | Compound 188 | 4.69 | 50 | 3492 | 6.98 | Blue | 356 |
| Example 17 | Compound 204 | 4.82 | 50 | 3540 | 7.08 | Blue | 381 |
| Example 18 | Compound 210 | 4.96 | 50 | 3987 | 7.97 | Blue | 351 |
| Example 19 | Compound 213 | 4.74 | 50 | 3859 | 7.72 | Blue | 348 |
| Example 20 | Compound 219 | 4.73 | 50 | 3565 | 7.13 | Blue | 316 |
| Example 21 | Compound 240 | 4.89 | 50 | 3529 | 7.06 | Blue | 355 |
| Example 22 | Compound 251 | 4.82 | 50 | 3983 | 7.97 | Blue | 327 |
| Example 23 | Compound 253 | 4.76 | 50 | 3852 | 7.70 | Blue | 331 |
| Example 24 | Compound 256 | 4.96 | 50 | 3874 | 7.75 | Blue | 326 |
| Example 25 | Compound 268 | 4.81 | 50 | 3721 | 7.44 | Blue | 312 |
| Example 26 | Compound 269 | 4.53 | 50 | 3652 | 7.30 | Blue | 332 |
| Example 27 | Compound 278 | 4.56 | 50 | 3552 | 7.10 | Blue | 351 |
| Example 28 | Compound 281 | 4.66 | 50 | 3512 | 7.02 | Blue | 366 |
| Comparati ve Example 1 | NPB | 7.01 | 50 | 2645 | 5.29 | Blue | 258 |
| Comparati ve Example 2 | Compound A | 6.50 | 50 | 2805 | 5.61 | Blue | 220 |
| Comparati ve Example 3 | Compound B | 5.81 | 50 | 3105 | 6.21 | Blue | 180 |
| Comparati ve Example 4 | Compound C | 6.02 | 50 | 3205 | 6.41 | Blue | 210 |
| Comparati ve Example 5 | Compound D | 8.21 | 50 | 1851 | 3.70 | Blue | 127 |
| Comparati ve Example 6 | Compound E | 6.01 | 50 | 2353 | 5.98 | Blue | 231 |
| Comparati ve Example 7 | Compound F | 7.32 | 50 | 2553 | 5.90 | Blue | 221 |
| Comparati ve Example 8 | Compound G | 5.88 | 50 | 2678 | 5.34 | Blue | 231 |
| Comparati ve Example 9 | Compound H | 5.68 | 50 | 2635 | 5.27 | Blue | 235 |

As apparent from Table 3, when the compound according to one or more embodiments is utilized as a hole transport material in organic light-emitting devices, the organic light-emitting devices of the Examples comprising the compound according to one or more embodiments were found to have improved driving voltage, excellent I-V-L characteristics with improved efficiency, and particularly, significant improvement of lifespan due to its lifespan improving effects, as compared with the organic light-emitting device of the Comparative Example 1 comprising NPB. In addition, even in comparison with Comparative Examples 2 to 9, in which Compounds A, B, C, D, E, F, G, and H were respectively utilized, the organic light-emitting device of the Examples were found to have improved driving voltage, improved luminance, improved luminescence efficiency, and improved half lifespan.

As apparent from the foregoing description, an organic light-emitting device comprising the amine-based compound may have a low driving voltage, high efficiency, long lifespan, and high maximum quantum efficiency.

It should be understood that embodiments described herein should be considered in a descriptive sense only and not for purposes of limitation.

While one or more embodiments have been described with reference to the figures, it will be understood by those of ordinary skill in the art that various changes in form and details may be made therein without departing from the scope as defined by the following claims, with due account being taken of equivalents thereof.

## Claims

1. An organic light-emitting device comprising:
a first electrode;
a second electrode facing the first electrode; and
an organic layer between the first electrode and the second electrode, and comprising an emission layer,
wherein the organic layer comprises at least one amine-based compound represented by Formula 1D: 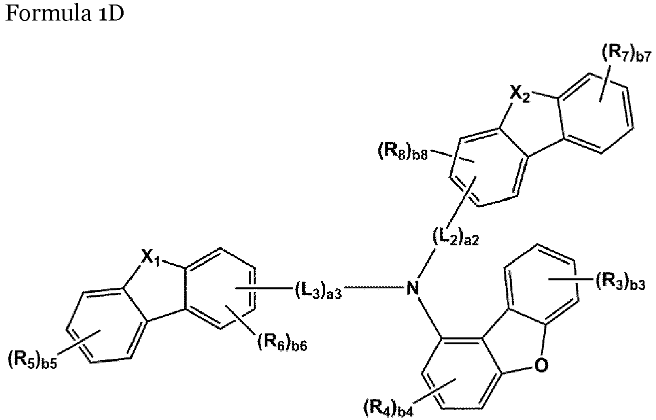
wherein, in Formula 1D,
X₁ is N-(L₁)ₐ₁-(R₁)_{b1},
X₂ is O, or S,
L₁ and L₂ are each independently selected from a single bond, a benzene group, a pentalene group, an indene group, a naphthalene group, an azulene group, a heptalene group, an indacene group, an acenaphthalene group, a phenalene group, a phenanthrene group, an anthracene group, a fluoranthene group, a triphenylene group, a pyrene group, a chrysene group, a naphthacene group, a picene group, a perylene group, a pentaphenylene group, a hexacene group, a pyrrole group, an imidazole group, a pyrazole group, a pyridine group, a pyrazine group, a pyrimidine group, a pyridazine group, an isoindole group, an indole group, an indazole group, a purine group, a quinoline group, an isoquinoline group, a benzoquinoline group, a phthalazine group, a naphthyridine group, a quinoxaline group, a quinazoline group, a cinnoline group, a carbazole group, a phenanthridine group, an acridine group, a phenanthroline group, a phenazine group, a benzimidazole group, a furan group, a benzofuran group, a thiophene group, a benzothiophene group, a thiazole group, an isothiazole group, a benzothiazole group, an iso-oxazole group, an oxazole group, a triazole group, a tetrazole group, an oxadiazole group, a triazine group, a benzoxazole group, a dibenzofuran group, a dibenzothiophene group, a benzocarbazole group, and a dibenzocarbazole group; and
a benzene group, a pentalene group, an indene group, a naphthalene group, an azulene group, a heptalene group, an indacene group, an acenaphthalene group, a phenalene group, a phenanthrene group, an anthracene group, a fluoranthene group, a triphenylene group, a pyrene group, a chrysene group, a naphthacene group, a picene group, a perylene group, a pentaphenylene group, a hexacene group, a pyrrole group, an imidazole group, a pyrazole group, a pyridine group, a pyrazine group, a pyrimidine group, a pyridazine group, an isoindole group, an indole group, an indazole group, a purine group, a quinoline group, an isoquinoline group, a benzoquinoline group, a phthalazine group, a naphthyridine group, a quinoxaline group, a quinazoline group, a cinnoline group, a carbazole group, a phenanthridine group, an acridine group, a phenanthroline group, a phenazine group, a benzimidazole group, a furan group, a benzofuran group, a thiophene group, a benzothiophene group, a thiazole group, an isothiazole group, a benzothiazole group, an iso-oxazole group, an oxazole group, a triazole group, a tetrazole group, an oxadiazole group, a triazine group, a benzoxazole group, a dibenzofuran group, a dibenzothiophene group, a benzocarbazole group, and a dibenzocarbazole group, each substituted with at least one selected from deuterium, -F, -Cl, -Br, -I, a hydroxyl group, a cyano group, a nitro group, an amino group, an amidino group, a hydrazino group, a hydrazono group, a C₁-C₂₀ alkyl group, a C₁-C₂₀ alkoxy group, a C₆-C₂₀ aryl group, a C₁-C₂₀ heteroaryl group, and -Si(Q₃₁)(Q₃₂)(Q₃₃),
L₃ is a single bond,
a1 and a2 are each independently an integer from 1 to 5, a3 is 1;
R₁ is independently selected from groups represented by Formulae 5-1 to 5-9, 5-13 to 5-19, and 5-21 to 5-79: 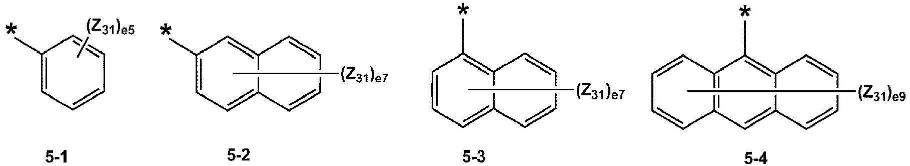 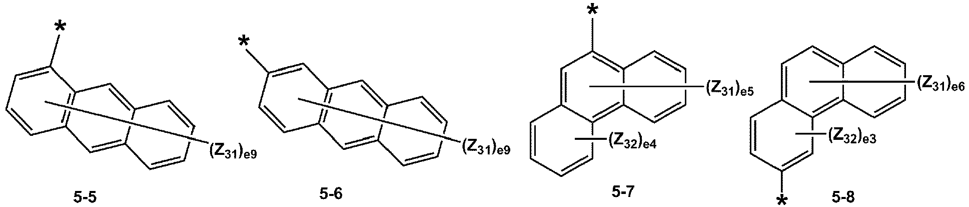 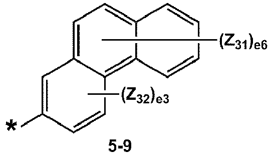 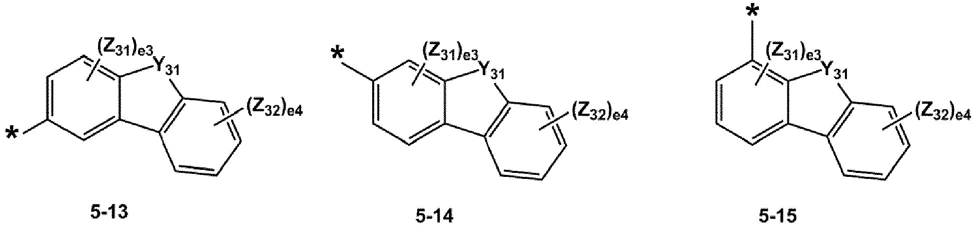 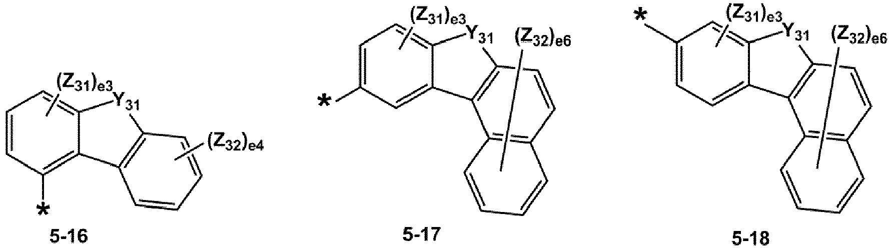 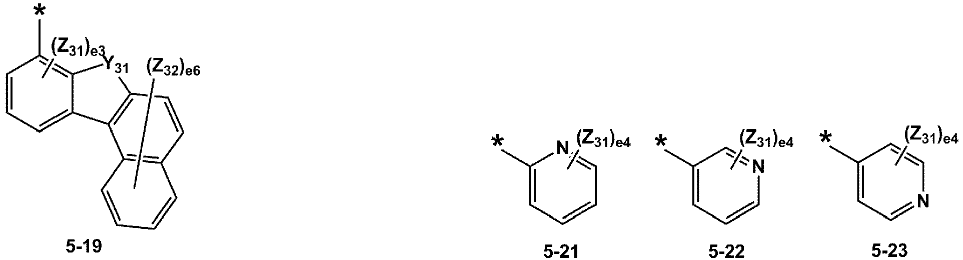 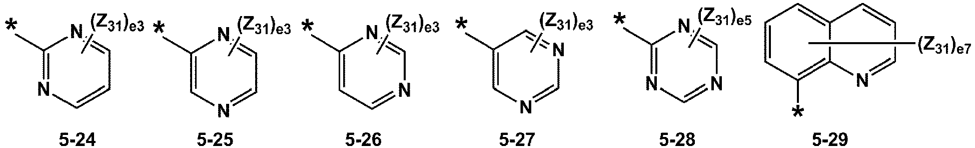 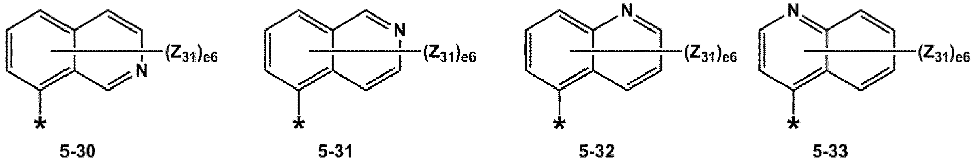 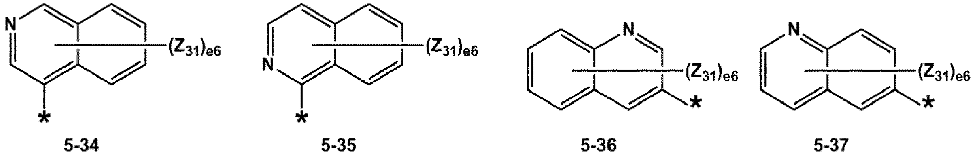 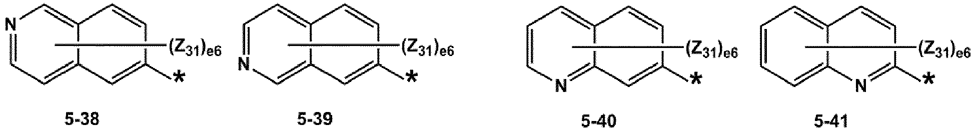 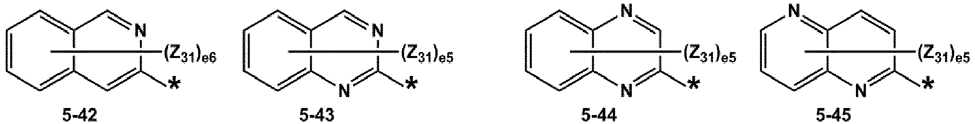 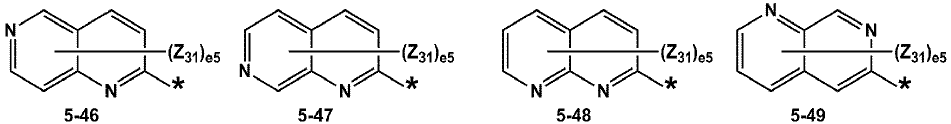 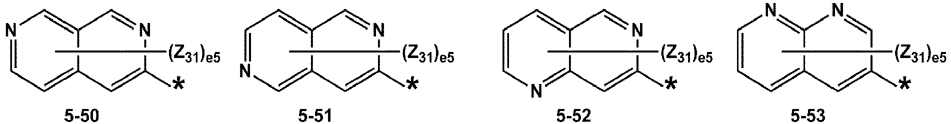 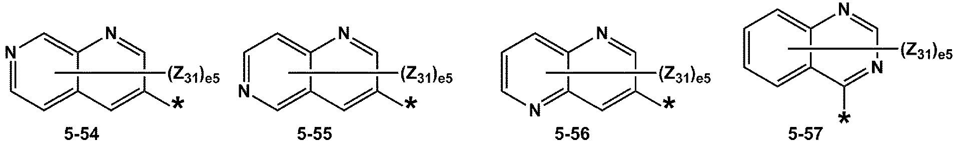 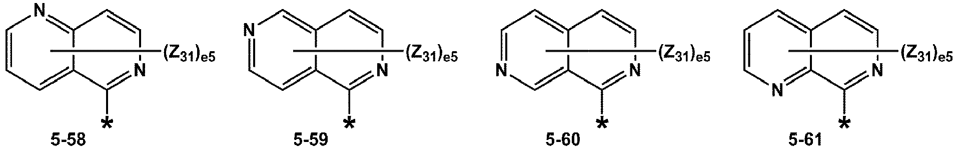 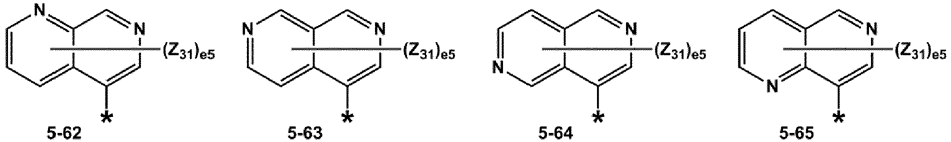 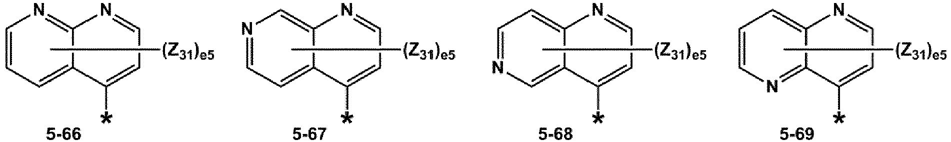 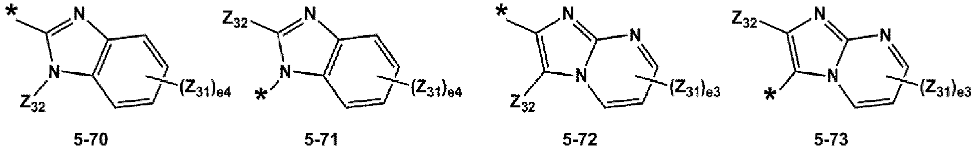 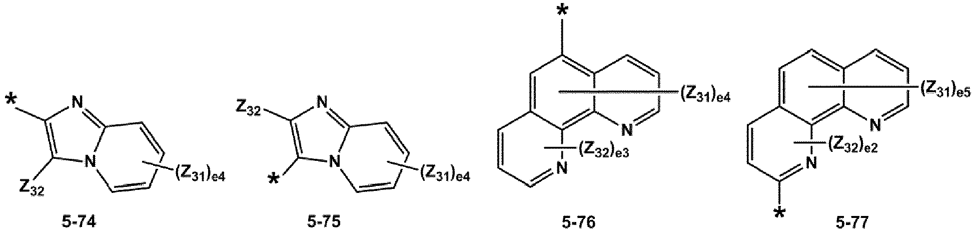 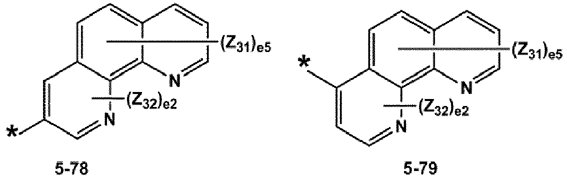 wherein, in Formulae 5-1 to 5-9, 5-13 to 5-19, and 5-21 to 5-79,
Y₃₁ is O, S, N(Z₃₅), or Si(Z₃₆)(Z₃₇),
Z₃₁, Z₃₂ and Z₃₅ to Z₃₇ are each independently selected from hydrogen, deuterium, - F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a naphthyl group, a phenalenyl group, a phenanthrenyl group, an anthracenyl group, a fluoranthenyl group, a triphenylenyl group, a pyridinyl group, a pyrazinyl group, a pyrimidinyl group, a quinolinyl group, an isoquinolinyl group, a benzoquinolinyl group, a naphthyridinyl group, a quinoxalinyl group, a quinazolinyl group, a carbazolyl group, a phenanthridinyl group, an acridinyl group, a phenanthrolinyl group, a phenazinyl group, a triazinyl group, a dibenzofuranyl group, a dibenzothiophenyl group, and -Si(Q₃₁)(Q₃₂)(Q₃₃),
e2 is an integer from 0 to 2; when e2 is 2, two Z₃₁ groups are identical to or different from each other, and two Z₃₂ groups are identical to or different from each other,
e3 is an integer from 0 to 3; when e3 is 2 or greater, at least two Z₃₁ groups are identical to or different from each other and at least two Z₃₂ groups are identical to or different from each other,
e4 is an integer from 0 to 4; when e4 is 2 or greater, at least two Z₃₁ groups are identical to or different from each other and at least two Z₃₂ groups are identical to or different from each other,
e5 is an integer from 0 to 5; when e5 is 2 or greater, at least two Z₃₁ groups are identical to or different from each other and at least two Z₃₂ groups are identical to or different from each other,
e6 is an integer from 0 to 6; when e6 is 2 or greater, at least two Z₃₁ groups are identical to or different from each other and at least two Z₃₂ groups are identical to or different from each other,
e₇ is an integer from 0 to 7; when e7 is 2 or greater, at least two Z₃₁ groups are identical to or different from each other,
e9 is an integer from 0 to 9; when e9 is 2 or greater, at least two Z₃₁ groups are identical to or different from each other, and
^{∗} indicates a binding site to an adjacent atom,
R₃ to R₈ are each independently selected from hydrogen, deuterium, -F, -Cl, -Br, -I, a hydroxyl group, a cyano group, a nitro group, an amino group, an amidino group, a hydrazino group, a hydrazono group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₁-C₆₀ heteroaryloxy group, a substituted or unsubstituted monovalent non-aromatic condensed C8 to C60 polycyclic group, a substituted or unsubstituted monovalent non-aromatic condensed 8- to 60-membered heteropolycyclic group, -Si(Q₁)(Q₂)(Q₃), -B(Q₁)(Q₂), and -C(=O)(Q₁),
b1 is an integer from 1 to 10,
b3, b5, and b7 are each independently an interger from 1 to 4,
b4, b6, and b8 are each independently an integer from 1 to 3, and
the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted C₁-C₆₀ heteroaryloxy group, the substituted monovalent non-aromatic condensed C8 to C60 polycyclic group, and the substituted monovalent non-aromatic condensed 8- to 60-membered heteropolycyclic group are each independently substituted with one or more substituents independently selected from:
deuterium, -F, -Cl, -Br, -I, a hydroxyl group, a cyano group, a nitro group, an amino group, an amidino group, a hydrazino group, a hydrazono group, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, -Br, -I, a hydroxyl group, a cyano group, a nitro group, an amino group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed C8 to C60 polycyclic group, a monovalent non-aromatic condensed 8- to 60-membered heteropolycyclic group, -Si(Q₁₁)(Q₁₂)(Q₁₃), -N(Q₁₁)(Q₁₂), - B(Q₁₁)(Q₁₂), -C(=O)(Q₁₁), -S(=O)₂(Q₁₁), and -P(=O)(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed C8 to C₆₀ polycyclic group, a monovalent non-aromatic condensed 8- to 60-membered heteropolycyclic group, a biphenyl group, and a terphenyl group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed C8 to C60 polycyclic group, a monovalent non-aromatic condensed 8- to 60-membered heteropolycyclic group, a biphenyl group, and a terphenyl group, each substituted with at least one selected from deuterium, -F, -Cl, -Br, -I, a hydroxyl group, a cyano group, a nitro group, an amino group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed C8 to C60 polycyclic group, a monovalent non-aromatic condensed 8- to 60-membered heteropolycyclic group, a biphenyl group, a terphenyl group, -Si(Q₂₁)(Q₂₂)(Q₂₃), - N(Q₂₁)(Q₂₂), -B(Q₂₁)(Q₂₂), -C(=O)(Q₂₁), -S(=O)₂(Q₂₁), and -P(=O)(Q₂₁)(Q₂₂); and
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and - P(=O)(Q₃₁)(Q₃₂),
wherein Q₁ to Q₃, Q₁₁ to Q₁₃, Q₂₁ to Q₂₃, and Q₃₁ to Q₃₃ are each independently selected from hydrogen, deuterium, -F, -Cl, -Br, -I, a hydroxyl group, a cyano group, a nitro group, an amino group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with a C₁-C₆₀ alkyl group, a C₁-C₆₀ heteroaryl group, a monovalent non-aromatic condensed C8 to C60 polycyclic group, a monovalent non-aromatic condensed 8- to 60-membered heteropolycyclic group, a biphenyl group, and a terphenyl group.

2. The organic light-emitting device of claim 1, wherein
the first electrode is an anode,
the second electrode is a cathode,
the organic layer further comprises a hole transport region between the first electrode and the emission layer and an electron transport region between the emission layer and the second electrode,
the hole transport region comprises a hole injection layer, a hole transport layer, an emission auxiliary layer, an electron blocking layer, or a combination thereof, and
the electron transport region comprises a hole blocking layer, an electron transport layer, an electron injection layer, or a combination thereof;
preferably wherein the hole transport region comprises the at least one amine-based compound;
more preferably wherein the hole transport region comprises the hole transport layer, and the hole transport layer comprises the at least one amine-based compound.

3. An amine-based compound represented by Formula 1D: 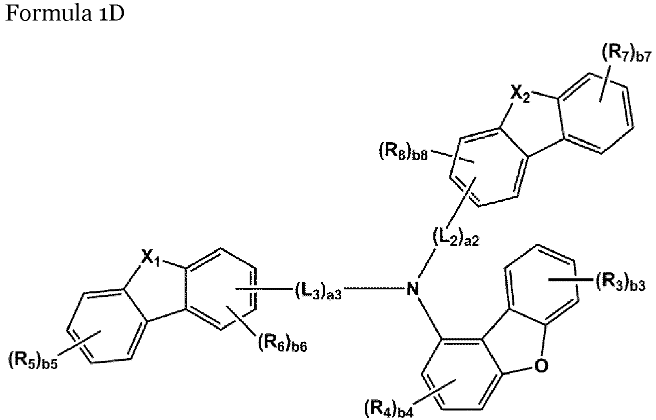 wherein, Formula 1D is as defined in claim 1.

4. The amine-based compound of any one of claims 3, wherein
(A) L₁ and L₂ are each independently selected from:
a single bond and a benzene group; and
a benzene group substituted with at least one selected from deuterium, -F, -Cl, -Br, -I, a hydroxyl group, a cyano group, a nitro group, an amino group, an amidino group, a hydrazino group, a hydrazono group, a C₁-C₂₀ alkyl group, a C₁-C₂₀ alkoxy group, a C₆-C₂₀ aryl group, a C₁-C₂₀ heteroaryl group, and -Si(Q₃₁)(Q₃₂)(Q₃₃);
L₃ is a single bond,
and/or
(B) R₃ to R₁₂ are each independently selected from hydrogen, deuterium, -F, - Cl, -Br, -I, a methyl group, an ethyl group, a propyl group, an iso-propyl group, an n-butyl group, an isobutyl group, a sec-butyl group, a ter-butyl group, a pentyl group, an iso-amyl group, a hexyl group, a phenyl group, and a biphenyl group.

5. The amine-based compound of any one of claims 3 to 4, wherein R₁ is selected from groups represented by Formulae 6-1 to 6-10 and 6-12 to 6-26: 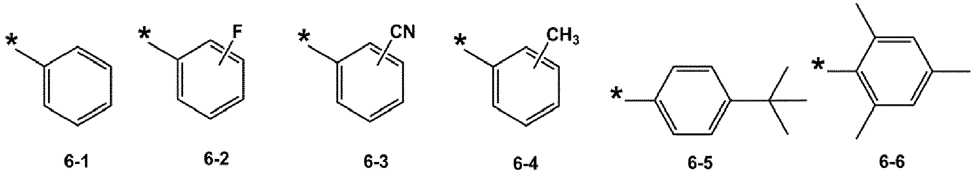 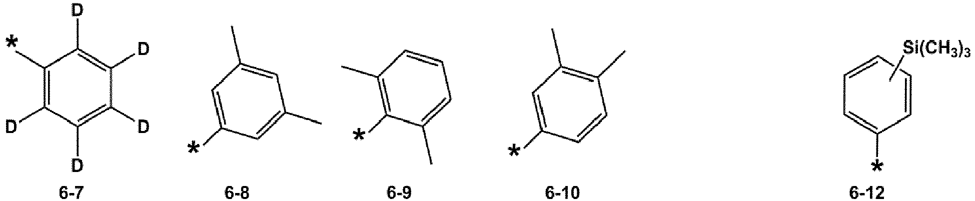 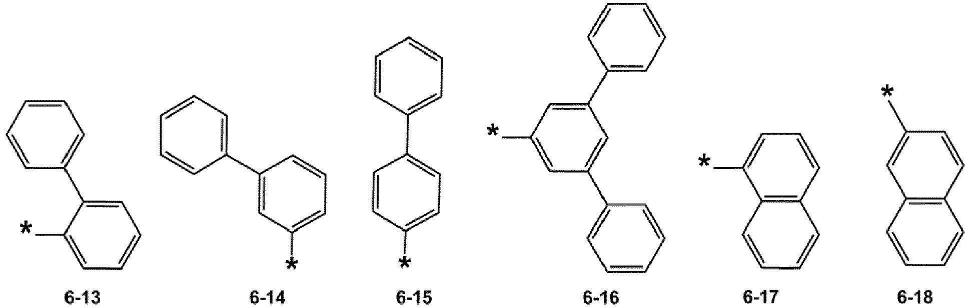 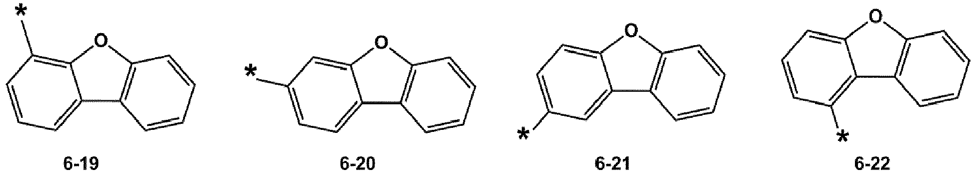 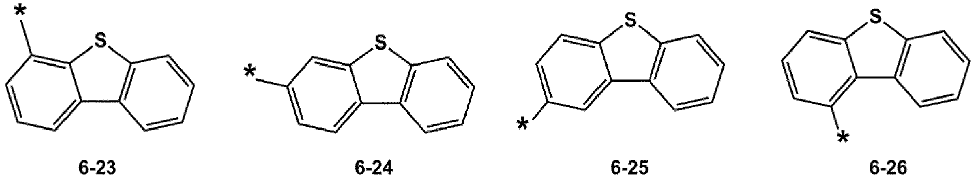 wherein, in Formulae 6-1 to 6-10 and 6-12 to 6-26,
^{∗}indicates a binding site to an adjacent atom.

6. The amine-based compound of any one of claims 3 to 5, wherein the amine-based compound represented by Formula 1D is represented by one of Formulae 1B-1 to 1B-16: 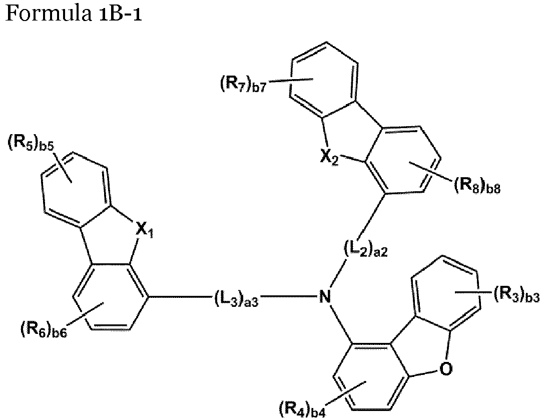 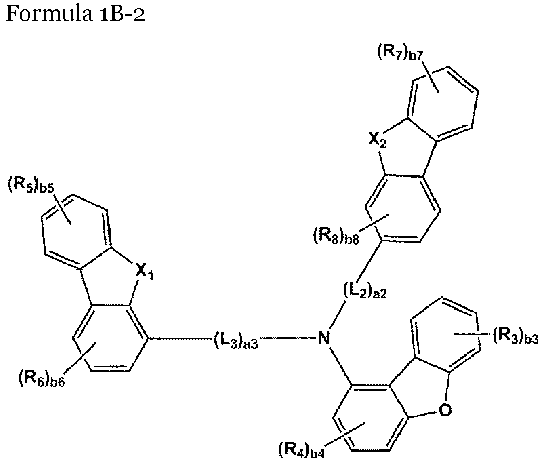 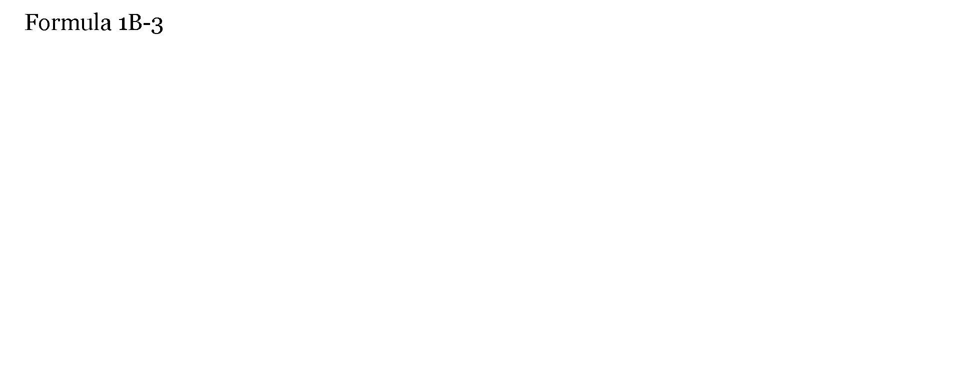 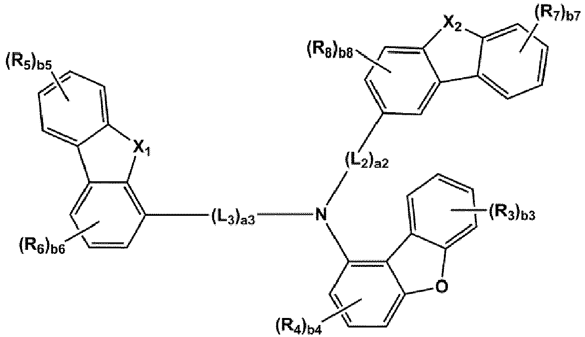 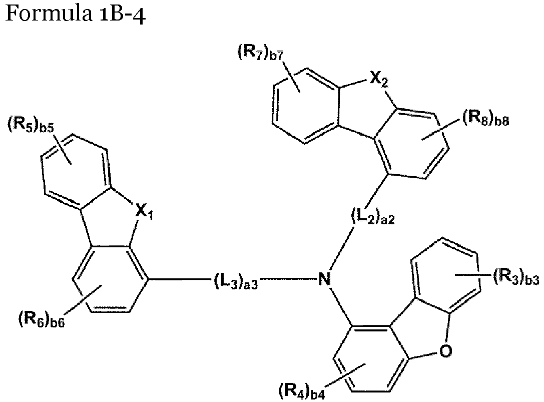 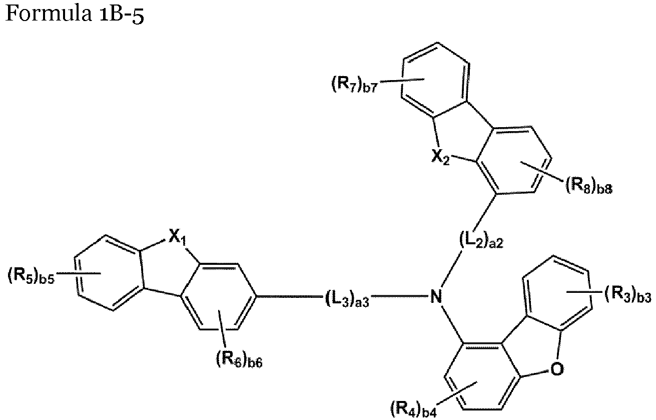 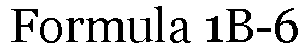 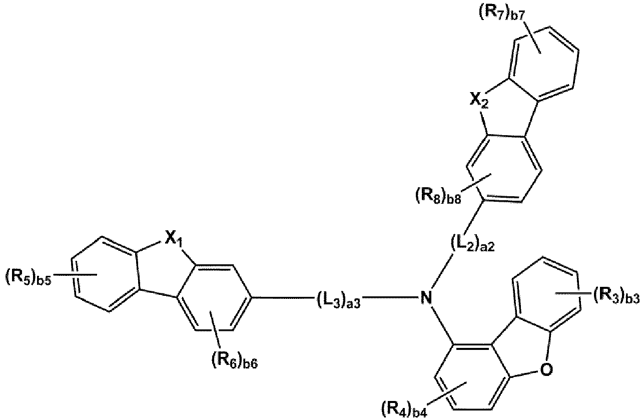 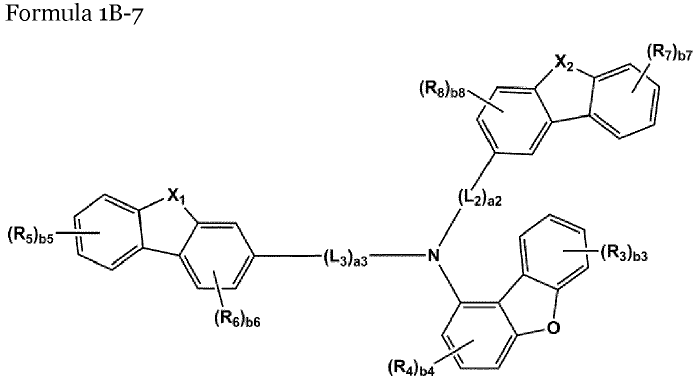 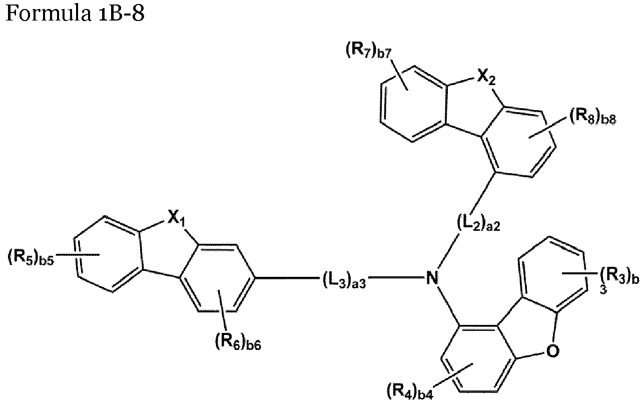 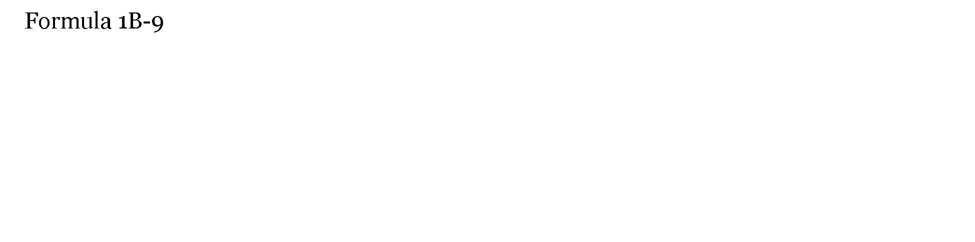 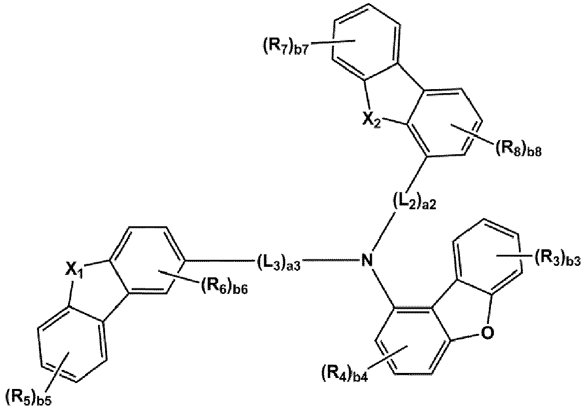 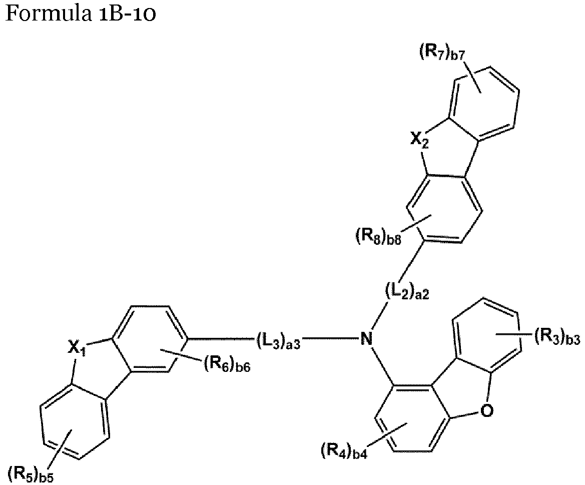 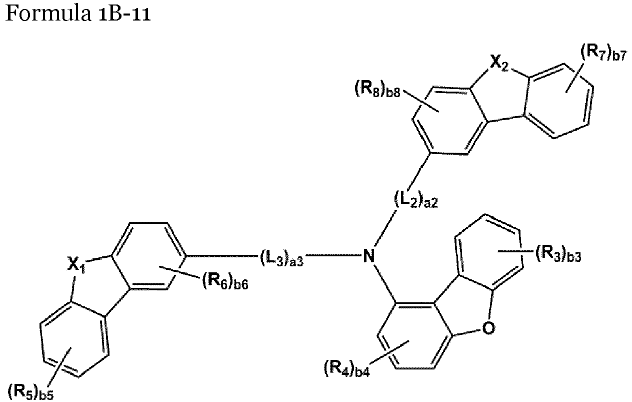 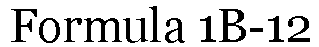 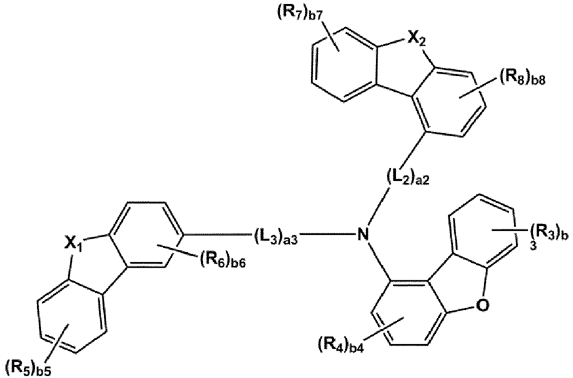 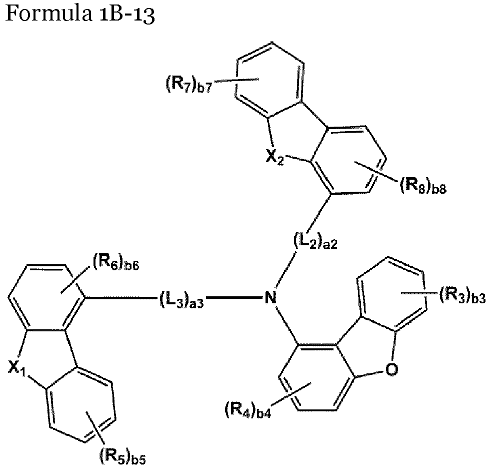 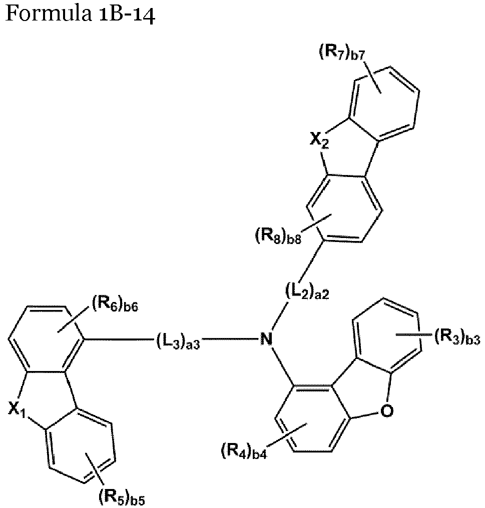 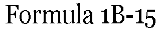 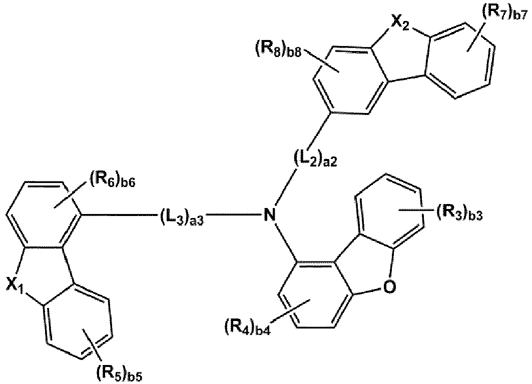 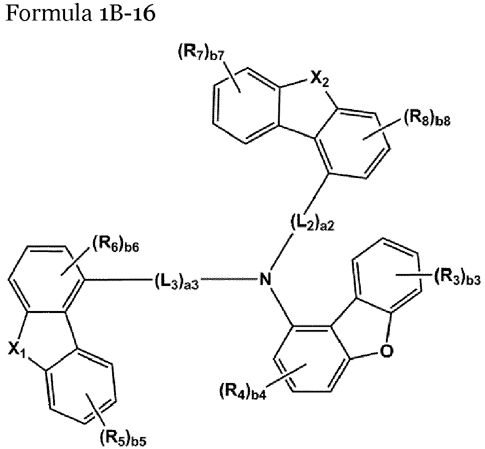 wherein, in Formulae 1B-1 to 1B-16,
X₁, X₂, L₂, L₃, a2, a3, R₃ to R₈, b3 to b8 are each independently the same as defined in claims 1 and 4 to 5.

7. An amine-based compound according to claim 3, wherein the amine-based compound is selected from Compounds 42-45, 47-50, 56-59, 61-64, 70-73, 75-78, 84-87, 89-92, 232-239, 243-250:

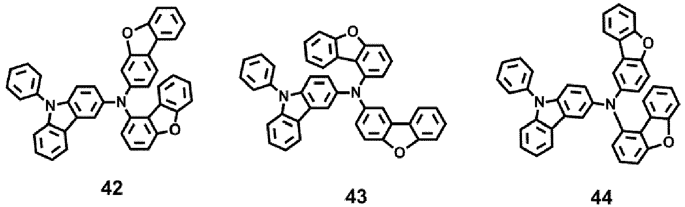

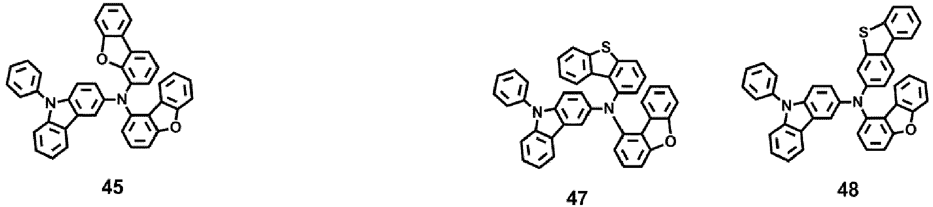

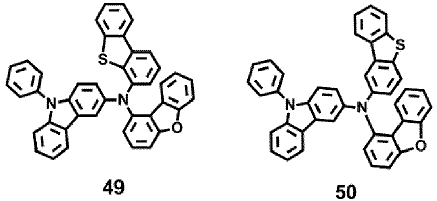

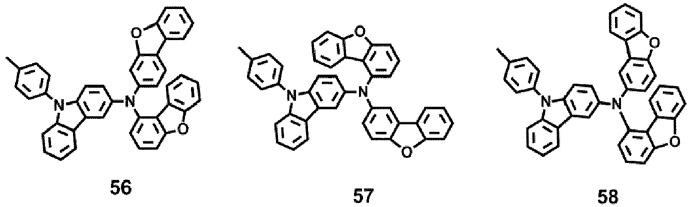

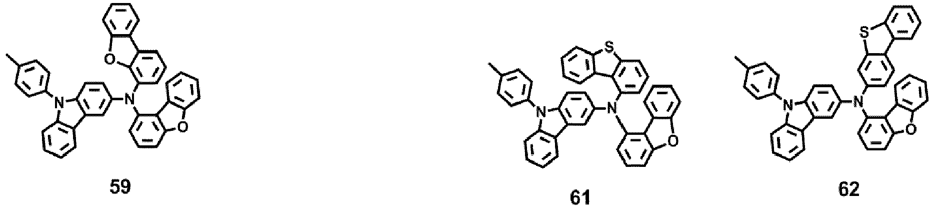

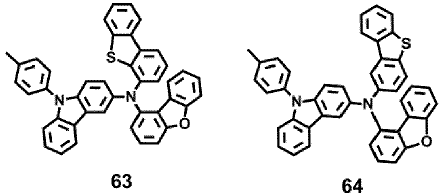

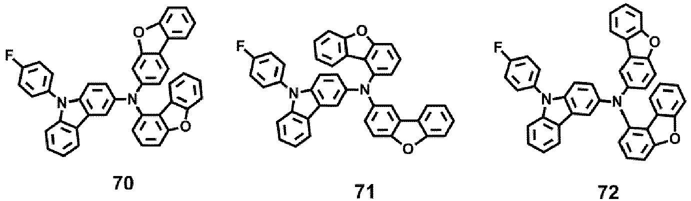

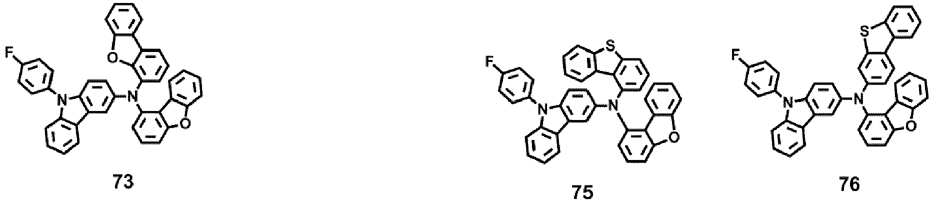

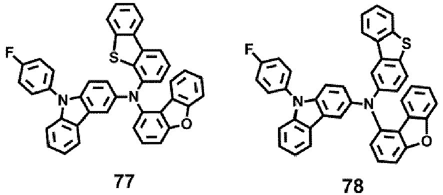

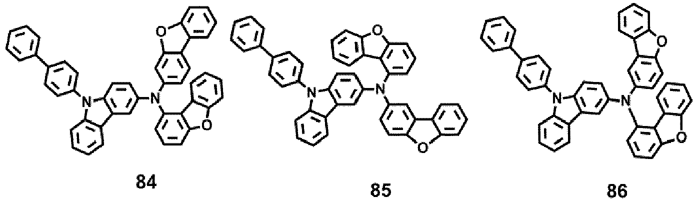

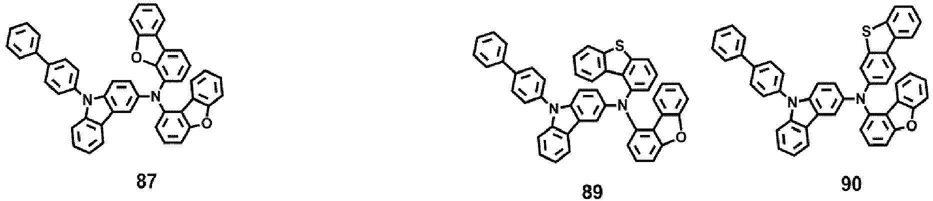

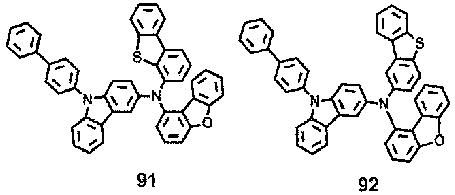

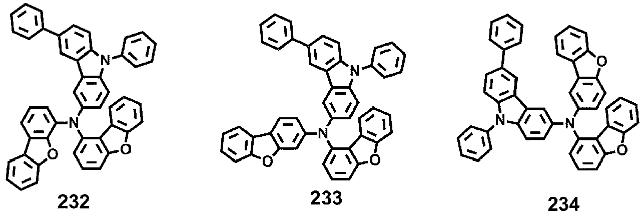

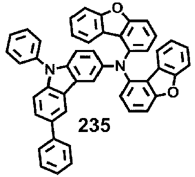

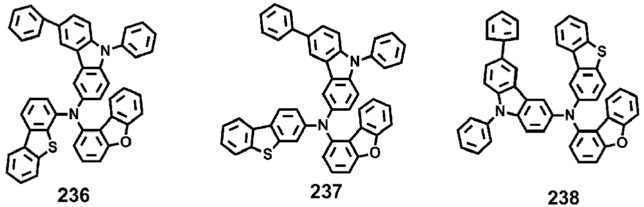

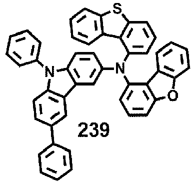

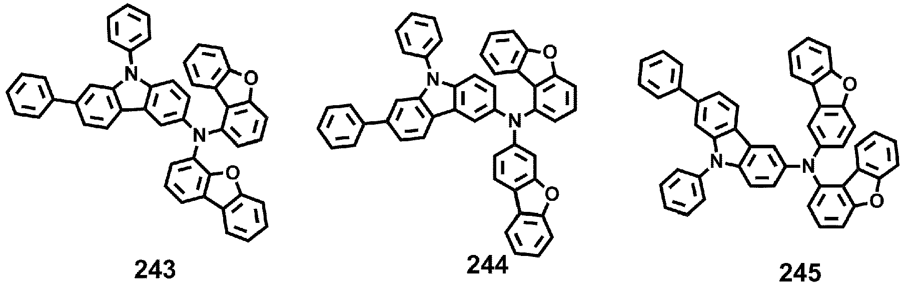

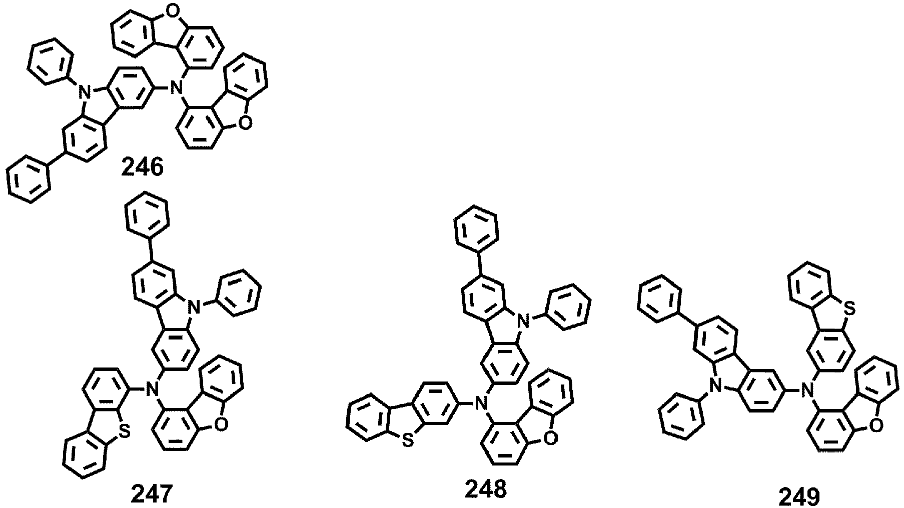

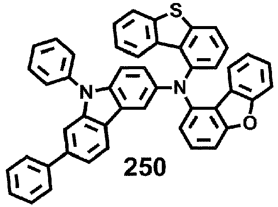

8. The organic light-emitting device according to any one of claims 1 or 2, wherein the amine based compound is as defined in any one of claims 3 to 7.

## Patentansprüche

1. Organische lichtemittierende Vorrichtung, umfassend:
eine erste Elektrode;
eine zweite Elektrode, die der ersten Elektrode gegenüberliegt; und
eine organische Schicht zwischen der ersten Elektrode und der zweiten Elektrode, und eine Emissionsschicht umfassend,
wobei die organische Schicht mindestens eine Verbindung auf Aminbasis, dargestellt durch Formel 1D, umfasst: 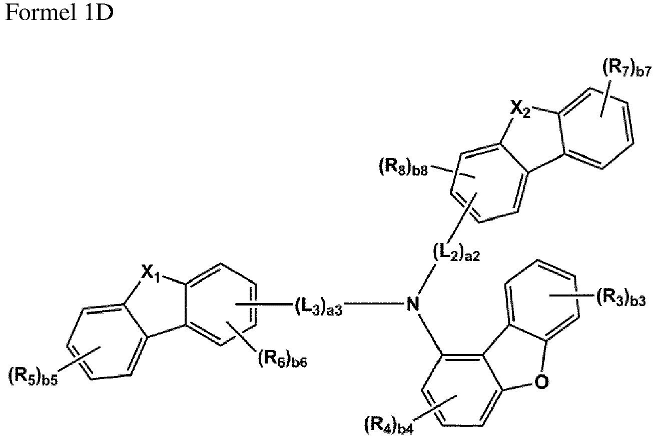 wobei, in Formel 1D,
X₁ N-(L₁)ₐ₁-(R₁)_{b1} ist,
X₂ O oder S ist,
L₁ und L₂ jeweils unabhängig voneinander ausgewählt sind aus einer Einfachbindung, einer Benzolgruppe, einer Pentalengruppe, einer Indengruppe, einer Naphthalingruppe, einer Azulengruppe, einer Heptalengruppe, einer Indacengruppe, einer Acenaphthalingruppe, einer Phenalengruppe, einer Phenanthrengruppe, einer Anthracengruppe, einer Fluoranthengruppe, einer Triphenylengruppe, einer Pyrengruppe, einer Chrysengruppe, einer Naphthacengruppe, einer Picengruppe, einer Perylengruppe, einer Pentaphenylengruppe, einer Hexacengruppe, einer Pyrrolgruppe, einer Imidazolgruppe, einer Pyrazolgruppe, einer Pyridingruppe, einer Pyrazingruppe, einer Pyrimidingruppe, einer Pyridazingruppe, einer Isoindolgruppe, einer Indolgruppe, einer Indazolgruppe, einer Puringruppe, einer Chinolingruppe, einer Isochinolingruppe, einer Benzochinolingruppe, einer Phthalazingruppe, einer Naphthyridingruppe, einer Chinoxalingruppe, einer Chinazolingruppe, einer Cinnolingruppe, einer Carbazolgruppe, einer Phenanthridingruppe, einer Acridingruppe, einer Phenanthrolingruppe, einer Phenazingruppe, einer Benzimidazolgruppe, einer Furangruppe, einer Benzofurangruppe, einer Thiophengruppe, einer Benzothiophengruppe, einer Thiazolgruppe, einer Isothiazolgruppe, einer Benzothiazolgruppe, einer Isooxazolgruppe, einer Oxazolgruppe, einer Triazolgruppe, einer Tetrazolgruppe, einer Oxadiazolgruppe, einer Triazingruppe, einer Benzoxazolgruppe, einer Dibenzofurangruppe, einer Dibenzothiophengruppe, einer Benzocarbazolgruppe und einer Dibenzocarbazolgruppe; und
einer Benzolgruppe, einer Pentalengruppe, einer Indengruppe, einer Naphthalingruppe, einer Azulengruppe, einer Heptalengruppe, einer Indacengruppe, einer Acenaphthalingruppe, einer Phenalengruppe, einer Phenanthrengruppe, einer Anthracengruppe, einer Fluoranthengruppe, einer Triphenylengruppe, einer Pyrengruppe, einer Chrysengruppe, einer Naphthacengruppe, einer Picengruppe, einer Perylengruppe, einer Pentaphenylengruppe, einer Hexacengruppe, einer Pyrrolgruppe, einer Imidazolgruppe, einer Pyrazolgruppe, einer Pyridingruppe, einer Pyrazingruppe, einer Pyrimidingruppe, einer Pyridazingruppe, einer Isoindolgruppe, einer Indolgruppe, einer Indazolgruppe, einer Puringruppe, einer Chinolingruppe, einer Isochinolingruppe, einer Benzochinolingruppe, einer Phthalazingruppe, einer Naphthyridingruppe, einer Chinoxalingruppe, einer Chinazolingruppe, einer Cinnolingruppe, einer Carbazolgruppe, einer Phenanthridingruppe, einer Acridingruppe, einer Phenanthrolingruppe, einer Phenazingruppe, einer Benzimidazolgruppe, einer Furangruppe, einer Benzofurangruppe, einer Thiophengruppe, einer Benzothiophengruppe, einer Thiazolgruppe, einer Isothiazolgruppe, einer Benzothiazolgruppe, einer Isooxazolgruppe, einer Oxazolgruppe, einer Triazolgruppe, einer Tetrazolgruppe, einer Oxadiazolgruppe, einer Triazingruppe, einer Benzoxazolgruppe, einer Dibenzofurangruppe, einer Dibenzothiophengruppe, einer Benzocarbazolgruppe und einer Dibenzocarbazolgruppe, jeweils substituiert mit mindestens einem, ausgewählt aus Deuterium, -F, -Cl, -Br, -I, einer Hydroxylgruppe, einer Cyanogruppe, einer Nitrogruppe, einer Aminogruppe, einer Amidinogruppe, einer Hydrazinogruppe, einer Hydrazonogruppe, einem C₁-C₂₀-Alkylrest, einem C₁-C₂₀-Alkoxyrest, einem C₆-C₂₀-Arylrest, einem C₁-C₂₀-Heteoarylrest und -Si(Q₃₁)(Q₃₂)(Q₃₃),
L3 eine Einfachbindung ist,
a1 und a2 jeweils unabhängig voneinander eine ganze Zahl von 1 bis 5 sind, a3 1 ist;
R₁ unabhängig ausgewählt ist aus Resten, dargestellt durch die Formeln 5-1 bis 5-9, 5-13 bis 5-19 und 5-21 bis 5-79: 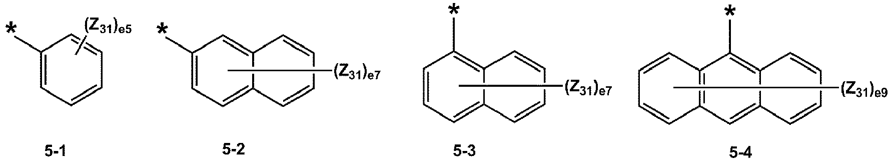 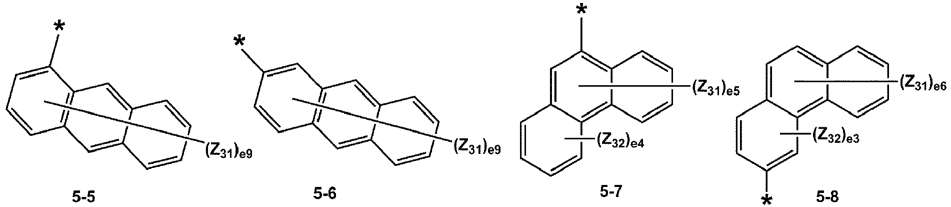 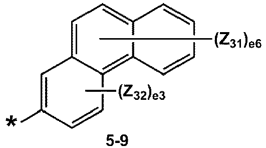 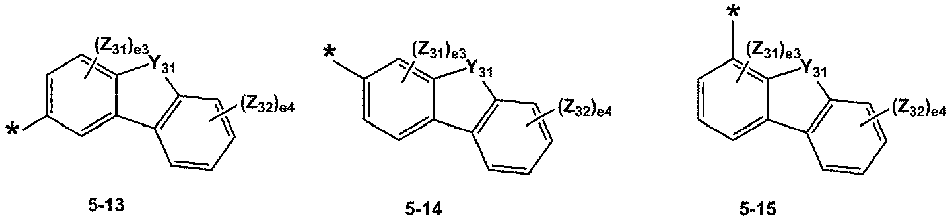 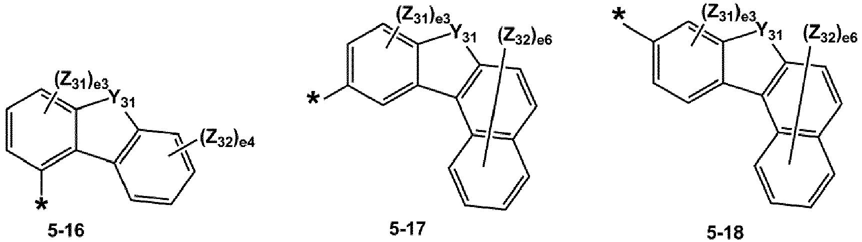 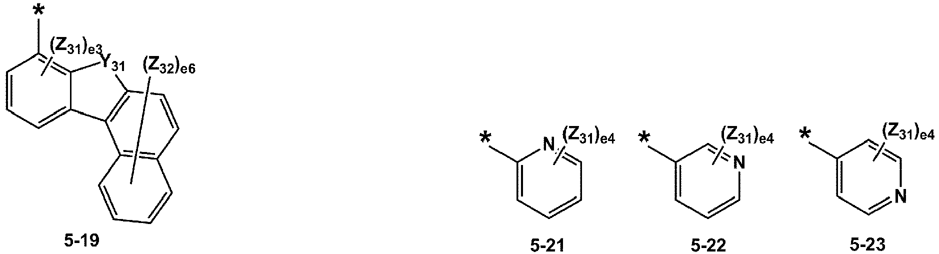 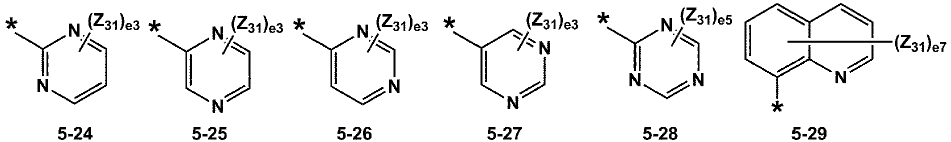 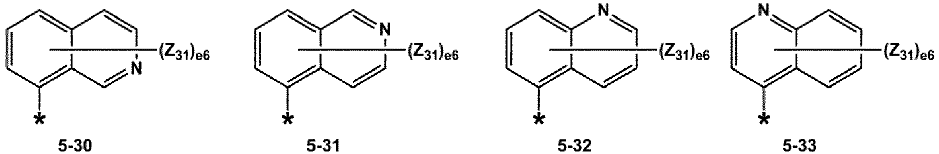 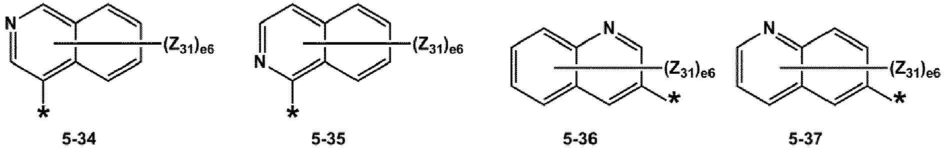 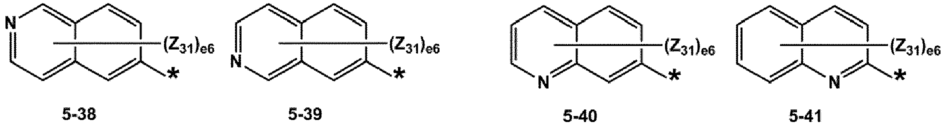 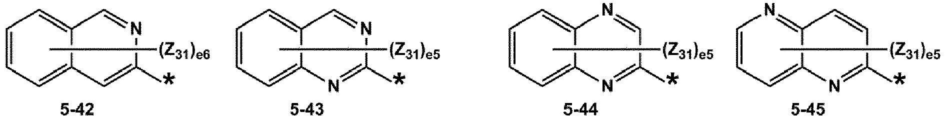 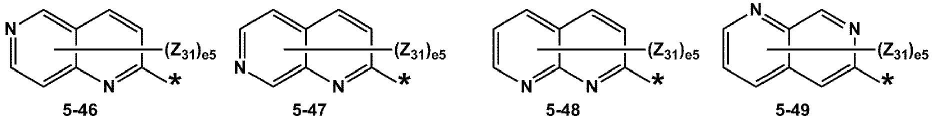 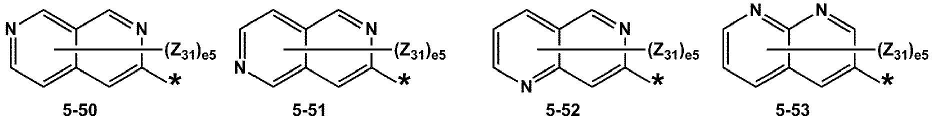 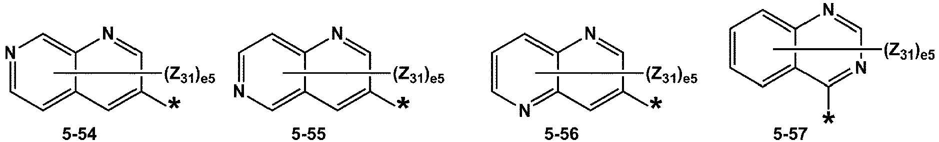 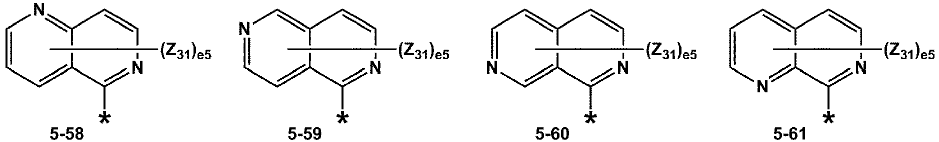 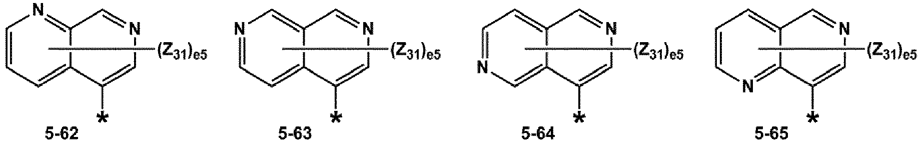 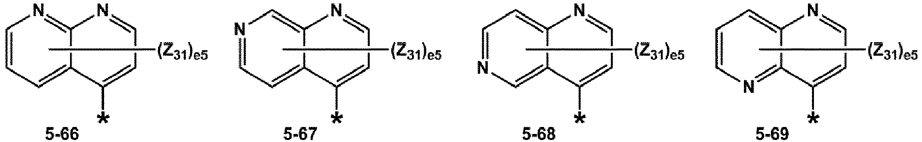 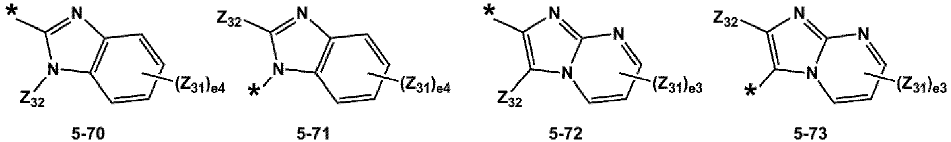 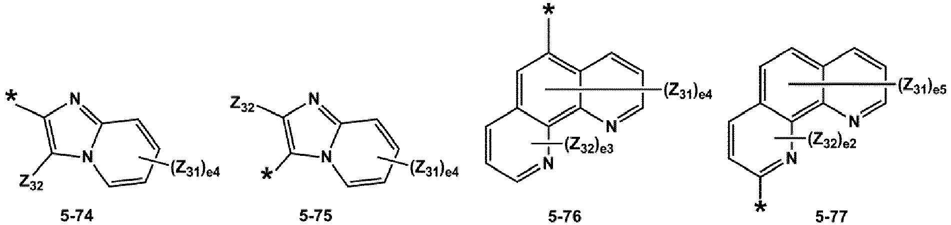 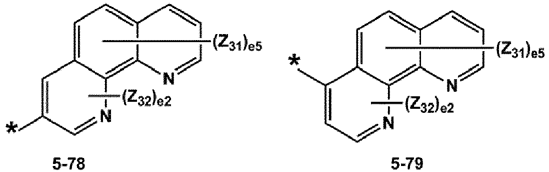 wobei, in den die Formeln 5-1 bis 5-9, 5-13 bis 5-19 und 5-21 bis 5-79,
Y₃₁ O, S, N(Z₃₅) oder Si(Z₃₆)(Z₃₇) ist,
Z₃₁, Z₃₂ und Z₃₅ bis Z₃₇ jeweils unabhängig voneinander ausgewählt sind aus Wasserstoff, Deuterium, -F, -Cl, -Br, -I, einer Hydroxylgruppe, einer Cyanogruppe, einer Nitrogruppe, einer Amidinogruppe, einer Hydrazinogruppe, einer Hydrazonogruppe, einem C₁-C₂₀-Alkylrest,einem C₁-C₂₀-Alkoxyrest, einer Cyclopentylgruppe, einer Cyclohexylgruppe, einer Cycloheptylgruppe, einer Cyclopentenylgruppe, einer Cyclohexenylgruppe, einer Phenylgruppe, einer Biphenylgruppe, einer Naphthylgruppe, einer Phenalenylgruppe, einer Phenanthrenylgruppe, einer Anthracenylgruppe, einer Fluoranthenylgruppe, einer Triphenylenylgruppe, Pyridinylgruppe, einer Pyrazinylgruppe, einer Pyrimidinylgruppe, einer Chinolinylgruppe, einer Isochinolinylgruppe, einer Benzochinolinylgruppe, einer Naphthyridinylgruppe, einer Chinoxalinylgruppe, einer Chinazolinylgruppe, einer Carbazolylgruppe, einer Phenanthridinylgruppe, einer Acridinylgruppe, einer Phenanthrolinylgruppe, einer Phenazinylgruppe, einer Triazinylgruppe, einer Dibenzofuranylgruppe, einer Dibenzothiophenylgruppe und -Si(Q₃₁)(Q₃₂)(Q₃₃),
e2 eine ganze Zahl von 0 bis 2 ist; wenn e2 2 ist, zwei Reste Z₃₁ miteinander identisch sind oder sich voneinander unterscheiden und zwei Reste Z₃₂ miteinander identisch sind oder sich voneinander unterscheiden,
e3 eine ganze Zahl von 0 bis 3 ist; wenn e3 2 oder größer ist, mindestens zwei Reste Z₃₁ miteinander identisch sind oder sich voneinander unterscheiden und mindestens zwei Reste Z₃₂ miteinander identisch sind oder sich voneinander unterscheiden,
e4 eine ganze Zahl von 0 bis 4 ist; wenn e4 2 oder größer ist, mindestens zwei Reste Z₃₁ miteinander identisch sind oder sich voneinander unterscheiden und mindestens zwei Reste Z₃₂ miteinander identisch sind oder sich voneinander unterscheiden,
e5 eine ganze Zahl von 0 bis 5 ist; wenn e5 2 oder größer ist, mindestens zwei Reste Z₃₁ miteinander identisch sind oder sich voneinander unterscheiden und mindestens zwei Reste Z₃₂ miteinander identisch oder sich voneinander unterscheiden,
e6 eine ganze Zahl von 0 bis 6 ist; wenn e6 2 oder größer ist, mindestens zwei Reste Z₃₁ miteinander identisch sind oder sich voneinander unterscheiden und mindestens zwei Reste Z₃₂ miteinander identisch sind oder sich voneinander unterscheiden,
e7 eine ganze Zahl von 0 bis 7 ist; wenn e7 2 oder größer ist, mindestens zwei Reste Z₃₁ miteinander identisch sind oder sich voneinander unterscheiden,
e9 eine ganze Zahl von 0 bis 9 ist; wenn e9 2 oder größer ist, mindestens zwei Reste Z₃₁ miteinander identisch sind oder sich voneinander unterscheiden, und
^{∗} eine Bindungsstelle zu einem benachbarten Atom kennzeichnet,
R₃ bis R₈ jeweils unabhängig voneinander ausgewählt sind aus Wasserstoff, Deuterium, -F, -Cl, -Br, -I, einer Hydroxylgruppe, einer Cyanogruppe, einer Nitrogruppe, einer Aminogruppe, einer Amidinogruppe, einer Hydrazinogruppe, einer Hydrazonogruppe, einem substituierten oder unsubstituierten C₁-C₆₀-Alkylrest, einem substituierten oder unsubstituierten C₂-C₆₀-Alkenylrest, einem substituierten oder unsubstituierten C₂-C₆₀-Alkinylrest, einem substituierten oder unsubstituierten C₁-C₆₀-Alkoxyrest, einem substituierten oder unsubstituierten C₃-C₁₀-Cycloalkylrest, einem substituierten oder unsubstituierten C₁-C₁₀-Heterocycloalkylrest, einem substituierten oder unsubstituierten C₃-C₁₀-Cycloalkenylrest, einem substituierten oder unsubstituierten C₁-C₁₀-Heterocycloalkenylrest, einem substituierten oder unsubstituierten C₆-C₆₀-Arylrest, einem substituierten oder unsubstituierten C₆-C₆₀-Aryloxyrest, einem substituierten oder unsubstituierten C₆-C₆₀-Arylthiorest, einem substituierten oder unsubstituierten C₁-C₆₀-Heteroarylrest, einem substituierten oder unsubstituierten C₁-C₆₀-Heteroaryloxyrest, einem substituierten oder unsubstituierten einwertigen, nichtaromatischen, kondensierten polycyclischen C8 bis C60-Rest , einem substituierten oder unsubstituierten einwertigen, nichtaromatischen, kondensierten heteropolycyclischen 8- bis 60-gliedrigen Rest, -Si(Q₁)(Q₂)(Q₃), -B(Q₁)(Q₂) und -C(=O)(Q₁),
b1 eine ganze Zahl von 1 bis 10 ist,
b3, b5 und b7 jeweils unabhängig voneinander eine ganze Zahl von 1 bis 4 sind,
b4, b6 und b8 jeweils unabhängig voneinander eine ganze Zahl von 1 bis 3 sind, und
der substituierte C₁-C₆₀-Alkylrest, der substituierte C₂-C₆₀-Alkenylrest, der substituierte C₂-C₆₀-Alkinylrest, der substituierte C₁-C₆₀-Alkoxyrest, der substituierte C₃-C₁₀-Cycloalkylrest, der substituierte C₁-C₁₀-Heterocycloalkylrest, der substituierte C₃-C₁₀-Cycloalkenylrest, der substituierte C₁-C₁₀-Heterocycloalkenylrest, der substituierte C₆-C₆₀-Arylrest, der substituierte C₆-C₆₀-Aryloxyrest, der substituierte C₆-C₆₀-Arylthiorest, der substituierte C₁-C₆₀-Heteroarylrest, der substituierte C₁-C₆₀-Heteroaryloxyrest, der substituierte einwertige, nichtaromatische, kondensierte polycyclische C8 bis C60-Rest und der substituierte einwertige, nichtaromatische, kondensierte heteropolycyclische 8- bis 60-gliedrige Rest jeweils unabhängig voneinander substituiert sind mit einem oder mehreren Substituenten, unabhängig voneinander ausgewählt aus:
Deuterium, -F, -Cl, -Br, -I, einer Hydroxylgruppe, einer Cyanogruppe, einer Nitrogruppe, einer Aminogruppe, einer Amidinogruppe, einer Hydrazinogruppe, einer Hydrazonogruppe, einem C₁-C₆₀-Alkylrest, einem C₂-C₆₀-Alkenylrest, einem C₂-C₆₀-Alkinylrest und einem C₁-C₆₀-Alkoxyrest;
einem C₁-C₆₀-Alkylrest, einem C₂-C₆₀-Alkenylrest, einem C₂-C₆₀-Alkinylrest und einem C₁-C₆₀-Alkoxyrest, jeweils substituiert mit mindestens einem, ausgewählt aus Deuterium, -F, -Cl, -Br, -I, einer Hydroxylgruppe, einer Cyanogruppe, einer Nitrogruppe, einer Aminogruppe, einer Amidinogruppe, einer Hydrazinogruppe, einer Hydrazonogruppe, einem C₃-C₁₀-Cycloalkylrest, einem C₁-C₁₀-Heterocycloalkylrest, einem C₃-C₁₀-Cycloalkenylrest, einem C₁-C₁₀-Heterocycloalkenylrest, einem C₆-C₆₀-Arylrest, einem C₆-C₆₀-Aryloxyrest, einem C₆-C₆₀-Arylthiorest, einem C₁-C₆₀-Heteoarylrest, einem einwertigen, nichtaromatischen, kondensierten polycyclischen C8 bis C60-Rest, einem einwertigen, nichtaromatischen, kondensierten heteropolycyclischen 8- bis 60-gliedrigen Rest, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁) und -P(=O)(Q₁₁)(Q₁₂);
einem C₃-C₁₀-Cycloalkylrest, einem C₁-C₁₀-Heterocycloalkylrest, einem C₃-C₁₀-Cycloalkenylrest, einem C₁-C₁₀-Heterocycloalkenylrest, einem C₆-C₆₀-Arylrest, einem C₆-C₆₀-Aryloxyrest, einem C₆-C₆₀-Arylthiorest, einem C₁-C₆₀-Heteoarylrest, einem einwertigen, nichtaromatischen, kondensierten polycyclischen C8 bis C60-Rest, einem einwertigen, nichtaromatischen, kondensierten heteropolycyclischen 8- bis 60-gliedrigen Rest, einer Biphenylgruppe und einer Terphenylgruppe;
einem C₃-C₁₀-Cycloalkylrest, einem C₁-C₁₀-Heterocycloalkylrest, einem C₃-C₁₀-Cycloalkenylrest, einem C₁-C₁₀-Heterocycloalkenylrest, einem C₆-C₆₀-Arylrest, einem C₆-C₆₀-Aryloxyrest, einem C₆-C₆₀-Arylthiorest, einem C₁-C₆₀-Heteoarylrest, einem einwertigen, nichtaromatischen, kondensierten polycyclischen C8 bis C60-Rest, einem einwertigen, nichtaromatischen, kondensierten heteropolycyclischen 8- bis 60-gliedrigen Rest, einer Biphenylgruppe und einer Terphenylgruppe, jeweils substituiert mit mindestens einem, ausgewählt aus Deuterium, -F, -Cl, -Br, -I, einer Hydroxylgruppe, einer Cyanogruppe, einer Nitrogruppe, einer Aminogruppe, einer Amidinogruppe, einer Hydrazinogruppe, einer Hydrazonogruppe, einem C₁-C₆₀-Alkylrest, einem C₂-C₆₀-Alkenylrest, einem C₂-C₆₀-Alkinylrest, einem C₁-C₆₀-Alkoxyrest, einem C₃-C₁₀-Cycloalkylrest, einem C₁-C₁₀-Heterocycloalkylrest, einem C₃-C₁₀-Cycloalkenylrest, einem C₁-C₁₀-Heterocycloalkenylrest, einem C₆-C₆₀-Arylrest, einem C₆-C₆₀-Aryloxyrest, einem C₆-C₆₀-Arylthiorest, einem C₁-C₆₀-Heteoarylrest, einem einwertigen, nichtaromatischen, kondensierten polycyclischen C8 bis C60-Rest, einem einwertigen, nichtaromatischen, kondensierten heteropolycyclischen 8- bis 60-gliedrigen Rest, einer Biphenylgruppe, einer Terphenylgruppe, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁) und -P(=O)(Q₂₁)(Q₂₂); und
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁) und -P(=O)(Q₃₁)(Q₃₂),
wobei Q₁ bis Q₃, Q₁₁ bis Q₁₃, Q₂₁ bis Q₂₃ und Q₃₁ bis Q₃₃ jeweils unabhängig voneinander ausgewählt sind aus Wasserstoff, Deuterium, -F, -Cl, -Br, -I, einer Hydroxylgruppe, einer Cyanogruppe, einer Nitrogruppe, einer Aminogruppe, einer Amidinogruppe, einer Hydrazinogruppe, einer Hydrazonogruppe, einem C₁-C₆₀-Alkylrest, einem C₂-C₆₀-Alkenylrest, einem C₂-C₆₀-Alkinylrest, einem C₁-C₆₀-Alkoxyrest, einem C₃-C₁₀-Cycloalkylrest, einem C₁-C₁₀-Heterocycloalkylrest, einem C₃-C₁₀-Cycloalkenylrest, einem C₁-C₁₀-Heterocycloalkenylrest, einem C₆-C₆₀-Arylrest, einem mit einem C₁-C₆₀-Alkylrest substituierten C₆-C₆₀-Arylrest, einem C₁-C₆₀-Heteoarylrest, einem einwertigen, nichtaromatischen, kondensierten polycyclischen C8 bis C60-Rest, einem einwertigen, nichtaromatischen, kondensierten heteropolycyclischen 8- bis 60-gliedrigen Rest, einer Biphenylgruppe und einer Terphenylgruppe.

2. Organische lichtemittierende Vorrichtung nach Anspruch 1, wobei
die erste Elektrode eine Anode ist,
die zweite Elektrode eine Kathode ist,
die organische Schicht weiterhin einen Lochtransportbereich zwischen der ersten Elektrode und der Emissionsschicht und einen Elektronentransportbereich zwischen der Emissionsschicht und der zweiten Elektrode umfasst,
der Lochtransportbereich eine Lochinjektionsschicht, eine Lochtransportschicht, eine Emissionshilfsschicht, eine Elektronenblockierschicht, oder eine Kombination davon umfasst, und
der Elektronentransportbereich eine Lochblockierschicht, eine Elektronentransportschicht, eine Elektroneninjektionsschicht, oder eine Kombination davon umfasst;
vorzugsweise wobei der Lochtransportbereich die mindestens eine Verbindung auf Aminbasis umfasst;
stärker bevorzugt wobei der Lochtransportbereich die Lochtransportschicht umfasst, und die Lochtransportschicht die mindestens eine Verbindung auf Aminbasis umfasst.

3. Verbindung auf Aminbasis, dargestellt durch Formel 1D: 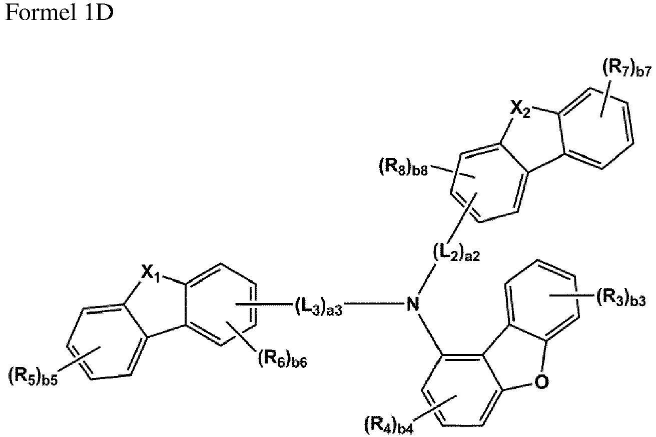 wobei Formel 1D wie in Anspruch 1 definiert ist.

4. Verbindung auf Aminbasis nach einem der Ansprüche 3, wobei
(A) L₁ und L2 jeweils unabhängig voneinander ausgewählt sind aus:
einer Einfachbindung und einer Benzolgruppe; und
einer Benzolgruppe, substituiert mit mindestens einem, ausgewählt aus Deuterium, -F, -Cl, -Br, -I, einer Hydroxylgruppe, einer Cyanogruppe, einer Nitrogruppe, einer Aminogruppe, einer Amidinogruppe, einer Hydrazinogruppe, einer Hydrazonogruppe, einem C₁-C₂₀-Alkylrest, einem C₁-C₂₀-Alkoxyrest, einem C₆-C₂₀-Arylrest, einem C₁-C₂₀-Heteoarylrest und -Si(Q₃₁)(Q₃₂)(Q₃₃);
L3 eine Einfachbindung ist,
und/oder
(B) R₃ bis R₁₂ jeweils unabhängig voneinander ausgewählt sind aus Wasserstoff, Deuterium, -F, -Cl, -Br, -I, einer Methylgruppe, einer Ethylgruppe, einer Propylgruppe, einer Isopropylgruppe, einer n-Butylgruppe, einer Isobutylgruppe, einer sec-Butylgruppe, einer tert-Butylgruppe, einer Pentylgruppe, einer Isoamylgruppe, einer Hexylgruppe, einer Phenylgruppe und einer Biphenylgruppe.

5. Verbindung auf Aminbasis nach einem der Ansprüche 3 bis 4, wobei R₁ ausgewählt ist aus Resten, dargestellt durch die Formeln 6-1 bis 6-10 und 6-12 bis 6-26: 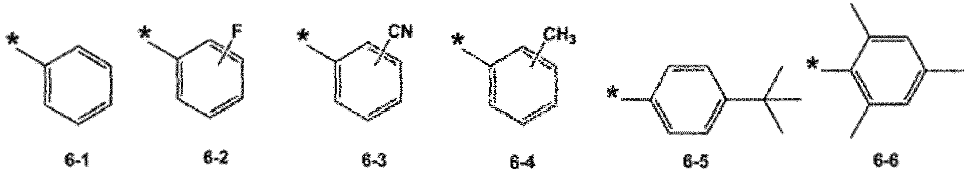 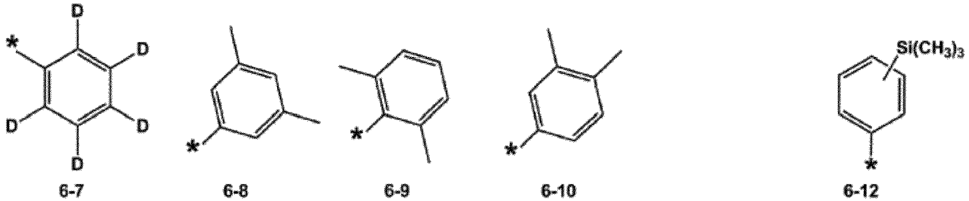 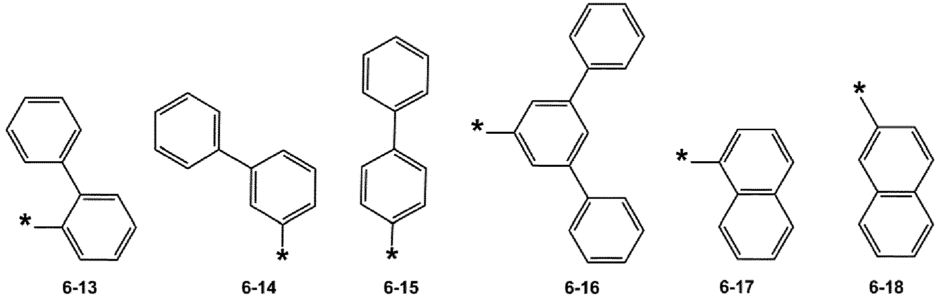 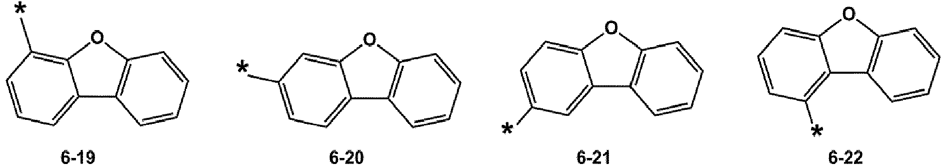 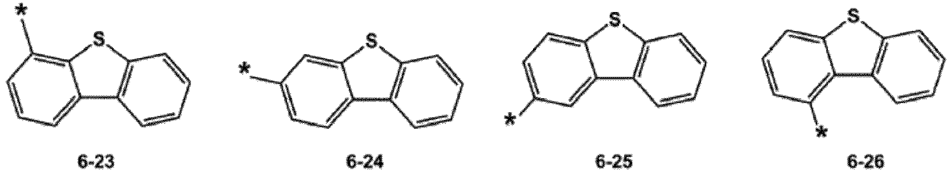 wobei, in den Formeln 6-1 bis 6-10 und 6-12 bis 6-26,
^{∗} eine Bindungsstelle zu einem benachbarten Atom kennzeichnet.

6. Verbindung auf Aminbasis nach einem der Ansprüche 3 bis 5, wobei die durch Formel 1D dargestellte Verbindung auf Aminbasis durch eine der Formeln 1B-1 bis 1B-16 wiedergegeben wird: 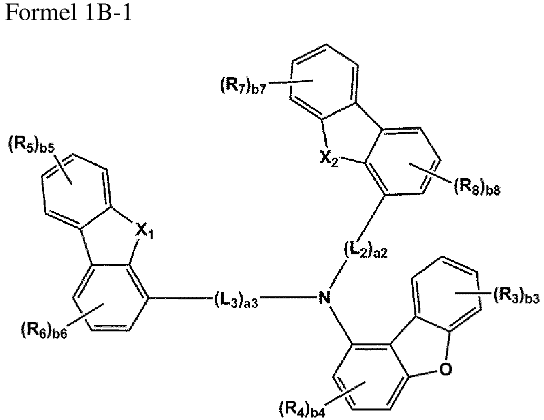 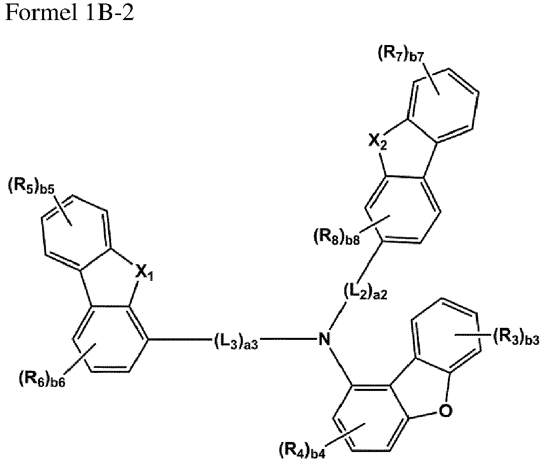 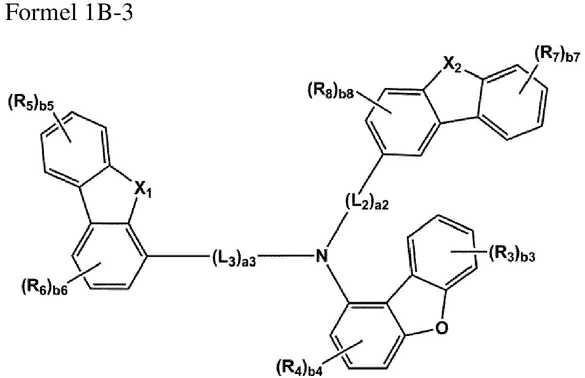 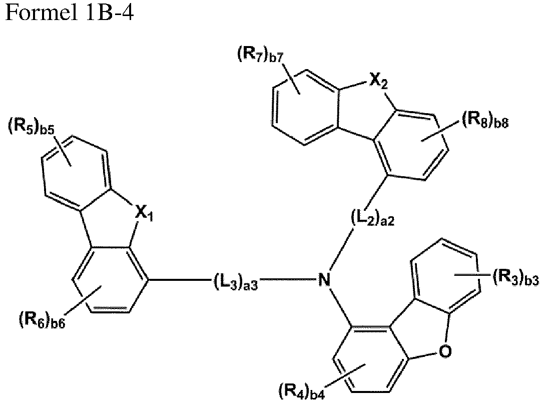 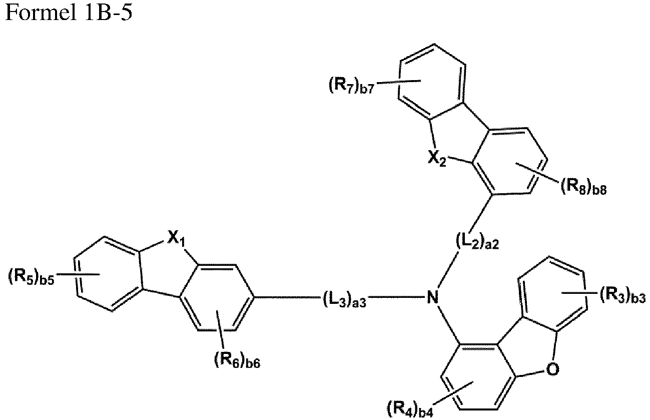 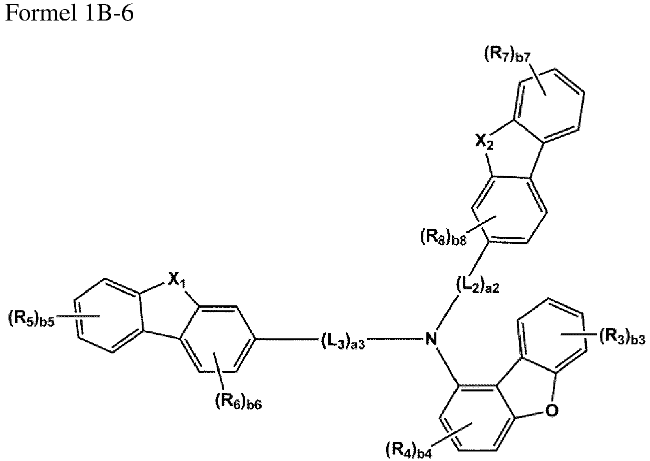 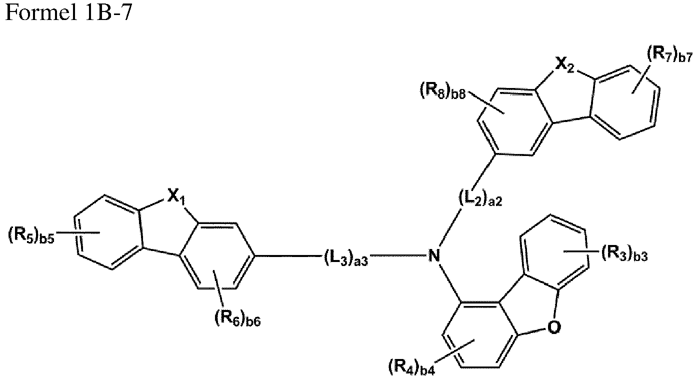 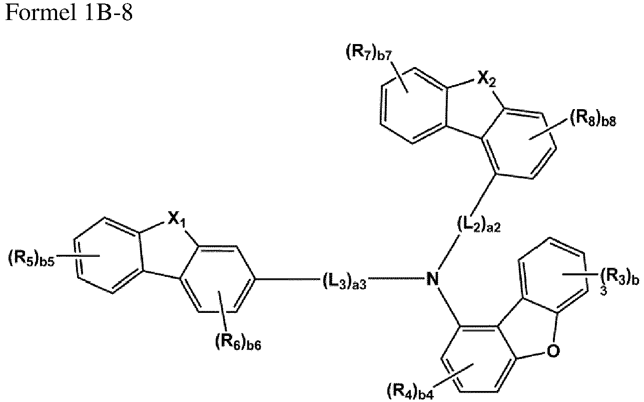 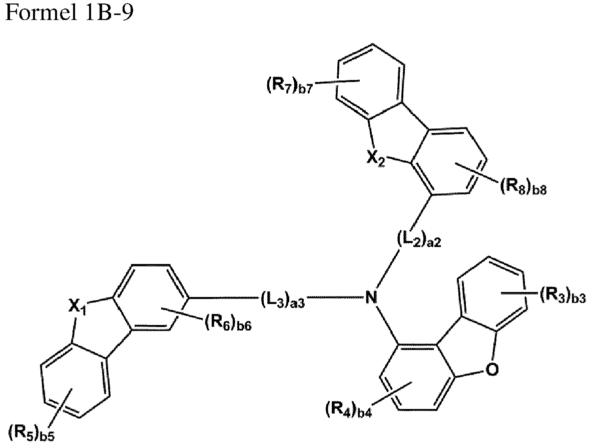 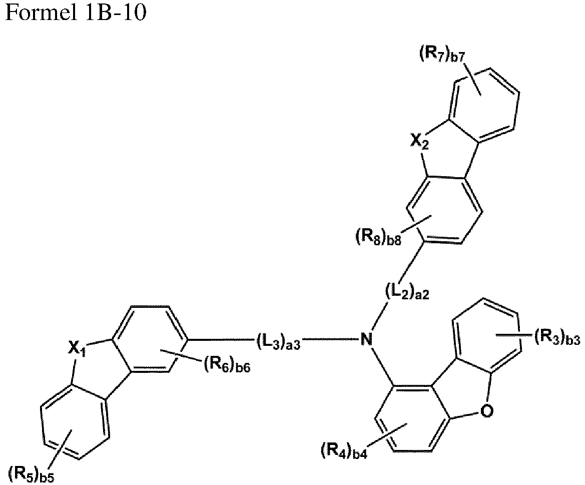 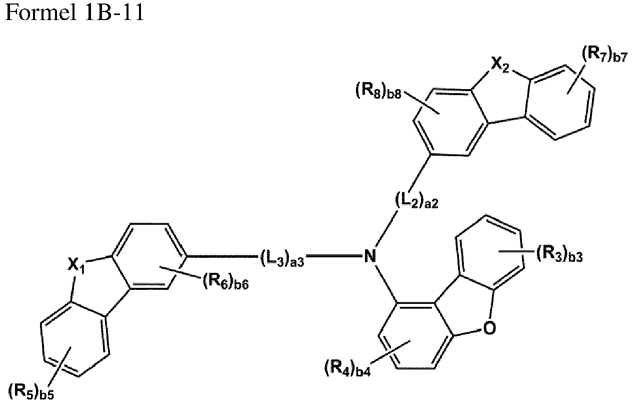 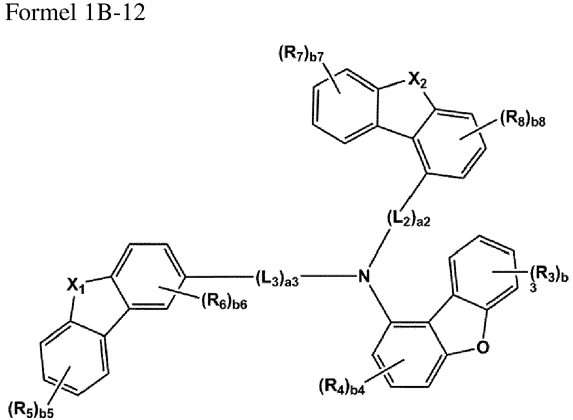 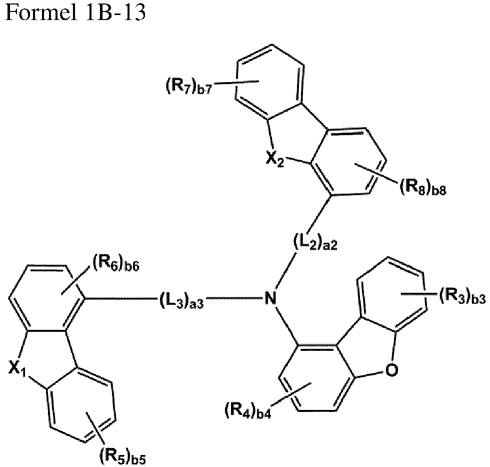 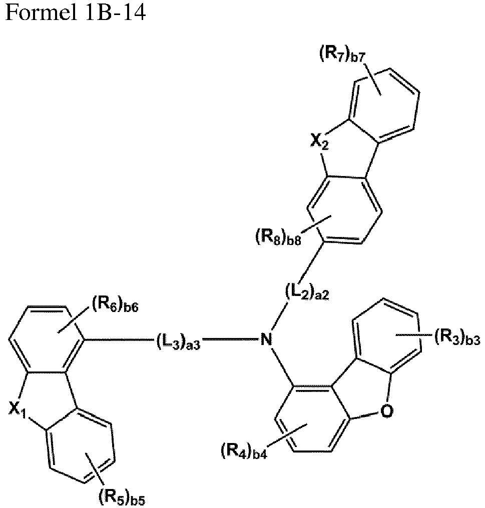 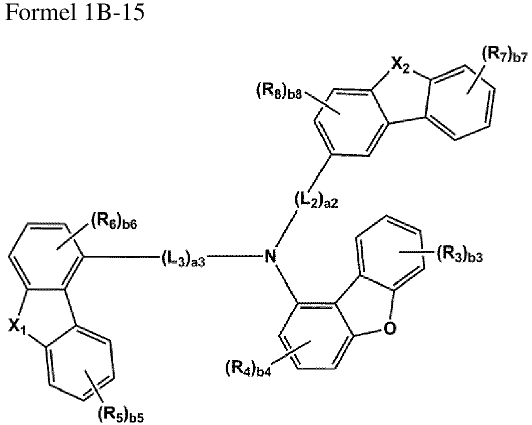 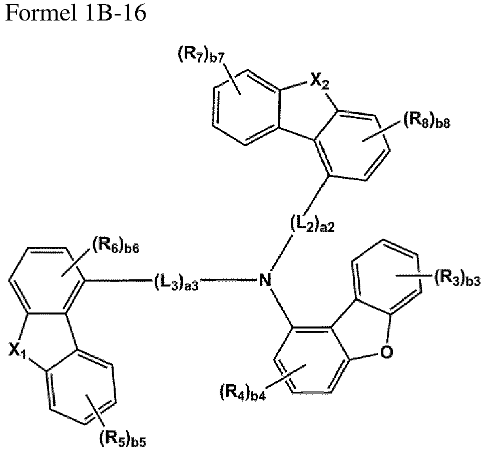 wobei, in den Formeln 1B-1 bis 1B-16,
X₁, X₂, L₂, L₃, a2, a3, R₃ bis R₈, b3 bis b8 unabhängig voneinander das Gleiche sind, wie in den Ansprüchen 1 und 4 bis 5 definiert.

7. Verbindung auf Aminbasis nach Anspruch 3, wobei die Verbindung auf Aminbasis ausgewählt ist aus den Verbindungen 42-45, 47-50, 56-59, 61-64, 70-73, 75-78, 84-87, 89-92, 232-239, 243-250:

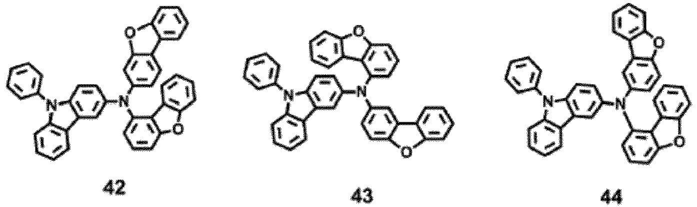

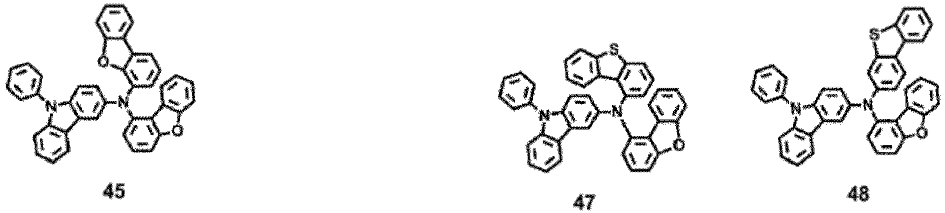

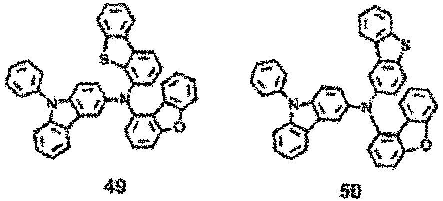

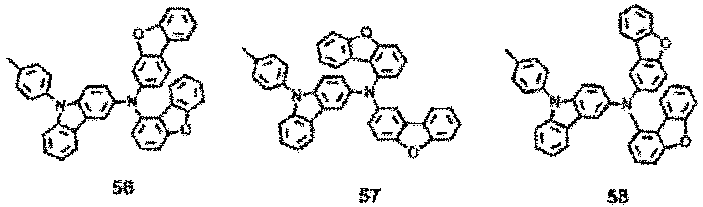

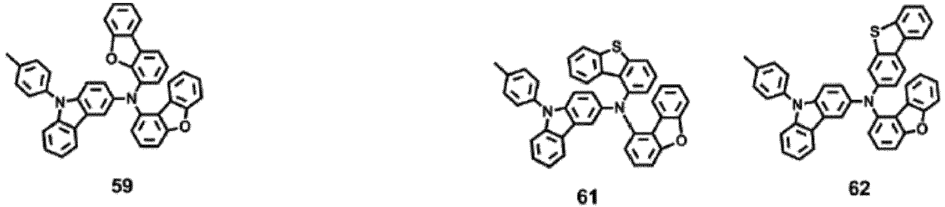

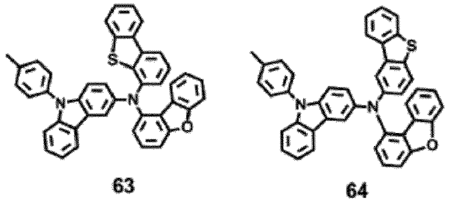

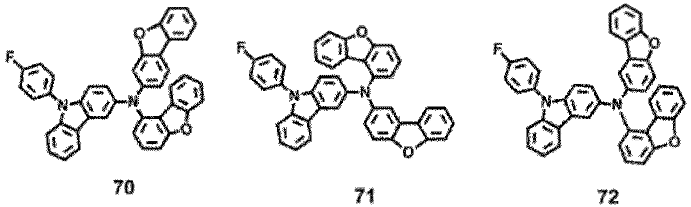

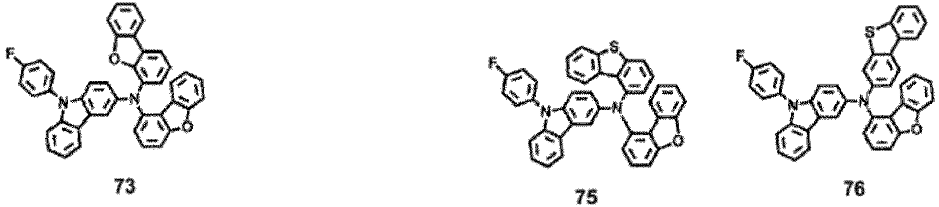

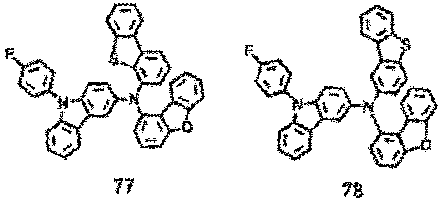

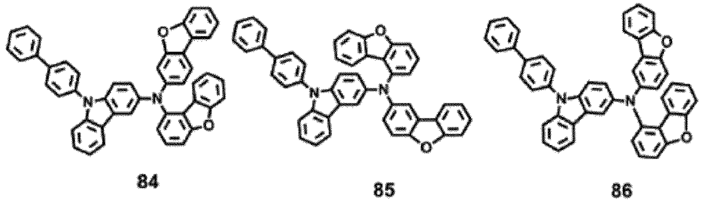

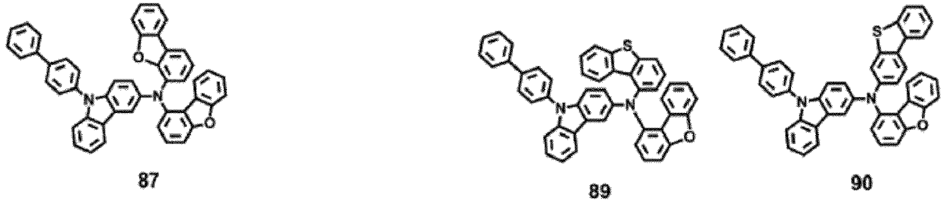

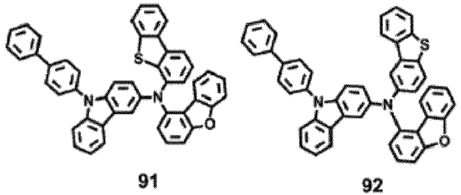

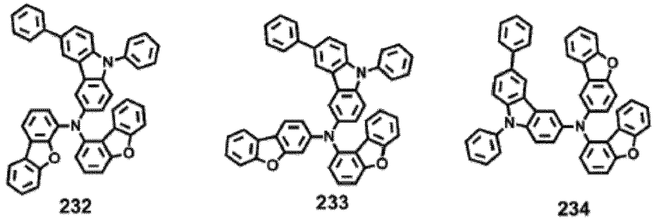

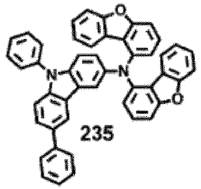

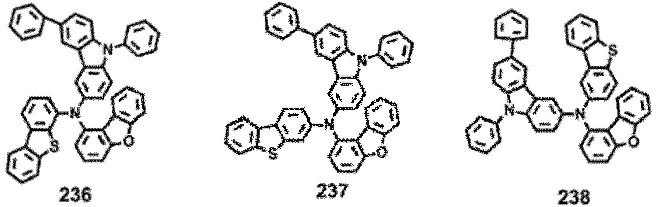

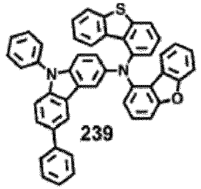

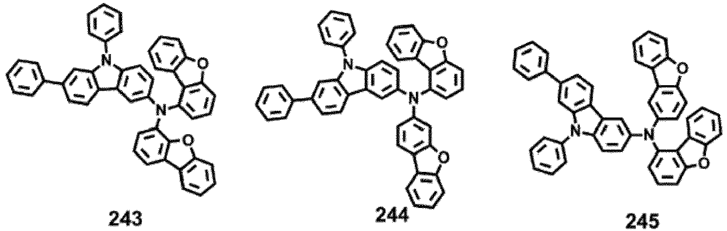

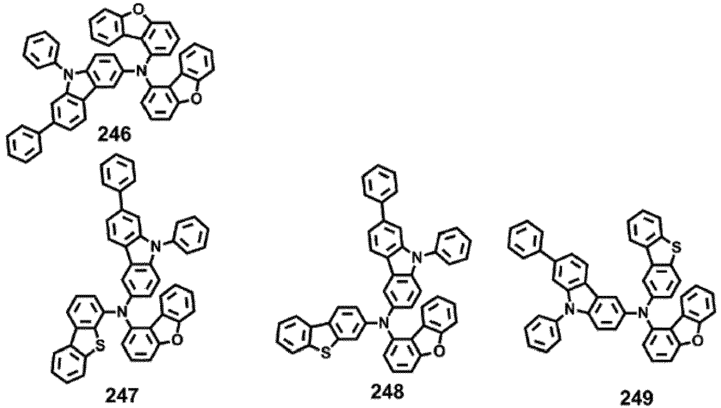

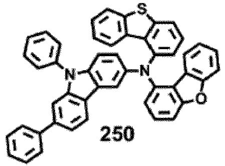

8. Organische lichtemittierende Vorrichtung nach einem der Ansprüche 1 oder 2, wobei die Verbindung auf Aminbasis wie in einem der Ansprüche 3 bis 7 definiert ist.

## Revendications

1. Dispositif électroluminescent organique comprenant :
une première électrode ;
une deuxième électrode faisant face à la première électrode ; et
une couche organique entre la première électrode et la deuxième électrode, et comprenant une couche d'émission,
dans lequel la couche organique comprend au moins un composé à base d'amine représenté par la formule 1D : 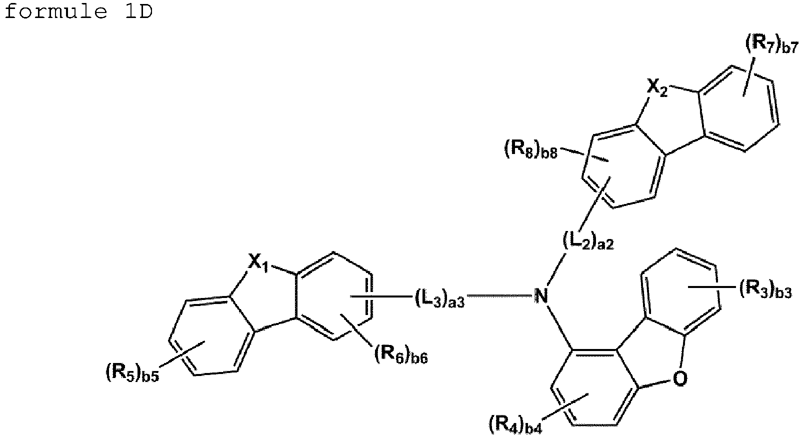 où, dans la formule 1D,
X₁ est N-(L₁)ₐ₁-(R₁)_{b1},
X₂ est O ou S,
chacun de L₁ et L₂ est indépendamment choisi parmi une liaison simple, un groupe benzène, un groupe pentalène, un groupe indène, un groupe naphtalène, un groupe azulène, un groupe heptalène, un groupe indacène, un groupe acénaphtalène, un groupe phénalène, un groupe phénanthrène, un groupe anthracène, un groupe fluoranthène, un groupe triphénylène, un groupe pyrène, un groupe chrysène, un groupe naphtacène, un groupe picène, un groupe pérylène, un groupe pentaphénylène, un groupe hexacène, un groupe pyrrole, un groupe imidazole, un groupe pyrazole, un groupe pyridine, un groupe pyrazine, un groupe pyrimidine, un groupe pyridazine, un groupe isoindole, un groupe indole, un groupe indazole, un groupe purine, un groupe quinoline, un groupe isoquinoline, un groupe benzoquinoline, un groupe phtalazine, un groupe naphtyridine, un groupe quinoxaline, un groupe quinazoline, un groupe cinnoline, un groupe carbazole, un groupe phénanthridine, un groupe acridine, un groupe phénanthroline, un groupe phénazine, un groupe benzimidazole, un groupe furane, un groupe benzofurane, un groupe thiophène, un groupe benzothiophène, un groupe thiazole, un groupe isothiazole, un groupe benzothiazole, un groupe isoxazole, un groupe oxazole, un groupe triazole, un groupe tétrazole, un groupe oxadiazole, un groupe triazine, un groupe benzoxazole, un groupe dibenzofurane, un groupe dibenzothiophène, un groupe benzocarbazole et un groupe dibenzocarbazole, et
un groupe benzène, un groupe pentalène, un groupe indène, un groupe naphtalène, un groupe azulène, un groupe heptalène, un groupe indacène, un groupe acénaphtalène, un groupe phénalène, un groupe phénanthrène, un groupe anthracène, un groupe fluoranthène, un groupe triphénylène, un groupe pyrène, un groupe chrysène, un groupe naphtacène, un groupe picène, un groupe pérylène, un groupe pentaphénylène, un groupe hexacène, un groupe pyrrole, un groupe imidazole, un groupe pyrazole, un groupe pyridine, un groupe pyrazine, un groupe pyrimidine, un groupe pyridazine, un groupe isoindole, un groupe indole, un groupe indazole, un groupe purine, un groupe quinoline, un groupe isoquinoline, un groupe benzoquinoline, un groupe phtalazine, un groupe naphtyridine, un groupe quinoxaline, un groupe quinazoline, un groupe cinnoline, un groupe carbazole, un groupe phénanthridine, un groupe acridine, un groupe phénanthroline, un groupe phénazine, un groupe benzimidazole, un groupe furane, un groupe benzofurane, un groupe thiophène, un groupe benzothiophène, un groupe thiazole, un groupe isothiazole, un groupe benzothiazole, un groupe isoxazole, un groupe oxazole, un groupe triazole, un groupe tétrazole, un groupe oxadiazole, un groupe triazine, un groupe benzoxazole, un groupe dibenzofurane, un groupe dibenzothiophène, un groupe benzocarbazole et un groupe dibenzocarbazole, chacun substitué par au moins l'un choisi parmi le deutérium, -F, -Cl, -Br, -I, un groupe hydroxyle, un groupe cyano, un groupe nitro, un groupe amino, un groupe amidino, un groupe hydrazino, un groupe hydrazono, un groupe alkyle en C₁ à C₂₀, un groupe alkoxy en C₁ à C₂₀, un groupe aryle en C₆ à C₂₀, un groupe hétéroaryle en C₁ à C₂₀, et -Si(Q₃₁)(Q₃₂)(Q₃₃),
L₃ est une liaison simple,
chacun de a1 et a2 est indépendamment un entier de 1 à 5, a3 vaut 1 ;
R₁ est indépendamment choisi parmi les groupes représentés par les formules 5-1 à 5-9, 5-13 à 5-19, et 5-21 à 5-79 : 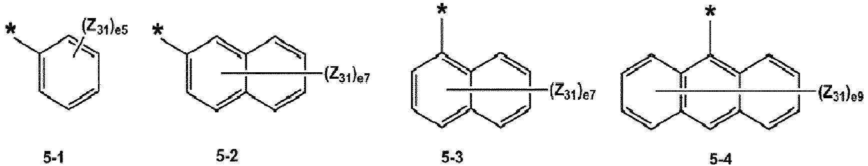 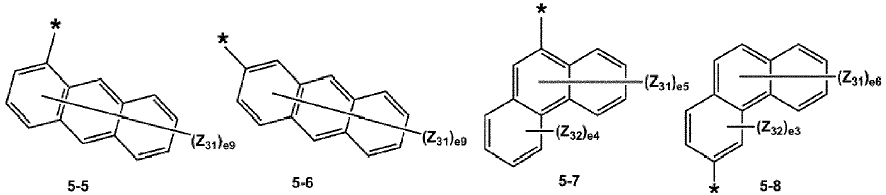 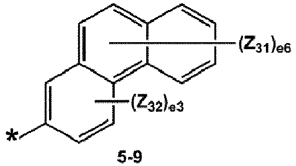 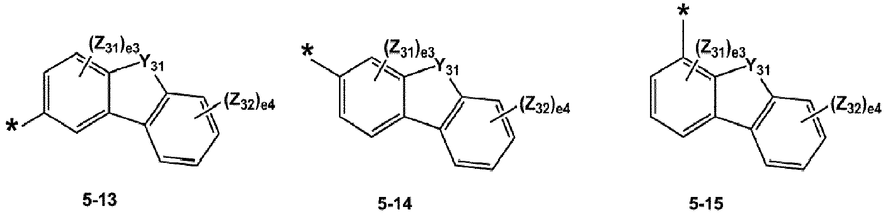 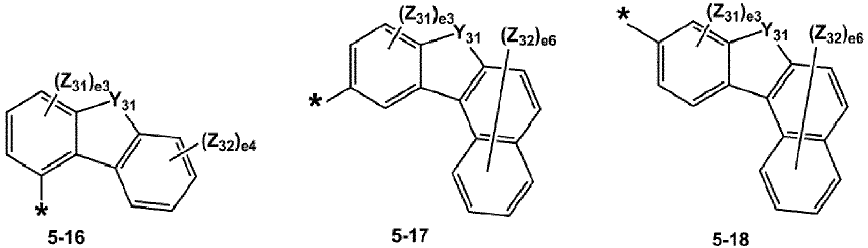 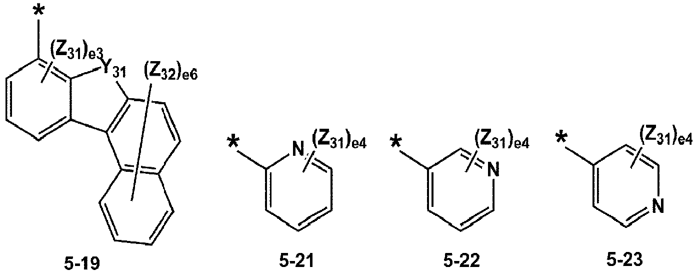 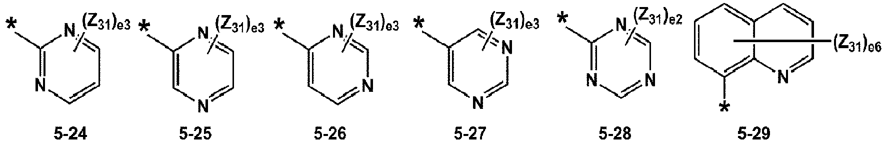 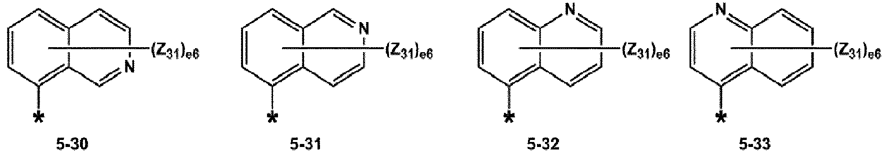 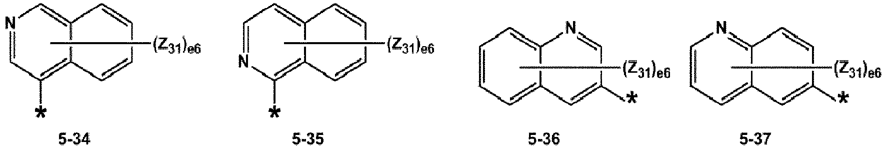 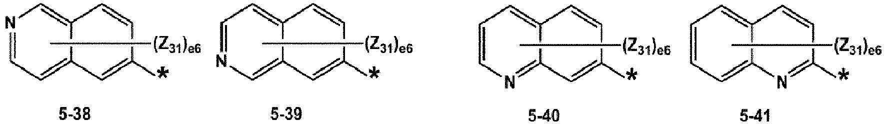 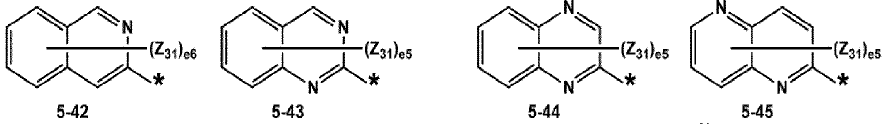 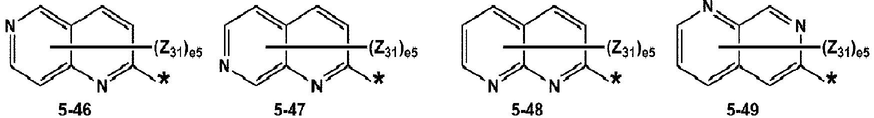 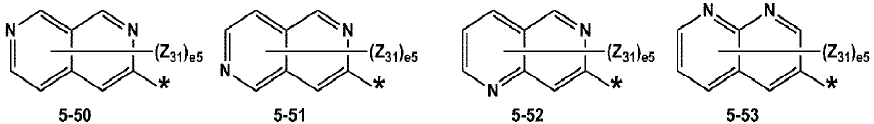 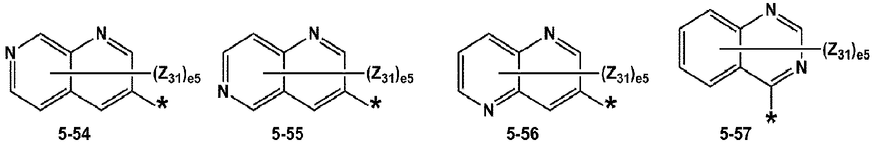 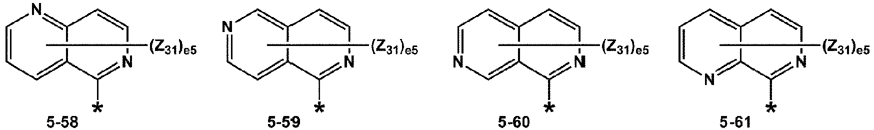 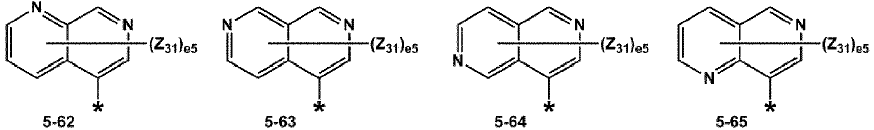 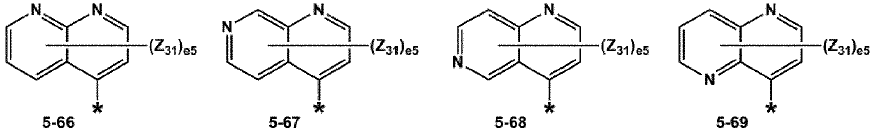 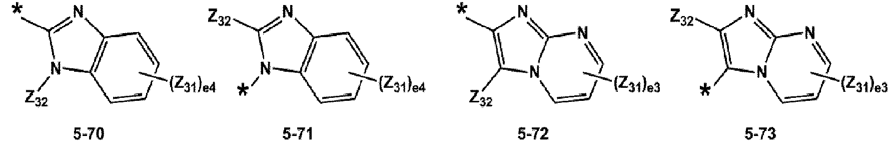 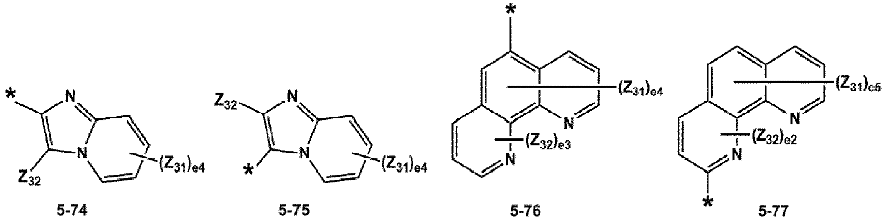 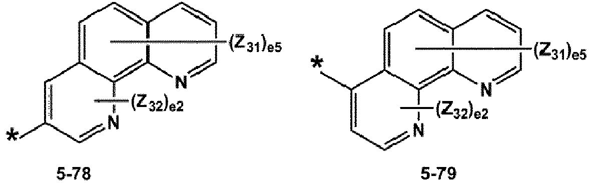
où, dans les formules 5-1 à 5-9, 5-13 à 5-19, et 5-21 à 5-79,
Y₃₁ est O, S, N(Z₃₅) ou Si(Z₃₆)(Z₃₇),
chacun de Z₃₁, Z₃₂ et Z₃₅ à Z₃₇ est indépendamment choisi parmi l'hydrogène, le deutérium, -F, -Cl, -Br, -I, un groupe hydroxyle, un groupe cyano, un groupe nitro, un groupe amidino, un groupe hydrazino, un groupe hydrazono, un groupe alkyle en C₁ à C₂₀, un groupe alkoxy en C₁ à C₂₀, un groupe cyclopentyle, un groupe cyclohexyle, un groupe cycloheptyle, un groupe cyclopentényle, un groupe cyclohexényle, un groupe phényle, un groupe biphényle, un groupe naphtyle, un groupe phénalényle, un groupe phénanthrényle, un groupe anthracényle, un groupe fluoranthényle, un groupe triphénylényle, un groupe pyridinyle, un groupe pyrazinyle, un groupe pyrimidinyle, un groupe quinolinyle, un groupe isoquinolinyle, un groupe benzoquinolinyle, un groupe naphtyridinyle, un groupe quinoxalinyle, un groupe quinazolinyle, un groupe carbazolyle, un groupe phénanthridinyle, un groupe acridinyle, un groupe phénanthrolinyle, un groupe phénazinyle, un groupe triazinyle, un groupe dibenzofuranyle, un groupe dibenzothiophényle, et -Si(Q₃₁)(Q₃₂)(Q₃₃),
e2 est un entier de 0 à 2 ; quand e2 vaut 2, deux groupes Z₃₁ sont identiques ou différents, et deux groupes Z₃₂ sont identiques ou différents,
e3 est un entier de 0 à 3 ; quand e3 vaut 2 ou plus, au moins deux groupes Z₃₁ sont identiques ou différents et au moins deux Z₃₂ sont identiques ou différents,
e4 est un entier de 0 à 4 ; quand e4 vaut 2 ou plus, au moins deux groupes Z₃₁ sont identiques ou différents et au moins deux Z₃₂ sont identiques ou différents,
e5 est un entier de 0 à 5 ; quand e5 vaut 2 ou plus, au moins deux groupes Z₃₁ sont identiques ou différents et au moins deux Z₃₂ sont identiques ou différents,
e6 est un entier de 0 à 6 ; quand e6 vaut 2 ou plus, au moins deux groupes Z₃₁ sont identiques ou différents et au moins deux Z₃₂ sont identiques ou différents,
e7 est un entier de 0 à 7 ; quand e7 vaut 2 ou plus, au moins deux groupes Z₃₁ sont identiques ou différents,
e9 est un entier de 0 à 9 ; quand e9 vaut 2 ou plus, au moins deux groupes Z₃₁ sont identiques ou différents, et
* indique un site de liaison à un atome adjacent,
chacun de R₃ à R₈ est indépendamment choisi parmi l'hydrogène, le deutérium, -F, -Cl, -Br, -I, un groupe hydroxyle, un groupe cyano, un groupe nitro, un groupe amino, un groupe amidino, un groupe hydrazino, un groupe hydrazono, un groupe alkyle en C₁ à C₆₀ substitué ou non substitué, un groupe alcényle en C₂ à C₆₀ substitué ou non substitué, un groupe alcynyle en C₂ à C₆₀ substitué ou non substitué, un groupe alkoxy en C₁ à C₆₀ substitué ou non substitué, un groupe cycloalkyle en C₃ à C₁₀ substitué ou non substitué, un groupe hétérocycloalkyle en C₁ à C₁₀ substitué ou non substitué, un groupe cycloalcényle en C₃ à C₁₀ substitué ou non substitué, un groupe hétérocycloalcényle en C₁ à C₁₀ substitué ou non substitué, un groupe aryle en C₆ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe hétéroaryloxy en C₁ à C₆₀ substitué ou non substitué, un groupe polycyclique en C₈ à C₆₀ condensé non aromatique monovalent substitué ou non substitué, un groupe hétéropolycyclique à 8 à 60 chaînons condensé non aromatique monovalent substitué ou non substitué, -Si(Q₁)(Q₂)(Q₃), -B(Q₁)(Q₂), et -C(=O)(Q₁),
b1 est un entier de 1 à 10,
chacun de b3, b5 et b7 est indépendamment un entier de 1 à 4,
chacun de b4, b6 et b8 est indépendamment un entier de 1 à 3, et
chacun du groupe alkyle en C₁ à C₆₀ substitué, du groupe alcényle en C₂ à C₆₀ substitué, du groupe alcynyle en C₂ à C₆₀ substitué, du groupe alkoxy en C₁ à C₆₀ substitué, du groupe cycloalkyle en C₃ à C₁₀ substitué, du groupe hétérocycloalkyle en C₁ à C₁₀ substitué, du groupe cyclo-alcényle en C₃ à C₁₀ substitué, du groupe hétérocycloalcényle en C₁ à C₁₀ substitué, du groupe aryle en C₆ à C₆₀ substitué, du groupe aryloxy en C₆ à C₆₀ substitué, du groupe arylthio en C₆ à C₆₀ substitué, du groupe hétéroaryle en C₁ à C₆₀ substitué, du groupe hétéroaryloxy en C₁ à C₆₀ substitué, du groupe polycyclique en C₈ à C₆₀ condensé non aromatique monovalent substitué, et du groupe hétéropolycyclique à 8 à 60 chaînons condensé non aromatique monovalent substitué est indépendamment substitué par un ou plusieurs substituants indépendamment choisis parmi :
le deutérium, -F, -Cl, -Br, -I, un groupe hydroxyle, un groupe cyano, un groupe nitro, un groupe amino, un groupe amidino, un groupe hydrazino, un groupe hydrazono, un groupe alkyle en C₁ à C₆₀, un groupe alcényle en C₂ à C₆₀, un groupe alcynyle en C₂ à C₆₀, et un groupe alkoxy en C₁ à C₆₀ ;
un groupe alkyle en C₁ à C₆₀, un groupe alcényle en C₂ à C₆₀, un groupe alcynyle en C₂ à C₆₀, et un groupe alkoxy en C₁ à C₆₀, chacun substitué par au moins l'un choisi parmi le deutérium, -F, -Cl, -Br, -I, un groupe hydroxyle, un groupe cyano, un groupe nitro, un groupe amino, un groupe amidino, un groupe hydrazino, un groupe hydrazono, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique en C₈ à C₆₀ condensé non aromatique monovalent, un groupe hétéropolycyclique à 8 à 60 chaînons condensé non aromatique monovalent, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), et -P(=O)(Q₁₁)(Q₁₂) ;
un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique en C₈ à C₆₀ condensé non aromatique monovalent, un groupe hétéropolycyclique à 8 à 60 chaînons condensé non aromatique monovalent, un groupe biphényle, et un groupe terphényle ;
un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique en C₈ à C₆₀ condensé non aromatique monovalent, un groupe hétéropolycyclique à 8 à 60 chaînons condensé non aromatique monovalent, un groupe biphényle, et un groupe terphényle, chacun substitué par au moins l'un choisi parmi le deutérium, -F, -Cl, -Br, -I, un groupe hydroxyle, un groupe cyano, un groupe nitro, un groupe amino, un groupe amidino, un groupe hydrazino, un groupe hydrazono, un groupe alkyle en C₁ à C₆₀, un groupe alcényle en C₂ à C₆₀, un groupe alcynyle en C₂ à C₆₀, un groupe alkoxy en C₁ à C₆₀, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique en C₈ à C₆₀ condensé non aromatique monovalent, un groupe hétéropolycyclique à 8 à 60 chaînons condensé non aromatique monovalent, un groupe biphényle, un groupe terphényle, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), et -P(=O)(Q₂₁)(Q₂₂) ; et
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), et -P(=O)(Q₃₁)(Q₃₂),
où chacun de Q₁ à Q₃, Q₁₁ à Q₁₃, Q₂₁ à Q₂₃ et Q₃₁ à Q₃₃ est indépendamment choisi parmi l'hydrogène, le deutérium, -F, -Cl, -Br, -I, un groupe hydroxyle, un groupe cyano, un groupe nitro, un groupe amino, un groupe amidino, un groupe hydrazino, un groupe hydrazono, un groupe alkyle en C₁ à C₆₀, un groupe alcényle en C₂ à C₆₀, un groupe alcynyle en C₂ à C₆₀, un groupe alkoxy en C₁ à C₆₀, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryle en C₆ à C₆₀ substitué par un groupe alkyle en C₁ à C₆₀, un groupe hétéroaryle en C₁ à C₆₀, un groupe polycyclique en C₈ à C₆₀ condensé non aromatique monovalent, un groupe hétéropolycyclique à 8 à 60 chaînons condensé non aromatique monovalent, un groupe biphényle, et un groupe terphényle.

2. Dispositif électroluminescent organique selon la revendication 1, dans lequel
la première électrode est une anode,
la deuxième électrode est une cathode,
la couche organique comprend en outre une région de transport de trous entre la première électrode et la couche d'émission et une région de transport d'électrons entre la couche d'émission et la deuxième électrode,
la région de transport de trous comprend une couche d'injection de trous, une couche de transport de trous, une couche auxiliaire d'émission, une couche de blocage d'électrons, ou une de leurs combinaisons, et
la région de transport d'électrons comprend une couche de blocage de trous, une couche de transport d'électrons, une couche d'injection d'électrons, ou une de leurs combinaisons ;
de préférence dans lequel la région de transport de trous comprend l'au moins un composé à base d'amine ;
mieux encore dans lequel la région de transport de trous comprend la couche de transport de trous, et la couche de transport de trous comprend l'au moins un composé à base d'aminé.

3. Composé à base d'aminé représenté par la formule 1D : 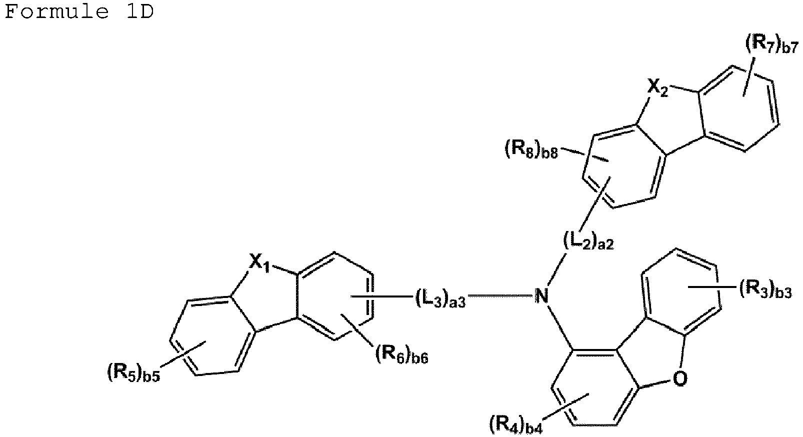 où la formule 1D est telle que définie dans la revendication 1.

4. Composé à base d'amine selon la revendication 3, dans lequel
(A) chacun de L₁ et L₂ est indépendamment choisi parmi :
une liaison simple et un groupe benzène ; et
un groupe benzène substitué par au moins l'un choisi parmi le deutérium, -F, -Cl, -Br, -I, un groupe hydroxyle, un groupe cyano, un groupe nitro, un groupe amino, un groupe amidino, un groupe hydrazino, un groupe hydrazono, un groupe alkyle en C₁ à C₂₀, un groupe alkoxy en C₁ à C₂₀, un groupe aryle en C₆ à C₂₀, un groupe hétéroaryle en C₁ à C₂₀, et -Si(Q₃₁)(Q₃₂)(Q₃₃) ;
L₃ est une liaison simple, et/ou
(B) chacun de R₃ à R₁₂ est indépendamment choisi parmi l'hydrogène, le deutérium, -F, -Cl, -Br, -I, un groupe méthyle, un groupe éthyle, un groupe propyle, un groupe isopropyle, un groupe n-butyle, un groupe isobutyle, un groupe sec-butyle, un groupe tert-butyle, un groupe pentyle, un groupe isoamyle, un groupe hexyle, un groupe phényle, et un groupe biphényle.

5. Composé à base d'amine selon l'une quelconque des revendications 3 et 4, dans lequel R₁ est choisi parmi les groupes représentés par les formules 6-1 à 6-10 et 6-12 à 6-26 : 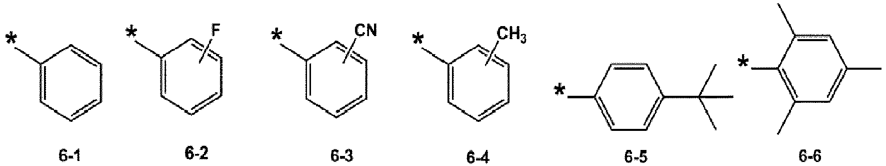 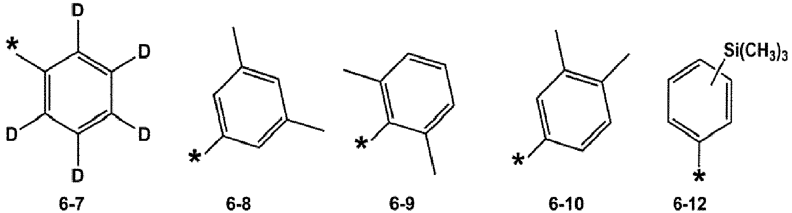 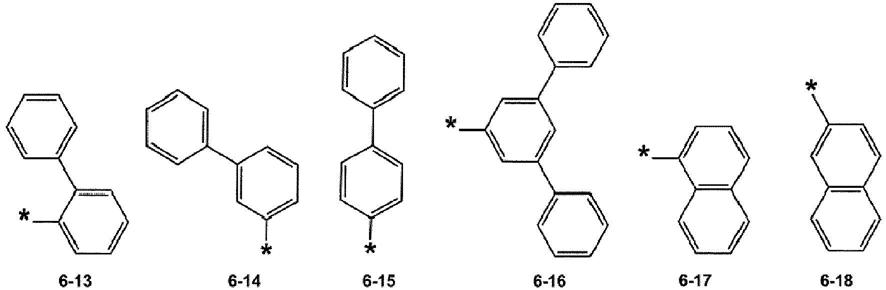 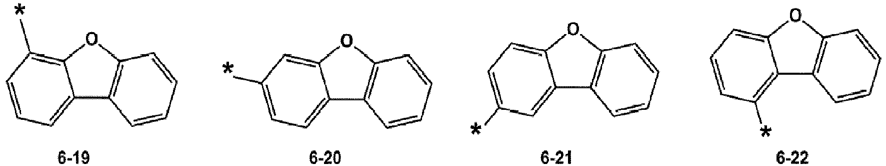 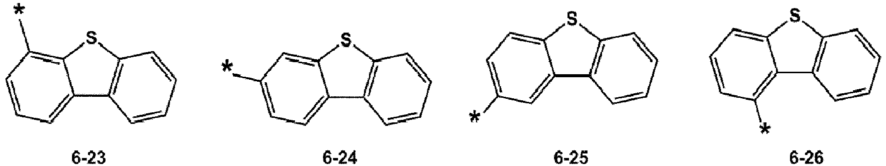 où, dans les formules 6-1 à 6-10 et 6-12 à 6-26,
* indique un site de liaison à un atome adjacent.

6. Composé à base d'amine selon l'une quelconque des revendications 3 à 5, où le composé à base d'amine représenté par la formule 1D est représenté par l'une des formules 1B-1 à 1B-16 : 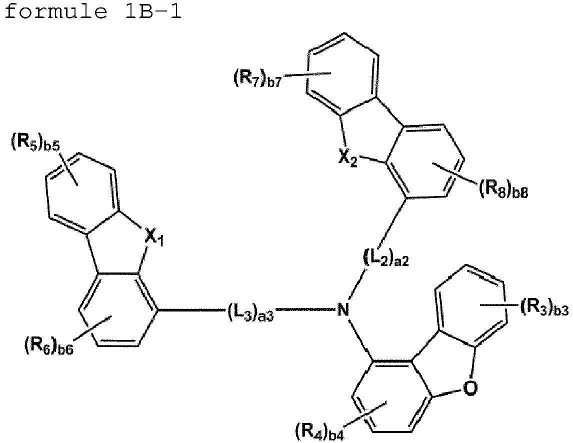 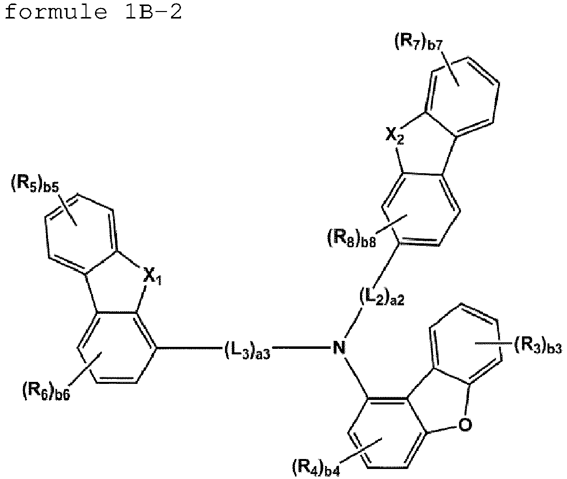 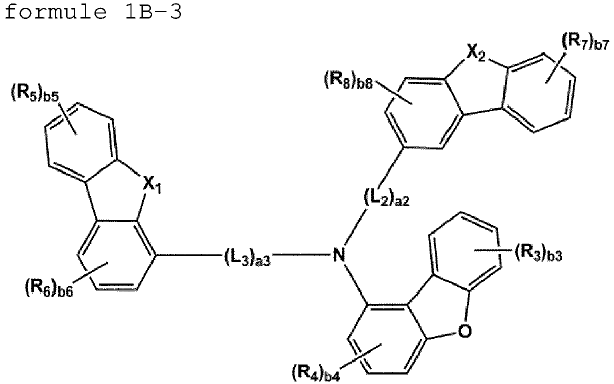 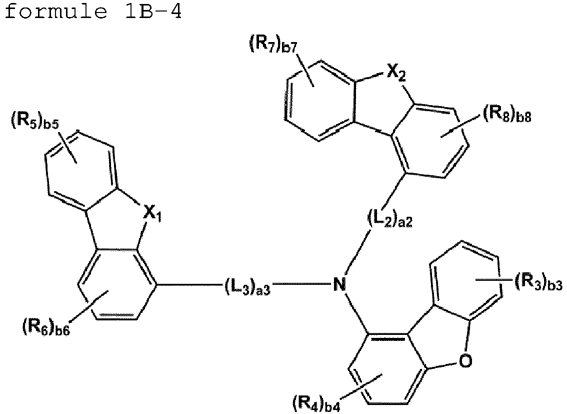 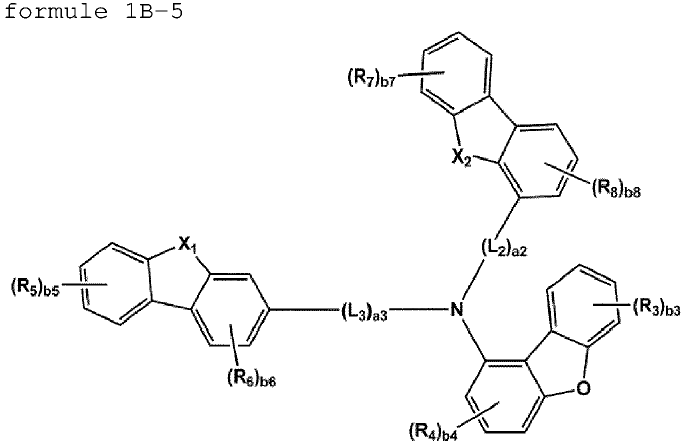 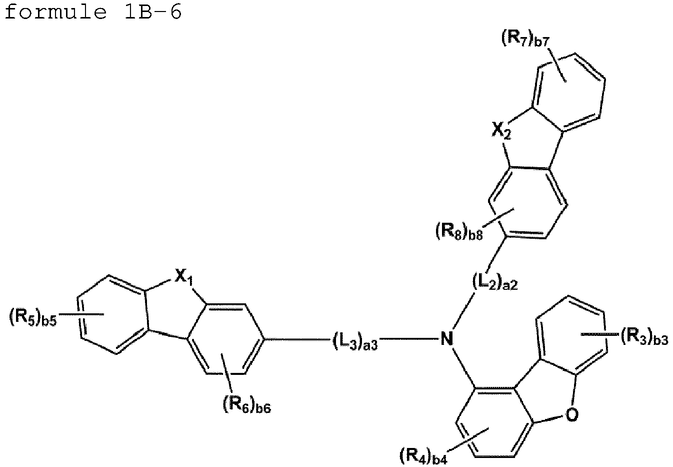 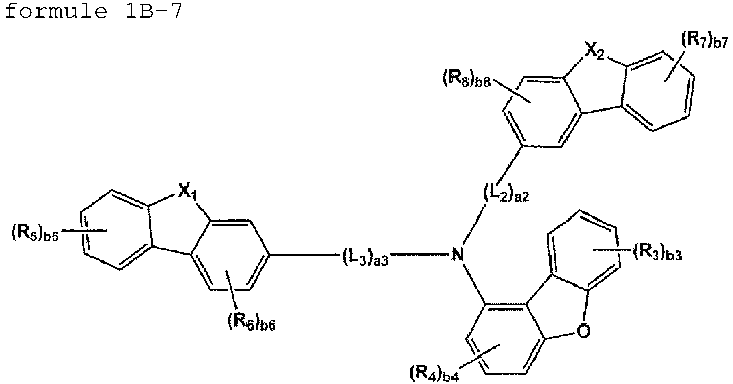 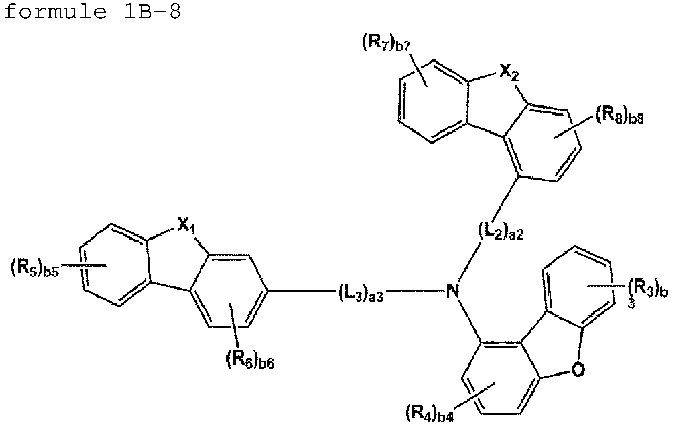 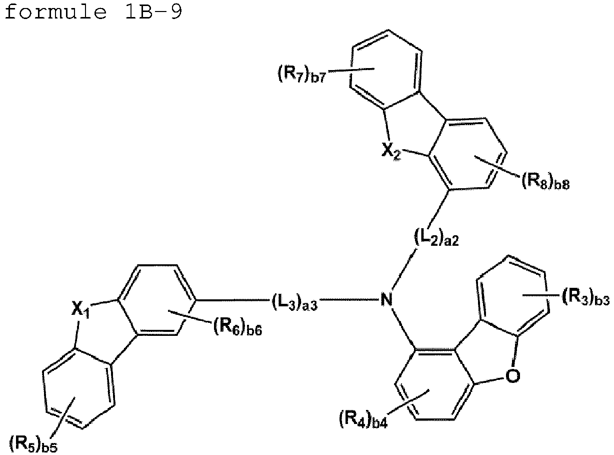 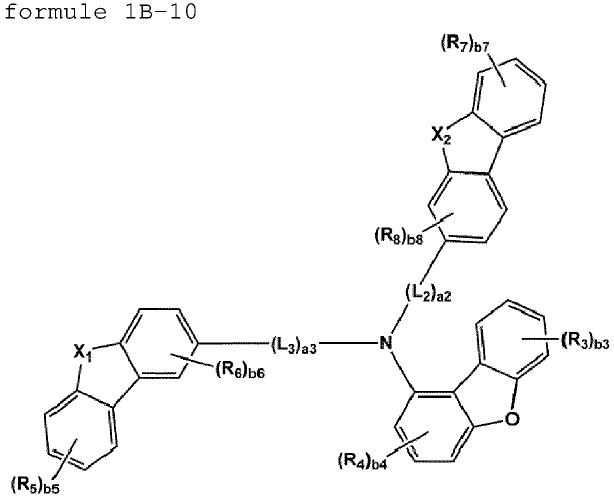 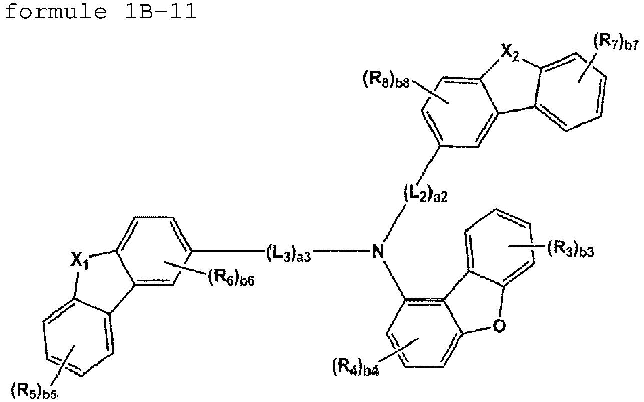 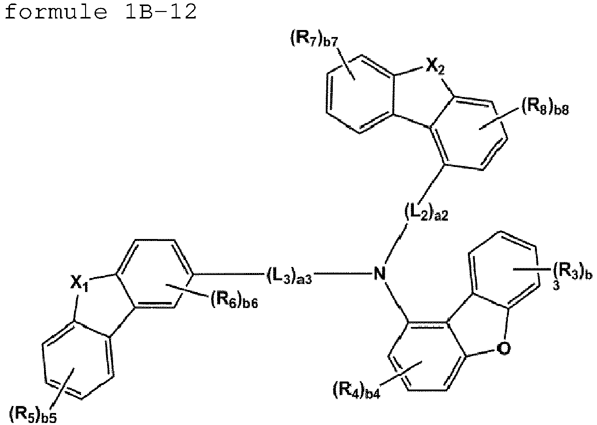 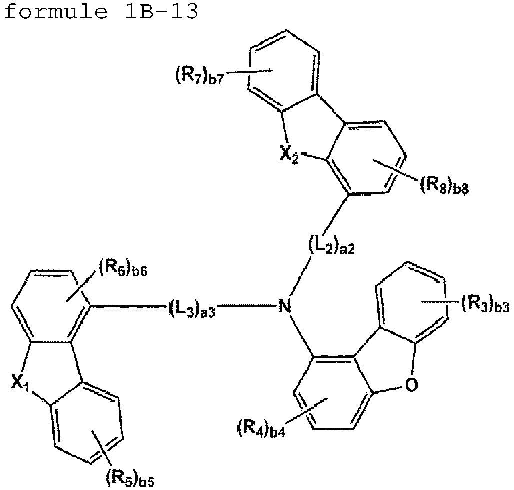 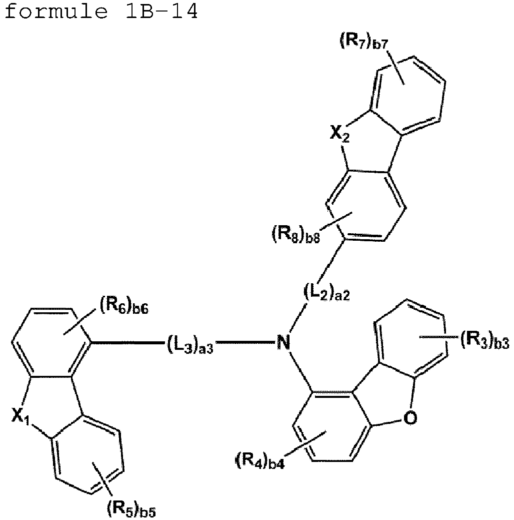 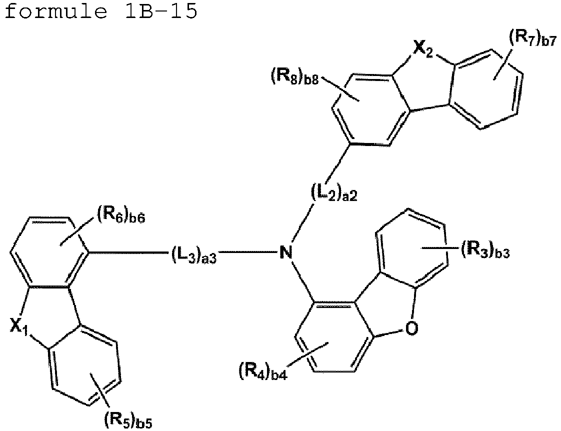 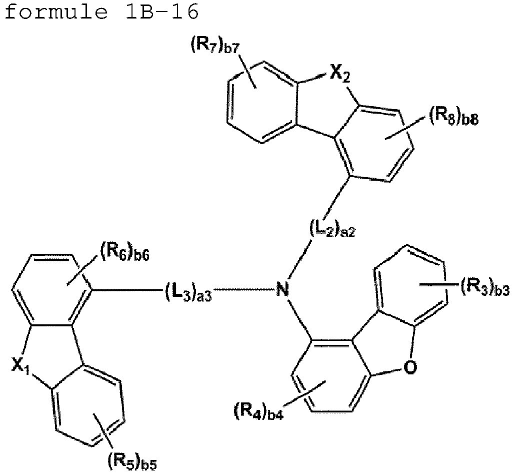 où, dans les formules 1B-1 à 1B-16,
chacun de X₁, X₂, L₂, L₃, a2, a3, R₃ à R₈, b3 à b8 sont indépendamment tels que définis dans les revendications 1, 4 et 5.

7. Composé à base d'amine selon la revendication 3, où le composé à base d'amine est choisi parmi les composés 42 à 45, 47 à 50, 56 à 59, 61 à 64, 70 à 73, 75 à 78, 84 à 87, 89 à 92, 232 à 239, 243 à 250 :

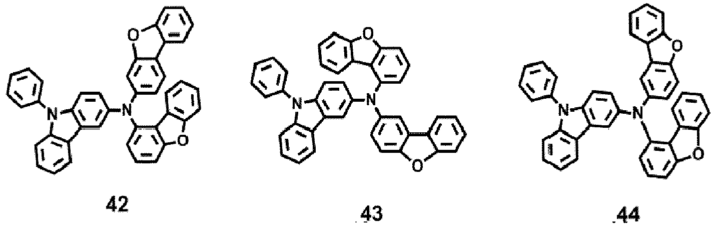

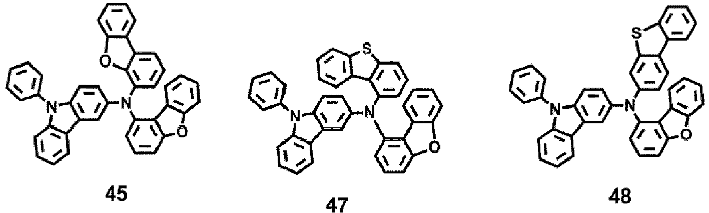

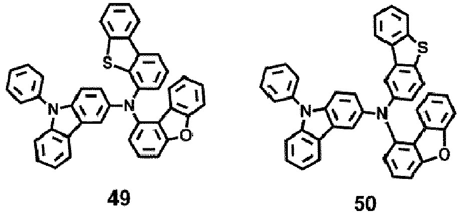

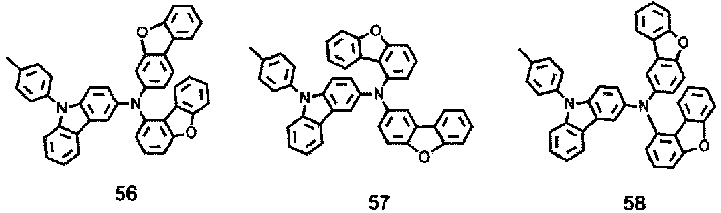

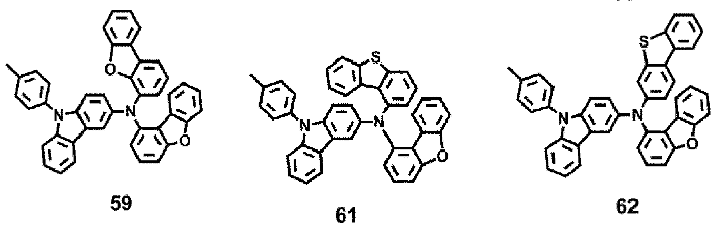

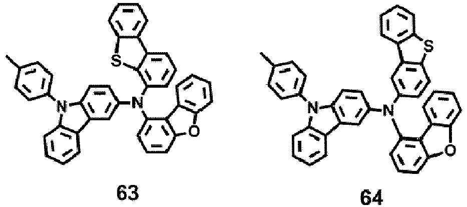

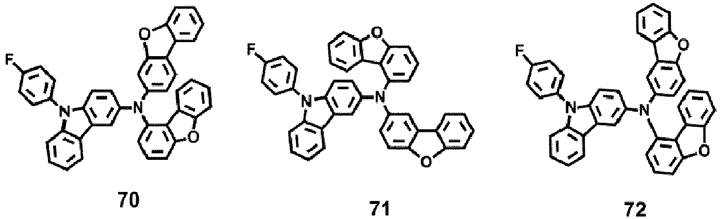

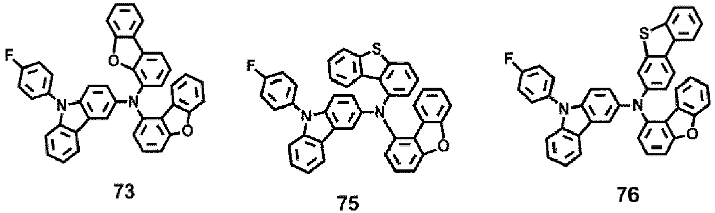

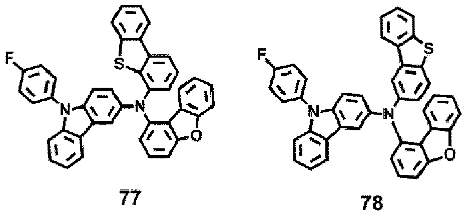

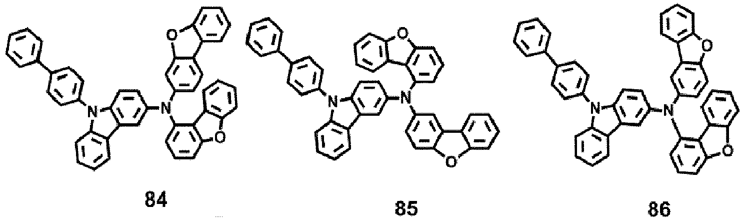

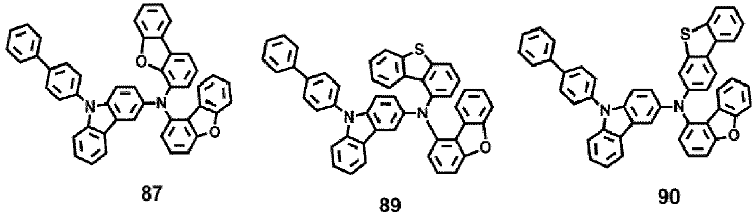

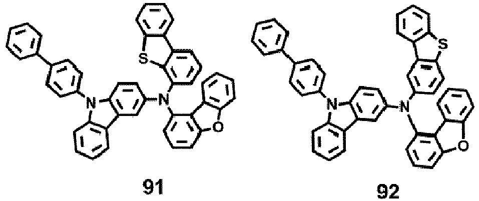

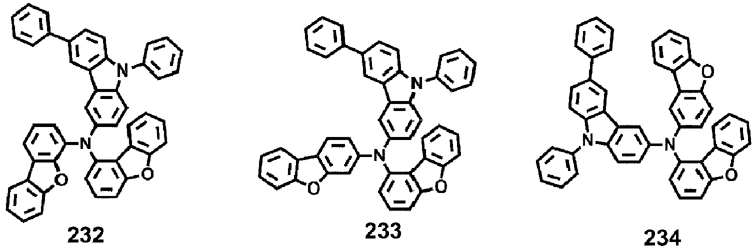

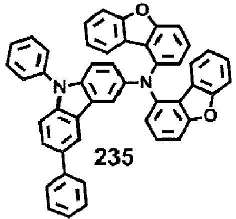

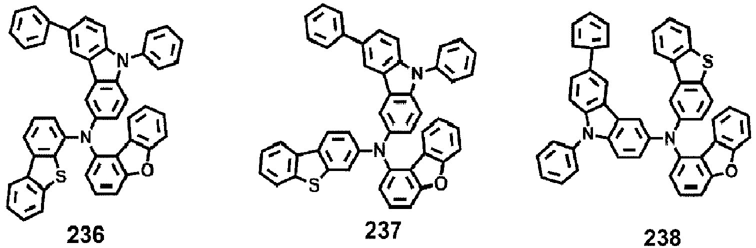

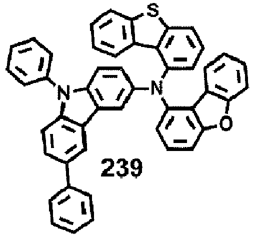

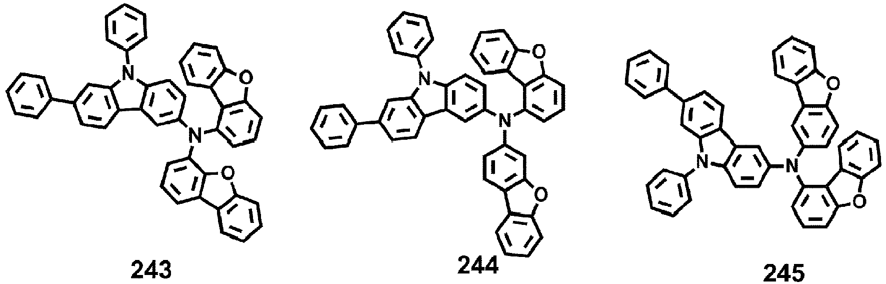

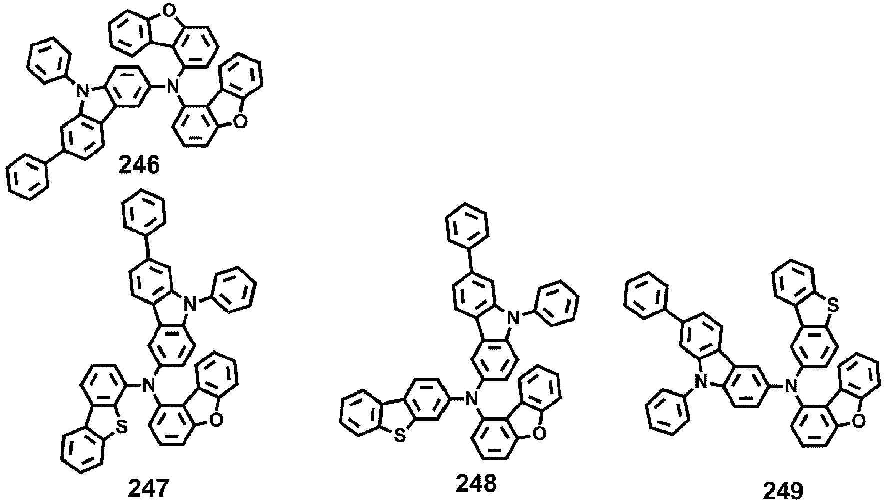

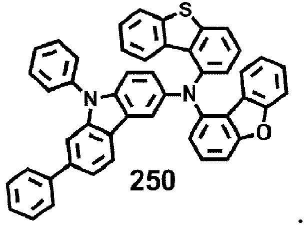

8. Dispositif électroluminescent organique selon l'une quelconque des revendications 1 et 2, dans lequel le composé à base d'amine est tel que défini dans l'une quelconque des revendications 3 à 7.
